(19) Europäisches Patentamt
European Patent Office
Office européen des brevets

(11) Veröffentlichungsnummer: **0 544 166 A2**

(12) # EUROPÄISCHE PATENTANMELDUNG

(21) Anmeldenummer: **92119508.7**

(51) Int. Cl.[5]: **C07D 501/20**, A61K 31/545

(22) Anmeldetag: **14.11.92**

(30) Priorität: **26.11.91 CH 3463/91**
**26.11.91 CH 3464/91**
**04.09.92 CH 2787/92**

(43) Veröffentlichungstag der Anmeldung:
**02.06.93 Patentblatt 93/22**

(84) Benannte Vertragsstaaten:
**AT BE CH DE DK ES FR GB GR IE IT LI LU MC NL PT SE**

(71) Anmelder: **F. HOFFMANN-LA ROCHE AG**
**Postfach 3255**
**CH-4002 Basel(CH)**

(72) Erfinder: **Angehrn, Peter**
**27 Bündtenweg**
**CH-4461 Böckten(CH)**
Erfinder: **Furlenmeier, André**
**119 Wettsteinallee**
**CH-4058 Basle(CH)**
Erfinder: **Hebeisen, Paul**
**102 Rennweg**
**CH-4052 Basel(CH)**
Erfinder: **Hofheinz, Werner**
**7 Talmattweg**
**CH-4103 Bottmingen(CH)**
Erfinder: **Link, Helmut**
**70 Dammerkirchstrasse**
**CH-4056 Basle(CH)**

(74) Vertreter: **Martin, Björn et al**
**Grenzacherstrasse 124 Postfach 3255**
**CH-4002 Basel (CH)**

(54) **Cephalosporinderivate.**

(57) Cephalosporinderivate der allgemeinen Formel

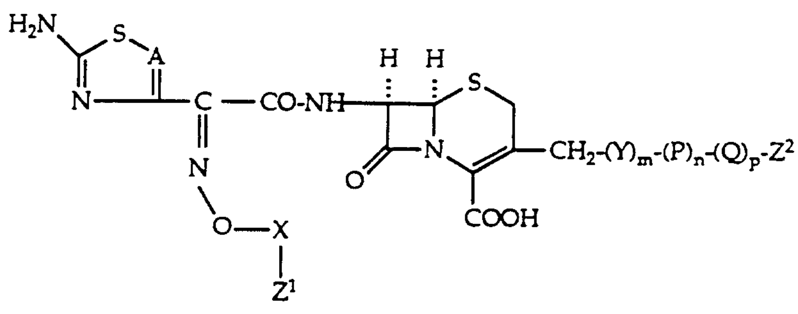

I

worin $Z^1$ und $Z^2$ je einzeln einen durch zwei vicinale Gruppen $-OR^1$ substituierten, 6-gliedrigen aromatischen Ring bedeutet (worin $R^1$ Wasserstoff oder niederes Alkanoyl darstellt), welcher zusätzlich noch 1 oder 2 O- oder N-Atome enthalten und/oder durch Halogen, Carboxy oder Carboxy-niederes Alkyl substituiert sein kann, oder $-X-Z^1$ und $-(Q)p-Z^2$ je einzeln eine Gruppe $-X^1-CONR^2OH$ bzw. $-(Q^1)p-CONR^2OH$, worin $R^2$ Wasserstoff, niederes Alkyl oder Phenyl darstellt, bedeutet; und worin ferner A einen Stickstoffatom oder eine Methingruppe (-CH=), X ggfs. durch Carboxy substituiertes und ggfs. mit einer der Gruppen -S-, -SO-, $-SO_2-$, -CO-, -OCO-, -NHCO-, $-NHSO_2-$ und -CONHNHCO- verknüpftes niederes Alkylen, Phenylen oder niederes Alkylen- Phenylen, $X^1$ niederes Alkylen, Phenylen oder niederes Alkylen- Phenylen, Y eine der Gruppen -O-, -OCO-, $-OCH_2-$, -S-, -SCO-, -SO- und $-SO_2-$, P einen ggfs. durch niederes Alkyl, Phenyl, Hydroxy oder Oxo substituierten 5- oder 6-

EP 0 544 166 A2

gliedrigen N-Monoheterocyclus oder einen ggfs. durch niederes Alkyl, Carboxy, niederes Alkoxycarbonyl, Carbamoyl, Hydroxymethyl oder niederes Alkanoyloxymethyl substituierten 8- bis 10-gliedrigen N-Biheterocyclus, Q ggfs. mit einer der Gruppen -CO-, -OCO-, $-SO_2-$, -NHCO- und $-NHSO_2-$ verknüpftes niederes Alkylen, Phenylen oder niederes Alkylen- Phenylen oder eine der Gruppen -S-, -SO-, $-SO_2-$, -CO-, -OCO-, -NHCO-, $-NHSO_2-$ und -CONHNHCO-, $Q^1$ niederes Alkylen, Phenylen oder niederes Alkylen-Phenylen und m, n und p jeweils die Zahl 0 oder 1 darstellt;
sowie leicht hydrolysierbare Ester und pharmazeutisch verträgliche Salze dieser Verbindungen und Hydrate von Verbindungen der Formel I bzw. von deren Estern und Salzen sowie ein Verfahren zu deren Herstellung und pharmazeutische Präparate, welche diese Verbindung enthalten, ferner auch Zwischenprodukte zur Herstellung dieser Verbindungen, weiter die Verwendung der Verbindungen bei der Bekämpfung von Krankheiten und bei der Herstellung der erwähnten Präparate.

Die vorliegende Erfindung betrifft neue Cephalosporinderivate der allgemeinen Formel

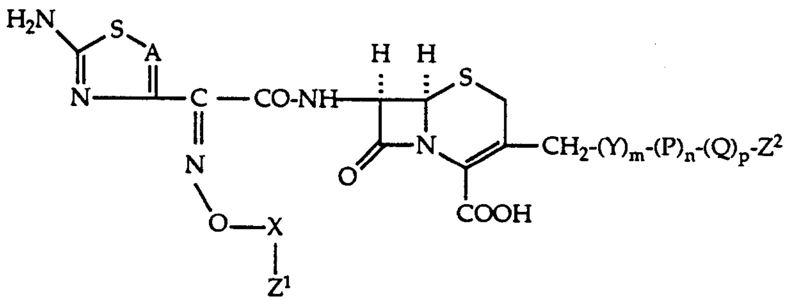

I

worin $Z^1$ und $Z^2$ je einzeln einen durch zwei vicinale Gruppen $-OR^1$ substituierten, 6-gliedrigen aromatischen Ring bedeutet (worin $R^1$ Wasserstoff oder niederes Alkanoyl darstellt), welcher zusätzlich noch 1 oder 2 O- oder N-Atome enthalten und/oder durch Halogen, Carboxy oder Carboxy-niederes Alkyl substituiert sein kann, oder $-X-Z^1$ und $-(Q)p-Z^2$ je einzeln eine Gruppe $-X^1-CONR^2OH$ bzw. $-(Q^1)p-CONR^2OH$, worin $R^2$ Wasserstoff, niederes Alkyl oder Phenyl darstellt, bedeutet; und worin ferner A ein Stickstoffatom oder eine Methingruppe (-CH = ), X ggfs. durch Carboxy substituiertes und ggfs. mit einer der Gruppen -S-, -SO-, $-SO_2-$, -CO-, -OCO-, -NHCO-, $-NHSO_2-$ und -CONHNHCO-verknüpftes niederes Alkylen, Phenylen oder niederes Alkylen-Phenylen, $X^1$ niederes Alkylen, Phenylen oder niederes Alkylen-Phenylen, Y eine der Gruppen -O-, -OCO-, $-OCH_2-$, -S-, -SCO-, -SO-und $-SO_2-$, P einen ggfs. durch niederes Alkyl, Phenyl, Hydroxy oder Oxo substituierten 5- oder 6-gliedrigen N-Monoheterocyclus oder einen ggfs. durch niederes Alkyl, Carboxy, niederes Alkoxycarbonyl, Carbamoyl, Hydroxymethyl oder niederes Alkanoyloxymethyl substituierten 8- bis 10-gliedrigen N-Biheterocyclus, Q ggfs. mit einer der Gruppen -CO-, -OCO-, $-SO_2-$, -NHCO- und $-NHSO_2-$verknüpftes niederes Alkylen, Phenylen oder niederes Alkylen-Phenylen oder eine der Gruppen -S-,-SO-, $-SO_2-$,-CO-, -OCO-, NHCO-, $-NHSO_2-$ und -CONHNHCO-, $Q^1$ niederes Alkylen, Phenylen oder niederes Alkylen-Phenylen und m, n und p jeweils die Zahl 0 oder 1 darstellt;
sowie leicht hydrolysierbare Ester und pharmazeutisch verträgliche Salze dieser Verbindungen und Hydrate von Verbindungen der Formel I bzw. von deren Estern und Salzen.

Diese erfindungsgemässen Produkte besitzen wertvolle antibiotische Eigenschaften. Sie können bei der Bekämpfung oder Verhütung von Infektionskrankheiten verwendet werden.

Die Verbindungen der Formel I liegen vorzugsweise in der obigen Formel dargestellten syn-isomeren Form vor. Sie können jedoch auch als Gemische mit der entsprechenden anti-isomeren Form vorliegen, in welchen die syn-isomere Form überwiegt.

Gegenstand der vorliegenden Erfindung sind: Die obigen Verbindungen der Formel I, deren leicht hydrolysierbare Ester und pharmazeutisch verträgliche Salze sowie die Hydrate dieser Stoffe als solche und zur Anwendung als therapeutische Wirkstoffe; ein Verfahren zur Herstellung dieser Produkte; Arzneimittel auf der Basis dieser Produkte und deren Herstellung; die Verwendung der erfindungsgemässen Produkte bei der Bekämpfung oder Verhütung von Krankheiten und die Verwendung der erfindungsgemässen Produkte zur Herstellung von antibiotisch wirksamen Arzneimitteln.

Der Ausdruck "nieder" bezeichnet Reste und Verbindungen mit höchstens 7, vorzugsweise höchstens 4 Kohlenstoffatomen. Der Ausdruck "Alkyl" bezeichnet geradkettige oder verzweigte, gesättigte Kohlenwasserstoffreste, wie Methyl, Aethyl, Propyl, Isopropyl und t-Butyl. Der Ausdruck "Alkylen" bezeichnet entsprechende Kohlenwasserstoffreste, welche jedoch zwei freie Valenzen besitzen, wie Methylen, Aethylen, Aethyliden, 1,3-Propylen, 1,4-Butylen, Butyliden, Isopropyliden und 1,2-Isobutylen. In "Phenylen" sitzen die zwei freien Valenzen vorzugsweise in 1,4-Stellung (1,4-Phenylen). Auch "niederes Alkylen-Phenylen" bezeichnet Gruppen mit zwei freien Valenzen, z.B. 1,4-Phenylen-methylen, 1,4-Phenylen-1,3-propylen, 1,4-Phenylen-dimethylen. Der Ausdruck "Alkoxy" allein oder in Zusammensetzungen, wie "Alkoxycarbonyl" bezeichnet über ein Sauerstoffatom gebundene Alkylgruppen, wie Methoxy und Aethoxy. Der Ausdruck "Alkanoyl" allein oder in Zusammensetzungen, wie "Alkanoyloxy" bezeichnet von geradkettigen oder verzweigten, gesättigten Fettsäuren abgeleitete Reste, wie Formyl und Acetyl.

Der Ausdruck "Halogen" bezeichnet die vier Formen Chlor, Fluor, Brom und Jod, wobei die Bedeutungsmöglichkeit Chlor bevorzugt ist.

Der Ausdruck "durch zwei vicinale Gruppen $-OR^1$ substituierter, 6-gliedriger aromatischer Ring" bedeutet vorzugsweise eine Dihydroxyphenylgruppe, wie 2,3-Dihydroxyphenyl oder, insbesondere, 3,4-Dihydroxyphenyl. Die Hydroxygruppen können durch niederes Alkanoyl substituiert sein, z.B. 3,4-Diacetoxyphenyl. Der Phenylkern kann wie oben angegeben weiter substituiert sein, z.B. durch Halogen, wie beispielsweise in 2-Fluor-3,4-dihydroxyphenyl, 3-Chlor-4,5-dihydroxyphenyl, 2,5-Dichlor-3,4-dihydroxyphenyl, 5-Brom-2-chlor-3,4-dihydroxyphenyl. Der aromatische Ring kann zusätzlich noch 1 oder 2 O- oder N-Atome enthalten, wobei tautomere Ketoformen entstehen, welche ebenfalls von der vorliegenden Erfindung umfasst sind, z.B. 1,4-Dihydro-5-hydroxy-4-oxo-2-pyrimidyl, 5-Hydroxy-4-oxo-4H-pyran-2-yl.

In einer bevorzugten Ausführungsform der erfindungsgemässen Produkte bedeuten beide der Substituenten $Z^1$ und $Z^2$ jeweils einen durch zwei vicinale Gruppen $-OR^1$ substituierten, 6-gliedrigen aromatischen Ring. Gemäss einer weiteren bevorzugten Ausführungsform ist nur $Z^2$ ein solcher Ring, während $-X-Z^1$ der Hydroxamsäurerest $-X^1-CONR^2OH$ darstellt. Gemäss einer weiteren Ausführungsform ist die Substitution umgekehrt, d.h. $Z^1$ ist der erwähnte Ring während $-(Q)_p-Z^2$ den Hydroxamsäurerest $-(Q^1)_p-CONR^2OH$ bedeutet.

Der unter dem Symbol P verwendete Ausdruck "5- oder 6-gliedriger Monoheterocyclus" bedeutet vorzugsweise partiell gesättigte oder aromatische Gruppen, welche als Heteroatome vorzugsweise ein Sauerstoff- oder Schwefelatom und/oder 1-4 Stickstoffatome enthalten. Im Rahmen der Erfindung besitzen diese Gruppen zwei freie Valenzen; Beispiele hierfür sind Oxadiazolylen, Pyrimidylen und Tetrazolylen. Der Monoheterocyclus kann auch substituiert sein, wie z.B. in 5-(2-Phenyl-pyrimidin-4-yl)-en, 5-(2-Methyl-pyrimidin-4-yl)-en.

Eine bevorzugte Bedeutung für P ist ein ggfs. durch niederes Alkyl, Carboxy, niederes Alkoxycarbonyl, Carbamoyl, Hydroxymethyl oder niederes Alkanoyloxymethyl substituierter 8- bis 10-gliedrigen N-Biheterocyclus. Dieser "8- bis 10-gliedrige N-Biheterocyclus" besitzt ebenfalls zwei freie Valenzen und bedeutet vorzugsweise Gruppen der allgemeinen Formel

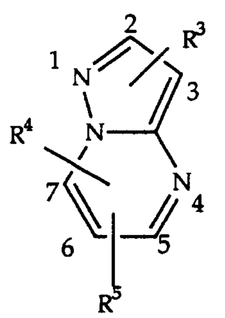
(a)

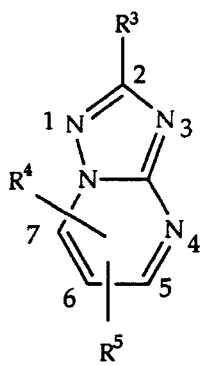
(b)

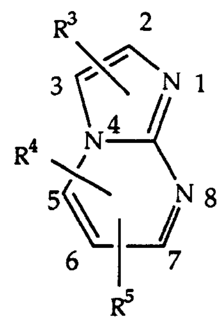
(c)

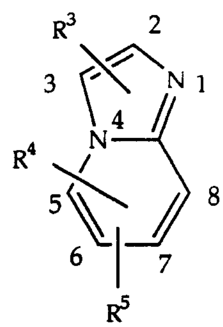
(d)

worin eines der Symbole $R^3$-$R^5$ Wasserstoff, niederes Alkyl, Carboxy, niederes Alkoxycarbonyl, Carbamoyl, Hydroxymethyl oder niederes Alkanoyloxymethyl bedeutet und die beiden verbleibenden Symbole $R^3$-$R^5$ jeweils kovalente Bindungen darstellen.

Bevorzugt ist die Gruppe (b) mit $R^5$ = 5-Methyl und $R^3$ und $R^4$ = kovalente Bindungen in 2- bzw. 7-Stellung, d.h. die 2-(5-Methyl-s-triazolo[1,5-a]pyrimidin-7-yl)-en-Gruppe.

Bevorzugte Substituenten in den Verbindungen der Formel I sowie den entsprechenden Estern, Salzen und Hydraten sind wie folgt:

| | |
|---|---|
| A: | Methin (-CH=); |
| $Z^1$: | 3,4-Dihydroxyphenyl, 2-Fluor-3,4-dihydroxyphenyl, 2,5-Dichlor-3,4-dihydroxyphenyl, 5-Brom-2-chlor-3,4-dihydroxyphenyl; 1,4-Dihydro-5-hydroxy-4-oxo-2-pyrimidyl, 5-Hydroxy-4-oxo-4H-pyran-2-yl; |
| X: | $-CH_2-$, $-CH_2-SO_2-$, $-CH(COOH)-$, $-C(CH_3)_2-$; |
| $Z^2$: | 3,4-Dihydroxyphenyl, 3-Chlor-4,5-dihydroxyphenyl; |
| $(Q)_p$: | $-CH_2NHSO_2-$, $-CH_2-OCO-$, $-CH_2-SO_2-$, abwesend (d.h. p = 0); |
| $(P)_n$: | 5-(1,3,4-Oxadiazol-2-yl)-en, 5-(Pyrimidin-4-yl)-en, 5-(2-Methyl-pyrimidin-4-yl)-en, 5-(2-Phenyl-pyrimidin-4-yl)-en, 5-(1H)-Tetrazol-1-yl)-en, 5-(2H-Tetrazol-2-yl)-en, 2-(5-Methyl-s-triazolo[1,5-a]pyrimidin-7-yl)-en, 5-(Pyrazolo-[1,5-a]pyrimidin-7-yl)-en, 5-(2-Methyl-s-triazolo[1,5-a]-pyrimidin-7-yl)-en, 2-(5,6-Dimethyl-s-triazolo[1,5-a]pyrimidin-7-yl)-en; |
| $(Y)_m$: | -S-, $-SO_2-$, abwesend (d.h. m = 0); |

X-$Z^1$: 1-(Hydroxycarbamoyl)-1-methyläthyl, 3,4-Dihydroxybenzyl, 3,4-Dihydroxyphenylsulfonylmethyl, (1,4-Dihydro-5-hydroxy-4-oxo-2-pyrimidyl)-methyl, 2-Fluor-3,4-dihydroxybenzyl, 2,5-Dichlor-3,4-dihydroxybenzyl, 5-Brom-2-chlor-3,4-dihydroxybenzyl, (5-Hydroxy-4-oxo-4H-pyran-2-yl)-methyl, α-Carboxy-3,4-dihydroxybenzyl und, insbesondere, 1-[3-(3,4-Dihydroxybenzoyl)carbazoyl]-1-methyläthyl;

$(Q)_p$-$Z^2$: (3,4-Dihydroxybenzolsulfonamido)-methyl, [(3,4-Dihydroxybenzoyl)oxy]methyl, [-(3,4-Dihydroxyphenyl)sulfonyl]methyl, [(3,4-Dihydroxybenzoyl)amino]methyl, 3,4-Dihydroxyphenyl (d.h. p = 0), 3-Chlor-4,5-dihydroxyphenyl (d.h. p = 0), Hydroxycarbamoyl (d.h. p = 0), N-Hydroxy-N-methyl-carbamoyl(d.h. p = 0), N-Methyl-N-hydroxycarbamoyl (d.h. p = 0);

$(P)_n$-$(Q)_pZ^2$: 2-[(3,4-Dihydroxybenzolsulfonamido)methyl]-5-methyl-s-triazolo[1,5-a)pyrimidin-7-yl, 2-[[(3,4-Dihydroxybenzoyl)oxy]methyl]-5-methyl-s-triazolo[1,5-a]pyrimidin-7-yl, 2-[[(3,4-Dihydroxyphenyl)sulfonyl]methyl]-5-methyl-s-triazolo[1,5-a]pyrimidin-7-yl, 2-[[(3,4-Dihydroxybenzoyl)amino]methyl]-5-methyl-s-triazolo[1,5-a]pyrimidin-7-yl, 5-(3,4-Dihydroxyphenyl)pyrazolo[1,5-a]pyrimidin-7-yl (d.h. p = 0), 5-(3,4-Dihydroxyphenyl)-1,3,4-oxadiazol-2-yl (d.h. p = 0), 5-(3,4-Dihydroxyphenyl)-2-phenyl-pyrimidin-4-yl (d.h. p = 0); 5-(3,4-Dihydroxyphenyl)-pyrimidin-4-yl (d.h. p = 0), 5-(3,4-Dihydroxyphenyl)-2-methyl-pyrimidin-4-yl (d.h. p = 0), 3,4-Dihydroxyphenyl (d.h. p = n = 0), 2-(Hydroxycarbamoyl)-5-methyl-s-triazolo[1,5-a]-pyrimidin-7-yl (d.h. p = 0), 5-(2-Hydroxycarbamoyl)-2-methyl-s-triazolo-[1,5-a]-pyrimidin-7-yl (d.h. p = 0), 5-(3,4-Dihydroxyphenyl)-1H-tetrazol-1-yl (d.h. p = 0), 5-(3,4-Dihydroxyphenyl)-2H-tetrazol-2-yl (d.h. p = 0), 5-(3,4-Dihydroxybenzoylamino)-1H-tetrazol-1-yl, 5-(3,4-Dihydroxybenzoylamino)-2H-tetrazol-2-yl, 2-[3,4-Dihydroxyphenyl]-5-methyl-s-triazolo[1,5-a]-pyrimidin-7-yl (d.h. p = 0), 2-[3-Chlor-3,4-dihydroxyphenyl]-5,6-dimethyl-s-triazolo[1,5-a]pyrimidin-7-yl (d.h. p = 0), 5-(3,4-Dihydroxyphenyl)pyrazolo[1,5-a]pyrimidin-7-yl (d.h. p = 0), 2-(N-Hydroxy-N-methylcarbamoyl)-5-methyl-s-triazolo[1,5-a]pyrimidin-7-yl (d.h. p = 0), 2-(Hydroxycarbamoyl)-5-hydroxymethyl-s-triazolo[1,5-a]pyrimidin-7-yl (d.h. p = 0), 5-(Hydroxycarbamoyl)-2-methyl-s-triazolo-[1,5-a]pyrimidin-7-yl (d.h. p = 0), 5-(Hydroxycarbamoyl)-2-hydroxymethyl-s-triazolo[1,5-a]pyrimidin-7-yl (d.h. p = 0), 5-[(2-Hydroxycarbamoyl)äthyl]-2-methyl-s-triazolo[1,5-a]-pyrimidin-7-yl;

$(Y)_m$-$(P)_n$-$Q_p$-$Z^2$: [2-[(3,4-Dihydroxybenzolsulfonamido)methyl]-5-methyl-s-triazolo[1,5-a]pyrimidin-7-yl]thio, [2-[[(3,4-Dihydroxybenzoyl)oxy]methyl]-5-methyl-s-triazolo[1,5-a]-pyrimidin-7-yl]thio, [2-[[(3,4-Dihydroxyphenyl)sulfonyl]methyl]-5-methyl-s-triazolo-[1,5-a]pyrimidin-7-yl]thio, [5-(3,4-Dihydroxyphenyl)pyrazolo[1,5-a]pyrimidin-7-yl]-thio (d.h. p = 0), [5-(3,4-Dihydroxyphenyl)-1,3,4-oxadiazol-2-yl]thio (d.h. p = 0), [5-(3,4-Dihydroxyphenyl)-2-phenyl-pyrimidin-4-yl]thio(d.h. p = 0), [5-(3,4-Dihydroxyphenyl)-pyrimidin-4-yl]thio (d.h. p = 0), [5-(3,4-Dihydroxyphenyl)-2-methylpyrimidin-4-yl]thio (d.h. p = 0), (3,4-Dihydroxyphenyl)-sulfonyl (d.h. p = n = 0), [2-(Hydroxycarbamoyl)-5-methyl-s-triazolo[1,5-a]pyrimidin-7-yl]thio (d.h. p = 0), [5-(Hydroxycarbamoyl)-2-methyl-s-triazolo[1,5-a]pyrimidin-7-yl]thio (d.h. p = 0), 5-(3,4-Dihydroxyphenyl)-1H-tetrazol-1-yl (d.h. p = m = 0), 5-(3,4-Dihydroxyphenyl)-2H-tetrazol-2-yl (d.h. p = m = 0), [5-(3,4-Dihydroxybenzoylamino)-1H-tetrazol-1-yl]thio (d.h. m = 0), [5-(3,4-Dihydroxybenzoylamino)-2H-tetrazol-2-yl]-thio (d.h. m = 0), [2-[3,4-Dihydroxyphenyl]-5-methyl-s-triazolo[1,5-a]pyrimidin-7-yl]thio (d.h. p = 0), [2-[3-Chlor-3,4-dihydroxyphenyl]-5,6-dimethyl-s-triazolo[1,5-a]- pyrimidin-7-yl]thio (d.h. p = 0), [5-(3,4-Dihydroxyphenyl)pyrazolo[1,5-a]-pyrimidin-7-yl]thio (d.h. p = 0), [2-(N-Hydroxy-N-methylcarbamoyl)-5-methyl-s-triazolo[1,5-a]pyrimidin-7-yl]thio (d.h. p = 0), [2-(Hydroxycarbamoyl)-5-hydroxymethyl-s-triazolo[1,5-a]pyrimidin-7-yl]thio (d.h. p = 0), [5-(Hydroxycarbamoyl)-2-methyl-s-triazolo[1,5-a]-pyrimidin-7-yl]thio (d.h. p = 0), [5-(Hydroxycarbamoyl)-2-hydroxymethyl-s-triazolo[1,5-a]pyrimidin-7-yl]thio (d.h. p = 0), [5-[(2-Hydroxycarbamoyl)äthyl]-2-methyl-s-triazolo-[1,5-a]pyrimidin-7-yl]-thio.

Eine Untergruppe der erfindungsgemässen Cephalosporinderivate sind diejenigen der Formel I, worin X ggfs. durch Carboxy substituiertes und ggfs. mit einer der Gruppen -S-,-SO-, $-SO_2-$, -CO-, -OCO-, -NHCO- und $-NHSO_2-$ verknüpftes niederes Alkylen, Phenylen oder niederes Alkylen-Phenylen darstellt sowie die entsprechenden Ester, Salze und Hydrate. Unter diese Untergruppe fallende, bevorzugte Produkte sind:

(6R,7R)-7-[(Z)-2-(2-Amino-4-thiazolyl)-2-[[(3,4-dihydroxybenzyl)oxy]imino]acetamido]-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]-oct-2-en-2-carbonsäure,

7-[(Z)-2-(2-Amino-4-thiazolyl)-2-[[(2-fluor-3,4-dihydroxybenzyl)oxy]imino]acetamido]-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]-oct-2-en-2-carbonsäure,

(6R,7R)-7-[(Z)-2-(2-Amino-4-thiazolyl)-2-[[(3,4-dihydroxybenzyl)oxy]imino]acetamido]-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo-[4.2.0]oct-2-en-2-carbonsäure,

(6R,7R)-7-[(Z)-2-(2-Amino-4-thiazolyl)-2-[[[(3,4-dihydroxy-2-fluorbenzyl)oxy]imino]acetamido]-3-[[[2-[[-(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure,

(6R,7R)-7-[(Z)-2-(2-Amino-4-thiazolyl)-2-[[(3,4-dihydroxybenzyl)oxy]imino]acetamido]-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure,

(6R,7R)-7-[(Z)-2-(2-Amino-4-thiazolyl)-2-[[(2-fluor-3,4-dihydroxybenzyl)oxy]imino]acetamido]-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo-[4.2.0]oct-2-en-2-carbonsäure
und deren pharmazeutisch verträgliche Salze.

Eine bevorzugte Untergruppe der erfindungsgemässen Cephalosporinderivate sind diejenigen der Formel I, worin X die Gruppe -(niederes Alkylen)-CONHNHCO- darstellt sowie die entsprechenden Ester, Salze und Hydrate. Unter diese Untergruppe fallende, besonders bevorzugte Produkte sind:

(6R,7R)-7-[(Z)-2-(2-Amino-4-thiazolyl)-2-[[1-[3-(3,4-dihydroxybenzoyl)carbazoyl]-1-methyläthoxy]imino]-acetamido]-3-[[[2-[(3,4-dihydroxybenzolsulfonamido)methyl]-5-methyl-s-triazolo[1,5-a]pyrimidin-7-yl]thio]-methyl]-8-oxo-5-thia-1-azabicyclo[4.2.0]oct-2-en-2-carbonsäure,

(6R,7R)-7-[(Z)-2-(2-Amino-4-thiazolyl)-2-[[1-[3-(3,4-dihydroxybenzoyl)carbazoyl]-1-methyläthoxy]-iminoacetamido]-3-[[[2-[[(3,4-dihydroxybenzoyl)oxy]methyl]-5-methyl-s-triazolo[1,5-a]pyrimidin-7-yl]thio]-methyl]-8-oxo-5-thia-1-azabicyclo[4.2.0]oct-2-en-2-carbonsäure,

(6R,7R)-7-[(Z)-2-(2-Amino-4-thiazolyl)-2-[[1-[3-(3,4-dihydroxybenzoyl)carbazoyl]-1-methyläthoxy]imino]-acetamido]-3-[[[2-[[(3,4-dihydroxyphenyl)sulfonyl]methyl]-5-methyl-s-triazolo[1,5-a]pyrimidin-7-yl]thio]-methyl]-8-oxo-5-thia-azabicyclo[4.2.0]oct-2-en-2-carbonsäure,

(6R,7R)-7-[(Z)-2-(2-Amino-4-thiazolyl)-2-[[1-[3-(3,4-dihydroxybenzoyl)carbazoyl]-1-methyläthoxy]imino]-acetamido]-3-[[[2-[[(3,4-dihydroxybenzoyl)amino]methyl]-5-methyl-s-triazolo[1,5-a]pyrimidin-7-yl]thio]-methyl]-8-oxo-5-thia-azabicyclo[4.2.0]oct-2-en-2-carbonsäure
und deren pharmazeutisch verträgliche Salze.

Weitere erfindungsgemässe Produkte sind als Endprodukte der nachstehenden Ausführungsbeispiele entnehmbar.

Die Verbindungen der Formel I bilden pharmazeutisch annehmbare Salze mit Basen. Beispiele von Salzen von Verbindungen der Formel I sind die Alkalimetallsalze, beispielsweise die Natrium- und Kaliumsalze, die Ammoniumsalze, die Salze mit organischen Basen, beispielsweise mit Aminen, wie Diisopropylamin, Benzylamin, Dibenzylamin, Triäthanolamin, Triäthylamin, N,N-Dibenzyläthylendiamin, N-Methylmorpholin, Pyridin, Piperazin, N-Aethylpiperidin, N-Methyl-D-glucamin und Procain, oder mit Aminosäuren, wie Arginin und Lysin. Je nach Zahl der sauren Gruppen der Verbindung der Formel I können Mono-, Di-, Trisalze etc. entstehen.

Die Verbindungen der Formel I bilden auch Säureadditionssalze mit organischen und anorganischen Säuren. Beispiele von Säureadditionssalzen von Verbindungen der Formel I sind Salze mit Mineralsäuren, beispielsweise Halogenwasserstoffsäuren, wie Salzsäure, Bromwasserstoff und Jodwasserstoff, Schwefelsäure, Salpetersäure, Phosphorsäure und dergleichen, Salze mit organischen Sulfonsäuren, beispielsweise mit Alkyl- und Arylsulfonsäuren, wie Aethansulfonsäure, p-Toluolsulfonsäure, Benzolsulfonsäure und dergleichen, sowie Salze mit organischen Carbonsäuren, beispielsweise mit Essigsäure, Weinsäure, Maleinsäure, Zitronensäure, Benzoesäure, Salicylsäure, Ascorbinsäure und dergleichen.

Bei den leicht hydrolysierbaren Estern von Verbindungen der Formel I handelt es sich vorzugsweise um Ester, welche unter milden Bedingungen hydrolysiert werden können, insbesondere um solche, welche unter physiologischen Bedingungen, beispielsweise enzymatisch hydrolysiert werden können. Beispiele solcher Ester, die herkömmlicher Art sein können, sind die 1-(niederen Alkanoyloxy)-niederen Alkylester, z.B. der Acetoxymethyl-, Pivaloyloxymethyl-, 1-Acetoxyäthyl- und der 1-Pivaloyloxyäthylester, die 1-(niederen Alkoxycarbonyloxy)niederen Alkylester, z.B. der (Methoxycarbonyloxy)methyl-, 1-(Aethoxycarbonyloxy)äthyl- und der 1-(Isopropoxycarbonyloxy)äthylester, die Laktonylester, z.B. der

Phthalidyl- und der Thiophthalidylester, die 1-(niederen Alkoxy)-niederen Alkylester, z.B. der Methoxymethylester, die 1-(niederen Alkanoylamino)-niederen Alkylester, z.B. der Acetamidomethylester, die Benzylester, die Cyanomethylester und die (2-Oxo-1,3-dioxol-4-yl)methylester.

Allfällig in einer Verbindung der Formel I zusätzlich vorhandene Carboxygruppen können ebenfalls in Form von leicht hydrolysierbaren Estergruppen vorliegen.

Die erfindungsgemässen Produkte, d.h. die Verbindungen der obigen Formel I, ihre leicht hydrolysierbaren Ester und pharmazeutisch verträglichen Salze, sowie die Hydrate dieser Verbindungen, Ester und Salze, können erfindungsgemäss dadurch hergestellt werden, dass man

a) eine Verbindung der allgemeinen Formel

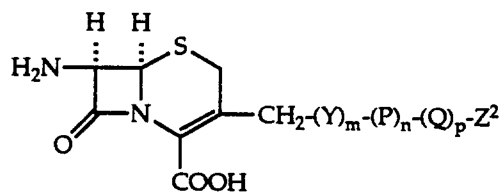

II

worin Y, P, Q, $Z^2$, m, n und p die oben angegebenen Bedeutungen haben,
oder einen leicht hydrolysierbaren Ester davon oder ein Säureadditionssalz einer dieser Verbindungen mit einer Carbonsäure der allgemeinen Formel

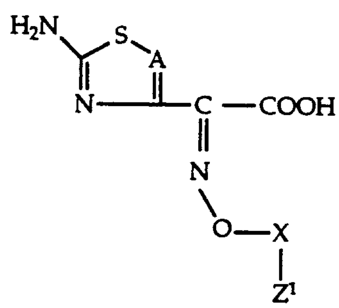

III

worin A, X und $Z^1$ die oben gegebenen Bedeutungen haben,
oder mit einem reaktionsfähigen Derivat dieser Verbindung acyliert, oder dass man

b) eine Verbindung der allgemeinen Formel

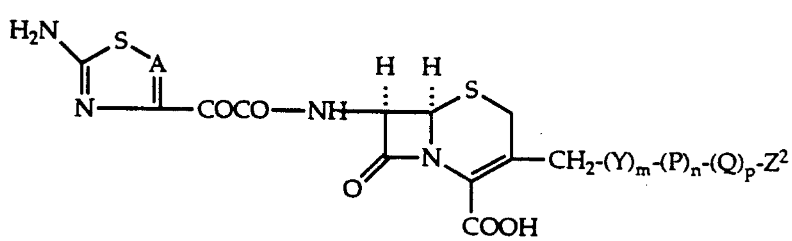

IV

worin A, Y, P, Q, $Z^2$, m, n und p die oben gegebenen Bedeutungen haben,
oder einen leicht hydrolysierbaren Ester davon mit einer Verbindung der allgemeinen Formel

$H_2N\text{-}O\text{-}X\text{-}Z^1$ V

worin X und $Z^1$ die oben gegebenen Bedeutungen haben, oder mit einem Säureadditionssalz davon umsetzt, oder dass man

c) zur Herstellung einer Verbindung der Formel I, worin m 1 und Y -S-darstellt, bzw. eines leicht hydrolysierbaren Esters davon eine Verbindung der allgemeinen Formel

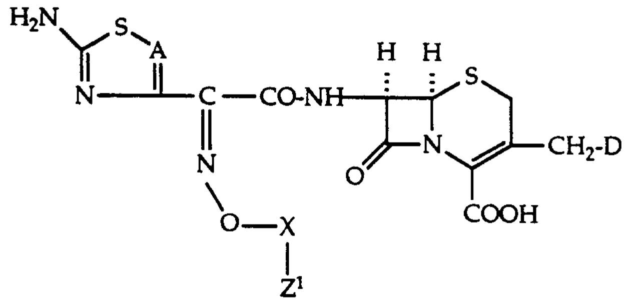

VI

worin A, X, $Z^1$ die oben gegebenen Bedeutungen haben und D eine Abgangsgruppe darstellt, oder einen leicht hydrolysierbaren Ester davon mit einer Verbindung der allgemeinen Formel

$HS(P)_n-(Q)_p-Z^2$ VII

worin P, Q, $Z^2$, n und p die oben gegebenen Bedeutungen haben, umsetzt, oder dass man

d) zur Herstellung eines leicht hydrolysierbaren Esters einer Verbindung der Formel I eine Carbonsäure der Formel I einer entsprechenden Veresterung unterwirft,

wobei in den Verfahrensvarianten a) - d) in $Z^1$ bzw. $Z^2$ vorhandene Hydroxygruppen geschützt sein können und die entsprechenden Schutzgruppen anschliessend notwendigenfalls abgespalten werden,

oder dass man

e) zur Herstellung von Salzen und Hydraten einer Verbindung der Formel I bzw., von Hydraten dieser Salze eine Verbindung der Formel I in ein Salz oder Hydrat bzw. ein Hydrat dieses Salzes überführt.

Bei verschiedenen der obigen erfindungsgemässen Verfahren müssen allfällig vorhandene reaktionsfähige Amino-, Hydroxyl- und/oder Carboxygruppen durch Schutzgruppen blockiert sein, wobei anschliessend diese Schutzgruppen in an sich bekannter Weise abgespalten werden. Diese Fälle sind für den Fachmann ohne weiteres erkennbar, und auch die Auswahl der jeweils geeigneten Schutzgruppen sowie der anschliessenden Abspaltungsmethoden bereitet ihm keine Schwierigkeiten.

Es ist ferner möglich, dass die erfindungsgemässen Produkte als Mischungen mit den den erfindungsgemässen Produkten entsprechenden isomeren Produkten anfallen. Dabei kann es sich beispielsweise um [E]-konfigurierte Oxime oder um $\Delta^2$-Isomere der erfindungsgemässen Cephalosporin-Derivate handeln. Die Abtrennung dieser Nebenprodukte, sowie deren allfällige Rückführung in erfindungsgemässe Produkte kann nach allgemein bekannten und jedem Fachmann geläufigen Methoden erfolgen. Es kommen dabei insbesondere chromatographische Methoden und Kristallisationsmethoden in Frage.

Die Acylierung gemäss Verfahrensvariante a) des erfindungsgemässen Verfahrens kann nach an sich bekannten und jedem Fachmann geläufigen Methoden durchgeführt werden. Man kann beispielsweise die Verbindung der Formel II mit der freien Carbonsäure der Formel III oder einem Salz davon mit einer Base acylieren, wobei man in diesem Fall in Gegenwart eines geeigneten Kondensationsmittels arbeitet und die Verbindung der Formel II vorgängig in einen leicht hydrolysierbaren Ester überführt. Geeignete Kondensationsmittel sind beispielsweise N,N'-disubstituierte Carbodiimide, wie N,N'-Dicyclohexylcarbodiimid, welche vorzugsweise zusammen mit N-Hydroxybenzotriazol oder N-Hydroxysuccinimid verwendet werden, 2-Halogenpyridiniumsalze, wie 2-Chlor-1-methylpyridiniumchlorid, Phosphoroxychlorid, Thionylchlorid und Oxalylchlorid.

Es ist aber auch möglich, die Carbonsäure der Formel III in Form eines reaktiven Derivates einzusetzen. Als reaktionsfähige Derivate kommen insbesondere Säurechloride, Säureanhydride, gemischte Anhydride (beispielsweise Anhydride mit Trifluoressigsäure, Benzolsulfonsäure, Mesitylensulfonsäure, p-Toluolsulfonsäure und p-Chlorsulfonsäure) und aktive Thiolester, beispielsweise S-(2-Benzothiazolyl)thioester in Frage. Bei Verwendung dieses S-(2-Benzothiazolyl)thioesters wird das Amin der Formel II vorzugsweise in Form eines Säureadditionssalzes, z.B. eines Toluolsulfonats, Methansulfonats oder Hydrochlorids, eingesetzt.

Die Acylierung kann gegebenenfalls in Gegenwart eines säurebindenden Mittels durchgeführt werden, wobei als säurebindende Mittel insbesondere Natriumhydrogencarbonat, Kaliumcarbonat, Triäthylamin, Pyridin oder N-Methylmorpholin in Frage kommen. Geeignete Lösungsmittel sind beispielsweise cyclische Aether, wie Tetrahydrofuran und Dioxan, halogenierte niedere Kohlenwasserstoffe, wie Chloroform und Methylenchlorid, Dimethylformamid, Dimethylacetamid, Acetonitril, Aceton und Wasser, sowie Mischungen davon. Die Reaktionstemperatur kann in einem weiten Bereich variieren und liegt in der Regel zwischen

-50 °C und 50 °C, vorzugsweise zwischen etwa -10 °C und 30 °C.

Die Oximbildung gemäss Verfahrensvariante b) des erfindungsgemässen Verfahrens kann ebenfalls nach an sich bekannten und jedem Fachmann geläufigen Methoden durchgeführt werden. Die Verbindung der Formel V wird dabei vorzugsweise in Form eines Säureadditionssalzes eingesetzt, beispielsweise als Hydrochlorid oder als p-Toluolsulfonat. Geeignete Lösungsmittel sind beispielsweise Wasser, niedere Alkohole, wie Methanol, cyclische Aether, wie Tetrahydrofuran und Dioxan, Acetonitril, N-Methylpyrrolidon, Dimethylformamid und Dimethylacetamid, sowie Mischungen davon. In einer bevorzugten Ausführungsform verwendet man Dimethylacetamid als Lösungsmittel. In einer weiteren bevorzugten Ausführungsform arbeitet man in Gegenwart eines Kupfersalzes, wobei sowohl Kupfer(I)- als auch Kupfer(II)salze in Frage kommen. Geeignete Salze sind beispielsweise die entsprechenden Halogenide, z.B. Chloride und Bromide, Sulfate, Acetate, Nitrate, Oxide, Carbonate, Perchlorate und die Tetrafluoroborate. Die Reaktionstemperatur liegt in einem Bereich von -20 °C bis 40 °C, vorzugsweise von 0 °C bis 30 °C.

Gemäss Variante c) des erfindungsgemässen Verfahrens können Verbindungen der allgemeinen Formel I hergestellt werden, worin m 1 und Y -S- darstellt und die übrigen Symbole die obige Bedeutung besitzen. Auch dieses Verfahrens kann nach an sich bekannten und jedem Fachmann geläufigen Methoden durchgeführt werden. Die mit D bezeichnete Abgangsgruppe ist vorzugsweise ein Halogenatom, z.B. Chlor, Brom oder Jod, eine niedere Alkyl- oder Arylsulfonyloxygruppe, wie Methansulfonyloxy oder p-Toluolsulfonyloxy, oder eine niedere Alkanoyloxygruppe, wie Acetoxy. Das Symbol D bedeutet vorzugsweise Acetoxy. Die Reaktion kann beispielsweise in einem inerten Lösungsmittel, beispielsweise in einem halogenierten niederen Kohlenwasserstoff, wie Methylenchlorid und Chloroform, in einem cyclischen Aether wie Tetrahydrofuran oder Dioxan, in einem offenkettigen Aether, wie Diäthyläther, in Dimethylformamid, Dimethylacetamid, Dimethylsulfoxid oder in Aceton, in Gegenwart einer Base, wie Kaliumcarbonat oder einem tertiären Amin, wie Triäthylamin, durchgeführt werden. Es ist aber auch möglich, das Mercaptan der Formel VII in Form eines Salzes einzusetzen, wobei insbesondere die Lithium-, Natrium-und Kaliumsalze für diesen Zweck in Frage kommen. Die Reaktion kann in einem weiten Temperaturbereich durchgeführt werden, wobei man jedoch vorzugsweise bei Raumtemperatur arbeitet.

Zur Herstellung der leicht hydrolysierbaren Ester der Carbonsäuren der Formel I gemäss Variante d) wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z.B. eines Alkalimetallhydroxids, Alkalimetallhydrogencarbonats oder -carbonats, oder eines organischen Amins, wie Triäthylamin, 1,8-Diazbicyclo[5.4.0]undec-7-en (DBU) oder Tetramethylguanidin, beschleunigt werden. Diese Reaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereich von etwa 0 bis 40 °C.

Falls gemäss den Verfahrensvarianten a) - d) ein Endprodukt der Formel I hergestellt werden soll, in der $Z^1$ und $Z^2$ zwei vicinale Hydroxygruppen enthalten, können in den entsprechenden Ausgangsverbindungen der Formel I-VII diese Hydroxygruppen geschützt sein; nach erfolgter Umsetzung werden die Hydroxygruppen wieder freigesetzt. In der Verfahrensvariante a), b) und c) sind vorzugsweise nur die in $Z^1$ vorhandenen Hydroxygruppen geschützt (in $Z^2$ ist das Schützen nicht notwendig) wohingegen in der Verfahrensvariante d) vorzugsweise sowohl in $Z^1$ als in $Z^2$ die Hydroxygruppen geschützt sind.

Die Hydroxyschutzgruppen werden vorzugsweise auf einer Vorproduktstufe eingeführt (vgl. die nachstehenden Ausführungsbeispiele). Vorzugsweise schützt man durch Diphenylmethyl (beispielsweise geht dann eine 3,4-Dihydroxyphenylgruppe in die 2,2-Diphenyl-1,3-benzodioxol-5-yl-gruppe über), indem man mit Diphenyldichlormethan erhitzt. Die Abspaltung der Diphenylmethylgruppe erfolgt vorzugsweise durch Einwirken eines sauren Mittels in einer Spur Wasser, z.B. durch konzentrierte wässrige Salzsäure oder, insbesondere durch Trifluoressigsäure mit einer Spur Wasser. Als Reaktionstemperatur eignet sich 0 °C bis etwa Raumtemperatur.

Die Hydroxygruppen können auch durch niedere Alkanoylgruppen, z.B. Acetyl, geschützt werden. Eingeführt wird z.B. durch Behandeln mit einem niederen Alkanoylhalogenid oder -anhydrid, z.B. dem Chlorid, in Gegenwart einer Base, wie Natriumhydroxid, DBU oder Diisopropyläthylamin. Die Abspaltung erfolgt unter milden alkalischen Bedingungen (pH etwa 7-8), z.B. mit Natriumhydroxid oder -carbonat, bei etwa 0° bis 50 °C.

Eine weitere Möglichkeit, die Hydroxygruppen zu schützen liegt in der Verwendung von Silylgruppen, z.B. Trimethylsilyl, t-Butyldimethylsilyl. Diese werden vorteilhaft eingeführt durch Behandeln mit dem entsprechenden Silylchlorid. Die Abspaltung kann durch Einwirken eines Fluorids erfolgen, vorzugsweise Tetrabutylammoniumfluorid, in einem organischen Lösungsmittel, z.B. Acetonitril, bei etwa 0 °C bis 50 °C.

Die Herstellung der Salze und Hydrate der Verbindungen der Formel I bzw. der Hydrate dieser Salze gemäss Variante e) kann in an sich bekannter Weise erfolgen, z.B. durch Umsetzung der Carbonsäure der

Formel I oder ein Salz davon mit einer äquivalenten Menge der gewünschten Base, zweckmässig in einem Lösungsmittel wie Wasser, oder in einem organischen Lösungsmittel, wie Aethanol, Methanol, Aceton oder anderen mehr. Entsprechend wird Salzbildung durch Addition einer organischen oder anorganischen Säure herbeigeführt. Die Temperatur der Salzbildung ist nicht kritisch. Sie liegt im allgemeinen bei Raumtemperatur, kann aber auch leicht darüber oder darunter, etwa im Bereiche von 0 °C bis +50 °C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester oder Salze davon) einer feuchten Atmosphäre, z.B. bei etwa +10° bis +40 °C, ausgesetzt werden.

Die verschiedenen als Ausgangsstoffe verwendeten Verbindungen sind bekannt oder können nach an sich bekannten Methoden und ausgehend von bekannten Ausgangsstoffen hergestellt werden. Die nachfolgenden Beispiele enthalten detaillierte Angaben betreffend die Herstellung dieser Ausgangsstoffe.

Wie bereits erwähnt besitzen die erfindungsgemässen Produkte wertvolle pharmakologische, insbesondere antibiotische Eigenschaften. Sie besitzen ein weites Wirkungsspektrum gegen gram-positive und gramnegative Mikroorganismen. Zum Nachweis dessen wurde folgender Wirkungsnachweis geführt:

1. Mindesthemmkonzentration in vitro

Zum Nachweis der antimikrobiellen Wirksamkeit der erfindungsgemässen Produkte wurden verschiedene der gemäss den nachstehenden Ausführungsbeispielen hergestellten Verbindungen auf ihre Aktivität in vitro mit der Agarverdünnungsmethode getestet. Die ermittelten Aktivitäten, Mindesthemmkonzentrationen (MHK) in µg/ml, sind in der nachfolgenden Tabelle 1 zusammengestellt:

**Tabelle 1**

Aktivitäten in vitro (MHK µg/ml)

| Endprodukt aus Beisp.No. | S.aureus 6538 | S.pyogenes 15 | S.pneumoniae BA | E.coli 25922 | K.pneumoniae 418 | K.oxytoca 1082 | S.marcescens 69438 |
|---|---|---|---|---|---|---|---|
| 1 | 16 | 4 | . | 0.25 | 0.12 | 0.5 | 0.25 |
| 2 | 32 | 2 | . | <0.1 | <0.25 | 4 | <0.25 |
| 3 | 32 | 4 | 2 | <0.1 | <0.1 | 0.25 | 0.12 |
| 4 | 4 | 0.25 | . | <0.1 | <0.1 | 16 | 0.12 |
| 5 | 4 | 0.5 | . | <0.1 | <0.1 | 0.25 | <0.1 |
| 6 | 16 | 2 | . | <0.1 | <0.1 | 0.12 | 0.12 |
| 7 | 32 | 4 | 2 | <0.1 | <0.1 | <0.1 | 0.12 |
| 8 | 4 | 1 | . | <0.1 | <0.1 | <0.1 | 0.12 |
| 9 | 8 | 1 | | <0.1 | <0.1 | <0.1 | <0.1 |
| 10 | 32 | 4 | . | <0.1 | <0.1 | 4 | 0.12 |
| 11 | 8 | 2 | . | <0.1 | <0.1 | <0.1 | 0.25 |
| 12 | 16 | 2 | . | <0.1 | <0.1 | 0.5 | <0.1 |
| 13 | 16 | 1 | . | 0.06 | 0.06 | 0.06 | 0.12 |
| 14 | 16 | 0.5 | 0.5 | 0.12 | 0.06 | 0.12 | 0.25 |
| 15 | 32 | 0.12 | . | <0.1 | <0.1 | 0.5 | 1 |
| 16 | 32 | 0.25 | | <0.1 | <0.1 | 0.25 | 1 |
| 17 | >32 | 0.12 | . | <0.1 | 0.25 | 2 | 0.5 |
| 18 | >32 | 1 | . | 0.12 | 0.12 | 0.25 | 1 |
| 19a | 16 | 0.5 | . | <0.1 | <0.1 | 1 | 0.12 |
| 19b | 16 | 0.25 | . | <0.1 | <0.1 | 0.5 | <0.1 |
| 21 | >32 | 16 | 8 | 1 | 0.5 | 1 | 1 |
| 22 | >32 | 16 | 16 | 8 | 0.5 | 0.5 | 16 |
| 23 | 16 | 4 | 4 | 0.25 | 0.12 | 16 | 2 |

**Tabelle 1** (Fortsetzung 1)

Aktivitäten in vitro (MHK µg/ml)

| Endprodukt aus Beisp.No. | E.cloacae 908SSi | E.cloacae 908R | E.cloacae P99 | E.aerogenes CF153 | C.freundii 902 | P.mirabilis 2117 |
|---|---|---|---|---|---|---|
| 1 | 4 | 16 | 32 | . | 1 | 0.12 |
| 2 | 4 | 1 | 16 | 0.5 | <0.1 | 0.25 |
| 3 | 8 | 1 | 0.5 | . | 0.12 | <0.1 |
| 4 | 4 | 1 | 32 | . | <0.1 | 0.25 |
| 5 | 1 | 0.25 | 4 | . | <0.1 | 0.25 |
| 6 | 4 | 0.12 | 4 | <0.1 | <0.1 | 0.25 |
| 7 | 4 | 2 | 1 | . | 0.12 | 0.12 |
| 8 | 2 | 0.25 | 8 | . | <0.1 | <0.1 |
| 9 | 4 | 0.12 | 8 | <0.1 | <0.1 | <0.1 |
| 10 | 8 | 2 | 16 | . | 0.12 | <0.1 |
| 11 | 4 | 2 | 16 | 1 | 0.12 | 0.12 |
| 12 | 4 | 2 | 8 | 4 | <0.1 | 0.12 |
| 13 | 4 | 1 | 8 | . | 0.25 | 0.06 |
| 14 | 1 | 2 | 16 | . | 1 | 0.5 |
| 15 | 16 | 2 | 16 | 0.12 | 0.25 | 0.12 |
| 16 | 8 | 0.25 | 8 | 0.12 | 0.12 | 0.25 |
| 17 | 8 | 1 | 16 | 0.25 | 0.5 | 0.5 |
| 18 | 8 | 2 | 16 | 0.12 | 0.12 | <0.1 |
| 19a | 2 | 16 | 16 | 0.12 | 0.25 | 0.25 |
| 19b | 2 | 2 | 8 | <0.1 | <0.1 | 0.25 |
| 21 | 8 | 8 | 16 | . | 4 | 1 |
| 22 | 16 | 32 | 32 | . | 16 | 16 |
| 23 | 16 | >32 | >32 | . | 4 | 1 |

**Tabelle 1** (Fortsetzung 2)

Aktivitäten in vitro (MHK µg/ml)

| Endprodukt aus Beisp.No. | P.vulgaris 1028 | P.aeruginosa BA | P.aeruginosa 799/WT | P.aeruginosa 799/61 | P.aeruginosa MK1184 | P.aeruginosa 27853 |
|---|---|---|---|---|---|---|
| 1 | 0.5 | 0.5 | 0.5 | 0.25 | 4 | 4 |
| 2 | 0.5 | <0.1 | 0.12 | <0.1 | 0.25 | <0.1 |
| 3 | <0.1 | <0.1 | 0.12 | <0.1 | <0.1 | 0.25 |
| 4 | 1 | <0.1 | 0.25 | 0.12 | 0.25 | 0.5 |
| 5 | 0.5 | 0.12 | <0.1 | <0.1 | <0.1 | 0.12 |
| 6 | <0.1 | <0.1 | <0.1 | <0.1 | <0.1 | <0.1 |
| 7 | 0.12 | 0.12 | 0.5 | 0.25 | <0.1 | 1 |
| 8 | <0.1 | <0.1 | <0.1 | <0.1 | <0.1 | 0.12 |
| 9 | <0.1 | <0.1 | . | <0.1 | <0.1 | <0.1 |
| 10 | <0.1 | <0.1 | <0.1 | <0.1 | <0.1 | <0.1 |
| 11 | 0.25 | 0.12 | 0.12 | <0.1 | <0.1 | <0.1 |
| 12 | 0.25 | <0.1 | <0.1 | <0.1 | <0.1 | <0.1 |
| 13 | 0.06 | 2 | 1 | 0.06 | 8 | 4 |
| 14 | 0.25 | 4 | 1 | 0.06 | 16 | 4 |
| 15 | 0.5 | 0.5 | 0.5 | <0.1 | 32 | 1 |
| 16 | 0.25 | 4 | 0.5 | <0.1 | >32 | 1 |
| 17 | 0.25 | 2 | 2 | <0.1 | 8 | 1 |
| 18 | 0.12 | 1 | 0.12 | <0.1 | 2 | 2 |
| 19a | 0.12 | 1 | 0.25 | <0.1 | 16 | 2 |
| 19b | <0.1 | 0.5 | 0.25 | <0.1 | 8 | 0.25 |
| 21 | 2 | 8 | 8 | 0.25 | >32 | 8 |
| 22 | 16 | >32 | 16 | 0.25 | >32 | 32 |
| 23 | 2 | 32 | 1 | 0.12 | >32 | 32 |

**Tabelle 1** Fortsetzung 3)

Aktivitäten in vitro (MHK µg/ml)

| Endprodukt aus Beisp.No. | S.aureus 6538 | S.pyogenes 15 | S.pneumoniae BA | E.coli 25922 | K.pneumoniae 418 | K.oxytoca 1082 | S.marcescens 69438 |
|---|---|---|---|---|---|---|---|
| 42 | 32 | 8 | 4 | <0.1 | <0.1 | <0.1 | 0.12 |
| 43 | 16 | 8 | 4 | <0.1 | <0.1 | <0.1 | 0.12 |
| 44 | 32 | 8 | 1 | <0.1 | <0.1 | <0.1 | 0.25 |
| 45 | 16 | 1 | 0.25 | 0.06 | 0.06 | 0.06 | 0.06 |
| 46 | >32 | 4 | . | 0.12 | 0.25 | 2 | 4 |
| 47 | 32 | 1 | 1 | 0.12 | <0.1 | 0.25 | 0.25 |
| 61 | 16 | 1 | 4 | <0.1 | <0.1 | <0.1 | <0.1 |

| Endprodukt aus Beisp.No. | E.cloacae 908SSi | E.cloacae 908R | E.cloacae P99 | E.aerogenes CF153 | C.freundii 902 | P.mirabilis 2117 |
|---|---|---|---|---|---|---|
| 42 | 4 | 0.5 | . | . | <0.1 | 0.12 |
| 43 | 2 | 0.12 | 0.12 | . | <0.1 | 0.12 |
| 44 | 8 | 1 | . | . | 0.25 | 0.25 |
| 45 | 2 | >32 | >32 | . | 0.06 | . |
| 46 | 16 | 1 | 16 | 0.25 | 0.5 | 0.25 |
| 47 | 8 | 1 | 16 | . | 0.5 | 0.12 |
| 61 | 1 | 0.5 | 1 | | <0.1 | <0.1 |

**Tabelle 1** (Fortsetzung 4)

Aktivitäten in vitro (MHK µg/ml)

| Endprodukt aus Beisp.No. | P.vulgaris 1028 | P.aeruginosa BA | P.aeruginosa 799/WT | P.aeruginosa 799/61 | P.aeruginosa MK1184 | P.aeruginosa 27853 |
|---|---|---|---|---|---|---|
| 42 | 0.25 | <0.1 | <0.1 | <0.1 | <0.1 | 0.25 |
| 43 | 0.12 | <0.1 | <0.1 | <0.1 | <0.1 | 0.25 |
| 44 | 0.25 | <0.1 | 0.12 | <0.1 | 0.12 | 0.5 |
| 45 | . | 0.06 | 0.06 | 0.64 | 1 | 0.12 |
| 46 | 0.5 | 0.5 | 0.25 | <0.1 | 2 | 0.25 |
| 47 | 0.25 | 4 | 4 | 0.25 | >32 | 4 |
| 61 | <0.1 | <0.1 | <0.1 | <0.1 | <0.1 | <0.1 |

2. Experimentelle systemische Septikämie an der Maus

Man verursacht eine experimentelle systemische Septikämie in 16-20 g schweren Albinomäusen, indem man den Versuchstieren eine Probe einer verdünnten Kultur des Testorganismus, welche über Nacht

gewachsen ist, intraperitoneal injiziert. Die verabreichte Probe wird dabei so bemessen, dass die verabreichte Dosis etwa 5-100 mal grösser ist, als die Dosis, welche benötigt wird, um 50% der unbehandelten Versuchstiere innerhalb von 48 Stunden zu töten. Die Bakterien wurden als Suspension in Kulturbrühe injiziert. Sowohl für die Kontrolle als auch für die Behandlung wurden für jede getestete Dosis Gruppen zu je 5 Versuchstieren verwendet. Die erfindungsgemässen Produkte wurden subkutan verabreicht, und zwar als Suspension in Tween 80 (2%), wobei die Konzentrationen so gewählt wurden, dass jedes Versuchstier pro Gabe 500 $\mu$l erhielt. Verabreicht wurden die jeweiligen Konzentrationen zweimal, und zwar 1 und 3 Stunden nach der Infektion (bei P. aeruginosa dreimal, und zwar 1,3 und 5 Stunden nach der Infektion). Die $ED_{50}$ ist diejenige Dosis, welche 50% der Versuchstiere schützt, und wurde ausgehend von der Ueberlebensrate nach 4 Tagen nach der Infektion mittels der Probitmethode berechnet. In der nachstehenden Tabelle 2 sind die $ED_{50}$-Werte in mg/kg s.c. für Vertreter der erfindungsgemässen Stoffklasse angegeben.

Tabelle 2

| Aktivitäten in mg/kg s.c. bei experimenteller systemischer Septikämie an der Maus | | | |
|---|---|---|---|
| Endprodukt aus Beispiel No. | S.aureus Schoch | E.coli 25922 | Pseudomonas aeruginosa BA |
| 2 | | 0.3 | |
| 3 | | 0.1 | 0.04 |
| 4 | 3.1 | <0.1 | 0.12 |
| 5 | 3.1 | <0.1 | 0.034 |
| 6 | | <0.2 | 0.3 |
| 7 | | <0.3 | 0.08 |
| 8 | 2 | <0.1 | 0.45 |
| 9 | | <0.05 | 0.24 |
| 10 | | <0.1 | 0.15 |
| 11 | 3.5 | <0.05 | 0.035 |
| 12 | 6.2 | <0.05 | 0.028 |
| 13 | | | 4 |
| 14 | >12 | 4.4 | 7.1 |
| 15 | | 0.9 | >2 |
| 16 | | <0.5 | >2 |
| 19b | | 0.78 | >3 |
| 21 | | <0.3 | 0.39 |
| 22 | | <0.05 | 1.8 |
| 42 | 10 | 0.005 | 0.017 |
| 43 | 12 | 0.05 | 0.019 |
| 44 | >12 | <0.05 | <0.01 |
| 45 | | 0.13 | 2.1 |
| 46 | | <0..5 | |
| 47 | | <0.1 | 0.1 |
| 61 | 8.4 | <0.1 | 0.06 |

Die erfindungsgemässen Produkte können als Heilmittel, z.B. in Form pharmazeutischer Präparate zur enteralen oder parenteralen Applikation, Verwendung finden. Die erfindungsgemässen Produkte können beispielsweise peroral, z.B. in Form von Tabletten, Lacktabletten, Dragées, Hart- und Weichgelatinekapseln, Lösungen, Emulsionen oder Suspensionen, rektal, z.B. in Form von Suppositorien, oder parenteral, z.B. in Form von Injektionslösungen, verabreicht werden.

Die Herstellung der pharmazeutischen Präparate kann in jedem Fachmann geläufiger Weise dadurch erfolgen, dass man die erfindungsgemässen Stoffe, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als solche Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Träger beispielsweise pflanzliche Oele, Wachse, Fette und halbfeste

und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole.

Als pharmazeutische Hilfsstoffe kommen die üblichen Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel und Antioxydantien in Frage.

Die Verbindungen der Formel I und ihre Salze bzw. Hydrate kommen insbesondere für die parenterale Applikation in Betracht und werden zu diesem Zweck vorzugsweise als Lyophilisat oder Trockenpulver zur Verdünnung mit üblichen Trägern, wie Wasser oder isotonischer Kochsalzlösung, zur Verfügung gestellt. Die leicht hydrolysierbaren Ester von Verbindungen der Formel I und ihre Salze und Hydrate kommen insbesondere für die enterale Applikation in Betracht.

Die pharmazeutischen Präparate können die erfindungsgemässen Stoffe in Mengen von etwa 25-2000 mg, vorzugsweise 100-1000 mg, pro Einzeldosierungsform enthalten. Für die Prophylaxe und Therapie von Injektionskrankheiten kommt für den Erwachsenen eine tägliche Dosis von etwa 0,05 g bis etwa 4 g, insbesondere etwa 0,1 g bis etwa 2 g in Betracht.

Die nachfolgenden Beispiele sollen die vorliegende Erfindung näher erläutern, ihren Umfang jedoch in keiner Weise beschränken.

## Beispiel 1

Herstellung von (6R,7R)-7-[(Z)-2-(2-Amino-4-thiazolyl)-2-[[(1-hydroxycarbamoyl)-1-methyläthoxy]imino]-acetamido]-3-[[[2-[(3,4-dihydroxybenzolsulfonamido)methyl]-5-methyl-s-triazolo[1.5-a]pyrimidin-7-yl]thio]-methyl]-8-oxo-5-thia-1-azabicyclo[4.2.0]oct-2-en-2-carbonsäure

0,05 g (6R,7R)-7-(2-Amino-4-thiazolglyoxylamido)-3-[[[2-[(3,4-dihydroxybenzolsulfonamido)methyl]-5-methyl-s-triazolo[1,5-a]pyrimidin-7-yl]thio]-methyl]-8-oxo-5-thia-1-azabicyclo[4.2.0]oct-2-en-2-carbonsäure und 0,02 g 2-(Aminooxy)-N-hydroxy-2-methylpropionamid-hydrochlorid werden in 0,5 ml Dimethylacetamid 16 Stunden bei Raumtemperatur gerührt. Das Dimethylacetamid wird am Hochvakuum abgedampft, der Rückstand wird mit Wasser digeriert und der gebildete Niederschlag filtriert und getrocknet. Man erhält 0,015 g (6R,7R)-7-[(Z)-2-(2-Amino-4-thiazolyl)-2-[(1-hydroxycarbamoyl)-1-methyläthoxy)imino]acetamido]-3-[[[2-[(3,4-dihydroxybenzolsulfonamido)methyl]-5-methyl-s-triazolo[1.5-a]pyrimidin-7-yl]thio]methyl]-8-oxo-5-thia-1-azabicyclo[4.2.0]oct-2-en-2-carbonsäure als leicht beiges Pulver.

$^1$H-NMR (DMSO-$d_6$, 250 MHz): Signale u.a. bei $\delta$(ppm) 1,40 (s, 3H), 1,42 (s, 3H), 2,57 (s, 3H), 3,62 (d, J = 18 Hz, 1H), 3,85 (d, J = 18 Hz, 1H), 4,13 (d, J = 6 Hz, 2H), 4,33 (d, J = 12,5 Hz, 1H), 4,50 (d, J = 12,5 Hz, 1H), 5,26 (d, J = 5 Hz, 1H), 5,90 (d,d J = 5 Hz, J = 8 Hz, 1H), 6,76 (d, J = 8,5 Hz, 1H), 6,80 (s, 1H), 7,06 (d,d J = 2,5 Hz, J = 8,5Hz, 1H), 7,15 (d, J = 2,5Hz, 1H), 7,25 (s, 1H), 7,37 (s, 2H), 8,05 (t, J = 6 Hz, 1H).

IR (KBr) 1775 $cm^{-1}$

MS 849,9 (M + $H^{\oplus}$)

Das als Ausgangsmaterial eingesetzte 2-(Aminooxy)-N-hydroxy-2-methylpropionamid-hydrochlorid kann wie folgt hergestellt werden:

Man verrührt 0,35 g Hydroxylamin-hydrochlorid, 0,7 ml Triäthylamin und 2,03 g Bis(trimethylsilyl)-acetamid in 20 ml Dichlormethan während 2 Stunden und gibt sodann 2 g 2-Methyl-2-(phthalimidooxy)-propionsäure-2-benzothiazol-thiolester zu (bekannt aus EPOS 286.145). Man rührt 3 Stunden lang weiter und dampft danach das Lösungsmittel ab, gibt 25 ml Aethanol zu und dampft wiederum ab. Danach löst man mit 50 ml Aethylacetat und 50 ml Wasser auf, trennt die organische Phase ab, wäscht sie mit Wasser und dampft sie ein. Nach Umkristallisieren aus 25 mi Aethylacetat erhält man 1 g N-Hydroxy-2-methyl-2-(phthalimidooxy)-2-methylpropionamid vom Smp. 184-187 °C (Zers.).

15,8 g N-Hydroxy-2-methyl-2-(phthalimidooxy)-2-methylpropionamid werden in 150 ml Aethanol und 110 ml Dimethylacetamid zur Lösung gebracht. Nach Zugabe von 3 g Hydrazinhydrat rührt man 1 Stunde bei 0 °C, filtriert vom Ausgefällten ab und dampft das Filtrat im Vakuum ein. Der Rückstand wird in 150 ml Wasser und 50 ml 1N wässriger Salzsäure verrührt. Nach nochmaligem Filtrieren dampft man ein und kristallisiert aus 100 ml Aethanol durch Zugabe von 450 ml Diäthyläther. Man erhält 7,8 g kristallines 2-(Aminooxy)-N-hydroxy-2-methylpropionamid-hydrochlorid vom Smp. 158-160 °C (Zers.).

Die als Ausgangsmaterial eingesetzte (6R,7R)-7-(2-Amino-4-thiazolglyoxylamido)-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo-

[4.2.0]oct-2-en-2-carbonsäure kann wie folgt hergestellt werden:

1,0 g 2-(Chlormethyl)-5-methyl-s-triazolo[1.5-a]pyrimidin-7-ol (bekannt aus C.A. 104/09-068678) werden in 20 ml konz. wässrigem Ammioniak gelöst und über Nacht bei Raumtemperatur gerührt. Vom gebildeten Niederschlag wird abgenutscht. Die Mutterlauge wird eingeengt und der Rückstand in Aethanol aufgenommen. Dabei kristallisiert das Produkt aus. Man erhält 0,66g 2-(Aminomethyl)-5-methyl-s-triazolo[1,5-a]-pyrimidin-7-ol als weisses Pulver vom Smp. 204-207 ° C.

0,35 g 2,2-Diphenyl-1,3-benzodioxol-5-sulfinsäure-Lithiumsalz (Herstellung siehe Beispiel 21) wird durch Zugabe eines Aequivalentes 1N wässriger Salzsäure in die freie Säure übergeführt. Letztere wird in 5 ml Methylenchlorid mit 0,140 g N-Chlorsuccinimid versetzt und 1 Stunde bei Raumtemperatur gerührt. Es wird vom entstandenen Niederschlag filtriert und die Mutterlauge zur Trockene eingeengt. Der Rückstand wird aus Acetonitril kristallisiert. Man erhält 0,27 g [3,4-[(Diphenylmethylen)dioxy]phenyl]sulfonylchlorid als weisse Kristalle vom Smp. 109-110 ° C.

0,37 g 2-(Aminomethyl)-5-methyl-s-triazolo[1,5-a]pyrimidin-7-ol werden in 3.0 ml Dimethylformamid gelöst, mit 0,17 ml N-Aethyl-diisopropylamin und 0,18 g [3,4-[(Diphenylmethylen)dioxy]phenylsulfonylchlorid versetzt und 4 Stunden bei Raumtemperatur gerührt. Das Dimethylformamid wird am Hochvakuum abgedampft und der Rückstand zwischen Wasser und Aethylacetat verteilt. Die organische Phase wird zweimal mit Wasser und einmal mit gesättigter wässriger Natriumchloridlösung gewaschen, mit Magnesiumsulfat getrocknet und eingeengt. Der Rückstand wird in Wasser aufgenommen. Es entsteht ein Niederschlag; dieser wird abfiltriert und getrocknet. Man erhält 0,370 g N-[[7-Hydroxy-5-methyl-s-triazolo[1,5-a]-pyridin-2-yl]methyl]-2,2-diphenyl-1,3-benzodioxol-5-sulfonamid als weisses Pulver.

0,26 g N-[[7-Hydroxy-5-methyl-s-triazolo[1,5-a]pyridin-2-yl]methyl]-2,2-diphenyl-1,3-benzodioxol-5-sulfonamid werden in 5,0 ml Phosphoroxychlorid 15 Minuten unter Rückflussbedingungen erhitzt. Das Phosphoroxychlorid wird am Wasserstrahlvakuum abgedampft und der Rückstand zwischen Methylenchlorid und Wasser verteilt. Die organische Phase wird mit Magnesiumsulfat getrocknet und eingeengt. Der Rückstand wird mit einer 5%-igen Lösung von Natriumhydrogensulfid in Dimethylformamid versetzt und 2 Stunden bei Raumtemperatur gerührt. Das Dimethylformamid wird abgedampft und der Rückstand in Wasser aufgenommen. Der pH-Wert wird mit 1N wässriger Salzsäure auf 2.0 eingestellt. Der gebildete Niederschlag wird abfiltriert und getrocknet. Man erhält 0,18 g N-[[7-Mercapto-5-methyl-s-triazolo[1,5-a]pyrimidin-2-yl]methyl]-2,2-diphenyl-1,3-benzodioxol-5-sulfonamid als gelbes Pulver.

0,27 g N-[[7-Mercapto-5-methyl-s-triazolo[1,5-a]pyrimidin-2-yl]methy]-2,2-diphenyl-1,3-benzodioxol-5-sulfonamid und 0,14 g 7-Aminocephalosporansäure werden mit 3,0 ml einer 20%-igen Lösung von Bortrifluorid in Acetonitril 4 Stunden bei Raumtemperatur gerührt. Nach Zugabe von 5 ml Eiswasser wird das Reaktionsgemisch zwischen Wasser und Aethylacetat verteilt. Der pH-Wert wird mit 3N wässriger Natronlauge auf 7,0 eingestellt. Die Phasen werden getrennt. Die wässrige Phase wird mit konzentrierter wässriger Salzsäure auf pH 2.0 eingestellt. Der gebildete Niederschlag wird abfiltriert. Der noch feuchte Niederschlag wird in Trifluoressigsäure gelöst und 2 Stunden bei Raumtemperatur gerührt. Die Trifluoressigsäure wird am Wasserstrahlvakuum abgedampft und der Rückstand mit Aethylacetat versetzt. Der Niederschlag wird filtriert und unter Hochvakuum getrocknet. Man erhält 0,2 g (6R,7R)-7-Amino-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure als leicht beiges Pulver.

$^1$H-NMR ($D_2O$, 250 MHz): Signale bei δ(ppm) 2,60 (s,3H), 3,50 (d, J = 18 Hz, 1H), 3,83 (d, J = 18 Hz, 1H), 4,11 (d, J = 12,5Hz, 1H), 4,44 (s, 2H), 4,77 (d, J = 5 Hz, 1H), 5,07 (d, J = 5 Hz, 1H), 6,46 (d, J = 8Hz, 1H), 6,85 (m, 2H), 7,07 (s, 1H).

IR: (KBr) 1781 $cm^{-1}$

MS: 580 (M + $H^{⊕}$)

0,15 g (6R,7R)-7-Amino-3-[[[2-[(3,4-dihydroxybenzolsulfonamido)methyl]-5-methyl-s-triazolo[1,5-a]-pyrimidin-7-yl]thio]methyl]-8-oxo-5-thia-1-azabicyclo[4.2.0]oct-2-en-2-carbonsäure wird in 1 ml Wasser und 1 ml Acetonitril suspendiert und durch Zugabe von 0,036 ml Triäthylamin in Lösung gebracht. Das Gemisch wird auf 0 ° C gekühlt, mit 0,20 g 2-Amino-4-thiazolthioglyoxylsäure-S-(2-benzothiazolyl)ester versetzt und 1 Stunde im Eisbad gerührt. Das Reaktionsgemisch wird 15 Minuten bei 3000 UpM zentrifugiert und die klare überstehende Lösung zwischen Wasser und Aethylacetat verteilt. Die wässrige Phase wird auf pH 2 gestellt. Es bildet sich ein gelber Niederschlag, welcher filtriert und getrocknet wird. Man erhält 0,12 g (6R,7R)-7-(2-Amino-4-thiazolglyoxylamido)-3[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure als gelbes Pulver.

$^1$H-NMR (DMSO-$d_6$, 250 MHz): Signale bei δ(ppm) 2,57 (s, 3H), 3,30 (d, J = 18 Hz, 1H), 3,81 (d, J = 18 Hz, 1H), 4,10 (d, J = 6 Hz, 2H), 4,32 (d, J = 12,5 Hz, 1H), 4,50 (d, J = 12,5 Hz, 1H), 5,21 (d, J = 5 Hz, 1H), 5,76 (d,d, J = 5 Hz, J = 8 Hz, 1H), 6,77 (d, J = 8,5 Hz, 1H), 7,06 (d,d, J = 2,5 Hz, J = 8,5 Hz, 1H), 7,14 (d, J = 2,5

Hz, 1H), 7,26 (s, 1H), 7,41 (s, 2H), 7,82 (s, 1H), 8,03 (t, J=6 Hz, 1H), 9,5 (s, breit 1H), 9,81 (d, J=8 Hz, 1H) auf s breit 1H, 14,0 (s, breit, 1H).
IR: KBr 1769 $cm^{-1}$
MS: 734 (M+H$^{⊕}$)

Beispiel 2

Herstellung von (6R,7R)-7-[(Z)-2-(2-Amino-4-thiazolyl)-2-[[(1,4-dihydro-5-hydroxy-4-oxo-2-pyrimidinyl)-methoxy]imino]acetamido]-3-[[[2-[(3,4-dihydroxybenzolsulfonamido)methyl]-5-methyl-s-triazolo[1,5-a]-pyrimidinyl-7-yl]thio]methyl]-8-oxo-5-thia-1-azabicyclo[4.2.0]oct-2-en-2-carbonsäure-Natriumsalz (1:1)

0,37 g (6R,7R)-7-(2-Amino-4-thiazolglyoxylamido)-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure, 0,16 g 2-(Aminooxy)methyl-5-hydroxy-4(1H)-pyrimidinon und 0,065 ml Methansulfonsäure werden in 4 ml Dimethylacetamid 16 Stunden bei Raumtemperatur gerührt. Das Dimethylacetamid wird unter Hochvakuum abgedampft und der Rückstand mit Wasser digeriert. Das Produkt wird abfiltriert und in Wasser resuspendiert, durch Zugabe von 1N wässriger Natronlauge bei pH 7,0 in Lösung gebracht und an RP12-Kieselgel mit Wasser/Acetonitril chromatographiert (RP12-Kieselgel = "Reversed phase" Kieselgel, z.B. Optiup $C_{12}$® der Firma ANTEC, Bennwil, Schweiz; ein Kieselgel mit Korngrösse 0.040-0.063 mm, behandelt mit Dodecyltrichlorsilan). Die Produktfraktionen werden eingeengt und lyophilisiert. Man erhält 0,15 g (6R,7R)-7-[(Z)-2-(2-Amino-4-thiazolyl)-2-[[(1,4-dihydro-5-hydroxy-4-oxo-2-pyrimidinyl)methoxy]imino]acetamido]-3-[[[2-[(3,4-dihydroxybenzolsulfonamido)methyl]-5-methyl-s-triazolo[1,5-a]pyrimidinyl-7-yl]thio]methyl]-8-oxo-5-thia-1-azabicyclo[4.2.0]-oct-2-en-2-carbonsäure-Natriumsalz (1:1) als leicht beiges Pulver.
$^{1}$H-NMR (DMSO-$d_6$, 250 MHz): Signale u.a. bei δ(ppm) 2,53 (s, 3H), 3,28 (d, J=18 Hz, 1H), 3,57 (d, J=18 Hz, 1H), 4,17 (m, 3H), 4,66 (d, J=12,5 Hz, 1H), 4,86 (s, 2H), 5,04 (d, J=5 Hz, 1H), 5,64 (d, J=5 Hz, 1H), 6,66 (d, J=8 Hz, 1H), 6,86 (s, 1H), 6,96 (m, 2H), 7,21 (s, 1H), 7,42 (s, 1H)
IR: (KBr) 1764 $cm^{-1}$
MS: 894,8 (M+H$^{⊕}$)

Das als Ausgangsverbindung eingesetzte 2-(Aminooxy)methyl-5-hydroxy-4(1H)-pyrimidinon kann wie folgt hergestellt werden:

0,696g (3 mMol) 5-(Benzyloxy)-2-(hydroxymethyl)-4(1H)-pyrimidinon, 0,787 g (3 mMol) Triphenylphosphin und 0,489 g (3 mMol) N-Hydroxyphthalimid werden in 50 ml Dimethylacetamid gelöst. Bei 20°C wird eine Lösung von 0,640 g (3,3 mMol) Azodicarbonsäurediäthylester in 2 ml Dimethylacetamid zugetropft. Man rührt 20 Stunden bei Raumtemperatur, dampft das Lösungsmittel im Hochvakuum ab und kristallisiert das verbleibende gelbe Oel aus Aethanol/Aether. Nach Umkristallisieren aus Aethanol erhält man N-[(5-Benzyloxy)-1,4-dihydro-4-oxo-2-pyrimidinyl]methoxy]phthalimid als weisse Kristalle vom Smp. 157°C.

0,754 g (2 mMol) N-[(5-Benzyloxy)-1,4-dihydro-4-oxo-2-pyridinyl]methoxy]phthalimid werden in 60 ml Methylenchlorid suspendiert. Bei -70°C werden 560 mg (2,2 mMol) Bortribromid zugetropft. Man rührt 4 Stunden bei -70°C, lässt auf Raumtemperatur erwärmen und rührt 16 Stunden weiter. Das Lösungsmittel wird im Vakuum abgedampft, der Rückstand mit 30 ml Wasser versetzt und gerührt. Das Produkt wird filtriert, mit Wasser und Aether gewaschen und im Hochvakuum bei 35°C getrocknet. Man erhält 5-Hydroxy-2-[(phthalimidooxy)methyl]-4(1H)-pyrimidinon als weisse Kristalle vom Smp. 174-175°C.

0,875 g (3 mMol) 5-Hydroxy-2-[(phthalimidooxy)methyl]4(1H)-pyrimidinon werden in 7 ml Dimethylformamid suspendiert. Bei 10°C gibt man 136 mg (3 mMol) Methylhydrazin dazu und rührt 1 Stunde bei Raumtemperatur. Die Lösung wird viermal mit 50 ml n-Pentan gewaschen, der feste Rückstand in Aether aufgenommen, das Produkt filtriert und aus Aethanol kristallisiert. Man erhält 2-(Aminooxy)methyl-5-hydroxy-4(1H)-pyrimidinon vom Smp. 163°C (Zers.) als weisse Kristalle.

Beispiel 3

Herstellung von (6R,7R)-7-[(Z)-2-(2-Amino-4-thiazolyl)-2-[[[(3,4-dihydroxyphenyl)sulfonyl]methoxy]imino]-acetamido]-3-[[[2-[(3,4-dihydroxybenzolsulfonamido)methyl]-5-methyl-s-triazolo[1,5-a]pyrimidin-7-yl]thio]-methyl]-8-oxo-5-thia-1-azabicyclo[4.2.0]oct-2-en-2-carbonsäure-Natriumsalz (1:1)

0,70 g (6R,7R)-7-(2-Amino-4-thiazolglyoxylamido)-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure und 0,32 g 4-[[(Aminooxy)methyl]sulfonyl]pyrocatechol-hydrochlorid (bekannt aus EPOS 303,172) werden in 7,0 ml Dimethylacetamid 2 Tage bei Raumtemperatur gerührt. Das Dimethylacetamid wird am Hochvakuum

abgedampft und der Rückstand in Wasser aufgenommen. Der entstandene Niederschlag wird abfiltriert, getrocknet, in Wasser resuspendiert, durch Zugabe von 1N wässriger Natronlauge bei pH 7,0 in Lösung gebracht und an RP12-Kieselgel mit Wasser/Acetonitril chromatographiert. Die einheitlichen Fraktionen werden eingeengt, das Produkt durch Ansäuern auf pH 2,0 ausgefällt, abfiltriert, getrocknet, in Wasser resuspendiert, durch Zugabe von 1N wässriger Natronlauge bei pH 7,0 in Lösung gebracht und lyophilisiert. Man erhält 0,44 g (6R,7R)-7-[(Z)-2-(2-Amino-4-thiazolyl)-2-[[[(3,4-dihydroxyphenyl)sulfonyl]methoxy]imino]-acetamido]-3-[[[2-[(3,4-dihydroxy benzolsulfonamido)methyl]-5-methyl-s-triazolo[1,5-a]pyrimidin-7-yl]thio]-methyl]-8-oxo-5-thia-1-azabicyclo[4.2.0]oct-2-en-2-carbonsäure-Natriumsalz (1:1) als leicht beiges Pulver.
$^1$H-NMR (DMSO-$d_6$, 250 MHz): Signale bei &(ppm) 2,54 (s, 3H), 3,16 (d, J = 18 Hz, 1H), 3,54 (d, J = 18 Hz, 1H), 4,14 (d, J = 12,5 Hz, 1H), 4,16 (s, 2H), 4,60 (d, J = 12,5 Hz, 1H), 4,95 (d, J = 5 Hz, 1H), 5,16 (m, 2H), 5,48 (m, 1H), 7 (m, 7H), 7,42 (s, 1H), 9,5 (m, breit, 1H)
IR: (KBr) 1765 $cm^{-1}$
MS: 935 ($M + H^{\oplus}$)

Beispiel 4

Herstellung von (6R,7R)-7-[(Z)-2-(2-Amino-4-thiazolyl)-2-[[(3,4-dihydroxybenzyl)oxy]imino]acetamido]-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure

0,370 g (6R,7R)-7-(2-Amino-4-thiazolglyoxylamido)-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure und 0,18 g O-[3,4-[(Diphenylmethylen)dioxy]benzyl]hydroxylamin-hydrochlorid werden in 4 ml Dimethylacetamid 16 Stunden bei Raumtemperatur gerührt. Das Dimethylacetamid wird am Hochvakuum abgedampft, der Rückstand mit Wasser digeriert, der gebildete Niederschlag in Wasser durch Zugabe von 1N wässriger Natronlauge bei pH 7,0 gelöst und an Kieselgel mit Wasser/Acetonitril chromatographiert. Die Produktfraktionen werden eingeengt und das Produkt durch Ansäuern auf pH 2,0 ausgefällt. Der Niederschlag wird abfiltriert, noch feucht in Trifluoressigsäure gelöst und 2 Stunden bei Raumtemperatur gerührt. Die Trifluoressigsäure wird am Wasserstrahlvakuum abgedampft und der Rückstand bei pH 7,0 (durch Zugabe von 1N wässriger Natronlauge) zwischen Wasser und Aethylacetat verteilt. Die wässrige Phase wird an Kieselgel mit Wasser/Acetonitril chromatographiert. Die Produktfraktionen werden eingeengt und bei pH 2,0 ausgefällt. Das Produkt wird abfiltriert und getrocknet. Man erhält 0,080 g (6R,7R)-7-[(Z)-2-(2-Amino-4-thiazolyl)-2-[[(3,4-dihydroxybenzyl)oxy]imino]acetamido]-3-[[[2-[(3,4-dihydroxybenzolsulfonamido)methyl]-5-methyl-s-triazolo[1,5-a]pyrimidin7-yl]thio]methyl]-8-oxo-5-thia-1-azabicyclo[4.2.0]oct-2-en-2-carbonsäure als leicht beiges Pulver.
$^1$H-NMR (DMSO-$d_6$, 250 MHz): Signale bei δ(ppm) 2,57 (s, 3H), 3,55 (d, J = 18 Hz, 1H), 3,77 (d, J = 18 Hz, 1H), 4,10 (d, J = 6 Hz, 2H), 4,32 (d, J = 12,5 Hz, 1H), 4,47 (d, J = 12,5 Hz, 1H), 4,92 (s, 2H), 5,15 (d, J = 5 Hz, 1H), 5,79 (d,d, J = 5 Hz, J = 8 Hz, 1H), 6,7 (m, 6H), 7,1 (m, 2H), 7,25(s,1H) überlagert von breitem s 7,27, 2H, 8,03 (t, J = 6Hz, 1H), 8,88 (s, breit, 2H), 9,54 (s, breit, 1H), 9,67 (d, J = 8 Hz, 1H), 9,80 (s, breit, 1H), 14,0 (s, sehr breit, 1H)
IR: (KBr) 1770 $cm^{-1}$
MS: 870.9 ($M + H^{\oplus}$)

Das als Ausgangsmaterial eingesetzte O-[3,4-[(Diphenylmethylen)dioxy]benzyl]hydroxylamin-hydrochlorid kann wie folgt hergestellt werden:

0,30 g 2,2-Diphenyl-1,3-benzodioxol-5-methanol, 0,40 g Triphenylphosphin und 0,33 g N-Hydroxyphthalimid werden in 5 ml Methylenchlorid gelöst und auf 0 °C gekühlt. Hierauf werden 0,30 ml Diäthylazodicarboxylat, gelöst in 2 ml Methylenchlorid, zugegeben und das Gemisch unter Auftauen auf Raumtemperatur 30 Minuten gerührt. Das Reaktionsgemisch wird auf 30 g Kieselgel aufgetragen und das Produkt mit Methylenchlorid eluiert. Die einheitlichen Fraktionen werden eingeengt. Dabei tritt spontan Kristallisation ein. Nach dem Trocknen erhält man 0,370 g N-[[3,4-[(Diphenylmethylen)dioxy]benzyl]oxy]phthalimid als weisse Kristalle vom Smp. 155 °C.

0,90 g N-[[3,4-[(Diphenylmethylen)dioxy]benzyl]oxy]phthalimid werden in 10 ml Aethanol suspendiert, mit 0,25 ml N,N-Dimethylaminopropylamin versetzt und 1 Stunde bei Raumtemperatur gerührt. Das Aethanol wird abgedampft, der Rückstand in Aethylacetat aufgenommen und an Kieselgel mit Aethylacetat als Eluens chromatographiert. Einheitliche Fraktionen werden eingeengt und das zurückbleibende Schaumharz aus n-Hexan kristallisiert. Man erhält 0,48 g O-[3,4-[(Diphenylmethylen)dioxy]benzyl]hydroxylamin als weisse Kristalle vom Smp. 159-163 °C. Daraus erhält man mit äthanolischer Salzsäure das entsprechende Hydrochlorid vom Smp. 154-157 °C.

Beispiel 5

Herstellung von 7-[(Z)-2-(2-Amino-4-thiazolyl)-2-[[(2-fluor-3,4-dihydroxybenzyl)oxy]imino]acetamido]-3[[[2-[-(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1)

0,37 g (6R,7R)-7-(2-Amino-4-thiazolglyoxylamido)-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure und 0,24 g O-[(4-Fluor-2,2-diphenyl-1,3-benzodioxol-5-yl)methyl]hydroxylamin-hydrochlorid werden in 4 ml Dimethylacetamid 16 Stunden bei Raumtemperatur gerührt. Das Dimethylacetamid wird am Hochvakuum abgedampft und der Rückstand mit Wasser digeriert. Der entstandene Niederschlag wird abfiltriert, noch feucht in 5 ml Trifluoressigsäure gelöst und 3 Stunden bei Raumtemperatur gerührt. Die Trifluoressigsäure wird am Wasserstrahlvakuum abgezogen und der Rückstand mit Aethylacetat digeriert. Der Niederschlag wird abfiltriert, getrocknet, in Wasser durch Zugabe von 1N wässriger Natronlauge bei pH 7,0 gelöst und an RP12-Kieselgel mit Wasser/Acetonitril chromatographiert. Die Produktfraktionen werden eingeengt und lyophilisiert. Man erhält 0,14 g 7-[(Z)-2-(2-Amino-4-thiazolyl)-2-[[(2-fluor-3,4-dihydroxybenzyl)oxy]imino]-acetamido]-3-[[[2-[(3,4-dihydroxybenzolsulfonamido)methyl]-5-methyl-s-triazolo[1,5-a]pyrimidin-7-yl]thio]-methyl]-8-oxo-5-thia-1-azabicyclo[4.2.0]oct-2-en-2-carbonsäure-Natriumsalz (1:1) als leicht beiges Pulver.
$^1$H-NMR (DMSO-$d_6$, 250 MHz): Signale bei δ(ppm) 2,54 (s, 3H), 3,30 (d, J = 18 Hz, 1H), 3,60 (d, J = 18 Hz, 1H), 4,12 (d, J = 12,5 Hz, 1H), 4,20 (m, 2H), 4,72 (d, J = 12,5 Hz, 1H), 5,01 (d, J = 5 Hz, 1H), 5,02 (s, 2H), 5,60 (d,d, J = 5Hz, J = 8 Hz, 1H), 6,8 (m, 8H), 7,23 (s, breit, 2H), 7,40 (s, 1H), 8,04 (m, breit, 1H), 9,51 (d, J = 8 Hz, 1H)
IR (KBr) 1765 $cm^{-1}$
MS: 889,3 ($M + H^{\oplus}$)

Das als Ausgangsmaterial eingesetzte O-[(4-Fluor-2,2-diphenyl-1,3-benzodioxol-5-yl)methyl]-hydroxylamin-hydrochlorid kann wie folgt hergestellt werden:

7,2 g 3-Fluorcatechol werden in 50 ml Tetrachlorkohlenstoff und 40 ml Chloroform bei -10 °C innerhalb 10 Minuten mit einer Lösung von 2,88 ml Brom in 30 ml Tetrachlorkohlenstoff versetzt. Nach 1-stündigem Rühren bei -10 °C wird das Reaktionsgemisch filtriert. Der zurückbleibende Feststoff wird mit wenig kaltem Tetrachlorkohlenstoff gewaschen und getrocknet. Man erhält 6,30 g 4-Brom-3-fluorcatechol als weisses Pulver. Dieses wird mit 6,0 ml Diphenyldichlormethan versetzt und innerhalb 1 Stunde auf 130 °C erhitzt. Anschliessend wird das Produkt bei 135 °C/0,04 Torr destilliert und aus Aethanol umkristallisiert. Man erhält 6,40 g 5-Brom-4-fluor-2,2-diphenyl-1,3-benzodioxol als weisses kristallines Pulver vom Smp. 61,5-63 °C.

| Elementaranalyse: | | | | | | | | |
|---|---|---|---|---|---|---|---|---|
| berechnet:<br>gefunden: | C | 61,48<br>61,42 | H | 3,26<br>3,48 | F | 5,12<br>5,26 | Br | 21,53 %<br>21,69 % |

17,3 g 5-Brom-4-fluor-2,2-dipheny-1,3-benzodioxol werden in 150 ml Tetrahydrofuran gelöst, bei -78 °C mit 29,1 ml einer 0,6M Lösung von n-Butyllithium in n-Hexan versetzt und 15 Minuten bei -78 °C gerührt. Hierauf werden 10 ml Dimethylformamid zugegeben, nach weiterem 15-minütigem Rühren mit halbgesättigter, wässriger Ammoniumchloridlösung versetzt und mit Aethylacetat extrahiert. Die organische Phase wird mit Magnesiumsulfat getrocknet und eingedampft. Der Rückstand wird aus Aethanol kristallisiert. Nach dem Trocknen erhält man 12,7 g 4-Fluor-2,2-diphenyl-1,3-benzodioxol-5-carboxaldehyd als weisse Kristalle vom Smp. 92-94 °C.

| Elementaranalyse: | | | | | | |
|---|---|---|---|---|---|---|
| berechnet:<br>gefunden: | C | 74,99<br>74,48 | H | 4,09<br>4,30 | F | 5,93 %<br>5,90 % |

0,32 g 4-Fluor-2,2-diphenyl-1,3-benzodioxol-5-carboxaldehyd werden in 5 ml Tetrahydrofuran gelöst und mit 20 mg Lithiumborhydrid versetzt. Nach 20-minütigem Rühren bei Raumtemperatur wird halbgesättigte, wässrige Ammoniumchloridlösung zugegeben und das Gemisch mit Aethylacetat extrahiert. Die organische Phase wird mit Magnesiumsulfat getrocknet, eingeengt und am Hochvakuum über Nacht getrocknet. Man erhält 0,320 g farbloses Oel, welches in 5 ml Methylenchlorid gelöst und nacheinander mit 0,40 g

Triphenylphosphin und 0,33 g N-Hydroxyphthalimid versetzt wird. Das Gemisch wird auf 0 ° C abgekühlt und mit 0,30 ml Diäthylazodicarboxylat, gelöst in 2 ml Methylenchlorid, versetzt. Nach 1-stündigem Rühren bei 0 ° C wird das gesamte Reaktionsgemisch an 30 g Kieselgel aufgetragen und das Produkt mit Methylenchlorid eluiert. Die einheitlichen Fraktionen werden eingeengt und der Rückstand aus Aethylacetat kristallisiert. Man erhält 0,35 g N-[(4-Fluor-2,2-diphenyl-1,3-benzodioxol-5-yl)methoxy]phthalimid als weisse Kristalle vom Smp. 177-179 ° C.

5,13 g N-[(4-Fluor-2,2-diphenyl-1,3-benzodioxol-5-yl)methoxy]phthalimid werden in 20 ml Aethanol suspendiert, mit 1,62 ml 3-Dimethylaminopropylamin versetzt und 1 Stunde bei Raumtemperatur gerührt. Das Aethanol wird am Wasserstrahlvakuum abgedampft und der Rückstand an Kieselgel (250 g) mit Aethylacetat chromatographiert. Die einheitlichen Fraktionen werden eingeengt; der ölige Rückstand wird mit 20 ml 1M äthanolischer Salzsäure versetzt. Es bildet sich ein weisser Niederschlag, der nach 1-stündigem Rühren im Eisbad abfiltriert und mit wenig kaltem Aethanol gewaschen wird. Nach dem Trocknen erhält man 3,80 g O-[(4-Fluor-2,2-diphenyl-1,3-benzodioxol-5-yl)methyl]hydroxylamin-hydrochlorid als weisses, kristallines Pulver vom Smp. 167-173 ° C.

$^1$H-NMR (DMSO-$d_6$, 250 MHz): Signale bei δ(ppm) 5,00 (s, 2H), 7,0 (m, 2H), 7,5 (m, 10H), 10,8 (s, breit, 3H)

Beispiel 6

Herstellung von (6R,7R)-7-[(Z)-2-(2-Amino-4-thiazolyl)-2-[[(1,4-dihydro-5-hydroxy-4-oxo-2-pyrimidnyl)-methoxy]imino]acetamido]-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1)

0,16 g 2-(Aminooxy)methyl-5-hydroxy-4(1H)-pyrimidinon, 0,065 ml Methansulfonsäure und 0,37 g (6R,7R)-7-(2-Amino-4-thiazolglyoxylamido)-3[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure werden 16 Stunden bei Raumtemperatur in 3,0 ml Dimethylacetamid gerührt. Das Dimethylacetamid wird am Hochvakuum abgedampft, der Rückstand mit Wasser digeriert und der entstandene Niederschlag filtriert. Dieser Feststoff wird in Wasser resuspendiert, durch Zugabe von 1N wässriger Natronlauge bei pH 7,0 in Lösung gebracht und an RP12-Kieselgel mit Wasser/Acetonitril chromatographiert. Einheitliche Fraktionen werden eingeengt und lyophilisiert. Man erhält 0,09 g (6R,7R)-7-[(Z)-2-(2-Amino-4-thiazolyl)-2-[[(1,4-dihydro-5-hydroxy-4-oxo-2-pyrimidinyl)methoxy]imino]acetamido]-3-[[[2-[[(3,4-dihydroxybenzoyl)oxy]methyl]-5-methyl-s-triazolo-[1,5-a]-pyrimidin-7-yl]thio]methyl]-8-oxo-5-thia-1-azabicyclo[4.2.0]oct-2-en-2-carbonsäure-Natriumsalz (1:1) als weisses Pulver.

$^1$H-NMR (DMSO-$d_6$, 250 MHz): Signale bei δ(ppm) 2,55 (s, 3H), 3,32 (d, J = 18 Hz, 1H), 3,62 (d, J = 18 Hz, 1H), 4,35 (d, J = 12,5 Hz, 1H), 4,68 (d, J = 12,5 Hz, 1H), 4,92 (s, 2H), 5,02 (d, J = 5 Hz, 1H), 5,47 (s, 2H), 5,62 (d,d, J = 5 Hz, J = 8 Hz, 1H), 6,84 (m, 2H), 7,28 (s, breit, 2H), 7,40 (m, 3H), 7,71 (s, 1H), 9,92 (d, J = 8 Hz, 1H)

IR (KBr) 1766 $cm^{-1}$

MS: 860 ($M + H^{\oplus}$)

Die als Ausgangsmaterial eingesetzte (6R,7R)-7-(2-Amino-4-thiazolglyoxylamido)-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure kann wie folgt hergestellt werden:

4,08 g (6R,7R)-7-Amino-3-[[[2-(hydroxymethyl)-5-methyl-s-triazolo-[1,5-a]pyrimidin-7-yl]thio]methyl]-8-oxo-5-thia-1-azabicyclo[4.2.0]oct-2-en-2-carbonsäure (bekannt aus EPOS 302,633) und 1,54 g 3,4-Dihydroxybenzoesäure werden mit 30 ml einer 20%igen Lösung von Bortrifluorid in Acetonitril versetzt und 2 Stunden bei Raumtemperatur gerührt. Es werden nochmals 25 ml einer 20%igen Lösung von Bortrifluorid in Acetonitril zugegeben; das Reaktionsgemisch wird 2 Tage unter Argon bei Raumtemperatur gerührt. Nach Zugabe von 100 ml Eiswasser wird das Reaktionsgemisch auf etwa die Hälfte eingeengt. Das Produkt wird durch Einstellen des pH-Wertes auf 2,0 mit konzentriertem wässrigem Ammoniak gefällt. Das ausgefällte Material wird in Wasser suspendiert, mit 1N wässriger Natronlauge in Lösung gebracht und durch Chromatographie an RP12-Kieselgel mit Wasser/Acetonitril gereinigt. Die Produktfraktionen werden auf etwa 50 ml eingeengt und das Produkt durch Ansäuern auf pH 2,0 gefällt. Nach Trocknen am Hochvakuum erhält man 1,60 g (6R,7R)-7-Amino-3-[[[2-[[(3,4-dihydroxybenzoyl)oxy]methyl]-5-methyl-s-triazolo[1,5-a]-pyrimidin-7-yl]thio]methyl]-8-oxo-5-thia-1-azabicyclo[4.2.0]oct-2-en-2-carbonsäure als weisses Pulver.

$^1$H-NMR (DMSO-d,) 250 MHz): Signale bei δ(ppm) 2,58 (s, 3H), 3,55 (d, J = 18 Hz, 1H), 3,76 (d, J = 18 Hz, 1H), 4,33 (d, J = 12,5 Hz, 1H), 4,45 (d, J = 12,5 Hz, 1H), 4,89 (d, J = 5 Hz, 1H), 5,06 (d, J = 5 Hz, 1H), 5,46 (s, 2H), 6,83 (d, J = 8 Hz, 1H), 7,36 (m, 3H), 9,44 (s, breit, 1H), 9,88 (s, breit, 1H)

IR: (KBr) 1778 $cm^{-1}$
MS: 545 ($M+H^{\oplus}$)

1,55 g (6R,7R)-7-Amino-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure werden in 12 ml Wasser und 12 ml Acetonitril suspendiert. Der pH-Wert wird durch Zugabe von 0,5 ml Triäthylamin auf 7,0 gestellt. Bei 0 °C werden anschliessend 1,80 g 2-Amino-4-thiazolthioglyoxylsäure-S-(2-benzothiazolyl)ester zugegeben und das Reaktionsgemisch 2 Stunden bei 0 °C gerührt. Das Reaktionsgemisch wird zentrifügiert und die klare überstehende Lösung zwischen Wasser und Aethylacetat verteilt. Die wässrige Phase wird abgetrennt, eingeengt und auf pH 2,0 angesäuert. Der entstandene gelbe Niederschlag wird abfiltriert und am Hochvakuum getrocknet. Man erhält 1,72 g (6R,7R)-7-(2-Amino-4-thiazolglyoxylamido)-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo-[4.2.0]oct-2-en-2-carbonsäure als gelbes Pulver.
$^1$H-NMR (DMSO-$d_6$, 250 MHz): Signale bei δ(ppm) 2,58 (s, 3H), 3,60 (d, J=18 Hz, 1H), 3,79 (d, J=18 Hz, 1H), 4,37 (d, J=12,5 Hz, 1H), 4,48 (d, J=12,5 Hz, 1H), 5,71 (d, J=5 Hz, 1H), 5,75 (dd, J=5 Hz, J=8 Hz, 1H), 6,83 (d, J=8 Hz, 1H), 7,35 (m, 5H), 7,82 (s, 1H), 9,43 (s, 1H), 9,81 (d, J=8 Hz, 1H), 9,87 (s, 1H)
IR: (KBr) 1776 $cm^{-1}$
MS: 699 ($M+H^{\oplus}$)

Beispiel 7

Herstellung von (6R,7R)-7-[(Z)-2-(2-Amino-4-thiazolyl)-2-[[[(3,4-dihydroxyphenyl)sulfonyl]methoxy]imino]-acetamido]-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1)

0,35 g (6R,7R)-7-(2-Amino-4-thiazolglyoxylamido)-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure und 0,13 g 4-[[(Aminooxy)methyl]sulfonyl]pyrocatechol-hydrochlorid werden in 4 ml Dimethylacetamid 72 Stunden bei Raumtemperatur gerührt. Das Dimethylacetamid wird am Hochvakuum abgedampft und der Rückstand in Wasser digeriert. Der Feststoff wird abfiltriert, in Wasser resuspendiert, durch Zugabe von 1N wässriger Natronlauge bei pH 7,0 in Lösung gebracht und an RP12-Kieselgel mit Wasser/Acetonitril chromatographiert. Die einheitlichen Fraktionen werden eingeengt und lyophilisiert. Man erhält 0,16 g (6R,7R)-7-[(Z)-2-(2-Amino-4-thiazolyl)-2-[[[(3,4-dihydroxyphenyl)sulfonyl]methoxy]imino]acetamido]-3-[[[2-[[(3,4-dihydroxybenzoyl)oxy]methyl]-5-methyl-s-triazolo-[1,5-a]pyrimidin-7-yl]thio]methyl]-8-oxo-5-thia-1-azabicyclo[4.2.0]oct-2-en-2-carbonsäure-Natriumsalz (1:1) als leicht beiges Pulver.
$^1$H-NMR (DMSO-$d_6$, 250 MHz): Signale bei δ(ppm) 2,60 (s, 3H), 3,05 (d, J=18 Hz, 1H), 3,48 (d, J=18 Hz, 1H), 4,28 (d, J=12,5 Hz, 1H), 4,53 (d, J=12,5 Hz, 1H), 4,91 (d, J=5 Hz, 1H), 5,16 (m, 2H), 5,41 (dd, J=5 Hz, J=8 Hz, 1H), 5,44 (s, 2H), 6,80 (m, 3H), 7,30 (m, 6H), 7,65 (s, 1H), 9,38 (d, J=8 Hz, 1H)
IR: (KBr) 1766 $cm^{-1}$
MS: 900 ($M+H^{\oplus}$)

Beispiel 8

Herstellung von (6R,7R)-7-[(Z)-2-(2-Amino-4-thiazolyl)-2-[[(3,4-dihydroxybenzyl)oxy]imino]acetamido]-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1)

0,42 g (6R,7R)-7-(2-Amino-4-thiazolglyoxylamido)-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure und 0,37 g O-[3,4-[(Diphenylmethylen)dioxy]benzyl]hydroxylamin werden 2 Tage in 5,0 ml Dimethylacetamid bei Raumtemperatur gerührt. Das Dimethylacetamid wird am Hochvakuum abgedampft und der Rückstand in Wasser digeriert. Der entstandene Feststoff wird filtriert, in Wasser resuspendiert, durch Zugabe von 1N wässriger Natronlauge bei pH 7,0 in Lösung gebracht und an RP12-Kieselgel mit Wasser/Acetonitril chromatographiert. Die einheitlichen Fraktionen werden eingeengt und das Produkt durch Ansäuern auf pH 2,0 ausgefällt. Dieser Feststoff wird in 10 ml Trifluoressigsäure und 5 Tropfen Wasser bei Raumtemperatur 2 Stunden gerührt. Die Trifluoressigsäure wird am Wasserstrahlvakuum abgedampft und der Rückstand mit Aethylacetat digeriert. Der Feststoff wird abfiltriert, in Wasser durch Zugabe von 1N wässriger Natronlauge bei pH 7,0 in Lösung gebracht und an RP12-Kieselgel mit Wasser/Acetonitril chromatographiert. Die einheitlichen Fraktionen werden eingeengt und lyophilisiert. Man erhält 0,07 g (6R,7R)-7-[(Z)-2-(2-Amino-4-

thiazolyl)-2-[[(3,4-dihydroxybenzyl)oxy]imino]acetamido]-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1) als beiges Pulver.
$^1$H-NMR (DMSO-$d_6$, 250 MHz): Signale bei δ(ppm) 2,55 (s, 3H), 3,30 (d, J = 18 Hz, 1H), 3,56 (d, J = 18 Hz, 1H), 4,36 (d, J = 12,5 Hz, 1H), 4,65 (d, J = 12,5 Hz, 1H), 4,90 (s, 2H), 4,98 (d, J = 5 Hz, 1H), 5,44 (s, 2H), 5,54 (dd, J = 5 Hz, J = 8 Hz, 1H), 6,70 (m, 5H), 7,21 (s, 2H), 7,40 (m, 2H), 7,76 (s, 1H), 8,90 (s, breit, 2H), 9,54 (d, J = 8 Hz, 1H), 9,84 (s, breit, 2H)
IR: (KBr) 1764 $cm^{-1}$

Beispiel 9

Herstellung von (6R,7R)-7-[(Z)-2-(2-Amino-4-thiazolyl)-2-[[[(3,4-dihydroxy-2-fluorbenzyl)oxy]imino]-acetamido]-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1)

0,42 g (6R,7R)-7-(2-Amino-4-thiazolglyoxylamido)-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure und 0,32 g O-[(4-Fluor-2,2-diphenyl-1,3-benzodioxol-5-yl)methyl]hydroxylamin-hydrochlorid werden in 5,0 ml Dimethylacetamid 24 Stunden bei Raumtemperatur gerührt. Das Dimethylacetamid wird am Hochvakuum abgedampft, der Rückstand mit Wasser digeriert, der entstandene Niederschlag filtriert, in Wasser resuspendiert, durch Zugabe von 1N wässrigem NaOH bei pH 7,0 in Lösung gebracht und an RP12-Kieselgel mit Wasser/Acetonitril chromatographiert. Die einheitlichen Fraktionen werden eingeengt und das Produkt durch Ansäuern auf pH 2,0 ausgefällt. Der Feststoff wird feucht in Trifluoressigsäure gelöst und 1 Stunde bei Raumtemperatur gerührt. Die Trifluoressigsäure wird am Wasserstrahlvakuum abgedampft, der Rückstand bei pH 7 mit 1N wässriger Natronlauge eingestellt und zwischen Wasser und Aethylacetat verteilt. Die wässrige Phase wird an Kieselgel mit Wasser/Acetonitril chromatographiert. Einheitliche Fraktionen werden eingeengt und lyophilisiert. Man erhält 0,17 g (6R,7R)-7-[(Z)-2-(2-Amino-4-thiazolyl)-2-[[[(3,4-dihydroxy-2-fluorbenzyl)oxy]imino]acetamido]-3-[[[2-[[(3,4-dihydroxybenzoyl)oxy]methyl]-5-methyl-s-triazolo[1,5-a]-pyrimidin7-yl]thio]methyl]-8-oxo-5-thia-1-azabicyclo[4.2.0]oct-2-en-2-carbonsäure-Natriumsalz (1:1) als weisses Pulver.
$^1$H-NMR (DMSO-$d_6$, 250 MHz): Signale bei δ(ppm) 2,55 (s, 3H), 3,31 (d, J = 18 Hz, 1H), 3,53 (d, J = 18 Hz, 1H), 4,36 (d, J = 12,5 Hz, 1H), 4,64 (d, J = 12,5 Hz, 1H), 4,97 (d, J = 5 Hz, 1H), 5,01 (s, 2H), 5,46 (s, 2H), 5,54 (d,d, J = 5 Hz, J = 8 Hz, 1H), 6,70 (m, 4H), 7,22 (s, breit, 2H), 7,40 (m, 2H), 7,74 (s, 1H), 9,54 (d, J = 8 Hz, 1H)
IR: (KBr) 1766 $cm^{-1}$
MS: 854,3 ($M + H^{\oplus}$)

Beispiel 10

Herstellung von (6R,7R)-7-[(Z)-2-(2-Amino-4-thiazolyl)-2-[[[(3,4-dihydroxyphenyl)sulfonyl]methoxy]imino]-acetamido]-3-[[[2-[[(3,4-dihydroxyphenyl)sulfonyl]methyl]-5-methyl-s-triazolo[1,5-a]pyrimidin-7-yl]thio]-methyl]-8-oxo-5-thia-1-azabicyclo[4.2.0]oct-2-en-2-carbonsäure-Natriumsalz (1:1)

0,30 g (6R,7R)-7-(2-Amino-4-thiazolglyoxylamido)-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure und 0,190 g 4-[[(Aminooxy)methyl]sulfonyl]pyrocatechol-hydrochlorid werden 2 Tage in 5 ml Dimethylacetamid bei Raumtemperatur gerührt. Das Dimethylacetamid wird am Hochvakuum abgedampft und der Rückstand in Wasser digeriert. Der entstandene Niederschlag wird abfiltriert, in Wasser suspendiert, bei pH 7,0 durch Zugabe von 1N wässriger Natronlauge in Lösung gebracht und mit Wasser/Acetonitril an RP12-Kieselgel chromatographiert. Nach Lyophilisation der Produktfraktionen erhält man 0,10 g (6R,7R)-7-[(Z)-2-(2-Amino-4-thiazolyl)-2-[[[(3,4-dihydroxyphenyl)sulfonyl]methoxy]imino]acetamido]-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1) als weisses Pulver.
$^1$H-NMR (DMSO-$d_6$, 250 MHz): Signale bei δ(ppm) 2,54 (s, 3H), 3,12 (d, J = 18 Hz, 1H), 3,40 (d, J = 18 Hz, 1H), 4,26 (d, J = 12,5 Hz, 1H), 4,70 (s, 2H), 4,83 (d, J = 5 Hz, 1H), 5,04 (d, J = 12 Hz, 1H), 5,15 (d, J = 12 Hz, 1H), 5,47 (d, breit, J = 5 Hz, 1H), 6,64 (m, 1H), 6,79 (m, 2H), 6,95 (m, 1H), 7,10 (m, 2H), 7,30 (s, breit, 2H), 7,67 (s, 1H)
IR: (KBr) 1765 $cm^{-1}$

MS: 942 (M + Na$^{\oplus}$)

Die als Ausgangsmaterial eingesetzte (6R,7R)-7-(2-Amino-4-thiazolglyoxylamido)-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo-[4.2.0]oct-2-en-2-carbonsäure kann wie folgt hergestellt werden:

5,00 g 2-(Chlormethyl)-5-methyl-s-triazolo[1,5-a]pyrimidin-7-ol und 4,20 g Natriumjodid werden in 75 ml Dimethylformamid 2 Stunden bei Raumtemperatur gerührt. Es werden 9,60 g 2,2-Diphenyl-1,3-benzodioxol-5-sulfinsäure-lithiumsalz (1:1) zugegeben. Nach 2-stündigem Rühren bei Raumtemperatur entsteht eine klare Lösung. Es wird noch 16 Stunden bei Raumtemperatur gerührt. Das Lösungsmittel wird unter Hochvakuum abgedampft. Der Rückstand wird zwischen Wasser und Aethylacetat verteilt. Die organische Phase wird mit Wasser (dreimal) und mit gesättigter wässriger Natriumchloridlösung gewaschen, mit Magnesiumsulfat getrocknet und eingeengt. Der Rückstand wird aus Tetrahydrofuran kristallisiert. Man erhält 5,70 g 2-[[[3,4-[(Diphenylmethylen)dioxy]phenyl]sulfonyl]methyl]-5-methyl-s-triazolo[1,5-a]pyrimidin-7-ol als weisses Pulver. Smp. 259-263 °C.

| Elementaranalyse: | | | | |
|---|---|---|---|---|
| berechnet: | C 62,39 | H 4,03 | N 11,19 | S 6,41 % |
| gefunden: | C 62,64 | H 4,08 | N 10,92 | S 6,22 % |

0,25 g 2-[[[3,4-[(Diphenylmethylen)dioxy]phenyl]sulfonyl]methyl]-5-methyl-s-triazolo[1,5-a]pyrimidin-7-ol werden in 2,0 ml Phosphoroxychlorid mit 0,040 ml Pyridin versetzt und anschliessend 30 Minuten auf 80 °C erhitzt. Das Reaktionsgemisch wird unter Wasserstrahlvakuum eingeengt und der Rückstand zwischen halbgesättigter, eiskalter wässriger Natriumchloridlösung und Aethylacetat verteilt. Die organische Phase wird mit eiskalter, wässriger Natriumchloridlösung gewaschen, mit Magnesiumsulfat getrocknet und eingeengt. Der Rückstand wird in 30 ml Aethanol mit 0,04g Thioharnstoff während 15 Minuten am Rückfluss erhitzt. Beim Abkühlen kristallisiert die Substanz als gelbe Kristalle. Man erhält 0,200 g 2-[[[3,4-[(Diphenylmethylen)dioxy]phenyl]sulfonyl]methyl]-5-methyl-s-triazolo[1,5-a]pyrimidin-7-thiol als gelbes Pulver. Smp. 272-275 °C (Zers.).

| Elementaranalyse: | | | | |
|---|---|---|---|---|
| berechnet: | C 60,45 | H 3,90 | N 10,85 | S 12,41 % |
| gefunden: | C 60,29 | H 3,81 | N 10,95 | S 12,35 % |

1,00 g 2-[[[3,4-[(Diphenylmethylen)dioxy]phenyl]sulfonyl]methyl]-5-methyl-s-triazolo[1,5-a]pyrimidin-7-thiol und 0,54 g 7-Aminocephalosporansäure werden in 10 ml Acetonitril aufgeschlämmt und mit 5,0 ml einer 20%igen Lösung von Bortrifluorid in Acetonitril versetzt. Nach 2-stündigem Rühren bei Raumtemperatur werden 20 ml Eiswasser zugegeben. Das Reaktionsgemisch wird auf 15 ml eingeengt und die entstandene Suspension im Eisbad 1 Stunde gerührt. Der entstandene weisse Niederschlag wird abfiltriert und unter Hochvakuum über Nacht getrocknet. Dieser Feststoff wird in 10 ml Trifluoressigsäure und 0,5 ml Wasser gelöst und 1 Stunde bei Raumtemperatur gerührt. Das Reaktionsgemisch wird zur Trockene eingeengt. Der Rückstand wird mit Aethylacetat digeriert. Man erhält 0,60 g (6R,7R)-7-Ammo-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure-Trifluoracetat (1:1) als leicht beiges Pulver.

$^{1}$H-NMR (DMSO-$d_6$, 250 MHz): Signale bei δ(ppm) 2,60 (s, 3H), 3,64 (d, J = 17,5 Hz, 1H), 3,83 (d, J = 17,5 Hz, 1H), 4,38 (d, J = 12,5 Hz, 1H), 4,48 (d, J = 12,5 Hz, 1H), 4,82 (s, 2H), 5,06 (d, J = 5 Hz, 1H), 5,18 (d, J = 5 Hz, 1H), 6,86 (d, J = 8 Hz, 1H), 7,11 (m, 3H), 7,35 (s, 1H), 9,74 (s, breit, 1H), 10,14 (s, breit, 1H)

IR (KBr): 1785 $cm^{-1}$

MS: 565 (M + H$^{\oplus}$

2,00 g (6R,7R)-7-Amino-3-[[[2-[[(3,4-dihydroxyphenyl)sulfonyl]methyl]-5-methyl-s-triazolo[1,5-a]-pyrimidin-7-yl]thio]methyl]-8-oxo-5-thia-1-azabicyclo[4.2.0]oct-2-en-2-carbonsäure-Trifluoracetat (1:1) werden in 10 ml Wasser und 10 ml Acetonitril suspendiert und durch Zugabe von Triäthylamin bei pH 7,0 gelöst. Das Reaktionsgemisch wird auf 0 °C gekühlt und mit 1,90 g 2-Amino-4-thiazolthioglyoxylsäure-S-(2-benzothiazolyl)ester 4 Stunden bei 0 °C gerührt. Das Reaktionsgemisch wird zentrifugiert und der klare Ueberstand zwischen Wasser und Aethylacetat verteilt. Die wässrige Phase wird leicht eingeengt und das Produkt durch Ansäuern auf pH 2,0 gefällt. Nach Filtration und Trocknen am Hochvakuum erhält man 2,46 g gelbes Pulver, welches durch Chromatographie an Kieselgel mit Wasser/Acetonitril gereinigt wird Man erhält

1,00 g (6R,7R)-7-(2-Amino-4-thiazolglyoxylamido)-3-[[[2-[[(3,4-dihydroxyphenyl)sulfonyl]methyl]-5-methyl-s-triazolo-[1,5-a]pyrimidin-7-yl]thio]methyl]-8-oxo-5-thia-1-azabicyclo[4.2.0]oct-2-en-2-carbonsäure als gelbes Pulver.

$^1$H-NMR (DMSO-$d_6$, 250 MHz): Signale bei δ(ppm) 2,58 (s, 3H), 3,60 (d, J = 18 Hz, 1H), 3,79 (d, J = 18 Hz, 1H), 4,36 (d, J = 12,5 Hz, 1H), 4,47 (d, J = 12,5 Hz, 1H), 4,81 (s, 2H), 5,21 (d, J = 5 Hz, 1H), 5,77 (d,d J = 5 Hz, J = 8 Hz, 1H), 6,67 (d, J = 9 Hz, 1H), 7,09 (m, 2H), 7,61 (s, 1H), 7,41 (s, breit, 2H), 7,63 (s, 1H), 9,70 (s, breit, 1H), 9,81 (d, J = 8 Hz, 1H), 10,10 (s, breit, 1H), 14,00 (s, sehr breit, 1H)

IR (KBr): 1774 $cm^{-1}$

MS: 719 ($M + H^⊕$)

## Beispiel 11

Herstellung von (6R,7R)-7-[(Z)-2-(2-Amino-4-thiazolyl)-2-[[(3,4-dihydroxybenzyl)oxy]imino]acetamido]-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1)

0,400 g (6R,7R)-7-(2-Amino-4-thiazolglyoxylamido)-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure und 0,38 g O-[3,4-[(Diphenylmethylen)dioxy]benzyl]hydroxylamin werden in 4 ml Dimethylacetamid 16 Stunden bei Raumtemperatur gerührt. Das Dimethylacetamid wird am Hochvakuum abgedampft, der Rückstand in Wasser digeriert, der entstandene Niederschlag abfiltriert, in Wasser suspendiert, durch Zugabe von 1N wässriger Natronlauge bei pH 7,0 in Lösung gebracht und an RP12-Kieselgel mit Wasser/Acetonitril chromatographiert. Aus den Produktfraktionen wird nach Einengen auf etwa 10 ml durch Ansäuern auf pH 2 ein weisser Niederschlag gefällt, welcher über Nacht am Hochvakuum getrocknet wird. Man erhält 0,330 g weisses Pulver, welches in 3 ml Trifluoressigsäure und 3 Tropfen Wasser gelöst und 1 Stunde bei Raumtemperatur gerührt wird. Die Trifluoressigsäure wird am Wasserstrahlvakuum abgedampft. Der Rückstand wird in Aethylacetat digeriert, filtriert, in Wasser suspendiert, durch Zugabe von 1N wässriger Natronlauge bei pH 7,0 in Lösung gebracht und mit Wasser/Acetonitril an RP12-Kieselgel chromatographiert. Die Produktfraktionen werden lyophilisiert. Man erhält 0,10 g (6R,7R)-7-[(Z)-2-(2-Amino-4-thiazolyl)-2-[[(3,4-dihydroxybenzyl)oxy]imino]acetamido]-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1) als weisses Pulver.

$^1$H-NMR (DMSO-$d_6$, 250 MHz): Signale bei δ(ppm) 2,56 (s, 3H), 3,35 (d, J = 18 Hz, 1H), 3,57 (d, J = 18 Hz, 1H), 4,34 (d, J = 12,5 Hz, 1H), 4,60 (d, J = 12,5 Hz, 1H), 4,72 (s, 2H), 4,92 (s, 2H), 5,02 (d, J = 5 Hz, 1H), 5,58 (d, breit, J = 5 Hz, 1H), 6,7 (m, ~6H), 6,98 (m, 2H), 7,23 (s, breit, 2H), 7,63 (s, 1H)

## Beispiel 12

Herstellung von (6R,7R)-7-[(Z)-2-(2-Amino-4-thiazolyl)-2-[[(2-fluor-3,4-dihydroxybenzyl)oxy]imino]acetamido]-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1)

0,50 g (6R,7R)-7-(2-Amino-4-thiazolglyoxylamido)-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure werden in 5,0 ml Dimethylacetamid gelöst, mit 0,40 g O-[(4-Fluor-2,2-diphenyl-1,3-benzodioxol-5-yl)methyl]-hydroxylamin-hydrochlorid versetzt und 16 Stunden bei Raumtemperatur gerührt. Das Dimethylacetamid wird am Hochvakuum abgedampft und der Rückstand mit Wasser digeriert und filtriert. Der resultierende Feststoff wird bei pH 7,0 in Wasser gelöst und an RP12-Kieselgel mit Wasser/Acetonitril chromatographiert. Die einheitlichen Fraktionen werden vereinigt und eingeengt und das Produkt durch Ansäuern auf pH 2,0 gefällt. Nach Trocknen am Hochvakuum erhält man 0,350 g eines weissen Pulvers. Dieses wird in 5 ml Trifluoressigsäure und 5 Tropfen Wasser gelöst und 1 Stunde bei Raumtemperatur gerührt. Die Trifluoressigsäure wird am Wasserstrahlvakuum abgezogen und der Rückstand bei pH 7,0 zwischen Wasser und Aethylacetat verteilt. Die wässrige Phase wird abgetrennt und leicht eingeengt und der Rückstand durch Ansäuern auf pH 2,0 gefällt. Das erhaltene, weisse Pulver wird in Wasser suspendiert und durch Zugabe von 1N wässriger Natronlauge bei pH 7,0 in Lösung gebracht. Nach Lyophilisation dieser Lösung erhält man 180 mg (6R,7R)-7-[(Z)-2-(2-Amino-4-thiazolyl)-2[[(2-fluor-3,4-dihydroxybenzyl)oxy]imino]acetemido]-3-[-[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1) als leicht beiges Pulver.

$^1$H-NMR (DMSO-$d_6$, 250 MHz): Signale bei δ(ppm) 2,58 (s, 3H), 3,48 (d, J = 18 Hz, 1H), 4,33 (d, J = 12,5 Hz, 1H), 4,46 (d, J = 12,5 Hz, 1H), 4,60 (s, 2H), 5,03 (s, 2H), 5,17 (d, J = 5 Hz, 1H), 5,79 (dd, J = 5 Hz, J = 8 Hz, 1H), 7 (m, 7H), 9,50 (d, J = 8 Hz, 1H)
IR (KBr): 1765 $cm^{-1}$
MS: 873,7 ($M + H^{⊕}$)

Beispiel 13

Herstellung von (6R,7R)-7-[(Z)-2-(2-Amino-4-thiazolyl)-2-[[(1,4-dihydro-5-hydroxy-4-oxo-2-pyrimidinyl)-methoxy]imino]acetamido]-3-[[[5-(3,4-dihydroxyphenyl)pyrazolo[1,5-a]pyrimidin-7-yl]thio]methyl]-8-oxo-5-thia-1-azabicyclo[4.2.0]oct-2-en-2-carbonsäure

125 mg (0,2 mMol) (6R,7R)-7-(2-Amino-4-thiazolglyoxylamido)-3-[[[5-(3,4-dihydroxyphenyl)pyrazolo[1,5-a]pyrimidin-7-yl]thio]methyl]-8-oxo-5-thia-1-azabicyclo[4.2.0]oct-2-en-2-carbonsäure, 41 mg (0,26 mMol) 2-(Aminooxy)methyl-5-hydroxy-4(1H)-pyrimidinon und 50 mg (0,26 mMol) p-Toluolsulfonsäurehydrat werden in 3 ml absolutem Dimethylacetamid gelöst. Nach 14-stündigem Rühren bei Zimmertemperatur wird im Hochvakuum bei Zimmertemperatur eingeengt und der Rückstand mit Wasser versetzt. Das Produkt wird abfiltriert und nacheinander mit Wasser, Aethanol und Diäthyläther gewaschen. Man erhält 160 mg (6R,7R)-7-[(Z)-2-(2-Amino-4-thiazolyl)-2-[[(1,4-dihydro-5-hydroxy-4-oxo-2-pyrimidinyl)methoxy]imino]acetamido]-3-[[-[5-(3,4-dihydroxyphenyl)pyrazolo[1,5-a]pyrimidin-7-yl]thio]methyl]-8-oxo-5-thia-1-azabicyclo[4.2.0]oct-2-en-2-carbonsäure als gelbes Pulver.
$^1$H-NMR (DMSO-$d_6$, 250 MHz): Signale bei δ (ppm) 3,60 (d, J = 18 Hz, 1H), 3,83 (d, J = 18 Hz, 1H), 4,48 (d, J = 12,5 Hz, 1H), 4,55 (d, J = 12,5 Hz, 1H), 4,91 (s, 2H), 5,22 (d, J = 5 Hz, 1H), 5,84 (dd, J = 5 Hz und J = 8 Hz, 1H), 6,65 (d, J = 2 Hz, 1H), 6,84 (s, 1H), 6,88 (d, J = 8 Hz, 1H), 7,28 (s, 2H), 7,40 (s, 1H), 7,42 (s, 1H), 7,54 (dd, J = 8 Hz und J = 2 Hz, 1H), 7,74 (d, J = 2 Hz, 1H), 8,19 (d, J = 2 Hz, 1H), 9,23 (s, 1H), 9,56 (s, 1H), 9,91 (d, J = 8 Hz, 1H).

Die als Ausgangsmaterial eingesetzte (6R,7R)-7-(2-Amino-4-thiazolglyoxylamido)-3-[[[5-(3,4-dihydroxyphenyl)pyrazolo[1,5-a]pyrimidin-7-yl]thio]methyl]-8-oxo-5-thia-1-azabicyclo[4.2.0]oct-2-en-2-carbonsäure kann wie folgt hergestellt werden:

33,2 g 3-Aminopyrazol und 110 g 3,4-Dimethoxybenzoyl-essigsäureäthylester werden in 500 mi Eisessig 6 Stunden bei 80 °C gerührt. Nach Abkühlen nutscht man das auskristallisierte Produkt ab, wäscht mit Diäthyläther und trocknet. Man erhält 45 g 5-(3,4-Dimethoxyphenyl)pyrazolo[1,5-a]pyrimidin-7-ol vom Smp. 268-270 °C.

15,3 g 5-(3,4-Dimethoxyphenyl)pyrazolo[1,5-a]pyrimidin-7-ol werden in 300 ml 47% Bromwasserstoff 5 Stunden unter Rückfluss gekocht. Danach wird die Lösung abgekühlt, im Vakuum eingeengt und mit 500 ml Wasser versetzt. Das ausgefällte 4-(7-Hydroxypyrazolo[1,5-a]pyrimidin-5-yl]pyrocatechol wird abgenutscht, mit Wasser gewaschen und getrocknet. Man erhält 13,5 g beige Kristalle, Smp. >270 °C.

12,16 g 4-(7-Hydroxypyrazolo[1,5-a]pyrimidin-5-yl]pyrocatechol werden in 100 ml Phosphoroxychlorid suspendiert. Nach Zutropfen von 19 ml N,N-Dimethylanilin rührt man 1 Stunde bei 80 °C, kühlt ab und dampft unter Wasserstrahlvakuum ein. Zum Rückstand gibt man 250 g Eis-Wasser, lässt die Mischung auf 25 °C erwärmen und extrahiert sodann mit 1000 ml Aethylacetat und 500 ml Aceton. Die organische Phase wird mit Wasser gewaschen und eingedampft. Der Rückstand wird über Kieselgel mittels Aceton/Aethylacetat 1:1 chromatographiert. Die das Produkt enthaltenden Fraktionen werden im Vakuum auf 50 ml eingeengt, wobei das Produkt auskristallisiert. Man erhält 5,35 g beige Kristalle des 4-(7-Chlorpyrazolo-[1,5-a]pyrimidin-5-yl)pyrocatechols, Smp. >270 °C. Dieses Produkt wird zu einer Lösung von 7,5 g $NaHS \cdot H_2O$ in 100 ml Wasser und 100 ml Aethanol gegeben. Man erwärmt auf 55 °C und dampft nach 1/2 Stunde ein. Zum Rückstand gibt man 100 ml Wasser, stellt mittels ca. 30 ml 3N wässriger Salzsäure auf pH 1 und nutscht das Reaktionsprodukt ab, wäscht mit Wasser und trocknet. Man erhält 4,85 g gelbes, kristallines 4-(7-Mercaptopyrazolo[1,5-a]pyrimidin-5-yl)pyrocatechol, Smp. >280 °C.

272 mg 7-Aminocephalosporansäure und 272 mg des obigen 4-(7-Mercaptopyrazolo[1,5-a]pyrimidin-5-yl)pyrocatechols werden in einer Mischung von 2 ml Sulfolan und 2 ml Dichlormethan suspendiert. Man gibt 0,75 mi Bortrifluoridätherat zu und rührt während 5 Stunden bei 25 °C. Danach versetzt man mit 20 ml Wasser, trennt die wässrige Phase ab, stellt deren pH mittels konzentrierter wässriger Ammoniaklösung auf 3,5 und nutscht das ausgefällte Produkt ab, wäscht mit Wasser, Methanol und zuletzt mit Diäthyläther und trocknet bei 0,1 mmHg und 40 °C. Man erhält 350 mg (6R,7R)-7-Amino-3-[[[5-(3,4-dihydroxyphenyl)-pyrazolo[1,5-a]pyrimidin-7-yl]thio]methyl]-8-oxo-5-thia-1-azabicyclo[4.2.0]oct-2-en-2-carbonsäure.

| Elementaranalyse: | | | | |
|---|---|---|---|---|
| Gefunden:<br>Berechnet: | 51,10%<br>50,95% | C, 3,74% H,<br>C, 3,63% H, | 14,74% N,<br>14,85% N, | 13.64% S;<br>13,60% S. |

$^1$H-NMR (DMSO-$d_6$, 250 MHz): Signale bei δ 3,60 (d, J = 18 Hz, 1H), 3,77 (d, J = 18 Hz, 1H), 4,48 (s, breit, 2H), 4,82 (d, J = 6 Hz, 1H), 5,02 (d, J = 6 Hz, 1H), 6,65 (d, J ca. 1 Hz, 1H), 6,87 (d, J = 9 Hz, 1H), 7,41 (s, 1H), 7,55 (d,d, J = ca. 1 und 9 Hz, 1H), 7,74 (d, J = ca. 1 Hz, 1H), 8,18 (d, J ca. 1 Hz, 1H), 9,25 (s, breit, 1H), 9,57 (s, breit, 1H).

1,41 g (6R,7R)-7-Amino-3-[[[5-(3,4-dihydroxyphenyl)pyrazolo[1,5-a]-pyrimidin-7-yl]thio]methyl]-8-oxo-5-thia-1-azabicyclo[4.2.0]oct-2-en-2-carbonsäure werden in 30 ml Dimethylacetamid gelöst und mit 1,45 g 2-Amino-4-thiazolthioglyoxylsäure-5-(2-benzothiazolyl)ester versetzt. Man rührt 1/2 Stunde bei Zimmertemperatur und engt dann im Vakuum bei 20 °C ein. Den Rückstand löst man in 25 ml Dimethylformamid und 25 ml Aceton auf, filtriert und gibt 2 ml einer 2m Lösung des Natrium-2-äthylcaproats in Aceton zu und danach 50 ml Diäthyläther. Das ausgefällte Material wird genutscht und über RP12-Kieselgel mit Wasser/Acetonitril chromatographiert. Die Produktfraktionen werden eingeengt und mit 1N wässriger Salzsäure auf pH 1 gestellt. Das ausgefallene Produkt wird abgenutscht, mit Wasser gewaschen und bei 0,1 mmHg/40 °C getrocknet. Man erhält 1,15 g (6R,7R)-7-(2-Amino-4-thiazolglyoxylamido)-3-[[[5-(3,4-dihydroxyphenyl)-pyrazolo[1,5-a]pyrimidin-7-yl]thio]methyl]-8-oxo-5-thia-1-azabicyclo[4.2.0]oct-2-en-2-carbonsäure.

| Elementaranalyse: | | | | |
|---|---|---|---|---|
| Gefunden:<br>Berechnet: | 48,32% C,<br>47,99% C, | 2,87% H,<br>3,06% H, | 15,93% N,<br>15,67% N, | 15,51% S;<br>15,37% S |

$^1$H-NMR (DMSO-$d_6$, 250 MHz): Signale bei δ 3,68 (d, J = 18 Hz, 1H), 3,82 (d, J = 18 Hz, 1H), 4,56 (s, breit, 2H), 5,25 (d, J = 5,5 Hz, 1H), 5,77 (dd, J = 5,5 und 8,5 Hz, 1H), 6,65 (d, J = ca. 1 Hz, 1H), 6,88 (d, J = 9 Hz, 1H), 7,42 (m, 3H), 7,55 (dd, J = ca. 1 und 9 Hz, 1H), 7,73 (d, J = ca. 1 Hz, 1H), 7,82 (s, 1H), 8,19 (d, J = ca. 1 Hz, 1H), 9,22 (breit, 1H), 9,57 (s, 1H), 9,83 (d, J = 8,5 Hz, 1H).

Beispiel 14

Herstellung von (6R,7R)-7-[(Z)-2-(2-Amino-4-thiazolyl)-2-[[(5-hydroxy-4-oxo-4H-pyran-2-yl)methoxy]imino]-acetamido]-3-[[[5-(3,4-dihydroxyphenyl)pyrazolo[1,5-a]pyrimidin-7-yl]thio]methyl]-8-oxo-5-thia-1-azabicyclo-[4.2.0]oct-2-en-2-carbonsäure

59 mg (0,095 mMol) (6R,7R)-7-(2-Amino-4-thiazolglyoxylamido)-3-[[[5-(3,4-dihydroxyphenyl)pyrazolo-[1,5-a]pyrimidin-7-yl]thio]methyl]-8-oxo-5-thia-1-azabicyclo[4.2.0]oct-2-en-2-carbonsäure und 41 mg (0,121 mMol) 2-[(Aminooxy)methyl]-5-hydroxy-4H-pyran-4-on-p-toluolsulfonat (1:1) werden in 2 ml absolutem Dimethylacetamid gelöst. Nach 24-stündigem Rühren bei Raumtemperatur wird das Lösungsmittel abgezogen und der Rückstand mit 4 ml Wasser versetzt. Das ausgefallene Produkt wird abfiltriert und mit Wasser gewaschen. Nach Trocknen im Hochvakuum bei Raumtemperatur erhält man 67 mg (6R,7R)-7-[(Z)-2-(2-Amino-4-thiazolyl)-2-[[(5-hydroxy-4-oxo-4H-pyran-2-yl)methoxy]imino]acetamido]-3-[[[5-(3,4-dihydroxyphenyl)pyrazolo-[1,5-a]pyrimidin-7-yl]thio]methyl]-8-oxo-5-thia-1-azabicyclo[4.2.0]oct-2-en-2-carbonsäure als gelbes Pulver.

$^1$H NMR (DMSO-$d_6$): Signale u.a. bei δ (ppm) 3,63 (d, J = 18Hz, 1H), 3,83 (d, J = 18Hz, 1H), 4,53 (s, 2H), 4,97 (s, 2H), 5,22 (d, J = 5Hz, 1H), 5,84 (dd, J = 5Hz und J = 8Hz, 1H), 6,48 (s, 1H), 6,62 (d, J = 2Hz, 1H), 6,82 (s, 1H), 6,85 (d, J = 8Hz, 1H), 7,40 (s, 1H), 7,54 (dd, J = 8Hz und J = 2Hz, 1H), 7,74 (d, J = 2Hz, 1H), 8,06 (s, 1H), 8,20 (d, J = 2Hz, 1H), 9,85 (d, J = 8Hz, 1H).

Das als Ausgangsmaterial eingesetzte 2-[(Aminooxy)methyl]-5-hydroxy-4H-pyran-4-on-p-toluolsulfonat (1:1) kann wie folgt hergestellt werden:

16,68 g (86 mMol) Azodicarbonsäurediäthylester werden in 1200 ml absoluten Tetrahydrofuran gelöst. Dazu gibt man ein Gemisch bestehend aus 22,6 g (86 mMol) Triphenylphosphin, 14,1 g (86 mMol) N-Hydroxyphthalimid und 20,0 g (86 mMol) 5-(Benzyloxy)-2-(hydroxymethyl)-4H-pyran-4-on (bekannt aus J. Med. Chem. 17, 1, 1974). Nach 20-stündigem Rühren bei Raumtemperatur wird filtriert und das Filtergut mit Aethanol ausgewaschen. Filtrat und Waschlösung werden vereinigt und eingeengt und der Rückstand mit Aethanol kristallisiert. Nach mehrmaligem Umkristallisieren aus Aethanol erhält man 23,5 g N-[[5-(Benzyloxy)-4-oxo-4H-pyran-2-yl]methoxy]phthalimid als weisse Kristalle vom Smp. 172 °C.

1,77 g (4,7 mMol) N-[[5-(Benzyloxy)-4-oxo-4H-pyran-2-yl]methoxy]phthalimid werden in 250 ml Dichlormethan suspendiert und bein-70 °C mit 1,32 g (5,2 mMol) Bortribromid (gelöst in 5 ml Dichlormethan) versetzt. Das Ganze wird 3 Stunden bei -70 °C und 20 Stunden bei Raumtemperatur gerührt. Das Lösungsmittel wird im Vakuum abgezogen und der Rückstand bei 0 °C mit ca. 70 ml Wasser und 10 ml Aethanol versetzt. Nach 2-stündigem Rühren wird das Produkt abfiltriert und nacheinander mit Wasser, Aethanol und Diäthyläther gewaschen. Man erhält 1,3 g N-[(5-Hydroxy-4-oxo-4H-pyran-2-yl)methoxy]-phthalimid als weisses Pulver vom Smp. 200-203 °C (Zers.).

570 mg (2 mMol) N-[(5-Hydroxy-4-oxo-4H-pyran-2-yl)methoxy]phthalimid werden in 5 ml absolutem Dimethylformamid suspendiert und bei -10 °C mit 100 mg (2 mMol) Methylhydrazin versetzt. Nach 1,5-stündigem Rühren bei Raumtemperatur wird das Ganze im Hochvakuum eingeengt und der Rückstand mit etwa 15 ml Aethanol versetzt. Man gibt 2 g p-Toluolsulfonsäurehydrat dazu und fällt das Produkt durch Zugabe von ca. 90 ml Diäthyläther aus. Nach zweimaligem Umkristallisieren aus Aethanol/Diäthyläther erhält man 460 mg 2-[(Aminooxy)methyl]-5-hydroxy-4H-pyran-4-on-p-toluolsulfonat (1:1) als weisse Kristalle vom Smp. 167 °C (Zers.)

Beispiel 15

Herstellung von (6R,7R)-7-[(Z)-2-(2-Amino-4-thiazolyl)-2-[[(2-fluor-(3,4-dihydroxybenzyl)oxy]imino]-acetamido]-3-[[[5-(3,4-dihydroxyphenyl)-2-phenyl-4-pyrimidnyl]thio]methyl]-8-oxo-5-thia-1-azabicyclo[4.2.0]-oct-2-en-2-carbonsäure

66 mg (0,1 mMol) (6R,7R)-7-(2-Amino-4-thiazolglyoxylamido)-3-[[(5-(3,4-dihydroxyphenyl)-2-phenyl-4-pyrimidinyl]thio]methyl]-8-oxo-5-thia-1-azabicyclo[4.2.0]oct-2-en-2-carbonsäure und 47 mg (0,13 mMol) O-[-(4-Fluor-2,2-diphenyl-1,3-benzodioxol-5-yl)methyl]hydroxylamin-hydrochlorid werden in 1 ml absolutem Dimethylacetamid gelöst. Nach 20-stündigem Rühren bei Raumtemperatur wird im Hochvakuum bei Raumtemperatur eingeengt und der Rückstand mit 0,5 mi Aethanol und 3 ml Wasser versetzt. Das ausgefallene Produkt wird abfiltriert und mit Wasser gewaschen. Nach dem Trockenen im Hochvakuum bei Raumtemperatur erhält man 98 mg (6R,7R)-7-[(Z)-2-(2-Amino-4-thiazolyl)-2-[[(4-fluor-2,2-diphenyl-1,3-benzodioxol-5-yl)-methoxy]imino]acetamido]-3-[[5-(3,4-dihydroxyphenyl)-2-phenyl-4-pyrimidinyl]thio]methyl]-8-oxo-5-thia-1-azabicyclo[4.2.0]oct-2-en-2-carbonsäure als beiges Pulver.

$^1$H NMR (DMSO-$d_6$): Signale u.a. bei 3,64 (d, J = 18Hz, 1H), 4,18 (d, J = 12,5Hz, 1H), 4,69 (d, J = 12,5Hz, 1H), 5,06 (s, 2H), 5,09 (d, J = 5Hz, 1H), 5,73 (dd, J = 5Hz und J = 8Hz, 1H), 6,71 (s, 1H), 6,75-7,0(5H), 7,22 (s, 2H), 7,3-7,6 (13H), 8,46 (s, 1H), 8,47 (2H), 9,25 (s, 1H), 9,33 (s, 1H), 9,60 (d, J = 8Hz, 1H).

95 mg (0,097 mMol) (6R,7R)-7-[(Z)-2-(2-Amino-4-thiazolyl)-2-[[(4-fluor-2,2-diphenyl-1,3-benzodioxol-5-yl)methoxy]imino]acetamido]-3-[[5-(3,4-dihydroxyphenyl)-2-phenyl-4-pyrimidinyl]thio]methy]-8-oxo-5-thia-1-azabicyclo[4.2.0]oct-2-en-2-carbonsäure werden in 3 ml Trifluoressigsäure 5,5 Stunden gerührt. Die Lösung wird eingedampft und der Rückstand mit 5 ml Aether kristallisiert. Das Produkt wird abgenutscht, mit Diäthyläther gewaschen und im Hochvakuum bei Raumtemperatur getrocknet. Man erhält 75 mg (6R,7R)-7-[(Z)-2-(2-Amino-4-thiazolyl)-2-[[(2-fluor-(3,4-dihydroxybenzyl)oxy]imino]acetamido]-3-[[[5-(3,4-dihydroxyphenyl)-2-phenyl-4-pyrimidinyl]thio]methyl]-8-oxo-5-thia-1-azabicyclo[4.2.0]oct-2-en-2-carbonsäure als gelbes Pulver.

$^1$H NMR (DMSO-$d_6$): Signale u.a. bei $\delta$ 3,52 (d, J = 18Hz, 1H), 3,78 (d, J = 18Hz, 1H), 4,17 (d, J = 12,5Hz), 4,74 (d, J = 12,5Hz, 1H), 5,01 (s, 2H), 5,14 (d, J = 5Hz, 1H), 5,76 (dd, J = 5Hz und J = 8Hz, 1H), 6,52 (dd, J = 8Hz und J = 2Hz, 1H), 6,66 (d, J = 8Hz, 1H), 6,69 (s, 1H), 6,78 (dd, J = 8Hz, J = 2Hz, 1H), 6,87 (d, J = 8Hz, 1H), 6,91 (d, J = 2Hz, 1H), 7,32 (2H), 7,57 (3H), 8,44 (s, 1H), 8,48 (2H), 9,64 (d, J = 8Hz, 1H).

Die als Ausgangsmaterial verwendete (6R,7R)-7-(2-Amino-4-thiazolglyoxylamido)-3-[[(5-(3,4-dihydroxyphenyl)-2-phenyl-4-pyrimidinyl]thio]methyl]-8-oxo-5-thia-1-azabicyclo[4.2.0]oct-2-en-2-carbonsäure kann wie folgt hergestellt werden:

7,2 g (30 mMol) (3,4-Dimethoxyphenyl)malonaldehydsäuremethylester und 10 g (42 mMol) Benzamidin werden bei 100 °C geschmolzen. Das Reaktionsgemisch wird an 250 g Kieselgel (0,063-0,2 mm) chromatographiert und das Produkt mit Dichlormethan/Methanol (9:1 v/v) eluiert. Das Eluat wird bei 45 °C im Vakuum konzentriert und das kristalline Produkt abgenutscht. Das Ganze wird mit Diäthyläther gewaschen und im Hochvakuum bei Raumtemperatur getrocknet. Man erhält 6,2 g 5-(3,4-Dimethoxyphenyl)-2-phenyl-6(1H)-pyrimidinon als gelbe Kristalle vom Smp. 230 °C.

7,4 g 5-(3,4-Dimethoxyphenyl)-2-phenyl-6(1H)-pyrimidinon werden in 250 ml 48%iger wässriger Bromwasserstoffsäure 24 Stunden bei 100 °C und 24 Stunden bei 140 °C gerührt. Nach dem Abkühlen werden die erhaltenen gelben Kristalle abfiltriert, mit Wasser gewaschen und in 200 ml Wasser suspendiert. Man

gibt Natriumhydrogencarbonat dazu, bis pH 7,5 erreicht ist. Nach 2-stündigem Rühren wird das Produkt abfiltriert, mit Wasser gewaschen und im Vakuum bei Raumtemperatur über Kaliumhydroxid getrocknet. Man erhält 6 g 5-(3,4-Dihydroxyphenyl)-2-phenyl-4(3H)-pyrimidinon-hydrobromid als gelbes Pulver vom Smp. >250 °C.
$^1$H NMR (DMSO-$d_6$): Signale bei δ 6,77 (d, J = 8Hz, 1H), 7,07 (dd, J = 8Hz und J = 2H, 1H), 7,28 (d, J = 2Hz, 1H), 7,5-7,6 (3H), 8,1-8,2 (3H), 9,07 (breit, 2H), 12,86 (breit, 1H).

6,0 g (21,4 mMol) 5-(3,4-Dihydroxyphenyl)-2-phenyl-4(3H)-pyrimidinonhydrobromid werden in 100 ml Pyridin gelöst und bei Raumtemperatur unter Rühren mit 5,2 g (51 mMol) Acetanhydrid versetzt. Nach 1,5 Stunden gibt man 200 ml Diäthyläther dazu und filtriert das ausgefallene Produkt ab. Der Feststoff wird mit Diäthyläther gewaschen und im Hochvakuum bei 35 °C getrocknet. Man erhält 7,1 g 4-(3,4-Dihydro-4-oxo-2-phenyl-5-pyrimidinyl)-o-phenylen-diacetat als weisse Kristalle vom Smp. 240 °C.

6,8 g (18,7 mMol) 4-(3,4-Dihydro-4-oxo-2-phenyl-5-pyrimidinyl)-o-phenylen-diacetat werden in 150 ml Phosphoroxychlorid gelöst. Nach 6-stündigem Rühren bei 100 °C wird bei 50 °C im Vakuum eingeengt und der Rückstand im Hochvakuum getrocknet. Man gibt nun Dichlormethan und Eis/Wasser dazu und trennt nach guter Durchmischung die Dichlormethanphase ab. Nach Trocknen über Natriumsulfat und Abdestillieren des Lösungsmittels kristallisiert man das Produkt aus Aethanol. Dieses wird abfiltriert und mit Aethanol/Diäthyläther gewaschen. Man erhält 6 g 4-(4-Chlor-2-phenyl-5-pyrimidinyl)-o-phenylen-diacetat als weisse Kristalle vom Smp. 152-153 °C.

6,0 g (15,7 mMol) 4-(4-Chlor-2-phenyl-5-pyrimidinyl)-o-phenylendiacetat werden in 200 ml Methanol suspendiert. Unter Rühren werden bei 50 °C 10 g Natriumhydrogensulfidhydrat zugegeben. Nach weiteren 4 Stunden bei 60 °C wird filtriert und das Filtrat eingeengt. Der Rückstand wird in ca. 150 ml Wasser gelöst und die Lösung mit 1N wässriger Salzsäure auf pH 5 gebracht. Das ausgefallene Produkt wird abgenutscht, mit Wasser gewaschen, bei Raumtemperatur getrocknet und aus Aethanol kristallisiert. Man erhält 3,6 g 5-(3,4-Dihydroxyphenyl)-2-phenyl-4(3H)-pyrimidinthion als gelbe Kristalle vom Smp. 220 °C.

162 mg (0,6 mMol) 7-Aminocephalosporansäure und 216 mg (0,66 mMol) 5-(3,4-Dihydroxyphenyl)-2-phenyl-4(3H)-pyrimidinthion werden in 7 ml Dichlormethan/Sulfolan (1:1 v/v) suspendiert. Unter Rühren gibt man 4 ml Bortrifluorid-diäthylätherat dazu. Nach 16 Stunden engt man im Hochvakuum bei Raumtemperatur ein, wäscht den Rückstand mit Diäthyläther und löst ihn in etwa 3 ml Wasser. Man gibt Natriumhydrogencarbonat dazu, bis pH 5 eingestellt ist. Dann werden 1 ml Diäthyläther und 0,5 ml Aethanol zugefügt. Das ausgefallene Produkt wird abgenutscht, nacheinander mit Wasser, Aethanol und Aether gewaschen und im Hochvakuum bei Raumtemperatur getrocknet. Man, erhält 250 mg (6R,7R)-7-Amino-3-[[[5-(3,4-dihydroxyphenyl)-2-phenyl-4-pyrimidinyl]thio]methyl]-8-oxo-5-thia-1-azabicyclo[4.2.0]oct-2-en-2-carbonsäure als beiges Pulver.
$^1$H NMR (DMSO-$d_6$): Signale u.a. bei δ 3,50 (d, J = 18Hz, 1H), 3,77 (d, J = 18Hz, 1H), 4,18 (d, J = 12,5Hz, 1H), 4,72 (d, J = 12,5Hz, 1H), 4,76 (d, J = 5Hz, 1H), 4,98 (d, J = 5Hz, 1H), 6,79 (dd, J = 8Hz und J = 2Hz, 1H), 6,88 (d, J = 8Hz, 1H), 6,92 (d, J = 2Hz, 1H), 7,5-7,6 (3H), 8,42 (s, 1H), 8,45 (2H), 9,25 (s, 1H), 9,32 (s, 1H).

1,02 g (2 mMol) (6R,7R)-7-Amino-3-[[[5-(3,4-dihydroxyphenyl)-2-phenyl-4-pyrimidinyl]thio]methyl]-8-oxo-5-thia-1-azabicyclo[4.2.0]oct-2-en-2-carbonsäure werden in 25 ml Wasser und 25 ml Acetonitril suspendiert. Unter Rühren tropft man bei 0 °C 27 ml 0,1N wässriges Kaliumhydroxid dazu und versetzt die Lösung portionenweise mit 1,0 g (3 mMol) 2-Amino-4-thiazolthioglyoxylsäure-S-(2-benzothiazolyl)ester. Man rührt 1 Stunde bei 0 °C und 2 Stunden bei 15-20 °C. Die Suspension wird nun filtriert und das Filtergut mit Wasser gewaschen. Mutterlauge und Waschlösung werden vereinigt, mit Aethylacetat gewaschen und schliesslich mit verdünnter, wässriger Salzsäure angesäuert (pH ~4). Das ausgefallene Produkt wird abgenutscht und nacheinander mit Wasser und Diäthyläther gewaschen. Man erhält nach Trocknen im Hochvakuum bei Raumtemperatur 0,5 g (6R,7R)-7-(2-Amino-4-thiazolglyoxylamido)-3-[[(5-(3,4-dihydroxyphenyl)-2-phenyl-4-pyrimidinyl]thio]methyl]-8-oxo-5-thia-1-azabicyclo[4.2.0]oct-2-en-2-carbonsäure als gelbes Pulver.
$^1$H NMR (DMSO-$d_6$): Signale u.a. bei δ 3,58 (d, J = 18Hz, 1H), 3,81 (d, J = 18Hz, 1H), 4,20 (d, J = 12,5Hz, 1H), 4,75 (d, J = 12,5Hz, 1H), 5,17 (d, J = 5Hz, 1H), 5,74 (dd, J = 5Hz und J = 8Hz, 1H), 6,78 (dd, J = 8Hz und J = 2Hz, 1H), 6,86 (d, J = 8Hz, 1H), 6,92 (d, J = 2Hz, 1H), 7,40 (s, 2H), 7,5-7,6 (3H), 7,80 (s, 1H), 8,44 (s, 1H), 8,48 (2H), 9,26 (s, 1H), 9,34 (s, 1H), 9,77 (d, J = 8Hz, 1H).

## Beispiel 16

Herstellung von (6R,7R)-7-[(Z)-2-(2-Amino-4-thiazolyl)-2-[[(1,4-dihydro-5-hydroxy-4-oxo-2-pyrimidinyl)-methoxy]imino]acetamido]-3-[[[5-(3,4-dihydroxyphenyl)-2-phenyl-4-pyrimidinyl]thio]methyl]-8-oxo-5-thia-1-azabicyclo[4.2.0]oct-2-en-2-carbonsäure

60 mg (0,09 mMol) (6R,7R)-7-(2-Amino-4-thiazolglyoxylamido)-3-[[(5-(3,4-dihydroxyphenyl)-2-phenyl-4-pyrimidinyl]thio]methyl]-8-oxo-5-thia-1-azabicyclo[4.2.0]oct-2-en-2-carbonsäure, 20,5 mg (0,13 mMol) 2-(Aminooxy)methyl-5-hydroxy-4(1H)-pyrimidinon und 13 mg (0,13 mMol) Methansulfonsäure werden in 0,6 ml absolutem Dimethylacetamid gelöst. Nach 18-stündigem Rühren bei Raumtemperatur wird im Hochvakuum bei Raumtemperatur eingeengt und der Rückstand mit Aethanol kristallisieret. Das Produkt wird mit Diäthyläther gewaschen und im Hochvakuum bei Raumtemperatur getrocknet. Man erhält 26 mg (6R,7R)-7-[(Z)-2-(2-Amino-4-thiazolyl)-2-[[(1,4-dihydro-5-hydroxy-4-oxo-2-pyrimidinyl)methoxy]imino]acetamido]-3-[[[5-(3,4-dihydroxyphenyl)-2-phenyl-4-pyrimidinyl]thio]methyl]-8-oxo-5-thia-1-azabicyclo[4.2.0]oct-2-en-2-carbonsäure als gelbes Pulver. Aus der Mutterlauge kann man nach Einengen weitere 28 mg gelbe Kristalle von derselben Verbindung erhalten.
$^1$H NMR (DMSO-$d_6$): Signale u.a. bei δ 3,53 (d, J = 18Hz, 1H), 3,83 (d, J = 18Hz, 1H), 4,19 (d, J = 12,5Hz, 1H), 4,75 (d, J = 12,5Hz, 1H), 4,88 (2H), 5,18 (d, J = 5Hz, 1H), 5,82 (dd, J = 5Hz und J = 8Hz, 1H), 6,79 (dd, J = 2Hz und J = 8Hz, 1H), 6,83 (s, 1H), 6,87 (d, J = 8Hz, 1H), 6,92 (d, J = 2Hz, 1H), 7,27 (s, 2H), 7,36 (s, 1H), 7,56 (3H), 8,43 (s, 1H), 8,46 (2H), 9,25 (s, 1H), 9,33 (s, 1H), 9,54 (s, 1H), 9,85 (d, J = 8Hz, 1H).

## Beispiel 17

Herstellung von (6R,7R)-7-[(Z)-2-(2-Amino-4-thiazolyl)-2-[[1-(hydroxycarbamoyl)-1-methyläthoxy]imino]-acetamido]-3-[[[5-(3,4-dihydroxyphenyl)-2-phenyl-4-pyrimidinyl]thio]methyl]-8-oxo-5-thia-1-azabicyclo[4.2.0]-oct-2-en-2-carbonsäure

33 mg (0,05 mMol) (6R,7R)-7-(2-Amino-4-thiazolglyoxylamido)-3-[[(5-(3,4-dihydroxyphenyl)-2-phenyl-4-pyrimidinyl]thio]methyl]-8-oxo-5-thia-1-azabicyclo[4.2.0]oct-2-en-2-carbonsäure und 11 mg (0,065mMol) 2-(Aminooxy)-N-hydroxy-2-methylpropionamid-hydrochlorid werden in 0,8 ml absolutem Dimethylacetamid gelöst. Nach 22-stündigem Rühren bei Raumtemperatur wird im Hochvakuum eingeengt und der Rückstand mit Wasser kristallisiert. Das Produkt wird abfiltriert, mit Wasser, Aethanol und Diäthyläther gewaschen und im Hochvakuum bei Raumtemperatur getrocknet. Man erhält 32 mg (6R,7R)-7-[(Z)-2-(2-Amino-4-thiazolyl)-2-[[1-(hydroxycarbamoyl)-1-methyläthoxy]imino]acetamido]-3-[[[5-(3,4-dihydroxyphenyl)-2-phenyl-4-pyrimidinyl]thio]methyl]-8-oxo-5-thia-1-azabicyclo[4.2.0]oct-2-en-2-carbonsäure als beiges Pulver.
$^1$H NMR (DMSO-$d_6$): Signale u.a. bei δ 1,36 (s, 6H), 3,58 (d, J = 18Hz, 1H), 3,83 (d, J = 18Hz, 1H), 4,20 (d, J = 12,5Hz, 1H), 4,76 (d, J = 12,5Hz, 1H), 5,20 (d, J = 5Hz, 1H), 5,88 (dd, J = 5Hz und J = 8Hz, 1H), 6,79(s, 1H), 6,79 (dd, J = 8Hz und J = 2Hz, 1H), 6,87 (d, J = 8Hz, 1H), 6,91 (d, J = 2Hz, 1H), 7,34 (s, 2H), 7,55 (3H), 8,44 (s, 1H), 8,46 (2H), 8,88 (1H), 9,23 (s, 1H), 9,33 (s, 1H), 9,62 (d, J = 8Hz, 1H), 10,05 (s, 1H).

## Beispiel 18

Herstellung von (6R,7R)-7-[(Z)-2-(2-Amino-4-thiazolyl)-2-[[(2,5-dichlor-3,4-dihydroxybenzyl)oxy]imino]-acetamido]-3-[[[5-(3,4-dihydroxyphenyl)-2-phenyl-4-pyrimidinyl]thio]methyl]-8-oxo-5-thia-1-azabicyclo[4.2.0]-oct-2-en-2-carbonsäure

66 mg (0,1 mMol) (6R,7R)-7-(2-Amino-4-thiazolglyoxylamido)-3-[[(5-(3,4-dihydroxyphenyl)-2-phenyl-4-pyrimidinyl]thio]methyl]-8-oxo-5-thia-1-azabicyclo[4.2.0]oct-2-en-2-carbonsäure und 55 mg (0,13 mMol) 5-[-(Aminooxy)methyl]-4,7-dichlor-2,2-diphenyl-1,3-benzodioxol-hydrochlorid werden in 2 ml absolutem Dimethylacetamid gelöst. Nach 24-stündigem Rühren bei Raumtemperatur wird im Hochvakuum bei Raumtemperatur eingeengt und der Rückstand aus Aethanol/Diäthyläther kristallisiert. Man erhält 130 mg (6R,7R)-7-[-(Z)-2-(2-Amino-4-thiazoyl)-2[[(4,7-dichlor-2,2-diphenyl-1,3-benzodioxol-5-yl)methoxy]imino]acetamido]-3-[[[5-(3,4-dihydroxyphenyl)-2-phenyl-4-pyrimidinyl]thio]methyl]-8-oxo-5-thia-1-azabicyclo[4.2.0]oct-2-en-2-carbonsäure als gelbliches Pulver.
$^1$H NMR (DMSO-$d_6$): Signale u.a. bei δ 3,40 (d, J = 18Hz, 1H), 3,67 (d, J = 18Hz, 1H), 4,17 (d, J = 12,5Hz, 1H), 4,73 (d, J = 12,5Hz, 1H), 5,11 (d, J = 5Hz, 1H), 5,12 (s, 2H), 5,76 (dd, J = 5Hz und J = 8Hz, 1H), 6,78 (dd, J = 8Hz und J = 2Hz, 1H), 6,81 (s, 1H), 6,87 (d, J = 8Hz, 1H), 6,92 (d, J = 2Hz, 1H), 7,14 (s, 1H), 7,4-7,6 (15H), 8,45 (s, 1H), 8,47 (2H), 9,72 (d, J = 8Hz, 1H).

102 mg (0,1 mMol) (6R,7R)-7-[(Z)-2-(2-Amino-4-thiazoyl)-2-[[(4,7-dichlor-2,2-diphenyl-1,3-benzodioxol-5-yl)methoxy]imino]acetamido]-3-[[[5-(3,4-dihydroxyphenyl)-2-phenyl-4-pyrimidinyl]thio]methyl]-8-oxo-5-thia-1-azabicyclo[4.2.0]oct-2-en-2-carbonsäure werden in 3 ml Trifluoressigsäure gelöst. Nach 5-stündigem Rühren bei Raumtemperatur wird bei Raumtemperatur im Vakuum eingeengt und der Rückstand aus Aethanol/Diäthyläther kristallisiert. Man erhält 63 mg (6R,7R)-7-[(Z)-2-(2-Amino-4-thiazolyl)-2-[[(2,5-dichlor-3,4-dihydroxybenzyl)oxy]imino]acetamido]-3-[[[5-(3,4-dihydroxyphenyl)-2-phenyl-4-pyrimidinyl]thio]methyl]-8-oxo-5-thia-1-azabicyclo[4.2.0]oct-2-en-2-carbonsäure als beiges Pulver.
$^1$H NMR (DMSO-$d_6$): Signale u.a. bei δ 3,52 (d, J=18Hz, 1H), 3,79 (d, J=18Hz, 1H), 4,18 (d, J=12,5Hz, 1H), 4,75 (d, J=12,5Hz, 1H), 5,08 (s, 2H), 5,16 (d, J=5Hz, 1H), 5,77 (dd, J=5Hz und J=8Hz, 1H), 6,74 (s, 1H), 6,78 (dd, J=8Hz und J=2Hz), 6,87 (d, J=8Hz, 1H), 6,92 (d, J=2Hz, 1H), 6,93 (s, 1H), 7,28 (2H), 7,56 (3H), 8,44 (s, 1H), 8,46 (2H), 9,24 (1H), 9,31 (1H), 9,67 (2H), 9,70 (d, J=8Hz, 1H).

Die Herstellung von 5-[(Aminooxy)methyl]-4,7-dichlor-2,2-diphenyl-1,3-benzodioxol-hydrochlorid wird in Beispiel 29 beschrieben.

Beispiel 19

Herstellung von a) (6R,7R)-7-[(Z)-2-(2-Amino-4-thiazolyl)-2-[[1-(hydroxycarbamoyl)-1-methyläthoxy]imino]-acetamido]-3-[[[5-(3,4-dihydroxyphenyl)-4-pyrimidinyl]thio]methyl]-8-oxo-5-thia-1-azabicyclo[4.2.0]oct-2-en-2-carbonsäure und b) (6R,7R)-7-[(Z)-2-(2-Amino-4-thiazolyl)-2-[[1-(hydroxycarbamoyl)-2-methyläthoxy]-imino]acetamido]-3-[[[5-(3,4-dihydroxyphenyl)-2-methyl-4-pyrimidinyl]thio]methyl]-8-oxo-5-thia-1-azabicyclo-[4.2.0]oct-2-en-2-carbonsäure

a) 90 mg (0,15 mMol) (6R,7R)-7-(2-Amino-4-thiazolglyoxylamido)-3-[[[5-(3,4-dihydroxyphenyl)-4-pyrimidinyl]thio]methyl]-8-oxo-5-thia-1-azabicyclo[4.2.0]-oct-2-en-2-carbonsäure und 34 mg (0,2 mMol) 2-(Aminooxy)-N-hydroxy-2-methylpropionamid-hydrochlorid werden in 2 ml absolutem Dimethylacetamid gelöst. Nach 24-stündigem Rühren bei Raumtemperatur wird im Hochvakuum bei Raumtemperatur eingeengt und der Rückstand aus Aethanol/Diäthyläther kristallisiert. Man nutscht ab und wäscht mit Aether und Wasser. Man erhält nach Trocknen im Hochvakuum bei Raumtemperatur 72 mg (6R,7R)-7-[-(Z)-2-(2-Amino-4-thiazolyl)-2-[[1-(hydroxycarbamoyl)-1-methyläthoxy]imino]acetamido]-3-[[[5-(3,4-dihydroxyphenyl)-4-pyrimidinyl]thio]methyl]-8-oxo-5-thia-1-azabicyclo[4.2.0]oct-2-en-2-carbonsäure als beiges Pulver.
$^1$H NMR (DMSO-$d_6$): Signale u.a. bei δ 1,40 (s, 3H), 1,42 (s, 3H), 3,50 (d, J=18Hz, 1H), 3,75 (d, J=18Hz, 1H), 3,90 (d, J=12,5Hz, 1H), 4,70 (d, J=12,5Hz, 1H), 5,16 (d, J=5Hz, 1H), 5,86 (dd, J=5Hz und J=8Hz, 1H), 6,74 (dd, J=2Hz und J=8Hz, 1H), 6,80 (s, 1H), 6,82 (d, J=8Hz, 1H), 6,86 (d, J=2Hz, 1H), 8,32 (s, 1H), 8,86 (s, 1H), 9,63 (d, J=8Hz, 1H), 10,07 (s, 1H).
b) (6R,7R)-7-[(Z)-2-(2-Amino-4-thiazolyl)-2-[[1-(hydroxycarbamoyl)-2-methyläthoxy]imino]acetamido]-3-[[-[5-(3,4-dihydroxyphenyl)-2-methyl-4-pyrimidinyl]thio]methyl]-8-oxo-5-thia-1-azabicyclo[4.2.0]oct-2-en-2-carbonsäure ist beige und wird analog hergestellt.
$^1$H NMR (DMSO-$d_6$): Signale u.a. bei δ 1,40 (s, 3H), 1,42 (s, 3H), 3,77 (d, J=18Hz, 1H), 3,92 (d, J=12,5Hz, 1H), 4,74 (d, J=12,5Hz, 1H), 5,18 (d, J=5Hz, 1H), 5,86 (dd, J=5Hz und J=8Hz, 1H), 6,69 (dd, J=2Hz und J=8Hz, 1H), 6,82 (s, 1H), 6,83 (d, J=2Hz, 1H), 6,84 (d, J=8Hz, 1H), 8,20 (s, 1H), 9,63 (d, J=8Hz, 1H), 10,08 (s, 1H).

Die als Ausgangsverbindung eingesetzte (6R,7R)-7-(2-Amino-4-thiazolglyoxylamido)-3-[[[5-(3,4-dihydroxyphenyl)-4-pyrimidinyl]thio]methyl]-8-oxo-5-thia-1-azabicyclo[4.2.0]oct-2-en-2-carbonsäure kann wie folgt hergestellt werden:

7,2 g (30 mMol) 2-Formyl-2-(3,4-dimethoxyphenyl)essigsäuremethylester, 4,8 g (60 mMol) Formamidinhydrochlorid und 7,74 g (60 mMol) N-Aethyldiisopropylamin (Hünig Base) werden in 60 ml Aethanol 55 Stunden bei 50 °C gerührt und anschliessend im Vakuum eingeengt. Der Rückstand wird mehrmals mit Diäthyläther gewaschen und dann mit Aethanol/Diäthyläther kristallisiert. Das gelbliche Produkt wird abgenutsch, mit Aethanol/Diäthyläther gewaschen und im Hochvakuum bei Raumtemperatur getrocknet. Man erhält 1,6 g 5-(3,4-Dimethoxyphenyl)-4(3H)-pyrimidinon vom Smp. 205-206 °C.

5-(3,4-Dimethoxyphenyl)-2-methyl-4(3H)-pyrimidinon wird analog hergestellt, Smp. 187-188 °C (weisser Feststoff).

9,28 g (40 mMol) 5-(3,4-Dimethoxyphenyl)-4(3H)-pyrimidinon werden in 300 ml 48%iger wässriger Bromwasserstofflösung 48 Stunden unter Rückfluss gesetzt. Die erhaltene Suspension wird genutscht, das Filtergut in Wasser aufgenommen und das Produkt durch Zugabe von Natriumhydrogencarbonat ausgefällt. Der Niederschlag wird abfiltriert, mit Wasser gewaschen und bei Raumtemperatur getrocknet. Man erhält 8,3 g 5-(3,4-Dihydroxyphenyl)-4(3H)-pyrimidinon-hydrobromid als beiges Pulver. Smp. >270 °C.
$^1$H NMR (DMSO-$d_6$): Signale bei δ 6,75 (d, J=8Hz, 1H), 6,97 (dd, J=8Hz und J=2Hz, 1H), 7,22 (d, J=2Hz, 1H), 8,01 (s, 1H), 8,11 (s, 1H), 8,9-9,2 (2H).

5-(3,4-Dihydroxyphenyl)-2-methyl-4(3H)-pyrimidinon wird analog hergestellt. Beige-farbener Feststoff vom Smp. 237 °C (Zers.).
$^1$H NMR (DMSO-$d_6$): Signale u.a. bei δ 2,29 (s, 3H), 6,73 (d, J=8Hz, 1H), 6,94 (dd, J=8Hz und J=2Hz, 1H), 7,20 (d, J=2Hz, 1H), 7,91 (s, 1H), 8,8-9,2(2H).

7,8 g (38 mMol) 5-(3,4-Dihydroxyphenyl)-4(3H)-pyrimidinon-hydrobromid werden in 60 ml absolutem Pyridin gelöst und mit 15,3 ml (115 mMol) Acetanhydrid versetzt. Die Lösung wird 5 Stunden bei Raumtemperatur gerührt und anschliessend im Vakuum eingeengt. Der Rückstand wird aus Diäthyläther kristallisiert und aus Aethanol umkristallisiert. Man erhält 7,3 g 4-(3,4-Dihydro-4-oxo-5-pyrimidinyl)-o-phenylen-diacetat als beige Kristalle vom Smp. 183-184 °C.

4-(3,4-Dihydro-2-methyl-4-oxo-5-pyrimidinyl)-o-phenylen-diacetat wird analog hergestellt. Weisse Kristalle vom Smp. 178-179 °C.

194 mg (0,67 mMol) 4-(3,4-Dihydro-4-oxo-5-pyrimidinyl)-o-phenylendiacetat werden zu 5 ml Phosphoroxychlorid gegeben. Nach 6-stündigem Rühren bei 100 °C wird im Vakuum bei 40 °C eingeengt. Der Rückstand wird in Dichlormethan/Methanol (99:1 v/v) gelöst und die Lösung mit Wasser gewaschen. Die Lösung wird über Natriumsulfat getrocknet und eingeengt. Der Rückstand wird mit Aethanol kristallisiert. Die beigen Kristalle werden abgenutscht, mit Petroläther gewaschen und im Hochvakuum bei Raumtemperatur getrocknet. Man erhält 90 mg 4-(4-Chlor-5-pyrimidinyl)-o-phenylen-diacetat vom Smp. 108 °C.

4-(4-Chlor-2-methyl-5-pyrimidinyl)-o-phenylen-diacetat wird analog hergestellt. Smp. 111 °C (weisse Kristalle).

1,1 g (3,6 mMol) 4-(4-Chlor-5-pyrimidinyl)-o-phenylen-diacetat werden in 30 ml Methanol gelöst. Man gibt 1,56 g Natriumhydrogensulfid-hydrat dazu und rührt 4,5 Stunden bei 60 °C. Das Lösungsmittel wird im Vakuum abdestilliert und der Rückstand in 20 ml Wasser gelöst. Man säuert mit verdünnter wässriger Salzsäure bis zum pH 5 an, nutscht das ausgefallene Produkt ab und wäscht es mit Wasser und Diäthyläther. Nach Trocknen im Hochvakuum erhält man 770 mg 5-(3,4-Dihydroxyphenyl)-4(3H)-pyrimidinthion als gelbe Kristalle vom Smp. 235 °C (Zers.).

$^1$H NMR (DMSO-d$_6$): Signale bei δ 6,74 (d, J = 8Hz, 1H), 6,81 (dd, J = 8Hz und J = 2Hz, 1H), 7,08 (d, J = 2Hz, 1H), 7,92 (s, 1H), 8,24 (1H), 8,98 (s, 1H), 9,08 (s, 1H), 14,18 (1H).

5-(3,4-Dihydroxyphenyl)-2-methyl-4(3H)-pyrimidinthion wird analog hergestellt. Gelber Feststoff vom Smp. 213-215 °C (Zers.).

$^1$H NMR (DMSO-d$_6$): Signale bei δ 2,42 (s, 3H), 6,73 (d, J = 8Hz, 1H), 6,78 (dd, J = 8Hz und J = 2Hz, 1H), 7,06 (d, J = 2Hz, 1H), 7,82 (s, 1H), 9,0 (2H), 14,0 (1H).

220 mg (0,81 mMol) 7-Aminocephalosporansäure und 250 mg (1,04 mMol) 5-(3,4-Dihydroxyphenyl)-4-(3H)-pyrimidinthion werden in 8 ml Sulfolan/Dichlormethan (1:1 v/v) suspendiert und unter Rühren mit 5 ml Bortrifluoriddiäthylätherat versetzt. Nach 18 Stunden wird im Vakuum eingeengt und der Rückstand mit Diäthyläther gewaschen. Dann gibt man 2 ml Aethanol und 4 ml Wasser dazu und fügt Natriumhydrogencarbonat zu, bis pH 5 erreicht ist. Das Produkt wird abgenutscht, mit Wasser und Diäthyläther gewaschen und im Hochvakuum bei Raumtemperatur getrocknet. Man erhält 311 mg (6R,7R)-7-Amino-3-[[[5-(3,4-dihydroxyphenyl)-4-pyrimidinyl]thio]methyl]-8-oxo-5-thia-1-azabicyclo[4.2.0]oct-2-en-2-carbonsäure als beiges Pulver.

$^1$H NMR (DMSO-d$_6$): Signale u.a. bei δ 3,41 (d, J = 18Hz, 1H), 3,70 (d, J = 18Hz, 1H), 3,89 (d, J = 12,5Hz, 1H), 4,66 (d, J = 12,5Hz, 1H), 4,73 (d, J = 5Hz, 1H), 4,94 (d, J = 5Hz, 1H), 6,72 (dd, J = 2Hz und J = 8Hz, 1H), 6,82 (d, J = 8Hz, 1H), 6,85 (d, J = 2Hz, 1H), 8,31 (s, 1H), 8,86 (s, 1H), 9,22 (s, 1H), 9,30 (s, 1H).

(6R,7R)-7-amino-3-[[5-(3,4-dihydroxyphenyl)-2-methyl-4-pyrimidinyl]-thio]methyl]-8-oxo-5-thia-1-azabicyclo[4.2.0]oct-2-en-2-carbonsäure wird analog hergestellt (beiger Feststoff).

$^1$H NMR (DMSO-d$_6$): Signale u.a. bei δ 3,42 (d, J = 18Hz, 1H), 3,68 (d, J = 18Hz, 1H), 3,87 (d, J = 12,5Hz, 1H), 4,68 (d, J = 12,5Hz, 1H), 4,73 (d, J = 5Hz, 1H), 4,96 (d, J = 5Hz, 1H), 6,69 (dd, J = 2Hz und J = 8Hz, 1H), 6,81 (d, J = 8Hz, 1H), 6,81 (d, J = 2Hz, 1H), 8,18 (s, 1H), 9,20 (s, 1H), 9,25 (s, 1H).

215 mg (0,5 mMol) (6R,7R)-7-Amino-3-[[[5-(3,4-dihydroxyphenyl)-4-pyrimidinyl]thio]methyl]-8-oxo-5-thia-1-azabicyclo[4.2.0]oct-2-en-2-carbonsäure werden in 10 ml Wasser und 5 ml Acetonitril suspendiert. Bei 0 °C tropft man unter Rühren 7,5 ml 0,1N wässrige Kaliumhydroxidlösung dazu und gibt nach 30 Minuten portionenweise 210 mg (0,65 mMol) 2-Amino-4-thiazolthioglyoxylsäure-S-(2-benzothiazolyl)ester innerhalb einer Stunde dazu. Nach weiteren 5 Stunden gibt man 0,1N wässrige Kalilauge dazu, bis pH 7,8 erreicht ist. Die erhaltene Suspension wird filtriert, das Filtrat mit Aethylacetat gewaschen und mit verdünnter wässriger Salzsäure angesäuert (pH 5). Das ausgefallene Produkt wird abgenutscht und mit Wasser und Diäthyläther gewaschen. Nach Trocknen im Hochvakuum erhält man 200 mg (6R,7R)-7-(2-Amino-4-thiazolglyoxylamido)-3-[[[5-(3,4-dihydroxyphenyl)-4-pyrimidinyl]thio]methyl]-8-oxo-5-thia-1-azabicyclo[4.2.0]oct-2-en-2-carbonsäure als beiges Pulver.

$^1$H NMR (DMSO-d$_6$):Signale u.a. bei δ 3,52 (d, J = 18Hz, 1H), 3,74 (d, J = 18Hz, 1H), 3,93 (d, J = 12,5Hz, 1H), 4,70 (d, J = 12,5Hz, 1H), 5,13 (d, J = 5Hz, 1H), 5,72 (dd, J = 5Hz und J = 8Hz, 1H), 6,73 (dd, J = 2Hz und J = 8Hz, 1H), 6,85 (d, J = 8Hz, 1H), 6,86 (d, J = 2Hz, 1H), 7,40 (s, 2H), 7,81 (s, 1H), 8,32 (s, 1H), 8,86 (s, 1H), 9,23 (s, 1H), 9,31 (s, 1H), 9,77 (d, J = 8Hz, 1H).

(6R,7R)-7-(2-Amino-4-thiazolglyoxylamido)-3-[[[5-(3,4-dihydroxyphenyl)-2-methyl-4-pyrimidinyl]thio]-methyl]-8-oxo-5-thia-1-azabicyclo[4.2.0]oct-2-en-2-carbonsäure wird in gleicher Weise hergestellt (beiges Pulver).
$^1$H NMR (DMSO-$d_6$): Signale u.a. bei δ 2,60 (s, 3H), 3,52 (d, J=18Hz, 1H), 3,73 (d, J=18Hz, 1H), 3,94 (d, J=12,5Hz, 1H), 4,70 (d, J=12,5Hz, 1H), 5,16 (d, J=5Hz, 1H), 5,72 (dd, J=5Hz und J=8Hz, 1H), 6,68 (dd, J=2Hz und J=8Hz, 1H), 6,82 (d, J=2Hz, 1H), 6,83 (d, J=8Hz, 1H), 7,40 (s, 2H), 7,80 (s, 1H), 8,19 (s, 1H), 9,18 (s, 1H), 9,24 (s, 1H), 9,75 (d, J=8Hz, 1H).

Beispiel 20

Herstellung von (6R,7R)-7-[(Z)-2-(2-Amino-4-thiazolyl)-2-[[(2,5-dichlor-3,4-dihydroxybenzyl)oxy]imino]-acetamido]-3-[[[5-(3,4-dihydroxypheny])-4-pyrimidinyl]thio]methyl]-8-oxo-5-thia-1-azabicyclo[4.2.0]oct-2-en-2-carbonsäure

100 mg (0,17 mMol) (6R,7R)-7-(2-Amino-4-thiazolglyoxylamido)-3-[[[5-(3,4-dihydroxyphenyl)-4-pyrimidinyl]thio]methyl]-8-oxo-5-thia-1-azabicyclo-[4.2.0]oct-2-en-2-carbonsäure und 93 mg (0,22 mMol) 5-[-(Aminooxy)methyl]-4,7-dichlor-2,2-diphenyl-1,3-benzodioxol-hydrochlorid werden in 2 ml absolutem Dimethylacetamid gelöst. Nach 20-stündigem Rühren bei Raumtemperatur wird das Lösungsmittel im Hochvakuum bei Raumtemperatur abdestilliert. Der Rückstand wird mit Diäthyläther kristallisiert. Das Produkt wird abgenutscht und mit Diäthyläther und Wasser gewaschen. Man erhalt 160 mg (6R,7R)-7-[(Z)-2-(2-Amino-4-thiazolyl)-2-[[(4,7-dichlor-2,2-diphenyl-1,3-benzodioxol-5-yl)methoxy]imino]acetamido]-3-[[[5-(3,4-dihydroxphenyl)-4-pyrimidinyl]thio]methyl]-8-oxo-5-thia -1-azabicyclo-[4.2.0]oct-2-en-2-carbonsäure als beiges Pulver nach Trocknen im Hochvakuum bei Raumtemperatur.
$^1$H NMR (DMSO-$d_6$): Signale u.a. bei δ 83,89 (d, J=12,5Hz, 1H), 4,66 (d, J=12,5Hz, 1H), 5,08 (d, J=5Hz, 1H), 5,11 (2H), 5,74 (dd, J=5Hz und J=8Hz, 1H), 6,72 (dd, J=2Hz und J=8Hz, 1H), 6,78 (s, 1H), 6,86 (d, J=2Hz, 1H), 6,86 (d, J=8Hz, 1H), 7,14 (s, 1H), 7,4-7,6 (10H), 8,32 (s, 1H), 8,84 (s, 1H), 9,67 (d, J=8Hz, 1H).

165 mg (0,17 mMol) (6R,7R)-7-[(Z)-2-(2-Amino-4-thiazolyl)-2-[[(4,7-dichlor-2,2-diphenyl-1,3-benzodioxol-5-yl)methoxy]imino]acetamido]-3-[[[5-(3,4-dihydroxyphenyl)-4-pyrimidinyl]thio]methyl]-8-oxo-5-thia-1-azabicyclo-[4.2.0]oct-2-en-2-carbonsäure werden in 3 ml Trifluoressigsäure gelöst. Nach 15-stündigem Rühren bei Raumtemperatur wird im Vakuum eingeengt und der Rückstand aus Diäthyläther kristallisiert. Die Kristalle werden abgenutscht und mit Diäthyläther/Aethanol (10:1 v/v) gewaschen. Nach Trocknen im Hochvakuum erhalt man 115 mg (6R,7R)-7-[(Z)-2-(2-Amino-4-thiazolyl)-2-[[(2,5-dichlor-3,4-dihydroxybenzyl)-oxy]imino]acetamido]-3-[[[5-(3,4-dihydroxyphenyl)-4-pyrimidinyl]thio]methyl]-8-oxo-5-thia-1-azabicyclo-[4.2.0]oct-2-en-2-carbonsäure als gelbliches Pulver.
$^1$H NMR (DMSO-$d_6$): Signale u.a. bei δ 3,41 (d, J=18Hz, 1H), 3,69 (d, J=18Hz, 1H), 3,87 (d, J=12,5Hz, 1H), 4,67 (d, J=12,5Hz, 1H), 5,08(2H), 5,10 (d, J=5Hz, 1H), 5,74 (dd, J=5Hz und J=8Hz, 1H), 6,72 (dd, J=2Hz und J=8Hz, 1H), 6,75 (s, 1H), 6,84 (d, J=8Hz, 1H), 6,84 (d, J=2Hz, 1H), 6,96 (s, 1H), 8,32 (s, 1H), 8,88 (s, 1H), 9,70 (d, J=8Hz, 1H).

Die Herstellung von 5-[(Aminooxy)methyl]-4,7-dichlor-2,2-diphenyl-1,3-benzodioxol-hydrochlorid wird in Beispiel 29 beschrieben.

Beispiel 21

Herstellung von (6R,7R)-7-[(Z)-2-(2-Amino-4-thiazolyl)-2-[[(1-hydroxycarbamoyl)-1-methyläthoxy]imino]-acetamido]-3-[[(3,4-dihydroxyphenyl)sulfonyl]methyl]-8-oxo-5-thia-1-azabicyclo[4.2.0]oct-2-en-2-carbonsäure

0,50 g (0,93 mMol) (6R,7R)-7-(2-Amino-4-thiazolglyoxylamido)-3-[[(3,4-dihydroxyphenyl)sulfonyl]methyl]-8-oxo-5-thia-1-azabicyclo[4.2.0]oct-2-en-2-carbonsäure werden in 5 ml Dimethylacetamid gelöst und mit 0,21 g (1,20 mMol) 2-(Aminooxy)-N-hydroxy-2-methylpropionamid-hydrochlorid 20 Stunden bei Raumtemperatur gerührt. Das Dimethylacetamid wird am Hochvakuum abgedampft und der Rückstand mit Wasser verrührt. Der Feststoff wird durch Filtration gesammelt und durch Chromatographie an Kieselgel mit Wasser/Acetonitril als Eluens gereinigt. Man erhält 240 mg (6R,7R)-7-[(Z)-2-(2-Amino-4-thiazolyl)-2-[[(1-hydroxycarbamoyl)-1-methyläthoxy]imino]acetamido]-3-[[(3,4-dihydroxphenyl)sulfonyl]methyl]-8-oxo-5-thia-1-azabicyclo[4.2.0]oct-2-en-2-carbonsäure als weisses Lyophilisat.
$^1$H-NMR ($D_2O$, 250 MHz): Signale bei δ(ppm): 1,56 (s, 3H), 1,57 (s, 3H), 3,37 (d, J=18Hz, 1H), 3,68 (d, J=18Hz, 1H), 4,13 (d, J=12,5Hz, 1H), 5,16 (d, J=12,5Hz, 1H), 5,22 (d, J=5Hz, 1H), 5,81 (d, J=5Hz, 1H), 7,00-7,40 (m, 4H).

IR: 1768 $cm^{-1}$
MS: 679 (M + $H^{⊕}$)

Die als Ausgangsmaterial benötigte (6R,7R)-7-(2-Amino-4-thiazolglyoxylamido)-3-[[3,4-dihydroxyphenyl)sulfonyl]methyl]-8-oxo-5-thia-1-azabicyclo[4.2.0]oct-2-en-2-carbonsäure kann wie folgt hergestellt werden:

74 ml einer 0,6M Lösung von Butyllithium in n-Hexan werden mit 116 ml Diäthyläther gemischt und auf -55°C gekühlt. Hierauf wird eine Lösung von 40,0 g 5-Brom-2,2-diphenyl-1,3-benzodioxol in 170 ml Diäthyläther zugetropft. Das Reaktionsgemisch wird 1 Stunde gerührt, wobei die Temperatur auf -15°C steigt. Anschliessend wird auf -50°C abgekühlt und 45 Minuten lang trockenes Schwefeldioxid-Gas eingeleitet. Der Ueberschuss an Schwefeldioxidgas wird durch Durchleiten von trockenem Argon wahrend 1 Stunde entfernt. Das Reaktionsgemisch wird auf Raumtemperatur aufgetaut. Dabei entsteht ein weisser Niederschlag; der filtriert und getrocknet wird. Man erhalt 22,2 g 2,2-Diphenyl-1,3-benzodioxol-5-sulfinsäure-Lithiumsalz als weisse Kristalle vom Smp. >300°C (Zers.).

6,00 g 7-Amino-3-chlormethyl-cephalosporansäure-p-methoxy-benzylester-hydrochlorid werden in 60 ml Dimethylsulfoxid gelöst, mit 3,00 g Natriumjodid und 7,20 g 2,2-Diphenyl-1,3-benzodioxol-5-sulfinsäure-Lithiumsalz versetzt und 2 Stunden bei Raumtemperatur gerührt. Das Lösungsmittel wird unter Hochvakuum abgedampft und der Rückstand zwischen Aethylacetat und gesättigter wässriger Kaliumhydrogencarbonatlösung verteilt. Dabei entsteht ein weisser Niederschlag, der abfiltriert und in 20 ml Trifluoressigsäure, 10 ml Anisol und 2 ml Wasser gelöst und 5 Stunden bei Raumtemperatur gerührt wird. Die Lösungsmittel werden zuerst am Wasserstrahlvakuum, dann am Hochvakuum abgedampft. Der dunkle Rückstand wird zwischen Aethylacetat und 1N wässriger Salzsäure verteilt. Die wässrige Phase wird mit Aktivkohle behandelt und das Produkt durch Einstellen des pH auf 3,50 gefällt. Man erhält 2,20 g (6R,7R)-7-Amino-3-[-[(3,4-dihydroxyphenyl)sulfonyl]methyl]-8-oxo-5-thia-1-azabicyclo[4.2.0]oct-2-en-2-carbonsäure als weisses Pulver.

$^1$H-NMR (DMSO-$d_6$, 250 MHz): Signale bei δ (ppm) 3,46 (s, 2H), 4,17 (d, J = 14 Hz, 1H), 4,76 (d, J = 5 Hz, H), 4,92 (d, J = 14 Hz, 1H), 4,98 (d, J = 5 Hz, 1H), 6,90 (d, J = 8,5 Hz, 1H), 7,10 (m, 2H), 9,8 (s, breit, 1H), 10,2 (s, breit, 1H)
IR (KBr) 1777 $cm^{-1}$
MS: 384 (M + $H^{⊕}$)

190 mg (0,50 mMol) (6R,7R)-7-Amino-3-[[(3,4-dihydroxyphenyl)sulfonyl]methyl]-8-oxo-5-thia-1-azabicyclo[4.2.0]oct-2-en-2-carbonsäure werden in 2,0 ml einer 1:1 Mischung von Wasser und Acetonitril durch Zugabe von 0,075 ml (0,50 mMol) Triäthylamin gelöst. Diese Lösung wird auf 0°C gekühlt und mit 280 mg (~0,75 mMol) 2-Amino-4-thiazolthioglyoxylsäure-S-(2-benzothiazolyl)ester versetzt und 1,5 Stunden gerührt. Die Feststoffe werden durch Zentrifugieren abgetrennt. Der klare Ueberstand wird zwischen Wasser und Aethylacetat verteilt, die wässrige Phase auf etwa die Hälfte eingeengt und das Produkt durch Ansäuern auf pH 2,0 gefällt. Nach Trocknen am Hochvakuum erhält man 180 mg (6R,7R)-7-(2-Amino-4-thiazolglyoxylamido)-3-[[(3,4-dihydroxyphenyl)sulfonyl]methyl]-8-oxo-5-thia-1-azabicyclo[4.2.0]oct-2-en-2-carbonsäure als gelbes Pulver.

$^1$H-NMR (DMSO-$d_6$, 250 MHz): Signale bei δ (ppm): 3,58 (m, 2H), 4,27 (d, J = 12,5Hz, 1H), 4,94 (d, J = 12,5Hz, 1H), 5,20 (d, J = 5Hz, 1H), 5,76 (dd, J = 5Hz, J = 7Hz, 1H), 6,92 (d, J = 8Hz, 1H), 7,14 (m, 2H), 7,41 (s, 2H), 7,83 (s, 1H), 9,77 (s, 1H), 9,81 (d, J = 8Hz), 10,18 (s, 1H), 13,62 (s, breit, 1H).
MS: 541 (M + $H^{⊕}$)
IR: 1773 $cm^{-1}$

## Beispiel 22

Herstellung von (6R,7R)-7-[(Z)-2-(2-Amino-4-thiazolyl)-2-[[(3,4-dihydroxysulfonyl)methoxy]imino]acetamido]-3-[[(3,4-dihydroxyphenyl)sulfonyl]methyl]-8-oxo-5-thia-1-azabicyclo[4.2.0]oct-2-en-2-carbonsäure-Natriumsalz

0,50 g (0,93 mMol) (6R,7R)-7-(2-Amino-4-thiazolglyoxylamido)-3-[[(3,4-dihydroxyphenyl)sulfonyl]methyl]-8-oxo-5-thia-1-azabicyclo[4.2.0]oct-2-en-2-carbonsäure werden in 5,00 ml Dimethylacetamid gelöst, mit 0,31 g (1,20 mMol) 4-[[(Aminooxy)methyl]sulfonyl]pyrocatechol-hydrochlorid versetzt und bei Raumtemperatur 20 Stunden gerührt. Das Dimethylacetamid wird am Hochvakuum abgedampft. Der Rückstand wird mit Wasser versetzt und der entstandene Niederschlag durch Filtration isoliert und in Wasser resuspendiert. Durch Zugabe von 1N wässriger Natronlauge wird dieser Niederschlag bei pH 6,6 in Lösung gebracht. Reinigung erfolgt durch Chromatographie an RP12-Kieselgel mit Wasser/Acetonitril als Eluens. Man erhält nach der Lyophilisation 520 mg (6R,7R)-7-[(Z)-2-(2-Amino-4-thiazolyl)-2-[[(3,4-dihydroxysulfonyl)methoxy]imino]-

acetamido]-3-[[(3,4-dihydroxyphenyl)sulfonyl]methyl]-8-oxo-5-thia-1-azabicyclo[4.2.0]oct-2-en-2-carbonsäure-Natriumsalz als hellgelben Feststoff.
$^1$H-NMR ($D_2O$, 250 MHz): Signale bei δ (ppm): 3,09 (d, J=18Hz, 1H), 3,50 (d, J=18Hz, 1H), 4,10 (d, J=12,5Hz, 1H), 4,96 (d, J=5Hz, 1H), 5,15 (d, J=12,5Hz, 1H), 5,45 (m, 2H), 5,61 (d, J=5Hz, 1H), 7,0-7,4 (m, 7H)
MS: 763,9 ($M+H^{⊕}$)
IR (KBr): 1767 $cm^{-1}$

Beispiel 23

Herstellung von (6R,7R)-7-[(Z)-2-(2-Amino-4-thiazolyl)-2-[[(3,4-dihydroxybenzyl)oxy]imino]acetamido]-3-[[-(3,4-dihydroxyphenyl)sulfonyl]methyl]-8-oxo-5-thia-1-azabicyclo[4.2.0]oct-2-en-2-carbonsäure

190 mg (0,50 mMol) (6R,7R)-7-(2-Amino-4-thiazolglyoxylamido)-3-[[(3,4-dihydroxyphenyl)sulfonyl]-methyl]-8-oxo-5-thia-1-azabicyclo[4.2.0]oct-2-en-2-carbonsäure werden in 3 ml Dimethylacetamid gelöst, mit 220 mg (0,62 mMol) O-[3,4-[(Diphenylmethylen)dioxy]benzyl]hydroxylamin-hydrochlorid versetzt und 20 Stunden bei Raumtemperatur gerührt. Das Dimethylacetamid wird am Hochvakuum abgedampft. Der Rückstand wird mit Wasser verrührt. Der Feststoff wird abfiltriert, in Trifluoressigsäure gelöst und 1 Stunde bei Raumtemperatur stehen gelassen. Die Trifluoressigsäure wird abgedampft und der Rückstand mit Aethylacetat verrührt. Der Feststoff wird abfiltriert und anschliessend durch Chromatographie an RP12-Kieselgel mit Wasser/Acetonitril als Eluens gereinigt. Man erhält nach Lyophilisation 100 mg (6R,7R)-7-[(Z)-2-(2-Amino-4-thiazolyl)-2-[[(3,4-dihydroxybenzyl)oxy]imino]acetamido]-3-[[(3,4-dihydroxyphenyl)sulfonyl]-methyl]-8-oxo-5-thia-1-azabicyclo[4.2.0]oct-2-en-2-carbonsäure als weisser Feststoff.
$^1$H-NMR (DMSO-$d_6$, 250 MHz): Signale bei δ (ppm) 3,37 (m, 2H), 3,86 (d, J=12,5Hz, 1H), 4,91 (s, 2H), 4,95 (d, J=5Hz, 1H), 5,54 (m, 2H), 6,6-7,2 (m, 6H, aromat.), 6,71 (s, 1H), 7,21 (s, 2H), 9,5 (d, J=8Hz, 1H)
MS: 678 ($M+H^{⊕}$)
IR: 1767 $cm^{-1}$

Beispiel 24

Herstellung von (6R,7R)-7-[(Z)-2-(2-Amino-4-thiazolyl)-2-[[(3,4-dihydroxybenzyl)oxy]imino]acetamido]-3-[[[2-(hydroxycarbamoyl)-5-methyl-s-triazolo-[1,5-a]pyrimidin-7-yl]thio]methyl]-8-oxo-5-thia-1-azabicyclo[4.2.0]oct-2-en-2-carbonsäure

Eine Lösung von 200 mg (0,457 mMol) (6R,7R)-7-Amino-3-[[[2-(hydroxycarbamoyl)-5-methyl-s-triazolo-[1,5-a]pyrimidin-7-yl]thio]methyl]-8-oxo-5-thia-1-azabicyclo[4.2.0]oct-2-en-2-carbonsäure und 346 mg (0,55 mMol) S-(2-Benzothiazolyl)-2-amino-4-thiazolglyoxylat-(Z)-O-[3,4-[(diphenylmethylen]dioxybenzyl]oxim in 19 ml Dimethylacetamid wird 7 Stunden bei Raumtemperatur gerührt und anschliessend im Hochvakuum eingeengt. Der Rückstand wird in Wasser suspendiert und abgenutscht. Das Nutschgut wird dreimal in Aethylacetat suspendiert, abgenutscht und anschliessend mit Aethylacetat, Aethanol, Dichlormethan und Diäthyläther gewaschen. Man erhält 295 mg (6R,7R)-7-[(Z)-2-(2-Amino-4-thiazolyl)-2-[[[3,4-[-(diphenylenmethylen)dioxy]benzyl]oxy]imino]acetamido]-3-[[[2-(hydroxycarbamoyl)-5-methyl-s-triazolo[1,5-a]pyridin-7-yl]thio]methyl]-8-oxo-5-thia-1-azabicyclo-[4.2.0]oct-2-en-2-carbonsäure als beiges Pulver.
$^1$H NMR (DMSO-$d_6$): Signale u.a. bei δ 2,60 (s, 3H), 3,52 (d, J=18Hz, 1H), 3,74 (d, J=18Hz, 1H), 4,36 (d, J=12,5Hz, 1H), 4,42 (d, J=12,5Hz, 1H), 5,02 (s, 2H), 5,18 (d, J=5Hz, 1H), 5,81 (dd, J=5Hz und 8Hz, 1H), 6,71 (s, 1H), 6,88 (dd, 1H), 6,98 (d, J=9Hz), 7,04 (d, 1H), 7,29 (s, 2H), 7,34-7,60 (10H), 9,72 d (d, J=8Hz, 1H).

250 mg (0,28 mMol) (6R,7R)-7-[(Z)-2-(2-Amino-4-thiazolyl)-2-[[[3,4-[(diphenylenmethylen)dioxy]benzyl]-oxy]imino]acetamido]-3[[[2-(hydroxycarbamoyl)-5-methyl-s-triazolo[1,5-a]pyridin-7-yl]thio]methyl]-8-oxo-5-thia-1-azabicyclo[4.2.0]oct-2-en-2-carbonsäure werden bei 0°C in 16 ml Trifluoressigsäure gelöst. Nach 5-stündigem Rühren bei 0°C wird das Lösungsmittel im Hochvakuum bei Raumtemperatur abgezogen. Der Rückstand wird in Aethanol/Diäthyläther suspendiert. Das Produkt wird abfiltriert und mit Diäthyläther gewaschen. Man erhält 80 mg (6R,7R)-7-[(Z)-2-(2-Amino-4-thiazolyl)-2-[[(3,4-dihydroxybenzyl)oxy]imino]-acetamido]-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure als leicht beige gefärbtes Pulver.
$^1$H NMR (DMSO-$d_6$): Signale u.a. bei δ 2,60 (s, 3H), 3,56 (d, J=18Hz), 3,78 (d, J=18Hz, 1H), 4,40 (d, J=12,5Hz, 1H), 4,47 (d, J=12,5Hz, 1H), 4,92 (s, 2H), 5,17 (d, J=5Hz, 1H), 5,80 (dd, J=5Hz und J=8Hz, 1H), 6,6-6,8 (4H), 7,23 (s (breit), 2H), 7,39 (s, 1H), 8,84 (s, 1H), 8,89 (s, 1H), 9,37 (s (breit), 1H), 9,66 (d,

J = 8Hz, 1H).

Die als Ausgangsmaterial eingesetzte (6R,7R)-7-Amino-3-[[[2-(hydroxycarbamoyl)-5-methyl-s-triazolo-[1,5-a]pyrimidin-7-yl]thio]methyl]-8-oxo-5-thia-1-azabicyclo[4.2.0]oct-2-en-2-carbonsäure kann wie folgt hergestellt werden:

Bei 0 °C werden 1,35 g (13,37 mMol) Triäthylamin zu einer Lösung von 0,62 g (8,92 mMol) Hydroxylamin-hydrochlorid in 15 ml absolutem Methanol getropft. Das Ganze wird 10 Minuten nachgerührt, und anschliessend wird eine Lösung von 1,0 g (4,46 mMol) 7-Mercapto-5-methyl-s-triazolo[1,5-a]pyrimidin-2-carbonsäuremethylester (bekannt aus EPOS 303.172) in 30 ml absolutem Methanol zugetropft. Nach 3-tägigem Rühren bei Raumtemperatur wird die beige Suspension mit 1N wässriger Salzsäure sauer gestellt (pH 4) und genutscht. Das Nutschgut wird mit Wasser und Diäthyläther gewaschen und aus Methanol/Dichlormethan kristallisiert. Man erhält 510 mg N-Hydroxy-7-mercapto-5-methyl-s-triazolo[1,5-a]-pyrimidin-2-carboxamid als beige Kristalle vom Smp. 213 °C (Zers.).

3,1 g (13,9 mMol) N-Hydroxy-7-mercapto-5-methyl-s-triazolo[1,5-a]pyrimidin-2-carboxamid und 3,4 g (12,5 mMol) 7-Aminocephalosporansäure werden in 75 ml Sulfolan/Dichlormethan (1:1 v/v) suspendiert. Bei 0 °C tropft man 7,5 ml Bortrifluoridätherat dazu und rührt 2,5 Stunden bei 0 °C und 15 Stunden bei Raumtemperatur. 200 ml Dichlormethan werden nun zugegeben. Nach 30-minütigem Rühren wird abgenutscht. Das Nutschgut wird in 100 ml Wasser gelöst. Das Produkt wird mit gesättigter Natriumhydrogencarbonatlösung ausgefällt (pH ~5), abfiltriert und mit Wasser, Aethanol und Aether gewaschen. Man erhält 3,5 g 6R,7R)-7-Amino-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure als beiges Pulver.

$^1$H NMR (DMSO-$d_6$): $\delta$ 2,60 (s, 3H), 3,56 (d, J = 18Hz, 1H), 3,77 (d, J = 18Hz, 1H), 4,36 (d, J = 12,5Hz, 1H), 4,45 (d, J = 12,5Hz, 1H), 4,83 (d, J = 5Hz, 1H), 5,04 (d, J = 5Hz, 1H), 7,38 (s, 1H), 9,36 (s, 1H), 11,68 (s, 1H).

Das als Ausgangsmaterial eingesetzte S-(2-Benzothiazolyl)-2-amino-4-thiazolglyoxylat-(Z)-O-[3,4-[(diphenylmethylen]dioxybenzyl]oxim kann wie folgt hergestellt werden:

81,4 g 3,4-[(Diphenylmethylen)dioxy]benzylchlorid [bekannt aus Tetrahedron Letters, 2745(1977)] und 53,5 g des Lithiumsalzes des 2-(2-Amino-4-thiazol)-2-hydroxyiminoessigsäure-allylesters (bekannt aus EPOS 96.297) werden in 1,2l Dimethylformamid 24 Stunden lang gerührt. Danach engt man im Vakuum auf ca. 300 ml ein, gibt 700 ml Wasser zu und extrahiert zweimal mit je 1 l Aethylacetat. Die vereinigten Aethylacetatextrakte werden mit Wasser gewaschen und auf ca. 350 ml eingeengt, wobei das Reaktionsprodukt auskristallisiert. Das Kristallisat wird abgenutscht und aus Dichlormethan/Aethanol umkristallisiert. Man erhält 73,8 g 2-(2-Amino-4-thiazol)-2-[3,4-[(diphenylmethylen)dioxy]benzyloxyiminoessigsäure-allylester vom Smp. 151-153 °C.

Dieses Produkt wird in 1,3 l Acetonitril suspendiert. Nach Zugabe von 344 mg Pd-(II)-acetat und 1,24 ml Triäthylphosphit kühlt man auf 0 °C, gibt 16,7 ml N-Methylpyrrolidon zu und rührt die erhaltene Suspension während 24 Stunden bei 0 °C. Danach nutscht man ab, wäscht mit Acetonitril und Diäthyläther und trocknet das entstandene N-Allyl-N-methylpyrtolidinium-Salz der 2-(2-Amino-4-thiazol)-2-[3,4-[(diphenylmethylen)-dioxy]benzyloxyiminoessigsäure ab. Man erhält 80,6 g.

46 g des obigen Salzes werden in 600 ml Acetonitril suspendiert. Nach Zugabe von 8,5 ml N-Methylmorpholin und 30,7 g 2,2-Dithio-bis-benzothiazol rührt man bei Zimmertemperatur und tropft über 2 Stunden die Lösung von 23 ml Triäthylphosphit in 150 ml Acetonitril zu. Nach weiteren 2 Stunden filtriert man und lässt das Filtrat 3 Tage lang stehen, wobei das Produkt kristallisiert. Man nutscht und wäscht mit Acetonitril und Diäthyläther und trocknet im Vakuum bei 35 °C. Man erhält 22,5 g S-(2-Benzothiazolyl)-2-amino-4-thiazolglyoxylat-(Z)-O-[3,4-[(diphenylmethylen]dioxybenzyl]oxim als gelbes Kristallpulver vom Smp. 155-157 °C.

| Elementaranalyse: | | | | |
|---|---|---|---|---|
| Gefunden; | 61,38% C, | 3,59% H, | 8,92% N, | 15,37% S; |
| Berechnet: | 61,72% C, | 3,56% H, | 9,00% N, | 15,44% S. |

Beispiel 25

Herstellung von (6R,7R)-7-[(Z)-2-(2-Amino-4-thiazolyl)-2-[[[(3,4-dihydroxyphenyl)sulfonyl]methoxy]imino]-acetamido]-3-[[[2-(hydroxycarbamoyl)-5-methyl-s-triazolo[1,5-a]pyrimidin-7-yl]thio]methyl]-8-oxo-5-thia-1-azabicyclo-[4.2.0]oct-2-en-2-carbonsäure

80 mg (0,135 mMol) (6R,7R)-7-(2-Amino-4-thiazolglyoxylamido)-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure und 45 mg (0,176 mMol) 4-[[(Aminooxy)methyl]sulfonyl]pyrocatechol-hydrochlorid werden in 4 ml absolutem Dimethylacetamid gelöst. Nach 24-stündigem Rühren bei Raumtemperatur werden nochmals 14 mg (0,06 mMol) 4-[(-(Aminooxy)methyl]sulfonyl]pyrocatechol-hydrochlorid zugefügt. Nach weiteren 24 Stunden wird das Lösungsmittel im Hochvakuum bei Raumtemperatur abgezogen und der Rückstand mit 4 ml Wasser versetzt. Das ausgefallene Produkt wird abgenutscht und nacheinander mit Wasser und Diäthyläther gewaschen. Man erhält 100 mg (6R,7R)-7-[(Z)-2-(2-Amino-4-thiazolyl)-2-[[[(3,4-dihydroxyphenyl)sulfonyl]methoxy]imino]-acetamido]-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure als braunes Pulver.
$^1$H NMR (DMSO-$d_6$): Signale u.a. bei $\delta$ 2,61 (s, 3H), 3,60 (d, J = 18Hz, 1H), 3,75 (d, J = 18Hz, 1H), 4,38 (d, J = 12,5Hz, 1H), 4,46 (d, J = 12,5Hz, 1H), 5,14 (d, J = 5Hz, 1H), 5,22(2H), 5,75 (dd, J = 5Hz und J = 8Hz, 1H), 6,70 (s, 1H), 6,91 (d, J = 7,5Hz, 1H), 7,2 (2H), 7,29 (s, 2H), 7,40 (s, 1H), 9,36 (s, 1H), 9,67 (s, 1H), 9,72 (d, J = 8Hz, 1H), 10,10 (s, 1H), 11,68 (s, 1H).

Die als Ausgangsverbindung eingesetzte (6R,7R)-7-(2-Amino-4-thiazolglyoxylamido)-3-[[[2-(hydroxycarbamoyl)-5-methyl-s-triazolo-[1,5-a]pyrimidin-7-yl]thio]methyl]-8-oxo-5-thia-1-azabicyclo[4.2.0]oct-2-en-2-carbonsäure kann wie folgt hergestellt werden:

43,7 mg (0,1 mMol) (6R,7R)-7-Amino-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure werden in einem Gemisch bestehend aus 2 ml Wasser und 1 ml Acetonitril suspendiert. Bei Raumtemperatur tropft man unter Rühren 2 ml 0,1N wässrige Kalilauge zu. Zur Lösung gibt man anschliessend 84 mg (0,26 mMol) 2-Amino-4-thiazolthioglyoxylsäure-S-(2-benzothiazolyl)ester. Die Suspension wird 3 Stunden bei Raumtemperatur gerührt und filtriert. Das Filtergut wird mit Wasser gewaschen, Filtrat und Waschlösung vereinigt und mit Aethylacetat nochmals gewaschen. Die wässrige Lösung wird mit verdünnter wässriger Salzsäure auf pH 5 gestellt. Das ausgefallene Produkt wird abgenutscht und mit Wasser und Diäthyläther gewaschen. Man erhält nach Trocknen im Hochvakuum 30 mg (6R,7R)-7-(2-Amino-4-thiazolglyoxylamido)-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure als gelbes Pulver.
$^1$H NMR (DMSO-$d_6$): Signale bei $\delta$ 2,61 (s, 3H), 3,63 (d, J = 18Hz, 1H), 3,80 (d, J = 18Hz, 1H), 4,40 (d, J = 12,5Hz, 1H), 4,48 (d, J = 12,5Hz, 1H), 5,21 (d, J = 5Hz, 1H), 5,77 (dd, J = 5Hz und J = 8Hz, 1H), 7,40 (s, 3H), 7,82 (s, 1H), 9,35 (s, 1H), 9,81 (d, J = 8Hz, 1H), 11,68 (s, 1H).

Beispiel 26

Herstellung von (6R,7R)-7-[(Z)-2-(2-Amino-4-thiazolyl)-2-[[(1,4-dihydro-5-hydroxy-4-oxo-2-pyrimidinyl)-methoxy]imino]acetamido]-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure

25 mg (0,042 mMol) (6R,7R)-7-(2-Amino-4-thiazolglyoxylamido)-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure, 8,6 mg (0,055 mMol) 2-(Aminooxy)methyl-5-hydroxy-4(1H)-pyrimidinon und 10,5 mg (0,055 mMol) p-Toluolsulfonsäurehydrat werden in 3 ml absolutem Dimethylacetamid gelöst. Nach 24-stündigem Rühren bei Raumtemperatur wird das Lösungsmittel im Hochvakuum bei Raumtemperatur abgezogen und der Rückstand mit 3 ml Wasser versetzt. Das ausgefallene Produkt wird abfiltriert und nacheinander mit Wasser und Diäthyläther gewaschen. Nach Trocknen im Hochvakuum erhält man 20 mg (6R,7R)-7-[(Z)-2-(2-Amino-4-thiazolyl)-2-[[-(1,4-dihydro-5-hydroxy-4-oxo-2-pyrimidinyl)-methoxy]imino]acetamido]-3-[[[2-(hydroxycarbamoyl)-5-methyl-s-triazolo-[1,5-a]pyrimidin-7-yl]thio]methyl]-8-oxo-5-thia-1-azabicyclo[4.2.0]oct-2-en-2-carbonsäure als beiges Pulver.
$^1$H NMR (DMSO-$d_6$): Signale u.a. bei $\delta$ 2,61 (s, 3H), 3,52 (d, J = 18Hz, 1H), 3,80 (d, J = 18Hz, 1H), 4,36 (d, J = 12,5Hz, 1H), 4,46 (d, J = 12,5Hz, 1H), 4,90 (2H), 5,20 (d, J = 5Hz, 1H), 5,84 (dd, J = 5Hz und J = 8Hz, 1H), 6,84 (s, 1H), 7,37 (s, 1H), 7,39 (s, 1H), 9,36 (1H), 9,55 (2H), 9,90 (d, J = 8Hz, 1H), 11,68 (s, 1H).

Beispiel 27

Herstellung von (6R,7R)-7-[(Z)-2-(2-Amino-4-thiazolyl)-2-[[(1,6-dihydro-5-hydroxy-1-methyl-6-oxo-2-pyrimidinyl)methoxy]imino]acetamido]-3-[[[2-(hydroxycarbamoyl)-5-methyl-s-triazolo[1,5-a]-pyrimidin-7-yl]thio]-methyl]-8-oxo-5-thia-1-azabicyclo[4.2.0]oct-2-en-2-carbonsäure

65 mg (0,11 mMol) (6R,7R)-7-(2-Amino-4-thiazolglyoxylamido)-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure werden in 4 ml absolutem Dimethylacetamid gelöst. 24 mg (0,14 mMol) 2-[(Aminooxy)methyl]-5-hydroxy-3-methyl-4-(3H)-pyrimidinon und 14 mg (0,14 mMol) Methansulfonsäure werden zugegeben. Nach 20-stündigem Rühren bei Raumtemperatur wird das Dimethylacetamid am Hochvakuum abgedampft und der Rückstand in Wasser aufgenommen und zur vollständigen Fällung des Produktes 15 Minuten gerührt. Das Produkt wird abfiltriert, mit Wasser und Diätbyläther gewaschen und im Hochvakuum bei Raumtemperatur getrocknet. Man erhält 48 mg (6R,7R)-7-[(Z)-2-(2-Amino-4-thiazolyl)-2-[[(1,6-dihydro-5-hydroxy-1-methyl-6-oxo-2-pyrimidinyl)methoxy]imino]acetamido]-3-[[[2-(hydroxycarbamoyl)-5-methyl-s-triazolo[1,5-a]pyrimidin-7-yl]-thio]methyl]-8-oxo-5-thia-1-azabicyclo[4.2.0]oct-2-en-2-carbonsäure als beiges Pulver.
$^{1}H$ NMR (DMSO-$d_6$): $\delta$ 2,61 (s, 3H), 3,52 (s, 3H), 4,38 (d, J = 15Hz, 1H), 4,47 (d, J = 15Hz, 1H), 5,09 (d, J = 14Hz, 1H), 5,19 (d, J = 14Hz, 1H), 5,19 (d, J = 5Hz, 1H), 5,82 (dd, J = 8Hz und J = 5Hz, 1H), 6,78 (s, 1H), 7,29 (s, 2H, breit), 7,4 (2H), 9,37 (s, 1H), 9,65 (s, 1H), 9,84 (d, J = 8Hz, 1H), 11,70 (s, 1H).

Das als Ausgangsmaterial eingesetzte 2-[(Aminooxy)methyl]-5-hydroxy-3-methyl-4(3H)-pyrimidinon kann wie folgt hergestellt werden:

2,3 g (10 mMol) 5-(Benzyloxy)-2-(hydroxymethyl)-4(1H)-pyrimidinon werden in 20 ml absolutem Methanol gelöst. Die Lösung wird mit 4,24 g (40 mMol) Natriumcarbonat und 10 ml Methyljodid versetzt und 4 Tage bei Raumtemperatur gerührt. Man gibt nun 400 ml Dichlormethan und 100 ml gesättigte Natriumchloridlösung hinzu, trennt die organische Phase ab, trocknet sie über Natriumsulfat und engt sie ein. Der Rückstand wird an 100 g Kieselgel (0,063-0,2 mm) chromatographiert. Die beiden Produkte werden nacheinander mit Dichlormethan/Methanol (9:1 v/v) eluiert. Man erhält 1,2 g 5-(Benzyloxy)-2-(hydroxymethyl)-3-methyl-4(3H)-pyrimidinon als weisse Kristalle aus Aether, Smp. 83-84 °C. 5-(Benzyloxy)-2-(hydroxymethyl)-1-methyl-4(1H)-pyrimidinon bildet ebenfalls weisse Kristalle aus Aether, Smp. 172-173 °C. Es werden 0,6 g erhalten.

4,8 g (20 mMol) Azodicarbonsäurediäthylester gibt man bei 0 °C tropfenweise unter Rühren zu einem Gemisch von 3,69 g (15 mMol) 5-(Benzyloxy)-2-(hydroxymethyl)-3-methyl-4(3H)-pyrimidinon, 3,26 g (20 mMol) N-Hydroxyphthalimid und 5,24 g (20 mMol) Triphenylphosphin in 100 ml absolutem Dimethylacetamid. Man rührt 2 Stunden bei 0 °C und 16 Stunden bei Raumtemperatur. Anschliessend wird das Lösungsmittel im Hochvakuum bei Raumtemperatur abgezogen. Der Rückstand wird aus Aethanol/Diäthyläther kristallisiert. Nach 18-stündigem Trocknen im Hochvakuum bei Raumtemperatur erhält man 5,5 g N-[[5-(Benzyloxy)-3,4-dihydro-3-methyl-4-oxo-2-pyrimidinyl]methoxy]phthalimid als weisse Kristalle vom Smp. 170 °C.

1,56 g (4 mMol) N-[[5-(Benzyloxy)-3,4-dihydro-3-methyl-4-oxo-2-pyrimidinyl]methoxy]phthalimid werden in 110 ml Dichiormethan gelöst und bei ca. -70 °C unter Rühren tropfenweise mit 0,42 ml (4,4 mMol) Bortribromid versetzt. Nach 4-stündigem Rühren bei ca. -70 °C und 16-stündigem Rühren bei Raumtemperatur wird das Lösungsmittel im Wasserstrahlvakuum bei Raumtemperatur abgezogen. Der Rückstand wird mit ca. 30 ml Wasser versetzt, das unlösliche Produkt abfiltriert und zuerst mit Wasser und anschliessend mit Diäthyläther gewaschen. Nach 18-stündigem Trocknen im Hochvakuum bei Raumtemperatur erhält man 1,1 g N-[(3,4-Dihydro-5-hydroxy-3-methyl-4-oxo-2-pyrimidinyl)methoxy]phthalimid als weisses Pulver, Smp. 172 °C (Zers.).

Zu einer Suspension von 301 mg (1 mMol) N-[(3,4-Dihydro-5-hydroxy-3-methyl-4-oxo-2-pyrimidinyl)-methoxy]phthalimid in 9 ml absolutem Dimethylformamid werden bei 0 °C 92 mg (2 mMol) Methylhydrazin gegeben. Dabei entsteht allmählich eine Lösung, welche 3 Stunden bei Raumtemperatur nachgerührt wird. Man engt im Hochvakuum bei Raumtemperatur ein, wäscht den Rückstand mit Petroläther und gibt schliesslich 10 ml Wasser dazu. Nach guter Durchmischung wird das Ganze filtriert. Das Filtrat wird eingeengt, zuletzt mit Toluol/Aethanol. Nach Kristallisation aus Aethanol erhält man 125 mg 2-[(Aminooxy)-methyl]-5-hydroxy-3-methyl-4(3H)-pyrimidinon als weisse Kristalle vom Smp. 106 °C.

Das analoge Ausgangsmaterial 2-[(Aminooxy)methyl]-5-hydroxy-1-methyl-4(1H)-pyrimidinon erhält man in analoger Weise wie 2-[(Aminooxy)methyl]-5-hydroxy-3-methyl-4(3H)-pyrimidinon. Es fallen als Zwischenprodukte an:

- N-[[5-(Benzyloxy)-1,4-dihydro-1-methyl-4-oxo-2-pyrmidinyl]methoxy]phthalimid (Smp. 154-155 °C; aus Aethanol/Aether)
- N-[(1,4-Dihydro-5-hydroxy-1-methyl-4-oxo-2-pyrimidinyl)methoxy]phthalimid (Smp. 145 °C, Zers.).

Das 2-[(Aminooxy)methyl]-5-hydroxy-1-methyl-4(1H)-pyrimidinon schmilzt, nach Umkristallisieren auf Aethanol, bei 115 °C (Zers.).

Beispiel 28

Herstellung von (6R,7R)-7-[(Z)-2-(2-Amino-4-thiazolyl)-2-[[[p-(hydroxycarbamoyl)benzyl]oxy]imino]-acetamido]-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure

17,7 mg (0,03 mMol) (6R,7R)-7-(2-Amino-4-thiazolglyoxylamido)-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure, 7 mg (0,039 mMol) α-(Aminooxy)-N-hydroxy-p-toluamid und 3,7 mg (0,039 mMol) Methansulfonsäure werden in 0,3 ml absolutem Dimethylacetamid gelöst. Nach 24-stündigem Rühren bei Raumtemperatur wird im Hochvakuum bei Raumtemperatur eingeengt und der Rückstand mit Wasser versetzt. Das ausgefallene Produkt wird abfiltriert, mit Wasser gewaschen und im Hochvakuum bei Raumtemperatur getrocknet. Man erhält 23 mg (6R,7R)-7-[(Z)-2-(2-Amino-4-thiazolyl)-2-[[[p-(hydroxycarbamoyl)benzyl]oxy]imino]acetamido]-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure als beiges Pulver vom Smp. 250 °C (Zers.).
$^1$H NMR (DMSO-$d_6$): Signale u.a. bei δ 2,52 (s, 3H), 3,58 (d, J = 18Hz, 1H), 3,80 (d, J = 18Hz, 1H), 4,38 (d, J = 12,5Hz, 1H), 4,46 (d, J = 12,5Hz, 1H), 5,18 (s, 2H), 5,21 (d, J = 5Hz, 1H), 5,85 (dd, J = 5Hz und J = 8Hz, 1H), 6,72 (s, 1H), 7,24 (s, 2H), 7,39 (s, 1H), 7,43 (d, J = 8Hz, 2H), 7,71 (d, J = 8Hz, 2H), 9,03 (1H), 9,36 (s, 1H), 9,76 (d, J = 8Hz, 1H), 11,20 (s, 1H), 11,68 (s, 1H).

Das als Ausgangsmaterial eingesetzte α-(Aminooxy)-N-hydroxy-p-toluamid kann wie folgt hergestellt werden:

18,9 g (0,1 Mol) 4-Chlormethyl-benzoylchlorid und 13,8 g (0,2 Mol) Hydroxylaminhydrochlorid werden in 200 ml Wasser und 150 ml Diäthyläther suspendiert. Unter Rühren werden 25,2 g (0,3 Mol) Natriumhydrogencarbonat zugegeben. Nach 30 Minuten wird das Produkt abgenutscht und mit Wasser und Diäthyläther gewaschen. Nach Trocknen im Hochvakuum bei Raumtemperatur erhält man 17 g weisses α-Chlor-N-hydroxy-p-toluamid vom Smp. 120-145 °C (Zers.).
$^1$H NMR (DMSO-$d_6$): Signale bei δ 4,80 (s, 2H), 7,51 (d, J = 8Hz, 1H), 7,75 (d, J = 8Hz, 1H), 9,07 (s, 1H), 11,25 (s, 1H).

7,4 g (40 mMol) α-Chlor-N-hydroxy-p-toluamid, 6,52 g (40 mMol) N-Hydroxyphthalimid und 0,28 g Kaliumjodid werden in 50 ml absolutem Dimethylformamid gelöst und unter Rühren mit 5,16 g (40 mMol) N-Aethyldiisopropylamin versetzt. Nach 16-stündigem Rühren bei Raumtemperatur werden 50 ml Diäthyläther zugefügt. Das ausgefallene Produkt wird abgenutscht und mit Diäthyläther gewaschen. Man erhält 7,5 g N-Hydroxy-α-(phthalimidooxy)-p-toluamid als weisses Pulver vom Smp. 202 °C (Zers.).
$^1$H NMR (DMSO-$d_6$): Signale bei δ 5,22 (s, 2H), 7,60 (d, J = 8Hz, 1H), 7,78 (d, J = 8Hz, 1H), 7,87 (s, 4H), 9,08 (s, 1H), 11,28 (s, 1H).

10,0 g (32 mMol) N-Hydroxy-α-(phthalimidooxy)-p-toluamid werden in 80 ml absolutem Dimethylformamid suspendiert und bei -5 °C mit 1,86 g (40 mMol) Methylhydrazin versetzt. Nach 2,5-stündigem Rühren bei Raumtemperatur werden 40 ml Lösungsmittel im Hochvakuum bei Raumtemperatur abdestilliert. Unlösliches Material wird abfiltriert und mit wenig Dimethylformamid gewaschen. Mutterlauge und Waschlösung werden mit 70 ml Aethanol versetzt. Nach 1/2-stündigem Rühren bei 0 °C wird das ausgefallene kristalline Produkt abgenutscht und mit wenig Aethanol gewaschen. Nach Trocknen im Hochvakuum bei Raumtemperatur erhält man 3,9 g α-(Aminooxy)-N-hydroxy-p-toluamid als weisse Kristalle vom Smp. 145 °C (Zers.).
$^1$H NMR (DMSO-$d_6$): Signale u.a. bei δ 4,60 (s, 2H), 6,12 (s, 2H), 7,38 (d, J = 8Hz, 1H), 7,73 (d, J = 8Hz, 1H).

Beispiel 29

Herstellung von (6R,7R)-7-[(Z)-2-(2-Amino-4-thiazolyl)-2-[[(2,5-dichlor-3,4-dihydroxybenzyl)oxy]imino]-acetamido]-3-[[[2-(hydroxycarbamoyl)-5-methyl-s-triazolo[1,5-a]pyrimidin-7-yl]thio]methyl]-8-oxo-5-thia-1-azabicyclo[4.2.0]-oct-2-en-2-carbonsäure

52 mg (0,088 mMol) (6R,7R)-7-(2-Amino-4-thiazolglyoxylamido)-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure und 47 mg (0,11 mMol) 5-[(Aminooxy)methyl]-4,7-dichlor-2,2-diphenyl-1,3-benzodioxol werden in 1 ml absolutem Dimethylacetamid gelöst. Nach 20-stündigem Rühren bei Raumtemperatur wird das Lösungsmittel im Hochvakuum bei Raumtemperatur abgezogen und der Rückstand durch Zugabe von Diäthyläther kristallisiert. Das Produkt wird abfiltriert und nacheinander mit Diäthyläther und Wasser gewaschen. Nach Trocknen im Hochvakuum bei Raumtemperatur erhält man 88 mg (6R,7R)-7-[(Z)-2-(2-Amino-4-thiazolyl)-2-[[(4,7-dichlor-2,2-diphenyl-1,3-benzodioxol-5-yl)methoxy]imino]acetamido]-3-[[[2-(hydroxycarbamoyl)-5-methyl-s-triazolo-[1,5-a]pyrimidin-7-yl]thio]methyl]-8-oxo-5-thia-1-azabicyclo[4.2.0]oct-2-en-2-carbonsäure als gelbes Pulver.
$^1H$ NMR (DMSO-$d_6$): Signale u.a. bei δ 2,57 (s, 3H), 3,46 (d, J=18Hz, 1H), 3,70 (d, J=18Hz, 1H), 4,37 (d, J=12,5Hz, 1H), 4,43 (d, J=12,5Hz, 1H), 5,11 (s, 2H), 5,17 (d, J=5Hz, 1H), 5,81 (dd, J=5Hz und J=8Hz, 1H), 6,76 (s, 1H), 7,14 (s, 1H), 7,24 (s, 2H), 7,40 (s, 1H), 7,5 (10H), 9,34 (s, 1H), 9,73 (d, J=8Hz, 1H), 11,69 (s, 1H).

80 mg (0,08 mMol) (6R,7R)-7-[(Z)-2-(2-Amino-4-thiazolyl)-2-[[(4,7-dichlor-2,2-diphenyl-1,3-benzodioxol-5-yl)methoxy]imino]acetamido]-3-[[[2-(hydroxycarbamoyl)-5-methyl-s-triazolo[1,5-a]pyrimidin-7-yi]thio]methyl]-8-oxo-5-thia-1-azabicyclo[4.2.0]oct-2-en-2-carbonsäure werden in 2 ml Trifluoressigsäure gelöst. Nach 16-stündigem Rühren bei Raumtemperatur wird das Lösungsmittel bei Raumtemperatur im Vakuum abgezogen und der Rückstand mit Diäthyläther kristallisiert. Das Produkt wird abfiltriert, mit Diäthyläther gewaschen und im Hochvakuum bei Raumtemperatur getrocknet. Man erhält 50 mg (6R,7R)-7-[(Z)-2-(2-Amino-4-thiazolyl)-2-[[(2,5-dichlor-3,4-dihydroxybenzyl)oxy]imino]acetamido]-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure als beiges Pulver.
$^1H$ NMR (DMSO-$d_6$): Signale u.a. bei δ 2,63 (s, 3H), 3,54 (d, J=18Hz, 1H), 3,77 (d, J=18Hz, 1H), 4,40 (d, J=12,5Hz, 1H), 4,47 (d, J=12,5Hz, 1H), 5,08 (s, 2H), 5,18 (d, J=5Hz, 1H), 5,81 (dd, J=5Hz und J=8Hz, 1H), 6,76 (s, 1H), 6,95 (s, 1H), 7,24 (s, 2H), 7,40 (s, 1H), 9,36 (s, 1H), 9,6-10,0 (2H), 9,74 (d, J=8Hz, 1H), 11,69 (s, 1H).

Das als Ausgangsmaterial eingesetzte 5-[(Aminooxy)methyl]-4,7-dichlor-2,2-diphenyl-1,3-benzodioxol kann wie folgt hergestellt werden:

1,1 g (ca. 4,6 mMol) 2,5-Dichlor-3,4-dihydroxybenzoesäuremethylester und 2,0 g (8,4 mMol) Dichlordiphenylmethan werden zusammen auf 150 °C erwärmt. Die Schmelze wird nach 1,5-stündigem Rühren abgekühlt und das Produkt aus Diäthyläther kristallisiert. Man erhält 1,3 g 4,7-Dichlor-2,2-diphenyl-1,3-benzodioxol-5-carbonsäuremethylester als weisse Kristalle vom Smp. 112 °C.

In einem anderen Versuch wird das Produkt aus Aethanol kristallisiert. Smp. 69-70 °C.

0,4 g (1 mMol) 4,7-Dichlor-2,2-diphenyl-1,3-benzodioxol-5-carbonsäuremethylester werden in 15 ml absolutem Tetrahydrofuran gelöst. Bei 0 °C werden unter Argon 5 ml einer 1M Lösung von Diisobutylaluminiumhydrid in Toluol zugetropft. Nach 4-stündigem Rühren bei 0 °C gibt man unter Kühlung gesättigte, wässrige Ammoniumchloridlösung dazu, bis pH 5 erreicht ist. Unlösliches Material wird abgenutscht und mit Diäthyläther und Methanol intensiv extrahiert. Nach Einengen der Extrakte verbleiben 0,3 g 4,7-Dichlor-2,2-diphenyl-1,3-benzodioxol-5-methanol als grünlich gefärbtes Oel.
$^1H$ NMR ($CDCl_3$): Signale bei δ 1,8 (breit, 1H), 4,67 (d, J=3Hz, 2H), 6,98 (s, 1H), 7,4 (6H), 7,6 (4H).

250 mg (0,67 mMol) 4,7-Dichlor-2,2-diphenyl-1,3-benzodioxol-5-methanol,108 mg (0,67 mMol) N-Hydroxyphthalimid und 175 mg (0,67 mMol) Triphenylphosphin werden in 20 ml absolutem Tetrahydrofuran suspendiert. Bei 0 °C werden unter Rühren 128 mg (0,73 mMol) Azodicarbonsäurediäthylester zugetropft. Das Ganze wird 1 Stunde bei 0 °C und 24 Stunden bei Raumtemperatur gerührt. Anschliessend wird das Lösungsmittel im Hochvakuum abgezogen. Der Rückstand wird aus Aethanol/Diäthyläther kristallisiert und umkristallisiert. Man erhält 230 mg N-[(4,7-Dichlor-2,2-diphenyl-1,3-benzodioxol-5-yl)methoxy]phthalimid als weisse Kristalle vom Smp. 127-129 °C.

Zu einer Lösung von 530 mg (1,02 mMol) N-[(4,7-Dichlor-2,2-diphenyl-1,3-benzodioxol-5-yl)methoxy]-phthalimid in 10 ml absolutem Dimethylformamid werden bei 0 °C 47 mg (1,02 mMol) Methylhydrazin zugegeben. Das Ganze wird 1 Stunde bei 0 °C und 4 Stunden bei Raumtemperatur gerührt. Das Lösungsmittel wird im Hochvakuum bei Raumtemperatur abgezogen und der Rückstand an 30 g Kieselgel (0,063-0,2 mm) chromatographiert. Das Produkt wird mit Dichlormethan/Aethanol (19:1 v/v) eluiert. Man erhält 310 mg 5-[(Aminooxy)methyl]-4,7-dichlor-2,2-diphenyl-1,3-benzodioxol als farbloses Oel.
$^1H$ NMR ($CDCl_3$): Signale bei δ 4,75 (s, 2H), 6,97 (s, 1H), 7,4 (6H), 7,6 (4H).

305 mg dieses Amins werden mit äthanolischer Chlorwasserstofflösung versetzt. Nach Zugabe von Diäthyläther kristallisiert das Aminhydrochlorid aus. Man erhält 310 mg 5-[(Aminooxy)methyl]-4,7-dichlor-2,2-diphenyl-1,3-benzodioxol-hydrochlorid als weisse Kristalle vom Smp. 133-134 °C, nach Umkristallisation aus Aethanol/Diäthyläther.

Beispiel 30

Herstellung von (6R,7R)-7-[(Z)-2-Amino-4-thiazolyl)-2-[[[(3,4-dihydroxyphenyl)sulfonyl]methoxy]imino]-acetamido]-3-[[[5-(hydroxycarbamoyl)-2-methyl-s-triazolo[1,5-a]pyrimidin-7-yl]thio]methyl]-8-oxo-5-thia-1-azabicyclo-[4.2.0]oct-2-en-2-carbonsäure

65 mg (0,11 mMol) (6R,7R)-7-(2-Amino-4-thiazolglyoxylamido)-3-[[[5-(hydroxycarbamoyl)-2-methyl-s-triazolo[1,5-a]pyrimidin-7-yl]thio]methyl]-8-oxo-5-thia-1-azabicyclo[4.2.0]oct-2-en-2-carbonsäure und 37 mg (0,14 mMol) 4-[[(Aminooxy)methyl]sulfonyl]pyrocatechol-hydrochlorid werden in 3 ml absolutem Dimethylacetamid gelöst. Nach 24 stündigem Rühren bei Raumtemperatur werden noch 4,2 mg (0,016 mMol) 4-[[-(Aminooxy)methyl]sulfonyl]pyrocatechol-hydrochlorid zugefügt. Nach weiteren 24 Stunden wird das Lösungsmittel im Hochvakuum bei Raumtemperatur abgezogen und der Rückstand mit Wasser versetzt. Das ausgefallene Produkt wird abfiltriert und nacheinander mit Wasser und Diäthyläther gewaschen. Nach Trocknen im Hochvakuum erhält man 55 mg (6R,7R)-7-[(Z)-2-Amino-4-thiazolyl)-2-[[[(3,4-dihydroxyphenyl)-sulfonyl]methoxy]imino]acetamido]-3-[[[5-(hydroxycarbamoyl)-2-methyl-s-triazolo[1,5-a]pyrimidin-7-yl]thio]-methyl]-8-oxo-5-thia-1-azabicyclo[4.2.0]oct-2-en-2-carbonsäure als beiges Pulver.
$^1$H NMR (DMSOd$_6$): Signale u.a. bei δ 2,54 (s, 3H), 3,55 (d, J = 18 Hz, 1H), 3,76 (d, J = 18 Hz, 1H), 4,46 (d, J = 12,5 Hz, 1H), 4,54 (d, J = 12,5 Hz, 1H), 5,11 (d, J = 5 Hz, 1H), 5,15 (d, J = 15 Hz, 1H), 5,25 (d, J = 15 Hz, 1H), 5,75 (dd, J = 5 Hz und J = 8 Hz, 1H), 6,69 (s, 1H), 6,91 (d, J = 8 Hz, 1H), 7,2 (2H), 7,29 (s, 2H), 7,74 (s, 1H), 9,34 (s, 1H), 9,66 (s, 1H), 9,73 (d, J = 8 Hz, 1H), 10,09 (s, 1H), 11,88 (s, 1H).

Die als Ausgangsverbindung eingesetzte (6R,7R)-7-(2-Amino-4-thiazolglyoxylamido)-3-[[[5-(hydroxycarbamoyl)-2-methyl-s-triazolo[1,5-a]pyrimidin-7-yl]thio]methyl]-8-oxo-5-thia-1-azabicyclo[4.2.0]oct-2-en-2-carbonsäure kann wie folgt hergestellt werden:

112 mg 3-Amino-5-methyl-1H-s-triazol und 320 mg 2-Oxo-bernsteinsäuredimethylester werden in 5 ml Eisessig gelöst. Nach 22-stündigem Kochen unter Rückfluss wird das Lösungsmittel im Vakuum abdestilliert. Der Rückstand wird in warmem Methanol aufgeschlämmt. Nach dem Abkühlen wird das Produkt abfiltriert und mit Methanol mehrmals gewaschen. Nach Trocknen im Hochvakuum erhält man 73 mg Methyl-7-hydroxy-2-methyl-s-triazolo[1,5-a]pyrimidin-5-carboxylat als beiges Pulver, Smp. >220 °C).
$^1$H NMR (DMSOd$_6$): Signale u.a. bei δ 2,44 (s, 3H), 3,88 (s, 3H), 6,54 (s, 1H).

1,0 g (4,8 mMol Methyl-7-hydroxy-2-methyl-s-triazolo[1,5-a]pyrimidin-5-carboxylat wird in 30 ml Phosphoroxychlorid suspendiert. Man gibt 0,38 ml (4,8 mMol) Pyridin dazu und rührt 5 Stunden bei 90 °C. Anschliessend engt man ein und gibt zum Rückstand Eis/Wasser und gesättigte wässrige Kochsalzlösung. Das Produkt wird mit Dichlormethan extrahiert. Nach Trocknen und Eindampfen des Extraktes verbleiben 0,81 g Methyl 7-chlor-2-methyl-s-triazolo[1,5-a]pyrimidin-5-carboxylat als beige Kristalle mit Schmelzpunkt 170-174 °C.
$^1$H NMR (DMSOd$_6$): Signale u.a. bei δ 2,61 (s, 3H), 3,97 (s, 3H), 8,08 (s, 1H).

0,80 g (3,53 mMol) Methyl-7-chlor-2-methyi-s-triazolo[1,5-a]pyrimidin-5-carboxylat werden in 35 ml Methanol suspendiert und mit 0,78 g (10,6 mMol) Natriumhydrogensulfidhydrat versetzt. Nach 5-stündigem Rühren bei 60 °C wird eingeengt und der Rückstand in 20 ml Wasser gelöst. Man säuert bis zu pH 3 mit 3N wässriger Salzsäure an. Das ausgefallene Produkt wird abgenutscht und mit Wasser und Methanol gewaschen. Nach Umkristallisation aus Methanol/Dichlormethan erhält man 0,66 g Methyl-7-mercapto-2-methyl-s-triazolo[1,5-a]pyrimidin-5-carboxylat als gelbe Kristalle vom Schmelzpunkt 237 °C (Zers.).
$^1$H NMR (DMSOd$_6$): Signale u.a. bei δ 2,54 (s, 3H), 3,88 (s, 3H), 7,51 (s, 1H).

500 mg (2,23 mMol) Methyl-7-mercapto-2-methyl-s-triazolo[1,5-a]pyrimidin-5-carboxylat werden in 10 ml absolutem Methanol suspendiert. Innerhalb 4 Tage werden unter Rühren 2,95 mg (8,9 mMol) Hydroxylamin zugegeben. Das Reaktionsgemisch wird mit 3N wässriger Salzsäure angesäuert (pH 6). Das Produkt wird abgenutscht und nacheinander mit Wasser, Methanol und Diäthyläther gewaschen. Man erhält 540 mg N-Hydroxy-7-mercapto-2-methyl-s-triazolo[1,5-a]pyrimidin-5-carboxamid als gelbe Kristalle vom Schmelzpunkt 178-179 °C.
$^1$H NMR (DMSOd$_6$): Signale u.a. bei δ 2,38 (s, 3H), 7,21 (s, 1H).

250 mg (1,1 mMol) N-Hydroxy-7-mercapto-2-methyl-s-triazolo[1,5-a]pyrimidin-5-carboxamid und 275 mg (1,0 mMol) 7-Amino-cephalosporinsäure werden in 15 ml Sulfolan/Dichlormethan (1:1 v/v) suspendiert und bei 0 °C mit 3,5 ml Bortrifluorid-diäthylätherat versetzt. Man rührt 2 Stunden bei 0-5 °C und 3 Stunden bei Raumtemperatur. Anschliessend fügt man 60 ml Dichlormethan zu. Unlösliches Material wird abgenutscht, in Wasser gelöst und mit gesättigter Natriumbicarbonatlösung versetzt, bis der pH-Wert 3 erreicht ist. Das ausgefallene Produkt wird abgenutscht und nacheinander mit Wasser und Diäthyläther gewaschen. Man erhält 295 mg (6R,7R)-7-Amino-3-[[[5-(hydroxycarbamoyl)-2-methyl-s-triazolo[1,5-a]pyrimidin-7-yl]thio]-methyl]-8-oxo-5-thia-1-azabicyclo[4.2.0]oct-2-en-2-carbonsäure als beiges Pulver.
$^1$H NMR (DMSOd$_6$): Signale u.a. bei δ 2,54 (s, 3H), 3,56 (d, J = 18 Hz, 1H), 3,78 (d, J = 18 Hz, 1H), 4,45 (d, J = 12,5 Hz, 1H), 4,51 (d, J = 12,5 Hz, 1H), 4,82 (d, J = 5 Hz, 1H), 4,98 (d, J = 5 Hz, 1H), 7,72 (s, 1H), 9,34 (s, 1H), 11,88 (s, 1H).

160 mg (0,36 mMol) (6R,7R)-7-Amino-3-[[[5-(hydroxycarbamoyl)-2-methyl-s-triazolo[1,5-a]pyrimidin-7-yl]thio]methyl]-8-oxo-5-thia-1-azabicyclo[4.2.0]oct-2-en-2-carbonsäure werden in 2 ml absolutem Dimethylacetamid suspendiert. Bei 0 °C gibt man 146 mg (0,72 mMol) O,N-Bistrimethylsilyl-acetamid dazu und rührt

2 Stunden. Die Lösung wird anschliessend mit 116 mg (0,36 mMol) 2-Amino-4-thiazol-thioglyoxylsäure-S-(2-benzothiazolyl)ester. versetzt und 2 Stunden bei 0°C und 16 Stunden bei Raumtemperatur gerührt. Man gibt etwas Aethanol dazu und engt nach 20 Minuten im Hochvakuum bei Raumtemperatur ein. Der Rückstand wird mit Wasser versetzt. Das ausgefallene Produkt wird abfiltriert und nacheinander mit Wasser, Aethanol, Essigester und Diäthyläther gewaschen. Man erhält 170 mg (6R,7R)-7-(2-Amino-4-thiazolglyoxylamido)-3-[[[5-(hydroxycarbamoyl)-2-methyl-s-triazolo[1,5-a]pyrimidin-7-yl]thio]methyl]-8-oxo-5-thia-1-azabicyclo[4.2.0]oct-2-en-2-carbonsäure als beiges Pulver.
$^1$H NMR (DMSOd$_6$): Signale u.a. bei δ 2,54 (s, 3H), 3,62 (d, J=18 Hz, 1H), 3,81 (d, J=18 Hz, 1H), 4,47 (d, J=12,5 Hz, 1H), 4,60 (d, J=12,5 Hz, 1H), 5,19 (d, J=5 Hz, 1H), 5,79 (dd, J=5 Hz und J=8 Hz, 1H), 7,41 (s, 2H), 7,74 (s, 1H), 7,82 (s, 1H), 9,34 (s, 1H), 9,80 (d, J=8 Hz, 1H), 11,89 (s, 1H).

Beispiel 31

Herstellung von (6R,7R)-7-[(Z)-2-(2-Amino-4-thiazolyl)-2-[[(1,4-dihydro-5-hydroxy-4-oxo-2-pyrimidinyl)-methoxy]imino]acetamido]-3-[[[5-(hydroxycarbamoyl)-2-methyl-s-triazolo[1,5-a]pyrimidin-7-yl]thio]methyl]-8-oxo-5-thia-1-azabicyclo[4.2.0]oct-2-en-2-carbonsäure

65 mg (0,11 mMol) (6R,7R)-7-(2-Amino-4-thiazolglyoxylamido)-3-[[[5-(hydroxycarbamoyl)-2-methyl-s-triazolo[1,5-a]pyrimidin-7-yl]thio]methyl]-8-oxo-5-thia-1-azabicyclo[4.2.0]oct-2-en-2-carbonsäure, 22,5 mg (0,143 Mol) 2-(Aminooxy)methyl-5-hydroxy-4(1H)-pyrimidinon und 27 mg (0,143 mMol) p-Toluolsulfonsäurehydrat werden in 2 ml absolutem Dimethylacetamid gelöst. Nach 24-stündigem Rühren bei Raumtemperatur wird das Lösungsmittel im Hochvakuum abgezogen und der Rückstand mit Wasser versetzt. Das ausgefallene Produkt wird abfiltriert und nacheinander mit Wasser und Diäthyläther gewaschen. Man erhält nach Trocknen im Hochvakuum 55 mg (6R,7R)-7-[(Z)-2-(2-Amino-4-thiazolyl)-2-[[(1,4-dihydro-5-hydroxy-4-oxo-2-pyrimidinyl)methoxy]imino]acetamido]-3-[[[5-(hydroxycarbamoyl)-2-methyl-s-triazolo[1,5-a]pyrimidin-7-yl]thio]methyl]-8-oxo-5-thia-1-azabicyclo[4.2.0]oct-2-en-2-carbonsäure als beiges Pulver.
$^1$H NMR (DMSOd$_6$): Signale u.a. bei δ 2,54 (s, 3H), 3,55 (d, J=18 Hz, 1H), 3,84 (d, J=18 Hz, 1H), 4,46 (d, J=12,5 Hz, 1H), 4,56 (d, J=12,5 Hz, 1H), 4,91 (s, 2H), 5,20 (d, J=5 Hz, 1H), 5,85 (dd, J=5 H und J=8 Hz, 1H), 6,85 (s, 1H), 7,38 (s, 1H), 7,73 (s, 1H), 9,90 (d, J=8 Hz, 1H), 11,88 (s, 1H).

Beispiel 32

Herstellung von (6R,7R)-7-[(Z)-2-(2-Amino-4-thiazolyl)-2-[(1-hydroxycarbamoyl)-1-methyläthoxy]imino]-acetamido]-3-[[5-(3,4-dihydroxyphenyl)-1H-tetrazol-1-yl]methyl]-8-oxo-5-thia-1-azabicyclo[4.2.0]oct-2-en-2-carbonsäure-Natriumsalz (1:1)

160 mg (6R,7R)-7-(2-Amino-4-thiazolglyoxylamido)-3-[[5-(3,4-dihydroxyphenyl)-1H-tetrazol-1-yl]methyl]-8-oxo-5-thia-1-azabicyclo[4.2.0]oct-2-en-2-carbonsäure und 80 mg 2-(Aminooxy)-N-hydroxy-2-methylpropionamidhydrochlorid werden in 2 ml Dimethylacetamid 24 Stunden bei Raumtemperatur gerührt. Das Dimethylacetamid wird am Hochvakuum abgedampft. Der Rückstand wird mit 3 ml Wasser versetzt. Der entstandene Niederschlag wird abfiltriert, mit wenig kaltem Wasser gewaschen und in etwa 3 ml Wasser resuspendiert. Der pH-Wert wird mit 1N wässriger Natronlauge auf 7 gestellt, worauf alles in Lösung geht. Die Lösung wird an Opti-up $C_{12}$® mit Wasser und 5% Acetonitril chromatographiert. Die einheitlichen Produktfraktionen werden eingeengt und lyophilisiert. Man erhält 91 mg (6R,7R)-7-[(Z)-2-(2-Amino-4-thiazolyl)-2-[(1-hydroxycarbamoyl)-1-methyläthoxy]imino]acetamido]-3-[[5-(3,4-dihydroxyphenyl)-1H-tetrazol-1-yl]-methyl]-8-oxo-5-thia-1-azabicyclo[4.2.0]oct-2-en-2-carbonsäure-Natriumsalz (1:1) als fast weisses Pulver.
$^1$H-NMR (DMSO-d$_6$, 250 MHz): Signale bei δ(ppm) 1, 43 (s, 3H), 1,46 (s, 3H), 3,29 (d, J=17 Hz, 1H) 3,48 (d, J=17 Hz, 1H), 5,05 (d, J=5 Hz, 1H), 5,16 (d, J=14 Hz, 1H), 5,74 (d,d, $J_1$=5 Hz, $J_2$=8 Hz, 1H), 5,79 (d, J=14 Hz, 1H), 6,82 (s, 1H), 6,88 (d, J=8 Hz, 1H), 7,15 (m, 2H), 7,35 (s, 2H), 8,90 (s, 1H), 9,40 (s, breit, 1H), 9,64 (d, J=8 Hz, 1H), 10,14 (s, 1H), 10,70 (s, breit, 1H).
IR: (KBr) 1771 $cm^{-1}$

Die als Ausgangsmaterial eingesetzte (6R,7R)-7-(2-Amino-4-thiazolglyoxylamido)-3-[[5-(3,4-dihydroxyphenyl)-1H-tetrazol-1-yl]methyl]-8-oxo-5-thia-1-azabicyclo[4.2.0]oct-2-en-2-carbonsäure kann wie folgt hergestellt werden:

70 g 5-Brom-2,2-diphenyl-1,3-benzodioxol werden in 440 ml Tetrahydrofuran bei -78°C mittels 125 ml einer 1,6M Lösung von Butyllithium in n-Hexan versetzt. Nach 10 Minuten gibt man 40 ml Dimethylformamid zu und lässt auf -10°C erwärmen. Danach gibt man 70 ml 20% wässrige Phosphorsäure und 500 ml Aethylacetat zu, trennt die organische Phase ab und dampft sie ein. Der Rückstand wird aus 130 ml

Aethanol umkristallisiert. Man erhält 49,5 g 2,2-Diphenyl-1,3-benzodioxol-5-carbaldehyd.

48,66 g 2,2-Diphenyl-1,3-benzodioxol-5-carbaldehyd und 22,40 g Hydroxylaminhydrochlorid werden in 130 ml Dimethylformamid 1 Tag bei Raumtemperatur gerührt. Das Dimethylformamid wird am Hochvakuum abgezogen. Der Rückstand wird zwischen Wasser und Aethylacetat verteilt. Die organische Phase wird mit Wasser und gesättigter Kochsalzlösung getrocknet, mit Magnesiumsulfat getrocknet und eingeengt. Das feste Produkt wird aus 230 ml Aethanol umkristallisiert. Man erhält 40,39 g 2,2-Diphenyl-1,3-benzodioxol-5-carboxaldehyd-(E und/oder Z)-oxim als weisse Kristalle vom Schmelzpunkt 157-159 °C.

6,00 g 2,2-Diphenyi-1,3-benzodioxol-5-carboxaldehyd-(E und/oder Z)-oxim werden in 5 ml Essigsäureanhydrid 1 Stunde am Rückfluss erhitzt. Das Reaktionsgemisch wird eingeengt und anschliessend bei 160 °C am Hochvakuum destilliert. Das feste Produkt wird aus Aethanol umkristallisiert. Man erhält 4,98 g 2,2-Diphenyl-1,3-benzodioxol-5-carbonitril als weisse Kristalle vom Schmelzpunkt 106-107 °C.

4,79 g 2,2-Diphenyl-1,3-benzodioxol-5-carbonitril werden mit 1,71 g Ammoniumchlorid und 2,08 g Natriumazid 5 Stunden bei 160 °C erhitzt. Das Reaktionsgemisch wird auf Raumtemperatur abgekühlt und bei pH 3 zwischen Wasser und Aethylacetat verteilt. Die organische Phase wird mit Wasser geschüttelt und der pH-Wert mit 1N wässriger Natronlauge auf 10 gestellt. Die wässrige Phase (pH 10) wird abgetrennt, mit Aethylacetat geschüttelt und der pH-Wert auf 4 eingestellt. Es bildet sich ein weisser Niederschlag. Dieser wird abfiltriert, getrocknet und aus 240 ml Acetonitril umkristallisiert. Man erhält 3,84 g 5-(2,2-Diphenyl-1,3-benzodioxol-5-yl)- 1H-tetrazol als weisse Kristalle vom Schmelzpunkt 212-215 °C/Zers.

2,57 g 5-(2,2-Diphenyl-1,3-benzodioxol-5-yl)-1H-tetrazol und 4,10 g 7-Aminocephalosporansäure werden in 25 ml Sulfolan gelöst, mit 7,50 ml Bortrifluoriddiäthylätherat versetzt und über Nacht bei Raumtemperatur gerührt. Das Reaktionsgemisch wird zwischen Wasser und Aethylacetat verteilt, der pH-Wert auf 7,0 eingestellt und die wässrige Phase mit Wasser und 10% Acetonitril an Opti-up $C_{12}$® chromatographiert. Man erhält nach Fällung bei pH 2,5 2,45 g beiges Produkt als Isomerengemisch. Dieses wird in 14 ml Wasser:Acetonitril 1:1 mit 0,63 ml Triäthylamin in Lösung gebracht und bei 0 °C mit 2,19 g 2-Amino-4-thiazol-thioglyoxylsäure-S-(2-benzothiazolyl)ester 3 Stunden gerührt. Das Reaktionsgemisch wird zentrifugiert und der klare gelbe Ueberstand zwischen Wasser und Aethylacetat verteilt (pH etwa 7,5). Der pH-Wert der wässrigen Phse wird auf 2,5 gestellt, wobei ein feiner gelber Niederschlag entsteht. Dieser wird abfiltriert, noch feucht in 20 ml Trifluoressigsäure gelöst und etwa 1 Stunde bei 0 °C gerührt. Die Trifluoressigsäure wird am Wasserstrahlvakuum abgedampft und der Rückstand bei pH 7,5 zwischen Wasser und Aethylacetat verteilt. Die wässrige Phase wird an Opti-up $C_{12}$® mit Wasser, 5% wässrigem Acetonitril und 10% wässrigem Acetonitril chromatographiert. Man erhält als polare Komponente (erste Fraktion) nach dem Fällen bei pH 2 305 mg (6R,7R)-7-(2-Amino-4-thiazolglyoxylamido)-3-[[5-(3,4-dihydroxyphenyl)-1H-tetrazol-1-yl]methyl]-8-oxo-5-thia-1-azabicyclo[4.2.0]oct-2-en-2-carbonsäure als gelben Feststoff.

$^1$H-NMR (DMSO-$d_6$): Signale bei δ(ppm): 3,44 (m, 2H), 5,14 (d, J=5Hz, 1H), 5,47 (d, J=15 Hz, 1H), 5,56 (d, J=15 Hz, 1H), 5,80 (d,d, J=5 Hz, J=8 Hz, 1H), 6,90 (d, J=7 Hz, 1H), 7,02 (d,d, J=7 Hz, J=3 Hz, 1H), 7,15 (d, J=3 Hz, 1H), 7,39 (s, 2H), 7,80 (s, 1H), 9,50 (s, breit, 1H), 9,69 (s, 1H), 9,82 (d, J=8 Hz, 1H), 14,0 (s, breit, 1H).

Man erhält als weniger polare Komponente (zweite Fraktion) nach Fällen bei pH 2 213 mg (6R,7R)-7-(2-Amino-4-thiazolglyoxylamido)-3-[[5-(3,4-dihydroxyphenyl)-2H-tetrazol-2-yl]methyl]-8-oxo-5-thia-1-azabicyclo-[4.2.0]oct-2-en-2-carbonsäure als gelben Feststoff.

$^1$H-NMR (DMSO-$d_6$): Signale bei δ(ppm): 3,54 (m, 2H), 5,20 (d, J=5 Hz, 1H), 5,57 (d, J=15 Hz, 1H), 5,82 (m, 3H), 6,87 (d, J=8 Hz, 1H), 7,36 (d,d, J=8 Hz, J=3 Hz, 1H), 7,39 (s, 2H), 7,45 (d, J=3 Hz, 1H), 7,81 (s, 1H), 9,35 (s, 1H), 9,44 (s, 1H), 9,82 (d, J=8 Hz, 1H), 14,0 (s, breit, 1H).

## Beispiel 33

Herstellung von (6R,7R)-7-[(Z)-2-(2-Amino-4-thiazolyl)-2-[[(2-fluor-3,4-dihydroxybenzyl)oxy]imino]acetamido]-3-[[5-(3,4-dihydroxyphenyl)-1H-tetrazol-1-yl]methyl]-8-oxo-5-thia-1-azabicyclo[4.2.0]oct-2-en-2-carbonsäure-Natriumsalz (1:1)

50 mg (6R,7R)-7-(2-Amino-4-thiazolglyoxylamido)-3-[[5-(3,4-dihydroxyphenyl)-1H-tetrazol-1-yl]methyl]-8-oxo-5-thia-1-azabicyclo[4.2.0]oct-2-en-2-carbonsäure und 67 mg O-[(4-Fluor-2,2-diphenyl-1,3-benzodioxol-5-yl)methyl]hydroxylamin-hydrochlorid werden in 2 ml Dimethylacetamid gelöst und 24 Stunden bei Raumtemperatur gerührt. Das Lösungsmittel wird am Hochvakuum abgedampft. Der Rückstand wird mit 2 ml Wasser versetzt. Der gebildete Niederschlag wird abfiltriert, in 1 ml Trifluoressigsäure gelöst und bei 0 °C 1 Stunde gerührt. Die Trifluoressigsäure wird am Rotationsverdampfer abgedampft. Der Rückstand wird zwischen Wasser und Aethylacetat bei pH 7 verteilt. Die wässrige Phase wird an Opti-up $C_{12}$® mit Wasser

und 5% Acetonitril chromatographiert. Die einheitlichen Produktfraktionen werden eingeengt und lyophilisiert. Man erhält 18 mg (6R,7R)-7-[(Z)-2-(2-Amino-4-thiazolyl)-2-[[(2-fluor-3,4-dihydroxybenzyl)oxy]imino]-acetamido]-3-[[5-(3,4-dihydroxyphenyl)-1H-tetrazol-1-yl]methyl]-8-oxo-5-thia-1-azabicyclo[4.2.0]oct-2-en-2-carbonsäure-Natriumsalz (1:1) als weisses Lyophilisat.
$^1$H-NMR (DMSO-$d_6$, 250 MHz): Signale bei δ(ppm) 3,22 (d, J = 17 Hz, 1H), 3,42 (d, J = 17 Hz, 1H), 4,96 (d, J = 5 Hz, 1H), 5,04 (s, 2H), 5,12 (d, J = 15 Hz, 1H), 5,64 (d,d, J = 5 Hz, J = 8 Hz, 1H), 5,79 (d, J = 15 Hz, 1H), 6,55 (m, 1H), 6,74 (m, 2H), 6,87 (d, J = 7 Hz, 1H), 7,12 (m, 1H), 7,17 (m, 1H), 7,25 (s, 2H), 9,61 (d, J = 8 Hz, 1H).
IR: (KBr) 1768 $cm^{-1}$

Beispiel 34

Herstellung von (6R,7R)-7-[(Z)-2-(2-Amino-4-thiazolyl)-2-[[(3,4-dihydroxybenzyl)oxy]imino]acetamido]-3-[[5-(3,4-dihydroxyphenyl)-1H-tetrazol-1-yl]methyl]-8-oxo-5-thia-1-azabicyclo[4.2.0]oct-2-en-2-carbonsäure

Diese Verbindung wird in Analogie zu Beispiel 33 aus (6R,7R)-7-(2-Amino-4-thiazolglyoxylamido)-3-[[5-(3,4-dihydroxyphenyl)-1H-tetrazol-1-yl]methyl]-8-oxo-5-thia-1-azabicyclo[4.2.0]oct-2-en-2-carbonsäure und O-[3,4-[(Diphenylmethylen)dioxy]benzyl]hydroxylamin hergestellt.
$^1$H-NMR (DMSO-$d_6$, 250 MHz): Signale bei δ(ppm) 3,40 (m, 2H), 4,92 (s, 2H), 5,10 (d, J = 5Hz, 1H), 5,33 (d, J = 14 Hz, 1H), 5,52 (d, J = 14 Hz, 1H), 5,80 (d,d, J = 5 Hz, J = 8 Hz, 1H), 6,7 (m, 4H), 6,9 (m, 1H), 7,0 (m, 1H), 7,15 (m, 1H), 7,23 (s, 2H), 8,86 (s, 1H), 8,89 (s, 1H), 9,50 (s, 1H), 9,68 (d, J = 8 Hz, 1H), 9,71 (s, 1H).

Beispiel 35

Herstellung von (6R,7R)-7-[(Z)-2-(2-Amino-4-thiazolyl)-2-[[(1,6-dihydro-5-hydroxy-6-oxo-2-pyrimidinyl)-methoxy]imino]acetamido]-3-[[5-(3,4-dihydroxyphenyl)-1H-tetrazol-1-yl]methyl]-8-oxo-5-thia-1-azabicyclo-[4.2.0]oct-2-en-2-carbonsäure-Natriumsalz (1:1)

Diese Verbindung wird in Analogie zu Beispiel 32 aus (6R,7R)-7-(2-Amino-4-thiazolglyoxylamido)-3-[[5-(3,4-dihydroxyphenyl)-1H-tetrazol-1-yl]methyl]-8-oxo-5-thia-1-azabicyclo[4.2.0]oct-2-en-2-carbonsäure, 2-(Aminooxy)methyl-5-hydroxy-4(1H)-pyrimidinon und Methansulfonsäure hergestellt.
$^1$H-NMR (DMSO-$d_6$, 250 MHz): Signale bei δ(ppm) 3,26 (s, J = 17 Hz, 1H), 3,42 (d, J = 17 Hz, 1H), 4,94 (s, 2H), 5,02 (d, J = 5 Hz, 1H), 5,14 (d, J = 15 Hz, 1H), 5,70 (d,d, J = 5 Hz, J = 8 Hz, 1H), 5,78 (d, J = 15 Hz, 1H), 6,87 (m, 2H), 7,14 (m, 2H), 7,29 (s, 2H), 7,40 (s, 1H), 9,93 (d, J = 8 Hz, 1H).
IR: (KBr) 1774 $cm^{-}$.

Beispiel 36

Wird in den Beispielen 32-35 (6R,7R)-7-(2-Amino-4-thiazolglyoxylamido)-3-[[5-(3,4-dihydroxyphenyl)-2H-tetrazol-2-yl]methyl]-8-oxo-5-thia-1-azabicyclo[4.2.0]oct-2-en-2-carbonsäure anstelle von (6R,7R)-7-(2-Amino-4-thiazolglyoxylamido)-3-[[5-(3,4-dihydroxyphenyl)-1H-tetrazol-1-yl]methyl]-8-oxo-5-thia-1-azabicyclo-[4.2.0]oct-2-en-2-carbonsäure eingesetzt, so erhält man unter sonst gleichen Bedingungen:

(6R,7R)-7-[(Z)-2-(2-Amino-4-thiazolyl)-2-[(1-(hydroxycarbamoyl)-1-methyläthoxy]imino]acetamido]-3-[[5-(3,4-dihydroxyphenyl)-2H-tetrazol-2-yl]methyl]-8-oxo-5-thia-1-azabicyclo[4.2.0]oct-2-en-2-carbonsäure-Natriumsalz (1:1):
$^1$H-NMR (DMSO-$d_6$, 250 MHz): Signale bei δ(ppm) 1,40 (s, 3H), 1,42 (s, 3H), 3,28 (m, 2H), 5,04 (d, J = 5 Hz, 1H), 5,47 (d, J = 14 Hz, 1H), 5,18 (d,d, J = 5 Hz, J = 8 Hz, 1H), 6,18 (d, J = 14 Hz, 1H), 6,80 (s, 1H), 6,87 (d, J = 7 Hz, 1H), 7,35 (m, 3H), 7,48 (m, 1H), 8,4 (s, 1H), 9,45 (s, breit, 1H), 9,5 (s, breit, 1H), 9,60 (d, J = 8 Hz, 1H), 10,12 (s, breit, 1H).
IR: (KBr) 1769 $cm^{-1}$.

(6R,7R)-7-[(Z)-2-(2-Amino-4-thiazolyl)-2-[[(3,4-dihydroxybenzyl)oxy]imino]acetamido]-3-[[5-(3,4-dihydroxyphenyl)-2H-tetrazol-2-yl]methyl]-8-oxo-5-thia-1-azabicyclo[4.2.0]oct-2-en-2-carbonsäure:
$^1$H-NMR (DMSO-$d_6$, 250 MHz): Signale bei δ(ppm) 3,48 (m, 2H), 4,93 (s, 2H), 5,16 (d, J = 5 Hz, 1H), 5,64 (d, J = 14 Hz, 1H), 5,80 (d,d, J = 5 Hz, J = 8 Hz, 1H), 5,82 (d, J = 14 Hz, 1H), 6,7 (m, H), 6,88 (d, J = 7 Hz) 7,24 (s, 2H), 7,38 (m, 1H), 7,48 (m, 1H), 8,86 (s, 1H), 8,91 (s, 1H), 9,20 (d, J = 8 Hz, 1H), 14,0 (s, breit, 1H).

(6R,7R)-7-[(Z)-2-(2-Amino-4-thiazolyl)-2-[[(2-fluor-3,4-dihydroxybenzyl)oxy]imino]acetamido]-3-[[5-(3,4-dihydroxyphenyl)-2H-tetrazol-2-yl]methyl]-8-oxo-5-thia-1-azabicyclo[4.2.0]oct-2-en-2-carbonsäure-Natriumsalz (1:1):

$^1$H-NMR (DMSO-$d_6$, 250 MHz): Signale bei δ(ppm) 3,24 (m, 2H), 4,97 (d, J=5 Hz, 1H), 5,02 (s, 2H), 5,46 (d, J=14 Hz, 1H), 5,57 (d,d, J=5 Hz, J=8 Hz, 1H), 6,16 (d, J=14 Hz, 1H), 6,54 (m, 1H), 6,70 (m, 2H), 6,86 (d, J=7 Hz, 1H), 7,24 (s, 2H), 7,36 (m, 1H), 7,48 (m, 1H), 9,56 (d, J=8 Hz, 1H).
IR: (KBr) 1767 $cm^{-1}$.

(6R,7R)-7-[(Z)-2-(2-Amino-4-thiazolyl)-2-[[(3,4-dihydro-5-hydroxy-4-oxo-2-pyrimidinyl)methoxy]imino]-acetamido]-3-[[5-(3,4-dihydroxyphenyl)-2H-tetrazol-2-yl]methyl]-8-oxo-5-thia-1-azabicyclo[4.2.0]oct-2-en-2-carbonsäure-Natriumsalz (1:1):
$^1$H-NMR (DMSO-$d_6$, 250 MHz): Signale bei δ(ppm) 3,23 (m, 2H), 4,93 (s, 2H), 5,02 (d, J=5 Hz, 1H), 5,43 (d, J=14 Hz, 1H), 5,62 (d,d, J=5 Hz, J=8 Hz, 1H), 6,17 (d, J=14 Hz, 1H), 6,35 (s, 1H), 6,36 (d, J=7 Hz, 1H), 7,27 (s, 1H), 7,35 (d,d, J=7 Hz, J=2,5 Hz, 1H), 7,38 (s, 1H), 7,48 (d, J=2,5 Hz, 1H), 9,90 (d, J=8 Hz, 1H).
IR: (KBr) 1768 $cm^{-1}$.

Beispiel 37

Herstellung von (6R,7R)-7-[(Z)-2-(2-Amino-4-thiazolyl)-2-[[[(S)-α-carboxy-3,4-dihydroxybenzyl]oxy]imino]-acetamido]-3-[[[2-[[(3,4-dihydroxyphenyl)sulfonyl]methyl]-5-methyl-s-triazolo[1,5-a]pyrimidin-7-yl]thio]-methyl]-8-oxo-5-thia-1-azabicyclo[4.2.0]oct-2-en-2-carbonsäure-Natriumsalz

250 mg (6R,7R)-7-(2-Amino-4-thiazolglyoxylamido)-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure und 230 mg tert-Butyl (S)-α-(aminooxy)-2,2-diphenyl-1,3-benzodioxolo-5-acetat werden in 30 ml Dimethylacetamid gelöst und 24 Stunden bei Raumtemperatur stehen gelassen. Das Dimethylacetamid wird am Hochvakuum abgedampft. Der Rückstand wird mit Wasser digeriert. Der Niederschlag wird abfiltriert, noch feucht in 10 ml Trifluoressigsäure gelöst und 2 Stunden bei Raumtemperatur gerührt. Die Trifluoressigsäure wird am Vakuum abgedampft. Der Rückstand wird bei pH 7 zwischen Wasser und Aethylacetat verteilt. Die wässrige Phase wird an Opti-up $c_{12}$® mit Wasser, 5% und 10% Acetonitril chromatographiert. Die einheitlichen Fraktionen werden eingeengt und lyophilisiert. Man erhält 160 mg (6R,7R)-7-[(Z)-2-(2-Amino-4-thiazolyl)-2-[[[(S)-α-carboxy-3,4-dihydroxybenzyl]oxy]imino]acetamido]-3-[[[2-[[(3,4-dihydroxyphenyl)-sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz als weisses Pulver.
$^1$H-NMR (DMSO-$d_6$, 250 MHz): Signale bei δ(ppm) 2,56 (s, 3H), 3,20 (d, J=17 Hz, 1H), 3,48 (d, J=17 Hz, 1H), 4,43 (m, 2H), 4,68 (s, 2H), 5,01 (d, J=5 Hz, 1H), 5,09 (s, 1H), 5,68 (d,d, J=5 Hz, J=8 Hz, 1H), 6,6-6,9 (m, 7H), 7,2 (s, breit, 2H), 7,64 (s, 1H), 11,61 (d, J=8 Hz, 1H).
IR: (KBr) 1766 $cm^{-1}$.

Das als Ausgangsmaterial eingesetzte tert-Butyl (S)-α-(aminooxy)-2,2-diphenyl-1,3-benzodioxolo-5-acetat kann wie folgt hergestellt werden:

70,6 g 5-Brom-2,2-diphenyl-1,3-benzodioxol werden in 300 ml Tetrahydrofuran gelöst, bei -78°C mit 125 ml n-Butyllithium in n-Hexan versetzt und anschliessend bei dieser Temperatur 10 Minuten gerührt. Diese Lösung wird auf einmal zu einer Lösung von 50,0 g Oxalsäure di-tert.-butylester in 300 ml Tetrahydrofuran zugegeben und weitere 10 Minuten bei -78°C gerührt. Hierauf wird 1 Liter halbkonzentrierte wässrige Ammoniumchloridlösung zugegeben und das Gemisch zur Phasentrennung zu einem Scheidetrichter übergeführt. Die organische Phase wird mit gesättigter wässriger Natriumchloridlösung gewaschen. Die wässrigen Phasen werden mit Aethylacetat extrahiert. Die vereinigten organischen Phasen werden über Magnesiumsulfat getrocknet und eingeengt. Der ölige Rückstand wird mit 70 ml t-Butylmethyläther versetzt und mit 300 ml n-Hexan verdünnt. Es tritt spontane Kristallisation ein. Die Kristalle werden abfiltriert und aus Aethanol umkristallisiert. Nach Trocknen erhält man 31,0 g tert-Butyl [3,4-[(diphenylmethylen)dioxy]phenyl]-glyoxylat als weisse Kristalle vom Schmelzpunkt 120,5-122°C.

15 g tert-Butyl [3,4-[(diphenylmethylen)dioxy]phenyl]glyoxylat werden in 250 ml Methanol:Methylenchlorid 2,5:1 gelöst und unter Zusatz von 0,5 Molprozent Rutheniumchlorid-[(R)-Biphemp]-Komplex unter 60 Atmosphären Wasserstoffdruck bei Raumtemperatur 144 Stunden hydriert. Das Reaktionsgemisch wird filtriert, eingeengt und der Rückstand aus Aethanol umkristallisiert. Man erhält 10,7g tert-Butyl (R)-α-hydroxy-2,2-diphenyl-1,3-benzodioxol-5-acetat vom Schmelzpunkt 153-154,5°C; $[\alpha]_D^{20}$ = -37,4°. Die optische Reinheit wurde mittels $^1$H NMR unter Benützung von chiralen Shift-Reagentien ermittelt. Nach diesen Kriterien war die Substanz enantiomerenrein.

2,2 g tert-Butyl (R)-α-hydroxy-2,2-diphenyl-1,3-benzodioxol-5-acetat, 1,80 g N-Hydroxyphthalimid und 2,2 g Triphenylphosphin werden in 25 ml Methylenchlorid gelöst und auf 0°C gekühlt. Hierauf werden tropfenweise 1,70 ml Diisopropylazodicarboxylat gelöst in 10 ml Methylenchlorid zugegeben und anschliessend 1 Stunde bei Raumtemperatur gerührt. Das Reaktionsgemisch wird mit Methylenchlorid an Kieselgel

chromatographiert. Das am wenigsten polare Produkt (zuletzt eluiert) wird gesammelt. Nach Einengen erhält man 0,9 g eines weissen Feststoffes vom Schmelzpunkt 132-133 °C. Dieser Feststoff wird in 10 ml Aethanol gelöst, mit 0,20 ml 3-Dimethylamino-1-propylamin versetzt und bei Raumtemperatur 1 Stunde gerührt. Es bildet sich ein weisser Niederschlag. Dieser wird filtriert und getrocknet. Man erhält 480 mg tert-Butyl (S)-α-(aminooxy)-2,2-diphenyl-1,3-benzodioxolo-5-acetat als weisse Kristalle.
$^1$H-NMR ($CDCl_3$, 250 MHz): Signale bei δ(ppm) 1,44 (s, 9H), 4,90 (s, 1H), 5,72 (s, breit, 2H), 6,8-7,0 (m, 3H), 7,35 (m, 6H), 7,55 (m, 4H).
ee ≧95% nach $^1$H NMR mit Hilfe von Shift-Reagentien.

Beispiel 38

Herstellung von (6R,7R)-7-[(Z)-2-(2-Amino-4-thiazolyl)-2-[[1-(hydroxycarbamoyl)-1-methyläthoxy]imino]-acetamido]-3-[[[2-(3,4-dihydroxyphenyl)-5-methyl-s-triazolo[1,5-a]pyrimidin-7-yl]thio]methyl]-8-oxo-5-thia-1-azabicyclo-[4.2.0]oct-2-en-2-carbonsäure

1,3 g (6R,7R)-7-(2-Amino-4-thiazolglyoxylamido)-3-[[[2-(3,4-dihydroxyphenyl)-5-methyl-s-triazolo[1,5-a]-pyrimidin-7-yl]thio]methyl]-8-oxo-5-thia-1-azabicyclo[4.2.0]oct-2-en-2-carbonsäure und 0,44 g 2-(Aminooxy)-N-hydroxy-2-methylpropionamid-hydrochlorid werden in 10 ml Dimethylacetamid gelöst. Nach Stehen über Nacht wird im Vakuum eingedampft. Der Rückstand wird mit 20 ml Wasser digeriert und der gebildete Niederschlag abgesaugt und getrocknet. Man erhält 1,15 g (6R,7R)-7-[(Z)-2-(2-Amino-4-thiazolyl)-2-[[1-(hydroxycarbamoyl)-1-methyläthoxy]imino]acetamido]-3-[[[2-(3,4-dihydroxyphenyl)-5-methyl-s-triazolo[1,5-a]-pyrimidin-7-yl]thio]methyl]-8-oxo-5-thia-1-azabicyclo[4.2.0]oct-2-en-2-carbonsäure.
$^1$H-NMR (DMSO-$d_6$, 250 MHz): Signale bei δ 1,41 (s, 3H), 1,43 (s, 3H), 2,58 (s, 3H), 3,63 (d, J = 18 Hz, 1H), 3,86 (d, J = 18 Hz, 1H), 4,38 (d, J = 13 Hz, 1H), 4,53 (d, J = 13 Hz, 1H), 5,25 (d, J = 5 Hz, 1H), 5,95 (dd, J = 5 und 8 Hz, 1H), 6,81 (s, 1H), 6,87 (d, J = 9 Hz, 1H), 7,23 (s, 1H), 7,52 (dd, J = 9 und 1 Hz, 1H), 7,62 (d, J = 1 Hz, 1H), 9,70 (d, J = 8 Hz, 1H), 10,09 (s, 1H).

Die als Ausgangsmaterial eingesetzte (6R,7R)-7-(2-Amino-4-thiazolglyoxylamido)-3-[[[2-(3,4-dihydroxyphenyl)-5-methyl-s-triazolo[1,5-a]pyrimidin-7-yl]thio]methyl]-8-oxo-5-thia-1-azabicyclo[4.2.0]oct-2-en-2-carbonsäure kann wie folgt hergestellt werden:

132 g 3,4-Dihydroxybenzohydrazid und 66 g Cyanamid werden in 1230 ml 0,64N äthanolischer Salzsäure und 465 ml Dimethylacetamid 16 Stunden unter Rückfluss gekocht. Nach Abkühlen kristallisiert das 1-(3,4-Dihydroxybenzamido)guanidin-hydrochlorid aus. Zur Vervollständigung der Fällung wird 5 l Diäthyläther zugesetzt. Das abgenutschte Rohprodukt wird sodann in 1,16 l 1N wässriger Natronlauge 1 Stunde auf dem Dampfbad erwärmt. Nach Filtrieren der heissen Lösung wird mit 90 ml 25%iger wässriger Salzsäure auf pH 6 gestellt, wobei das gebildete 4-(5-Amino-1H-1,2,4-triazol-3-yl)pyrocatechol ausfällt. Man erhält 87 g kristallines Produkt vom Smp. 292-295 °C (Zers.).

87 g des obigen Produktes werden mit 106 ml Acetessigsäureäthylester in 870 ml Eisessig 17 Stunden lang bei 80 °C gerührt. Nach Abkühlen filtriert man die entstandene Fallung ab, nimmt sie in 1200 ml Wasser auf und fällt durch Einstellen des pH-Wertes auf 6 das entstandene 2-(3,4-Dihydroxyphenyl)-5-methyl-s-triazolo[1,5-a]pyrimidin-7(4H)-on aus. Man erhält nach Trocknen im Vakuum 90,6 g farbloses Kristallisat, Smp. über 310 °C. Zur weiteren Umsetzung wird das Produkt in 900 ml Pyridin mit 95 ml Acetanhydrid acetyliert. Man isoliert das Produkt durch Abdampfen des Lösungsmittels und Umkristallisieren aus 2 l Eisessig. Man erhält 103,5 g 2-(3,4-Diacetexyphenyl)-5-methyl-s-triazolo[1,5-a[pyrimidin-7(4H)-on vom Smp. 286-289 °C.

69 g des vorgenannten Produktes wird in 350 ml Phosphoroxychlorid vorgelegt. Zum Gemisch gibt man 25 ml Dimethylanilin und rührt sodann 5 Stunden bei 80 °C. Nach Abkühlen im Eisbad nutscht man über eine Glasnutsche ab, wäscht die Fallung mit wenig Aethylacetat nach und trocknet im Vakuum. Das rohe Zwischenprodukt wird sodann im Verlaufe einer Stunde in eine Lösung von 756 g Natriumhydrogensulfid in 850 ml Wasser eingetragen. Man rührt über Nacht weiter, senkt durch Zugabe von ca. 60 ml 3N wässriger Salzsäure den pH-Wert auf 7, nutscht das pastenförmige Produkt ab und wäscht mit wenig Wasser nach. Nach Aufschlämmen in 400 ml Aceton nutscht man erneut ab und erhält 32,6 g farbloses 4-(7-Mercapto-5-methyl-s-trisazolo[1,5-a]pyrimidin-2-yl)pyrocatechol. $C_{12}H_{16}N_4O_2S \cdot H_2O$ (berechnet: C = 49,31%, H = 4,14%, N = 19,17%, S = 10,97%; gefunden: C = 48,99%, H = 4,01%, N = 18,84%, S = 11,03%).

1,8 g 4-(7-Mercapto-5-methyl-s-triazolo[1,5-a]pyrimidin-2-yl)pyrocatechol und 1,63 g 7-Amino-cephalosporansäure werden in 30 ml eines 1:1 Gemisches von Dichlormethan und Sulfolan suspendiert. Bei 3 °C gibt man 2,63 g Bortrifluorid-ätherat zu und belässt die Lösung während 11/2 Stunden bei der gleichen Temperatur. Danach verdünnt man tropfenweise mit 60 ml Dichlormethan, nutscht die entstandene Fällung ab, wäscht mit Diäthyläther nach und löst sie unmittelbar in 40 ml Wasser auf. Unter Eiskühlung gibt man

solange 20%ige wässrige Natronlauge zu, bis pH 3,1 erreicht ist, wobei das Produkt ausfällt. Dieses wird abgenutscht, mit wenig Wasser und schliesslich mit 20 ml Aceton gewaschen und im Vakuum bei 0,1 mmHg und Raumtemperatur getrocknet. Man erhält 2,17 g (6R,7R)-7-Amino-3-[[[2-(3,4-dihydroxyphenyl)-5-methyl-s-triazolo[1,5-a]pyrimidin-7-yl]thio]methyl]-8-oxo-5-thia-1-azabicyclo[4.2.0]oct-2-en-2-carbonsäure.
$^1$H-NMR (DMSO-$d_6$, 250 MHz): Signale bei δ: 2,57 (s, 3H), 3,59 (d, J = 18 Hz, 1H), 3,76 (d, J = 18 Hz, 1H), 4,36 (d, J = 12,5 Hz, 1H), 4,42 (d, J = 12,5 Hz, 1H), 4,83 (d, J = 6 Hz, 1H), 5,40 (d, J = 6 Hz, 1H), 6,87 (d, J = 9 Hz, 1H), 7,22 (s, 1H), 7,51 (m, 1H), 7,63 (d, J = 1 Hz, 1H), 9,4 (breit, 2H).

4,86 g (6R,7R)-7-Amino-3-[[[2-(3,4-dihydroxyphenyl)-5-methyl-s-triazolo-[1,5-a]pyrimidin-7-yl]thio]-methyl]-8-oxo-5-thia-1-azabicyclo[4.2.0]oct-2-en-2-carbonsäure und 4,01 g 2-Amino-4-thiazol-thioglyoxylsäure-5-(2-benzothiazolyl)ester werden in 60 ml Dimethylacetamid während 3 Stunden bei Zimmertemperatur reagieren gelassen. Danach wird bei 25 °C und 0,1 bar das Lösungsmittel abgedampft und der Rückstand mit 125 ml Aceton digeriert. Die entstandene Fällung wird abgenutscht. Nach wiederholter Behandlung mit Aceton erhält man nach Trocknen im Vakuum 4,8 g (6R,7R)-7-(2-Amino-4-thiazolglyoxylamido)-3-[[[2-(3,4-dihydroxyphenyl)-5-methyl-s-triazolo[1,5-a]pyrimidin-7-yl]thio]methyl]-8-oxo-5-thia-1-azabicyclo-[4.2.0]oct-2-en-2-carbonsäure.
$^1$H-NMR (DMSO-$d_6$, 250 MHz): Signale bei δ 2,58 (s, 3H), 3,65 (d, J = 18 Hz, 1H), 3,82 (d, J = 18 Hz, 1H), 4,39 (d, J = 13 Hz, 1H), 4,50 (d, J = 13 Hz, 1H), 5,24 (d, J = 6 Hz, 1H), 5,81 (dd, J = 6 und 9 Hz, 1H), 6,86 (d, J = 8 Hz, 1H), 7,24 (s, 1H), 7,42 (s, breit, 2H), 7,51 (m, 1H), 7,62 (d, J etwa 1 Hz, 1H), 7,83 (s, 1H), 9,31 (s, breit, 1H), 9,47 (s, breit, 1H), 9,82 (d, J = 9 Hz, 1H).

## Beispiel 39

Herstellung von (6R,7R)-[(Z)-2-(2-Amino-4-thiazolyl)-2-[[(3,4-dihydroxybenzyl)oxy]imino]acetamido]-3-[[[2-(3,4-dihydroxyphenyl)-5-methyl-s-triazolo[1,5-a]pyrmidin-7-yl]thio]methyl]-8-oxo-5-thia-1-azabicyclo[4.2.0]-oct-2-en-2-carbonsäure-Natriumsalz (1:1)

1,87 g S-(2-Benzothiazolyl)-2-amino-4-thiazolglyoxylat-(Z)-O-[3,4-[(diphenylmethylen]dioxybenzyl]oxim und 1,46 g (6R,7R)-7-Amino-3-[[[2-(3,4-dihydroxyphenyl)-5-methyl-s-triazolo[1,5-a]pyrimidin-7-yl]thio]-methyl]-8-oxo-5-thia-1-azabicyclo[4.2.0]oct-2-en-2-carbonsäure werden in 20 ml Dimethylacetamid gelöst. Nach 48-stündigem Stehen setzt man 50 ml Wasser zu und filtriert das ausgefällte Rohprodukt ab. Dieses wird in 50 ml Wasser resuspendiert und durch Zugabe von Natriumbicarbonat bei pH 7,8 partiell in Lösung gebracht. Nach Filtration fällt man sodann das Reaktionsprodukt durch Zugabe von 1N wässriger Salzsäure aus, filtriert und trocknet. Das derart gereinigte Produkt wird sodann in 25 ml Trifluoressigsäure und 0,1 ml Wasser eingetragen. Nach 1 Stunde dampft man ein, gibt 35 ml Diäthyläther zu und nutscht die Fällung ab. Man löst erneut in 20 ml Dimethylacetamid auf, und gibt 1,4 ml einer 2m Lösung von Natrium-2-äthylcaproat in Aethylacetat zu und fällt das Natriumsalz der (6R,7R)-[(Z)-2-(2-Amino-4-thiazolyl)-2-[[(3,4-dihydroxybenzyl)oxy]imino]acetamido]-3-[[[2-(3,4-dihydroxyphenyl)-5-methyl-s-triazolo[1,5-a]pyrimidin-7-yl]-thio]methyl]-8-oxo-5-thia-1-azabicyclo[4.2.0]oct-2-en-2-carbonsäure durch tropfenweise Zugabe von 100 ml Aethylacetat als feinkristallines Produkt aus. Man erhält 1,75 g beiges Pulver.
$^1$H-NMR (DMSO-$d_6$): Signale bei δ 2,54 (s, 3H), 3,46 (d, J = 18 Hz, 1H), 3,62 (d, J = 18 Hz, 1H), 4,40 (d, J = 13 Hz, 1H), 4,61 (d, J = 13 Hz, 1H), 4,92 (s, 1H), 5,08 (d, J = 5 Hz, 1H), 5,66 (dd, J = 5 und 8 Hz, 1H), 6,7 (s, 1H), 6,6-6,76 (m, 3H), 6,85 (d, J = 8,5 Hz, 1H), 7,5 (m, 2H), 7,63 (m, 1H), 9,58 (d, J = 8 Hz, 1H).

## Beispiel 40

Herstellung von (6R,7R)-7-[(Z)-2-(2-Amino-4-thiazolyl)-2-(Z)-[[1-hydroxycarbamoyl)-1-methyläthoxy]imino]-acetamido]-3-[[[2-(3-chlor-4,5-dihydroxyphenyl)-5,6-dimethyl-s-triazolo[1,5-a]pyrrimidin-7-yl]thio]methyl]-8-oxo-5-thia-1-azabicyclo[4.2.0]oct-2-en-2-carbonsäure-Natriumsalz (1:1)

In gleicher Weise wie in Beispiel 38 wird ausgehend von (6R,7R)-7-(2-Amino-4-thiazolglyoxylamido)-3-[-[[2-(3-chlor-4,5-dihydroxyphenyl)-5,6-dimethyl-s-triazolo[1,5-a]pyrimidin-7-yl]thio]methyl]-8-oxo-5-thia-1-azabicyclo[4.2.0]oct-2-en-2-carbonsäure die Verbindung (6R,7R)-7-[(Z)-2-(2-Amino-4-thiazolyl)-2-(Z)-[[1-hydroxycarbamoyl)-1-methyläthoxy]imino]acetamido]-3-[[[2-(3-chlor-4,5-dihydroxyphenyl)-5,6-dimethyl-s-triazolo[1,5-a]pyrimidin-7-yl]thio]methyl]-8-oxo-5-thia-1-azabicyclo[4.2.0]oct-2-en-2-carbonsäure-Natriumsalz (1:1) hergestellt.
$^1$H-NMR (DMSO-$d_6$, 250 MHz): Signale bei δ 1,41 (s, 3H), 1,44 (s, 3H), 2,45 (s, 3H), 2,59 (s, 3H), 3,63 (d, J = 18 Hz, 1H), 3,79 (d, J = 18 Hz, 1H), 4,31 (d, J = 13 Hz, 1H), 4,43 (d, J = 13 Hz, 1H), 5,11 (d, J = 6 Hz, 1H), 5,80 (dd, J = 6 und 9 Hz, 1H), 6,79 (s, 1H), 7,34 (s, breit, 2H), 7,6-7,65 (m, 2H).

Die dazu benötigte (6R,7R)-7-(2-Amino-4-thiazolglyoxylamido)-3-[[[2-(3-chlor-4,5-dihydroxyphenyl)-5,6-dimethyl-s-triazolo[1,5-a]pyrimidin-7-yl]thio]methyl]-8-oxo-5-thia-1-azabicyclo[4.2.0]oct-2-en-2-carbonsäure kann analog zu (6R,7R)-7-(2-Amino-4-thiazolglyoxylamido)-3-[[[2-(3,4-dihydroxyphenyl)-5-methyl-s-triazolo-[1,5-a]pyrimidin-7-yl]thio]methyl]-8-oxo-5-thia-1-azabicyclo[4.2.0]oct-2-en-2-carbonsäure hergestellt werden. Ausgegangen wird von 3-Chlor-4,5-dihydroxybenzohydrazid, das zu 3-Chlor-5-(5-amino-1H-1,2,4-triazol-3-yl)pyrocatechol und dieses mittels 2-Methyl-acetessigsäureäthylester zum 2-(3-Chlor-4,5-dihydroxyphenyl)-5-methyl-s-triazolo[1,5-a]pyrimidin-7(4H)-on umgesetzt wird. Man erhält zunächst (6R,7R)-7-Amino-3-[[[2-(3-chlor-4,5-dihydroxyphenyl)-5,6-dimethyl-s-triazolo[1,5-a]pyrimidin-7-yl]thio]methyl]-8-oxo-5-thia-1-azabicyclo-[4.2.0]oct-2-en-2-carbonsäure:
[$^1$H-NMR (DMSO-$d_6$, 250 MHz): Signale bei $\delta$ 2,43 (s, 1H), 2,57 (s, 1H), 3,60 (d, J = 18 Hz, 1H), 3,75 (d, J = 18 Hz, 1H), 4,25 (d, J = 12,5 Hz, 1H), 4,32 (d, J = 12,5 Hz, 1H), 4,70 (d, J = 6 Hz, 1H), 4,81 (d, J = 6 Hz, 1H), 7,59 (d, J = 1 Hz, 1H), 7,62 (d, J = 1 Hz, 1H)]
und aus diesem die (6R,7R)-7-(2-Amino-4-thiazolglyoxylamido)-3-[[[2-(3-chlor-4,5-dihydroxyphenyl)-5,6-dimethyl-s-triazolo[1,5-a]pyrimdin-7-yl]thio]methyl]-8-oxo-5-thia-1-azabicyclo[4.2.0]oct-2-en-2-carbonsäure:
$^1$H-NMR (DMSO-$d_6$, 250 MHz): Signale bei $\delta$ 2,40 (s, 3H), 2,53 (s, 3H), 3,38 (d, J = 18 Hz, 1H), 3,58 (d, J = 18 Hz, 1H), 4,61 (d, J = 12,5 Hz, 1H), 4,51 (d, J = 12,5 Hz, 1H), 5,00 (d, J = 6 Hz, 1H), 5,60 (m, 1H), 7,43 (s, breit, 2H), 7,51 (d, J = 1 Hz, 1H), 7,63 (d, J = 1 Hz, 1H), 7,80 (s, 1H), 9,73 (d, breit, 1H).

## Beispiel 41

Herstellung von (6R,7R)-7-[(Z)-2-(2-Amino-4-thiazolyl)-2-[[(3,4-dihydroxybenzyl)oxy]imino]acetamido]-3-[[[5-(3,4-dihydroxyphenyl)pyrazolo[1,5-a]pyrimidin-7-yl]thio]methyl]-8-oxo-5-thia-1-azabicyclo[4.2.0]oct-2-en-2-carbonsäure-Natriumsalz (1:1)

1,252 g (6R,7R)-7-(2-Amino-4-thiazolglyoxylamdo)-3-[[[5-(3,4-dihydroxyphenyl)pyrazolo[1,5-a]pyrimidin-7-yl]thio]methyl]-8-oxo-5-thia-1-azabicyclo-[4.2.0]oct-2-en-2-carbonsäure und 1,065 g O-[3,4-[-(Diphenylmethylen)dioxy]benzyl]hydroxylamin werden in 20 ml Dimethylacetamid gelöst. Man belässt 24 Stunden bei Raumtemperatur und 3 Tage im Kühlschrank, dampft im Vakuum bei 40 °C ein und fällt das Reaktionsprodukt durch Verrühren mit 100 ml Wasser. Man wäscht mit Wasser und trocknet. Das Rohprodukt wird sodann in 25 ml Trifluoressigsäure bei 0 °C gelöst. Nach Zugabe von 25 Tropfen Wasser lässt man ohne weitere Kühlung 1 Stunde lang stehen und dampft im Vakuum ein. Der Rückstand wird mit 100 ml Diäthyläther verrührt, wobei das Produkt in fester Form anfällt. Es wird abfiltriert, unter Zusatz von Natriumbicarbonat bei pH 7 in Wasser gelöst und über RP12-Kieselgel mit Wasser/Acetonitril chromatographiert. Die Produktfraktion wird eingeengt und lyophilisiert. Man erhält 530 mg (6R,7R)-7-[(Z)-2-(2-Amino-4-thiazolyl)-2-[[(3,4-dihydroxybenzyl)oxy]imino]acetamido]-3-[[[5-(3,4-dihydroxyphenyl)pyrazolo[1,5-a]-pyrimidin-7-yl]thio]methyl]-8-oxo-5-thia-1-azabicyclo[4.2.0]oct-2-en-2-carbonsäure-Natriumsalz (1:1) als beiges Pulver.
$^1$H-NMR (DMSO-$d_6$, 250 MHz): Signale bei $\delta$ 3,46 (d, J = 18 Hz, 1H), 3,61 (d, J = 18 Hz, 1H), 4,59 (d, J = 13 Hz, 1H), 4,88 (d, J = 13 Hz, 1H), 4,90 (s, 2H), 5,04 (d, J = 5 Hz, 1H), 5,59 (dd, J = 5 und 8 Hz, 1H), 6,59-6,72 (m, 5H), 6,87 (d, J = 9 Hz, 1H), 7,21 (s, breit, 2H), 7,64-7,68 (m, 2H), 7,82 (m, 1H), 8,14 (d, J = 2 Hz, 1H), 8,95 (breit, 2H), 9,59 (m, 3H).

## Beispiel 42

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-[(3,4-dihydroxybenzolsulfonamido)methyl]-5-methyl-s-triazolo[1,5-a]-pyrimidin-7-yl]thio]methyl]-8-oxo-5-thia-1-azabicyclo[4.2.0]oct-2-en-2-carbonsäure-Natriumsalz (1:1)

0,10 g (6R,7R)-7-(2-Amino-4-thiazolglyoxylamido)-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure und 0,10 g 1-[2-(Aminooxy)-2-methylpropionyl]-2-(3,4-dihydroxybenzoyl)hydrazin-hydrochiorid (bekannt aus EPOS 286,145) werden in 1 ml Dimethylacetamid 16 Stunden bei Raumtemperatur gerührt. Das Dimethylacetamid wird am Hochvakuum abgedampft und der Rückstand mit Wasser digeriert. Der Niederschlag wird abfiltriert, in Wasser resuspendiert, durch Zugabe von 1N Natronlauge bei pH 7,0 in Lösung gebracht und lyophilisiert. Man erhält 0,11 g (6R,7R)-7-[(Z)-2-(2-Amino-4-thiazolyl)-2[[1-[3-(3,4-dihydroxybenzoyl)-carbazoyl]-1-methyläthoxy]imino]acetamido]-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure-Natriumsalz (1:1) als leicht beiges Pulver.

$^1$H-NMR (DMSO-$d_6$, 250 MHz): Signale bei δ(ppm) 1,47 (s, 3H), 1,52 (s, 3H), 2,53 (s, 3H), 3,39 (d, J = 18 Hz, 1H), 3,67 (d, J = 18 Hz, 1H), 4,13 (d, J = 12,5 Hz, 1H), 4,15 (s, verbreitert, 2H), 4,72 (d, J = 12,5 Hz, 1H), 5,12 (d, J = 5 Hz, 1H), 5,71 (d,d, J = 5 Hz, J = 8 Hz, 1H), 6,7 (m, 2H), 6,86 (s, 1H), 6,9 (m, 2H), 7,3 (m, 4H), 7,38 (s, 1H), 8,0 (s, stark verbreitert, 1H), 9,26 (s, breit, 1H), 9,60 (d, J = 8 Hz, 1H)
IR: (KBr) 1766 $cm^{-1}$
MS: 985,1 ($M + H^{\oplus}$)

Beispiel 43

Herstellung von (6R,7R)-7-[(Z)-2-(2-Amino-4-thiazolyl)-2-[[1-[3-(3,4-dihydroxybenzoyl)carbazoyl]-1-methyläthoxy]iminoacetamido]-3-[[[2-[[(3,4-dihydroxybenzoyl)oxy]methyl]-5-methyl-s-triazolo[1,5-a]-pyrimidin-7-yl]thio]methyl]-8-oxo-5-thia-1-azabicyclo[4.2.0]oct-2-en-2-carbonsäure-Natriumsalz (1:1)

0,50 g (6R,7R)-7-(2-Amino-4-thiazolglyoxylamido)-3-[[[2-[[(3,4-dihydroxybenzoyl)oxy]methyl]-5-methyi-s-triazolo[1,5-a]pyrimidin-7-yl]thio]methyl]-8-oxo-5-thia-1-azabicyclo[4.2.0]oct-2-en-2-carbonsäure und 0,25 g 1-[2-(Aminooxy)-2-methylpropionyl]-2-(3,4-dihydroxybenzoyl)hydrazin-hydrochlorid werden in 5 ml Dimethylacetamid 16 Stunden bei Raumtemperatur gerührt. Das Dimethylacetamid wird am Hochvakuum abgedampft, der Rückstand in Wasser digeriert und der entstandene Niederschlag abfiltriert. Der Feststoff wird in Wasser resuspendiert, mit 1N wässriger Natronlauge bei pH 7,0 in Lösung gebracht und an RP12-Kieselgel mit Wasser/Acetonitril chromatographiert (RP12-Kieselgel = "Reversed phase" Kieselgel, z.B. Opti-up $C_{12}$® der Firma ANTEC, Bennwil, Schweiz; ein Kieselgel mit Korngrösse 0.040-0.063 mm, behandelt mit Dodecyltrichlorsilan). Die einheitlichen Fraktionen werden eingeengt und lyophilisiert. Man erhält 0,40 g (6R,7R)-7-[(Z)-2-(2-Amino-4-thiazolyl)-2-[[1-[3-(3,4-dihydroxybenzoyl)carbazoyl]-1-methyläthoxy]iminoacetamido]-3-[[[2-[[(3,4-dihydroxybenzoyl)oxy]methyl]-5-methyl-s-triazolo[1,5-a]-pyrimidin-7-yl]thio]methyl]-8-oxo-5-thia-1-azabicyclo[4.2.0]oct-2-en-2-carbonsäure-Natriumsalz (1:1) als leicht beiges Pulver.
$^1$H-NMR (DMSO-$d_6$, 250 MHz): Signale bei δ(ppm) 2,55 (s, 3H), 3,41 (d, J = 18 Hz, 1H), 3,64 (d, J = 18 Hz, 1H), 4,31 (d, J = 12,5 Hz, 1H), 4,68 (d, J = 12,5 Hz, 1H), 5,09 (d, J = 5 Hz, 1H), 5,44 (s, 2H), 5,71 (dd, J = 5 Hz, J = 8 Hz, 1H), 6,8 (m, 3H), 7,30 (m, 6H), 7,65 (s, 1H), 9,26 (s, 1H), 9,54 (d, J = 8 Hz, 1H)
IR (KBr) 1768 $cm^{-1}$
MS: 972 ($M + Na^{\oplus}$), 950 ($M + H^{\oplus}$)

Beispiel 44

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-[[(3,4-dihydroxyphenyl)sulfonyl]methyl]-5-methyl-s-triazolo[1,5-a]-pyrimidin-7-yl]thio]methyl]-8-oxo-5-thia-azabicyclo[4.2.0]oct-2-en-2-carbonsäure-Natriumsalz (1:1)

0,50 g (6R,7R)-7-(2-Amino-4-thiazolglyoxylamido)-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-carbonsäure und 0,35 g 1-[2-(Aminooxy)-2-methylpropionyl]-2-(3,4-dihydroxybenzoyl)hydrazin-hydrochlorid werden in 5 ml Dimethylacetamid gelöst und bei Raumtemperatur 16 Stunden gerührt. Das Dimethylacetamid wird am Hochvakuum abgedampft und der Rückstand mit Wasser digeriert. Der gebildete Niederschlag wird abfiltriert, mit Wasser gewaschen, in Wasser resuspendiert, bei pH 7,0 (mit 1N wässriger Natronlauge eingestellt) in Lösung gebracht und an RP12-Kieselgel chromatographiert. Nach Lyophilisation der Produktfraktionen erhält man 0,490 g (6R,7R)-7-[(Z)-2-(2-Amino-4-thiazolyl)-2-[[1-[3-(3,4-dihydroxybenzoyl)-carbazoyl]-1-methyläthoxy]imino]acetamido]-3-[[[2-[[(3,4-dihydroxyphenyl)sulfonyl]methyl]-5-methyl-s-triazolo[1,5-a]pyrimidin-7-yl]thio]methyl]-8-oxo-5-thia-azabicyclo[4.2.0]oct-2-en-2-carbonsäure-Natriumsalz (1:1) als leicht beiges Pulver.
$^1$H-NMR (DMSO-$d_6$, 250 MHz): Signal bei δ(ppm) 1,46 (s, 3H), 1,52 (s, 3H), 2,55 (s, 3H), 3,38 (d, J = 18 Hz, 1H), 3,62 (d, J = 18 Hz, 1H), 4,32 (d, J = 12,5 Hz, 1H), 4,54 (d, J = 12,5 Hz, 1H), 4,71 (s, 2H), 5,09 (d, J = 5 Hz, 1H), 5,70 (d, breit, J = 5 Hz, 1H), 6,71 (m, 2H), 6,65 (s, 1H), 6,93 (m, 2H), 7,20-7,30 (m, 4H), 7,60 (s, 1H)
IR: (KBr) 1765 $cm^{-1}$
MS: 992 ($M + H^{\oplus}$)

Beispiel 45

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[5-(3,4-dihydroxyphenyl)-1,3,4-oxadiazol-2-yl]thio]methyl]-8-oxo-5-thia-1-azabicyclo[4.2.0]oct-2-en-2-carbonsäure

80 mg (0,1 mMol) (6R,7R)-7-(2-Amino-4-thiazolglyoxylamido)-3-[[[5-(3,4-dihydroxyphenyl)-1,3,4-oxadiazol-2-yl]thio]methyl]-8-oxo-5-thia-1-azabicyclo-[4.2.0]oct-2-en-2-carbonsäure und 42 mg (0,13 mMol) 1-[2-(Aminooxy)-2-methylpropionyl]-2-(3,4-dihydroxybenzoyl)hydrazin-hydrochlorid werden in 2 ml absolutem Dimethylacetamid gelöst. Nach 20-stündigem Rühren wird filtriert und die Mutterlauge im Hochvakuum bei Raumtemperatur eingeengt. Der Rückstand wird mit Aethanol/Diäthyiäther kristallisiert. Das Produkt wird filtriert und mit Wasser gewaschen. Nach Trocknen im Hochvakuum bei Raumtemperatur erhält man 65 mg (6R,7R)-7-[(Z)-2-(2-Amino-4-thiazolyl)-2-[[1-[3-(3,4-dihydroxybenzoyl)carbazoyl]-1-methyläthoxy]imino]-acetamido]-3-[[[5-(3,4-dihydroxyphenyl)-1,3,4-oxadiazol-2-yl]thio]methyl]-8-oxo-5-thia-1-azabicyclo[4.2.0]oct-2-en-2-carbonsäure als gelbes Pulver.
$^1H$ NMR (DMSO-$d_6$): Signale u.a. bei $\delta$ 1,46 (s, 3H), 1,52 (s, 3H), 3,65 (d, J = 18Hz, 1H), 3,84 (d, J = 18Hz, 1H), 4,26 (d, J = 12,5Hz, 1H), 4,43 (d, J = 12,5Hz, 1H), 5,20 (d, J = 5Hz, 1H), 5,88 (dd, J = 5Hz und J = 8Hz, 1H), 6,75 (d, J = 8Hz, 1H), 6,90 (d, J = 8Hz, 1H), 6,91 (s, 1H), 7,1-7,4 (4H), 9,23 (s, 1H), 9,64 (d, J = 8Hz, 1H), 10,00 (s, 1H).

Die als Ausgangsmaterial eingesetzte (6R,7R)-7-(2-Amino-4-thiazolglyoxylamido)-3-[[[5-(3,4-dihydroxyphenyl)-1,3,4-oxadiazol-2-yl]thio]methyl]-8-oxo-5-thia-1-azabicyclo[4.2.0]oct-2-en-2-carbonsäure kann wie folgt hergestellt werden:

10,38 g (38,4 mMol) 7-Aminocephalosporansäure und 9,6 g (42,24 mMol) 5-(3,4-Dihydroxyphenyl)-1,3,4-oxadiazol-2(3H)-thion-Dimethylformamidkomplex (bekannt aus EPOS 241.901) werden in 210 ml Wasser suspendiert und unter Rühren portionenweise mit 3,84 g (42,24 mMol) Natriumhydrogencarbonat versetzt. Nach 3-tägigem Rühren bei 40 °C wird die erhaltene braune Suspension abgenutscht und nacheinander mit Wasser, Aethanol und Diäthyläther gewaschen. Nach Trocknen im Hochvakuum bei Raumtemperatur erhält man 11,7 g weisses Material. Dieses wird in Aethanol gelöst und mit p-Toluolsulfosäure versetzt. Anschliessend wird die Lösung eingeengt und das Salz aus Aethanol/Diäthyläther kristallisiert. Man erhält weisses, kristallines (6R,7R)-7-Amino-3-[[[5-(3,4-dihydroxyphenyl)-1,3,4-oxadiazol-2-yl]thio]-methyl]-8-oxo-5-thia-1-azabicyclo[4.2.0]oct-2-en-2-carbonsäure-p-toluolsulfonsäure.
$^1H$ NMR (DMSO-$d_6$): 2,29 (s, 3H), 3,78 (d, J = 18Hz, 1H), 3,87 (d, J = 18Hz, 1H), 4,32 (d, J = 12,5Hz, 1H), 4,46 (d, J = 12,5Hz, 1H), 5,2 (2H), 6,89 (d, J = 8Hz, 1H), 7,11 (d, J = 8Hz, 2H), 7,27 (dd, J = 8Hz und J = 2Hz, 1H), 7,34 (d, J = 2Hz, 1H), 7,47 (d, J = 8Hz, 2H).

470 mg (0,7 mMol) (6R,7R)-7-Amino-3-[[[5-(3,4-dihydroxyphenyl)-1,3,4-oxadiazol-2-yl]thio]methyl]-8-oxo-5-thia-1-azabicyclo[4.2.0]oct-2-en-2-carbonsäure-p-toluolsulfonsäure und 500 mg (1,5 mMol) 2-Amino-4-thiazolthioglyoxylsäure-S-(2-benzothiazolyl)ester werden in 20 ml absolutem Dimethylacetamid gelöst. Nach 24-stündigem Rühren bei Raumtemperatur wird im Hochvakuum bei Raumtemperatur eingeengt. Der Rückstand wird in etwa 20 ml Aethanol aufgenommen und filtriert. Die Mutterlauge wird mit Diäthyläther versetzt. Das ausgefallene Produkt wird abfiltriert und mit Diäthyläther/Aethanol (98:2 v/v) gewaschen. Nach Trocknen im Hochvakuum erhält man 400 mg (6R,7R)-7-(2-Amino-4-thiazolglyoxylamido)-3-[[[5-(3,4-dihydroxyphenyl)-1,3,4-oxadiazol-2-yl]thio]methyl]-8-oxo-5-thia-1-azabicyclo[4.2.0]oct-2-en-2-carbonsäure-p-toluolsulfonsäure als gelbes Pulver.
$^1H$ NMR (DMSO-$d_6$): Signale u.a. bei $\delta$ 2,29 (s, 3,9H), 3,69 (d, J = 18Hz, 1H), 3,81 (d, J = 18Hz, 1H), 4,29 (d, J = 12,5Hz, 1H), 4,45 (d, J = 12,5Hz), 5,18 (d, J = 5Hz, 1H), 5,76 (dd, J = 5Hz und J = 8Hz, 1H), 6,89 (d, J = 8Hz, 1H), 7,12 (d, J = 7,5Hz, 2,6H), 7,27 (dd, J = 8Hz und J = 2Hz, 1H), 7,34 (d, J = 2Hz, 1H), 7,48 (d, J = 7,5Hz, 2,6H), 7,96 (s, 1H), 9,88 (d, J = 8Hz, 1H).

Beispiel 46

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[5-(3,4-dihydroxyphenyl)-2-phenyl-4-pyrimidinyl]thio]methyl]-8-oxo-5-thia-1-azabicyclo[4.2.0]-oct-2-en-2-carbonsäure

33 mg (0,05 mMol) (6R,7R)-7-(2-Amino-4-thiazolglyoxylamido)-3-[[(5-(3,4-dihydroxyphenyl)-2-phenyl-4-pyrimidinyl]thio]methyl]-8-oxo-5-thia-1-azabicyclo[4.2.0]oct-2-en-2-carbonsäure und 22 mg (0,065 mMol) 1-[2-Aminooxy)-2-methylpropionyl]-2-(3,4-dihydroxybenzoyl)hydrazin-hydrochlorid werden in 0,6 ml absolutem Dimethylacetamid gelöst. Nach 18-stündigem Rühren bei Raumtemperatur wird das Lösungsmittel im

Hochvakuum abdestilliert. Der Rückstand wird mit 4 ml Wasser und 0,3 ml Aethanol versetzt. Das ausgefallene Produkt wird abfiltriert und mit Wasser und Diäthyläther gewaschen. Nach Trocknen im Hochvakuum bei Raumtemperatur erhält man 40 mg (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[5-(3,4-dihydroxyphenyl)-2-phenyl-4-pyrimidinyl]thio]methyl]-8-oxo-5-thia-1-azabicyclo[4.2.0]-oct-2-en-2-carbonsäure als beiges Pulver.
$^1$H NMR (DMSO-$d_6$): Signale u.a. bei δ 1,46 (s, 3H), 1,48 (s, 3H), 3,60 (d, J = 18Hz, 1H), 3,85 (d, J = 18Hz, 1H), 4,19 (d, J = 12,5Hz, 1H), 4,78 (d, J = 12,5Hz, 1H), 5,21 (d, J = 5Hz, 1H), 5,86 (dd, J = 5Hz und J = 8Hz, 1H), 6,72-6,94 (5H), 7,20-7,34(4H), 7,58(3H), 8,44 (s, 1H), 8,46(2H), 9,19 (s, 2H), 9,23 (s, 1H), 9,30 (s, 1H), 9,56 (s, 1H), 9,63 (d, J = 8Hz, 1H), 10,00 (s, 1H).

Beispiel 47

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[(3,4-dihydroxyphenyl)sulfonyl]methyl]-8-oxo-5-thia-1-azabicyclo[4.2.0]-oct-2-en-2-carbonsäure

160 mg (0,30 mMol) (6R,7R)-7-(2-Amino-4-thiazolglyoxylamido)-3-[[(3,4-dihydroxyphenyl)sulfonyl]-methyl]-8-oxo-5-thia-1-azabicyclo[4.2.0]oct-2-en-2-carbonsäure werden in 2,0 ml Dimethylacetamid gelöst, mit 130 mg (0,39 mMol) 1-[2-(Aminooxy)-2-methylpropionyl]-2-(3,4-dihydroxybenzoyl)hydrazin-hydrochlorid versetzt und bei Raumtemperatur 20 Stunden gerührt. Das Dimethylacetamid wird unter Hochvakuum abgedampft und das Produkt durch Zugabe von Wasser gefällt. Die Reinigung erfolgt durch Chromatographie an Kieselgel mit Wasser/Acetonitril als Eluens. Man erhält nach dem Lyophilisieren 200 mg (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[-(3,4-dihydroxyphenyl)sulfonyl]methyl]-8-oxo-5-thia-1-azabicyclo[4.2.0]oct-2-en-2-carbonsäure als weissen Feststoff.
$^1$H-NMR ($D_2O$, 250 MHz): Signale bei δ (ppm): 1,65 (s, 6H), 3,46 (m, 2H), 4,02 (d, J = 12,5Hz, 1H), 4,84 (s, 2H), 5,11 (d, J = 12,5Hz, 1H), 5,21 (d, J = 5Hz, 1H), 5,80 (d, J = 5Hz, 1H), 7,00 (m, 2H), 7,10 (s, 1H), 7,30 (m, 4H).
MS: 814 ($M + H^{\oplus}$)
IR: 1767 $cm^{-1}$

Beispiel 48

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-(hydroxycarbamoyl)-5-methyl-s-triazolo[1,5-a]pyrimidin-7-yl]thio]-methyl]-8-oxo-5-thia-1-azabicyclo[4.2.0]oct-2-en-2-carbonsäure

60 mg (0,101 mMol) (6R,7R)-7-(2-Amino-4-thiazolglyoxylamido)-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure und 40 mg (0,1318 mMol) 1-[2-(Aminooxy)-2-methylpropionyl]-2-(3,4-dihydroxybenzoyl)hydrazin-hydrochlorid werden in 3 ml absolutem Dimethylacetamid gelöst. Nach 24-stündigem Rühren bei Raumtemperatur wird das Lösungsmittel im Hochvakuum bei Raumtemperatur abgezogen. Das verbleibende Oel wird mit Wasser versetzt und das ausgefallene Produkt abfiltriert. Das Ganze wird nacheinander mit Wasser, Aethanol und Diäthyläther gewaschen und im Hochvakuum bei Raumtemperatur getrocknet. Man erhält 62 mg (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[-[2-(hydroxycarbamoyl)-5-methyl-s-triazolo-[1,5-a]pyrimidin-7-yl]thio]methyl]-8-oxo-5-thia-1-azabicyclo[4.2.0]-oct-2-en-2-carbonsäure als beiges Pulver.
$^1$H NMR (DMSO-$d_6$): Signale u.a. bei δ 1,47 (s, 3H), 1,50 (s, 3H), 2,60 (s, 3H), 3,64 (d, J = 18Hz, 1H), 3,84 (d, J = 18Hz, 1H), 4,36 (d, J = 12,5Hz, 1H), 4,48 (d, J = 12,5Hz, 1H), 5,23 (d, J = 5Hz, 1H), 5,90 (dd, J = 5Hz und J = 8Hz, 1H), 6,74 (d, J = 7,5Hz, 1H), 6,86 (s, 1H), 7,21 (dd, J = 7,5Hz und J = 2Hz, 1H), 7,29 (d, J = 2Hz, 1H), 7,35 (s, 1H), 9,17 (s, 1H), 9,20 (s, 1H), 9,34 (s, 1H), 9,54 (s, 1H), 9,66 (d, J = 8Hz, 1H), 10,00 (s, 1H), 11,66 (s, 1H).

Die als Ausgangsverbindung eingesetzte (6R,7R)-7-(2-Amino-4-thiazolglyoxylamido)-3-[[[2-(hydroxycarbamoyl)-5-methyl-s-triazolo-[1,5-a]pyrimidin-7-yl]thio]methyl]-8-oxo-5-thia-1-azabicyclo[4.2.0]oct-2-en-2-carbonsäure kann wie folgt hergestellt werden:

43,7 mg (0,1 mMol) (6R,7R)-7-Amino-3-[[[2-(hydroxycarbamoyl)-5-methyl-s-triazolo[1,5-a]pyrimidin-7-yl]-thio]methyl]-8-oxo-5-thia-1-azabicyclo[4.2.0]oct-2-en-2-carbonsäure werden in einem Gemisch bestehend aus 2 ml Wasser und 1 ml Acetonitril suspendiert. Bei Raumtemperatur tropft man unter Rühren 2 ml 0,1N

wässrige Kalilauge zu. Zur Lösung gibt man anschliessend 84 mg (0,26 mMol) 2-Amino-4-thiazolthioglyoxylsäure-S-(2-benzothiazolyl)ester. Die Suspension wird 3 Stunden bei Raumtemperatur gerührt und filtriert. Das Filtergut wird mit Wasser gewaschen, Filtrat und Waschlösung vereinigt und mit Aethylacetat nochmals gewaschen. Die wässrige Lösung wird mit verdünnter wässriger Salzsäure auf pH 5 gestellt. Das ausgefallene Produkt wird abgenutscht und mit Wasser und Diäthyläther gewaschen. Man erhält nach Trocknen im Hochvakuum 30 mg (6R,7R)-7-(2-Amino-4-thiazolglyoxylamido)-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure als gelbes Pulver.
$^1$H NMR (DMSO-$d_6$): Signale bei $\delta$ 2,61 (s, 3H), 3,63 (d, J=18Hz, 1H), 3,80 (d, J=18Hz, 1H), 4,40 (d, J=12,5Hz, 1H), 4,48 (d, J=12,5Hz, 1H), 5,21 (d, J=5Hz, 1H), 5,77 (dd, J=5Hz und J=8Hz, 1H), 7,40 (s, 3H), 7,82 (s, 1H), 9,35 (s, 1H), 9,81 (d, J=8Hz, 1H), 11,68 (s, 1H).

Beispiel 49

Herstellung von (6R,7R)-7-[(Z)-2-(2-Amino-4-thiazolyl)-2-[(1-[3-(2,5-dichlor-3,4-dihydroxybenzoyl)carbazoyl]-1-methyläthoxy]imino]acetamido]-3-[[(2-(hydroxycarbamoyl)-5-methyl-s-triazolo[1,5-a]pyrimidin-7-yl]thio]-methyl]-8-oxo-5-thia-1-azabicyclo[4.2.0]oct-2-en-2-carbonsäure

150 mg (0,25 mMol) (6R,7R)-7-(2-Amino-4-thiazolglyoxylamido)-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure und 175 mg (0,32 mMol) 1-[2-(Aminooxy)-2-methylpropionyl]-2-[(4,7-dichlor-2,2-diphenyl-1,3-benzodioxol-5-yl)carbonyl]-hydrazin-hydrochlorid werden in 4 ml absolutem Dimethylacetamid gelöst. Nach 20-stündigem Rühren bei Raumtemperatur wird das Lösungsmittel im Hochvakuum abdestilliert. Der Rückstand wird mehrmals mit Diäthyläther gewaschen und anschliessend mit Wasser kristallisiert. Das Produkt wird abgenutscht, mit Wasser und Diäthyläther gewaschen und im Hochvakuum bei Raumtemperatur getrocknet. Man erhält 260 mg (6R,7R)-7-[(Z)-2-(2-Amino-4-thiazolyl)-2-[[1-[3-[(4,7-dichlor-2,2-diphenyl-1,3-benzodioxol-5-yl)carbazoyl]-1-methyläthoxy]imino]acetmido]-3-[[[2-(hydroxycarbamoyl)-5-methyl-s-triazolo[1,5-a]pyrimidin-7-yl)thio]-methyl]-8-oxo-5-thia-1-azabicyclo[4.2.0]oct-2-en-2-carbonsäure als beiges Pulver.
$^1$H NMR (DMSO-$d_6$): Signale u.a. bei $\delta$ 1,46 (s, 3H), 1,48 (s, 3H), 2,60 (s, 3H), 3,64 (d, J=18Hz, 1H), 3,86 (d, J=18Hz, 1H), 4,38 (d, J=12,5Hz, 1H), 4,50 (d, J=12,5Hz, 1H), 5,25 (d, J=5Hz, 1H), 5,91 (dd, J=5Hz und J=8Hz, 1H), 6,86 (s, 1H), 7,18 (s, 1H), 7,38 (s, 1H), 7,4-7,6 (10H), 9,46 (s, 1H), 9,66 (d, J=8Hz, 1H), 10,26 (s, 1H), 11,70 (s, 1H).

125 mg (0,116 mMol) (6R,7R)-7-[(Z)-2-(2-Amino-4-thiazolyl)-2-[[1-[3-[(4,7-dichlor-2,2-diphenyl-1,3-benzodioxol-5-yl)carbazoyl]-1-methyläthoxy]imino]acetamido]-3-[[[2-(hydroxycarbamoyl)-5-methyl-s-triazolo-[1,5-a]pyrimidin-7-yl)thio]methyl]-8-oxo-5-thia-1-azabicyclo[4.2.0]oct-2-en-2-carbonsäure werden in 2 ml Trifluoressigsäure gelöst. Nach 5 Stunden wird das Lösungsmittel bei Raumtemperatur im Vakuum abdestilliert. Der Rückstand wird mit Diäthyläther kristallisiert. Das Produkt wird abgenutscht und nacheinander mit Diäthyläther, Wasser und Diäthyläther gewaschen. Nach Trocknen im Hochvakuum bei Raumtemperatur erhält man 90 mg (6R,7R)-7-[(Z)-2-(2-Amino-4-thiazolyl)-2-[(1-[3-(2,5-dichlor-3,4-dihydroxybenzoyl)-carbazoyl]-1-methyläthoxy]imino]acetamido]-3-[[(2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure als beiges Pulver.
$^1$H NMR (DMSO-$d_6$): Signale u.a. bei $\delta$ 1,47 (s, 3H), 1,48 (s, 3H), 2,61 (s, 3H), 3,64 (d, J=18Hz, 1H), 3,86 (d, J=18Hz, 1H), 4,36 (d, J=12,5Hz, 1H), 4,49 (d, J=12,5Hz, 1H), 5,25 (d, J=5Hz, 1H), 5,92 (dd, J=5Hz und J=8Hz, 1H), 6,86 (s, 1H), 7,00 (s, 1H), 7,32 (s, 3H), 7,38 (s, 1H), 9,34 (s, 1H), 9,40 (s, 1H), 9,67 (d, J=8Hz, 1H), 9,96 (s, 1H), 10,11 (s, 1H), 10,17 (s, 1H), 11,68 (s, 1H).

Das als Ausgangsmaterial eingesetzte 1-[2-(Aminooxy)-2-methylpropionyl]-2-[(4,7-dichlor-2,2-diphenyl-1,3-benzodioxol-5-yl)carbonyl]hydrazinhydrochlorid kann wie folgt hergestellt werden:

0,50 g (1,24 mMol) 4,7-Dichlor-2,2-diphenyl-1,3-benzodioxol-5-carbonsäuremethylester und 0,62 g (12 mMol) Hydrazinhydrat werden zusammen 20 Stunden bei 80 °C gerührt. Das Reaktionsgemisch wird im Hochvakuum konzentriert und der Rückstand aus Aethanol/Wasser kristallisiert. Nach Umkristallisation aus Aethanol/Wasser erhält man 310 mg 4,7-Dichlor-2,2-diphenyl-1,3-benzodioxol-5-carboxyhydrazid als weisse Kristalle vom Smp. 131 °C.

3,00 g (7,42 mMol) 4,7-Dichlor-2,2-diphenyl-1,3-benzodioxol-5-carboxyhydrazid, 2,92 g (7,42 mMol) N-[-[2-Methyl-2-(phthalimidooxy)propionyl]oxy]phthalimid und 0,62 g (7,42 mMol) Natriumhydrogencarbonat werden in 100 ml absolutem Tetrahydrofuran suspendiert. Nach 24-stündigem Rühren bei Raumtemperatur wird das Lösungsmittel im Vakuum abdestilliert und der Rückstand an 100 g Kieselgel (0,063-0,2 mm) chromatographiert. Das Produkt wird mit Dichlormethan/Aethanol (98:2 v/v) eluiert. Nach Eindampfen des Eluats verbleiben 3,1 g 1-[(4,7-Dichlor-2,2-diphenyl-1,3-benzodioxol-5-yl)carbonyl]-2-[2-phthalimidooxy)-2-methylpropionyl]hydrazin als farbloses Oel.
$^1H$ NMR (DMSO-$d_6$): Signale u.a. bei δ 1,55 (s, 6H), 7,20 (s, 1H), 7,5 (10H), 7,92 (s, 1H), 10,10 (s, 1H), 10,38 (s, 1H).

3,0 g (4,7 mMol) 1-[(4,7-Dichlor-2,2-diphenyl-1,3-benzodioxol-5-yl)carbonyl]-2-[2-phthalimidooxy)-2-methylpropionyl]hydrazin werden in 120 ml absolutem Tetrahydrofuran gelöst und bei 0°C unter Rühren mit 0,219 g (4,7 mMol) Methylhydrazin versetzt. Nach einer Stunde wird das Reaktionsgemisch auf Zimmertemperatur erwärmt, und nach weiteren 4 Stunden engt man es ein. Der Rückstand wird an 100 g Kieselgel (0,063-0,2 mm) chromatographiert. Man eluiert das Produkt mit Dichlormethan/Aethanol (95:5 v/v) und engt das Eluat ein. Das verbleibende Oel wird in äthanolischer Salzsäure gelöst und durch Zugabe von Diäthyläther kristallisiert. Man erhält 1,6 g 1-[2-(Aminooxy)-2-methylpropionyl]-2-[(4,7-dichlor-2,2-diphenyl-1,3-benzodioxol-5-yl)carbonyl]hydrazin-hydrochlorid als weisse Kristalle, Smp. 96-99°C.
$^1H$ NMR (DMSO-$d_6$): Signale u.a. bei δ 1,52 (s, 6H), 7,17 (s, 1H), 7,5 (12H), 10,26 (1H), 10,40 (s, 1H).

Beispiel 50

Herstellung von (6R,7R)-7-[(Z)-2-(2-Amino-4-thiazolyl)-2-[[1-[3-(5-brom-2-chlor-3,4-dihydroxybenzoyl)-carbazoyl]-1-methyläthoxy]imino]acetamido]-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure

59 mg (0,10 mMol) (6R,7R)-7-(2-Amino-4-thiazolglyoxylamido)-3-[[[2-(hydroxycarbamoyl)-5-methyl-s-triazolo[1,5-a]pyrimidin-7-yl]thio]methyl]-8-oxo-5-thia-1-azabicyclo[4.2.0]oct-2-en-2-carbonsäure und 76 mg (0,13 mMol) 1-[2-(Aminooxy)-2-methylpropionyl]-2-[(7-brom-4-chlor-2,2-diphenyl-1,3-benzodioxol-5-yl)-carbonyl]hydrazin-hydrochlorid (1:1) werden in 2 ml absolutem Dimethylacetamid gelöst. Nach 20-stündigem Rühren bei Raumtemperatur wird das Lösungsmittel im Hochvakuum abdestilliert. Der Rückstand wird mehrmals mit Diäthyläther gewaschen und anschliessend mit Wasser kristallisiert. Das Produkt wird abgenutscht, mit Wasser und Diäthyläther gewaschen und im Hochvakuum bei Raumtemperatur getrocknet. Man erhält 110 mg (6R,7R)-7-[(Z)-2-(2-Amino-4-thiazolyl)-2-[[1-[3-(7-brom-4-chlor-2,2-diphenyl-1,3-benzodioxol-5-yl)carbazoyl]-1-methyläthoxy]imino]acetamido]-3-[[[2-(hydroxycarbamoyl)-5-methyl-s-triazolo-[1,5-a]pyrimidin-7-yl]thio]methyl]-8-oxo-5-thia-1-azabicyclo[4.2.0]oct-2-en-2-carbonsäure als beiges Pulver.
$^1H$ NMR (DMSO-$d_6$): Signale u.a. bei δ 1,46 (s, 3H), 1,50 (s, 3H), 2,60 (s, 3H), 3,64 (d, J=18 Hz, 1H), 3,86 (d, J=18 Hz, 1H), 4,38 (d, J=12,5 Hz, 1H), 4,50 (d, J=12,5 Hz, 1H), 5,25 (d, J=5 Hz, 1H), 5,92 (dd, J=5 Hz und J=8 Hz, 1H), 6,85 (s, 1H), 7,27 (s, 1H), 7,31 (2H), 7,48 (s, 1H), 7,52 (10), 9,36 (1H), 9,48 (s, 1H), 9,65 (d, J=8 Hz, 1H), 10,26 (1H), 11,69 (s, 1H).

105 mg (0,093 mMol) (6R,7R)-7-[(Z)-2-(2-Amino-4-thiazolyl)-2-[[1-[3-(7-brom-4-chlor-2,2-diphenyl-1,3-benzodioxol-5-yl)carbazoyl]-1-methyläthoxy]imino]acetamido]-3-[[[2-(hydroxycarbamoyl)-5-methyl-s-triazolo-[1,5-a]pyrimidin-7-yl]thio]methyl]-8-oxo-5-thia-1-azabicyclo[4.2.0]oct-2-en-2-carbonsäure werden in 2 ml Trifluoressigsäure gelöst. Nach 5 Stunden wird die Trifluoressigsäure im Vakuum abdestilliert. Der Rückstand wird durch Zugabe von Diäthyläther kristallisiert. Man erhält 90 mg (6R,7R)-7-[(Z)-2-(2-Amino-4-thiazolyl)-2-[[1-[3-(5-brom-2-chlor-3,4-dihydroxybenzoyl)carbazoyl]-1-methyläthoxy]imino]acetamido]-3-[[[2-(hydroxycarbamoyl)-5-methyl-s-triazolo[1,5-a]pyrimidm-7-yl]thio]methyl]-8-oxo-5-thia-1-azabicyclo[4.2.0]oct-2-en-2-carbonsäure als beiges Pulver.
$^1H$ NMR (DMSO-$d_6$): Signale u.a. bei δ 1,48 (s, 3H), 1,50 (s, 3H), 2,60 (s, 3H), 3,64 (d, J=18 Hz, 1H), 3,85 (d, J=18 Hz, 1H), 4,38 (d, J=12,5 Hz, 1H), 4,50 (d, J=12,5 Hz, 1H), 5,26 (d, J=5 Hz), 5,92 (dd, J=5 Hz und J=8 Hz, 1H), 6,86 (s, 1H), 7,15 (s, 1H), 7,32 (2H), 7,36 (s, 1H), 9,35 (1H), 9,42 (s, 1H), 9,66 (d, J=8 Hz, 1H), 9,97 (1H), 10,11 (s, 2H), 11,69 (s, 1H).

Das als Ausgangsmaterial eingesetzte 1-[2-(Aminooxy)-2-methylpropionyl]-2-[(7-brom-4-chlor-2,2-diphenyl-1,3-benzodioxol-5-yl)carbonyl]hydrazin-hydrochlorid kann wie folgt hergestellt werden:

Ein Gemisch von 9,6 g (40 mMol) Methyl 3-brom-4,5-dihydroxybenzoat, 8,4 g (62 mMol) Sulfurylchlorid und 400 ml Eisessig wird 60 Stunden bei 55°C gerührt und anschliessend bei 40°C im Vakuum eingeengt. Der Rückstand wird in 200 ml Methanol gelöst und mit Chlorwasserstoff gesättigt. Nach 5-stündigem Stehen bei Raumtemperatur wird das Lösungsmittel im Vakuum abdestilliert. Das verbleibende Material wird mit ca. 100 ml Dichlormethan/Aethylacetat/Petroläther (1:1:1 v/v) versetzt und filtriert. Das Filtrat wird eingeengt und der Rückstand aus Diäthyläther/Petroläther kristallisiert und umkristallisiert. Man erhält 3,4 g Methyl 5-brom-2-chlor-3,4-dihydroxybenzoat als weisse Kristalle vom Schmelzpunkt 103°C.

Ein Gemisch von 1,0 g (3,5 mMol) Methyl 5-brom-2-chlor-3,4-dihydroxybenzoatund 7,5 ml Dichlordiphenylmethan wird unter Argon 2 1/2 Minuten bei 150°C gehalten. Nach raschem Abkühlen wird das Produkt aus n-Pentan kristallisiert. Man erhält 1,3 g Methyl 7-brom-4-chlor-2,2-diphenyl-1,3-benzodioxol-5-carboxylat als weisse Kristalle vom Schmelzpunkt 137-138°C.

Ein Gemisch von 620 mg (1,39 mMol) Methyl 7-brom-4-chlor-2,2-diphenyl-1,3-benzodioxol-5-carboxylat und 700 mg (13,9 mMol) Hydrazinhydrat wird 20 Stunden bei 80 °C gehalten. Das Reaktionsgemisch wird im Hochvakuum eingeengt und der Rückstand mit Aethanol/Wasser versetzt. Das unlösliche Produkt wird abgenutscht und mit Aethanol/Diäthyläther gewaschen. Man erhält 580 mg 7-Brom-4-chlor-2,2-diphenyl-1,3-benzodioxol-5-carboxyhydrazid als weisse Kristalle vom Schmelzpunkt 124 °C.

Ein Gemisch von 500 mg (1,122 mMol) 7-Brom-4-chlor-2,2-diphenyl-1,3-benzodioxol-5-carboxyhydrazid, 442 mg (1,122 mMol) N-[[2-Methyl-2-(phthalimidooxy)propionyl]oxy]phthalimid und 94 mg (1,122 mMol) Natriumhydrogencarbonat in 30 ml Tetrahydrofuran wird 20 Stunden bei Raumtemperatur gerührt. Das Lösungsmittel wird im Vakuum abdestilliert und der Rückstand an 30 g Kieselgel (0,06-0,2 mm) chromatographiert. Das Produkt wird mit Dichlormethan/Aethanol (98:2 v/v) eluiert. Nach Einengen des Eluats erhält man 510 mg 1-[(7-Brom-4-chlor-2,2-diphenyl-1,3-benzodioxol-5-yl)carbonyl]-2-[2-methyl-2-(phthalimidooxy)-propionyl]hydrazin als farbloses Oel.
$^1$H NMR (DMSO-$d_6$): Signale bei δ 1,55 (s, 6H), 7,29 (s, 1H), 7,5 (10H), 7,92 (s, 4H), 10,10 (s, 1H), 10,37 (s, 1H).

500 mg (0,77 mMol) 1-[(7-Brom-4-chlor-2,2-diphenyl-1,3-benzodioxol-5-yl)carbonyl]-2-[2-methyl-2-(phthalimidooxy)propionyl]hydrazin werden in 10 ml Tetrahydrofuran gelöst und bei 0 °C mit 35 mg (0,77 mMol) Methylhydrazin versetzt. Nach 2-stündigem Rühren bei 0 °C und 3-stündigem Rühren bei Raumtemperatur wird im Vakuum eingeengt. Der Rückstand wird an 20 g Kieselgel (0,06-02 mm) chromatographiert. Das Produkt wird mit Dichlormethan/Aethanol (95:5 v/v) eluiert. Das Eluat wird eingeengt und der Rückstand mit äthanolischer Salzsäure versetzt. Nach Kristallisation aus Aethanol/Diäthyläther erhält man 220 mg 1-[2-(Aminooxy)-2-methylpropionyl]-2-[(7-brom-4-chlor-2,2-diphenyl-1,3-benzodioxol-5-yl)carbonyl]hydrazin-hydrochlorid als weisse Kristalle vom Schmelzpunkt 216-218 °C.

Beispiel 51

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-(N-hydroxy-N-methylcarbamoyl)-5-methyl-s-triazolo[1,5-a]pyrimidin-7-yl]thio]methyl]-8-oxo-5-thia-1-azabicyclo[4.2.0]oct-2-en-2-carbonsäure

40 mg (0,066 mMol) (6R,7R)-7-(2-Amino-4-thiazolglyoxylamido)-3-[[[2-(N-hydroxy-N-methylcarbamoyl)-5-methyl-s-triazolo[1,5-a]pyrimidin-7-yl]thio]methyl]-8-oxo-5-thia-1-azabicyclo[4.2.0]oct-2-en-2-carbonsäure und 29 mg (0.086 mMol) 1-[2-(Aminooxy)-2-methylpropionyl]-2-(3,4-dihydroxybenzoyl)hydrazin-hydrochlorid werden in 1 ml absolutem Dimethylacetamid gelöst. Nach 20-stündigem Rühren wird die Lösung bei Raumtemperatur im Hochvakuum eingeengt. Der Rückstand wird aus Aethanol kristallisiert. Man erhält 16 mg (6R,7R)-7-[(Z)-2-(2-Amino-4-thiazolyl)-2-[[1-[3-(3,4-dihydroxybenzoyl)carbazoyl]-1-methyläthoxy]imino]-acetamido]-3-[[[2-(N-hydroxy-N-methylcarbamoyl)-5-methyl-s-triazolo[1,5-a]pyrimidin-7-yl]thio]methyl]-8-oxo-5-thia-1-azabicyclo[4.2.0]oct-2-en-2-carbonsäure als braunes Pulver.
$^1$H NMR (DMSO-$d_6$): Signale u.a. bei δ 1,44 (s, 3H), 1,46 (s, 3H), 2,61 (s, 3H), 3,30 (s, 3H), 5,25 (d, J = 5 Hz, 1H), 5,91 (dd, J = 5 Hz und J = 8 Hz, 1H), 6,76 (d, J = 7,5 Hz, 1H), 6,88 (s, 1H), 7,25 (dd, J = 7,5 Hz und J = 2 Hz, 1H), 7,30 (d, J = 2 Hz, 1H), 9,24 (s, 1H), 9,68 (d, J = 8 Hz, 1H), 9,98 (s, 1H), 10,44 (breit, 1H).

Die als Ausgangsmaterial eingesetzte (6R,7R)-7-(2-Amino-4-thiazolglyoxylamido)-3-[[[2-(N-hydroxy-N-methylcarbamoyl)-5-methyl-s-triazol[1,5-a]pyrimidin-7-yl]thio]methyl]-8-oxo-5-thia-1-azabicyclo[4.2.0]oct-2-en-2-carbonsäure kann wie folgt hergestellt werden:

3,36 g (15 mMol) 7-Mercapto-5-methyl-s-triazolo[1,5-a]pyrimidin-2-carbonsäuremethylester werden in 30 ml 2N wässriger Natronlauge gelöst. Nach 18-stündigem Rühren bei Raumtemperatur fällt man das Produkt durch Zugabe von 1N wässriger Salzsäure aus. Der Niederschlag wird abgenutscht und mit Wasser und Aceton gewaschen. Nach Kristallisation aus Dimethylacetamid/Diäthyläther erhält man 3,0 g 7-Mercapto-5-methyl-s-triazolo[1,5-a]pyrimidin-2-carbonsäure-Dimethylacetamid vom Schmelzpunkt 180 °C (Zers.).

0,99 g (3,33 mMol) 7-Mercapto-5-methyl-s-triazolo[1,5-a]pyrimidin-2-carbonsäure-Dimethylacetamid werden in 15 ml absolutem Dimethylacetamid suspendiert und unter Rühren mit 530 mg (3,96 mMol) 1-Chlor-N,N,2-trimethyl-1-propenamin versetzt. Nach 4 Stunden wird die Lösung auf -10 °C abgekühlt und unter Rühren mit 550 mg (6,6 mMol) N-Methylhydroxylaminhydrochloridund 860 mg (6,6 mMol) Aethyl-diisopropylamin versetzt. Das Reaktionsgemisch wird 18 Stunden bei Raumtemperatur gerührt und anschliessend im Hochvakuum eingeengt. Der Rückstand wird mit Wasser kristlalisiert und aus Dimethylformamid/Diäthyläther umkristallisiert. Man erhält 0,50 g N-Hydroxy-7-mercapto-N,5-dimethyl-s-triazolo[1,5-a]pyrimidin-2-carboxamid als gelbe Kristalle vom Schmelzpunkt 215 °C (Zers.).
$^1$H NMR (DMSO-$d_6$): Signale u.a. bei δ 2,35 (s, 3H), 3,29 (s, 3H), 6,91 (s, 1H), 10,46 (breit, 1H).

53 mg (0,22 mMol) N-Hydroxy-7-mercapto-N,5-dimethyl-s-triazolo[1,5-a]pyrimidin-2-carboxamid und 54 mg (0,2 mMol) 7-Aminocephalosporansäure werden in 1 ml Dichlormethan/Sulfolan (1:1 v/v) suspendiert. Bei 10 °C gibt man unter Rühren 0,5 ml Bortrifluorid-diäthylätherat dazu und rührt 20 Stunden bei Raumtemperatur weiter. Anschliessend gibt man ca. 10 ml Dichlormethan dazu, nutscht das unlösliche Material ab, löst es in Wasser auf und fällt durch Zugabe von Natriumhydrogencarbonat (pH→4) das Produkt aus. Der Niederschlag wird abgenutscht und mit Wasser und Aethanol gewaschen. Man erhält 66 mg (6R,7R)-7-Amino-3-[[[2-(N-hydroxy-N-methylcarbamoyl)-5-methyl-s-triazolo[1,5-a]pyrimidin-7-yl]thio]-methyl]-8-oxo-5-thia-1-azabicyclo[4,2,0]oct-2-en-2-carbonsäure als weisses Pulver.

$^1H$ NMR (DMSO-$d_6$): Signale u.a. bei $\delta$ 2,60 (s, 3H), 3,29 (s, 3H), 3,55 (d, J = 18 Hz, 1H), 3,76 (d, J = 18 Hz, 1H), 4,34 (d, J = 12,5 Hz, 1H), 4,43 (d, J = 12,5 Hz, 1H), 4,82 (d, J = 5 Hz, 1H), 5,02 (d, J = 5 Hz, 1H), 7,36 (s, 1H).

45 mg (0,1 mMol) (6R,7R)-7-Amino-3-[[[2-(N-hydroxy-N-methylcarbamoyl)-5-methyl-s-triazolo[1,5-a]-pyrimidin-7-yl]thio]methyl]-8-oxo-5-thia-1-azabicyclo[4,2,0]oct-2-en-2-carbonsäure werden in 1 ml absolutem Dimethylacetamid suspendiert. Bei 0 °C gibt man unter Rühren 0,052 ml (0,2 mMol) O,N-Bis-trimethylsilyl-acetamid dazu, rührt 1 Stunde bei 0 °C und 2 Stunden bei Raumtemperatur. Anschliessend versetzt man die Lösung mit 40 mg (0,125 mMol) 2-Amino-4-thiazol-thioglyoxylsäure-S-(2-benzothiazolyl)ester. Nach 18-stündigem Rühren bei Raumtemperatur zieht man das Lösungsmittel bei Raumtemperatur im Hochvakuum ab. Das Produkt wird durch Zugabe von Aethanol kristallisiert. Dieses wird abgenutscht und nacheinander mit Aethanol und Diäthyläther gewaschen. Man erhält 50 mg (6R,7R)-7-(2-Amino-4-thiazolglyoxylamido)-3-[-[[2-(N-hydroxy-N-methylcarbamoyl)-5-methyl-s-triazolo[1,5-a]pyrimidin-7-yl]thio]methyl]-8-oxo-5-thia-1-azabicyclo[4.2.0]oct-2-en-2-carbonsäure als braunes Pulver.

$^1H$ NMR (DMSO-$d_6$): Signale u.a. bei $\delta$ 2,61 (s, 3H), 3,29 (s, 3H), 3,63 (d, J = 18 Hz, 1H), 3,82 (d, J = 18 Hz, 1H), 4,41 (d, J = 12,5 Hz, 1H), 4,49 (d, J = 12,5 Hz, 1H), 5,21 (d, J = 5 Hz, 1H), 5,78 (dd, J = 5 Hz und J = 8 Hz, 1H), 7,4 (3H), 7,82 (s, 1H), 9,80 (d, J = 8 Hz, 1H), 10,44 (breit, 1H).

## Beispiel 52

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-(hydroxycarbamoyl)-5-(hydroxymethyl)-s-triazolo[1,5-a]pyrimidin-7-yl]-thio]methyl]-8-oxo-5-thia-1-azabicyclo[4.2.0]oct-2-en-2-carbonsäure

45 mg (0,074 mMol) (6R,7R)-7-(2-Amino-4-thiazolglyoxylamido)-3-[[[2-(hydroxycarbamoyl)-5-(hydroxymethyl)-s-triazolo[1,5-a]pyrimidin-7-yl]thio]methyl]-8-oxo-5-thia-1-azabicyclo[4.2.0]oct-2-en-2-carbonsäure und 31 mg (0,096 mMol) 1-[2-(Aminooxy)-2-methylpropionyl]-2-(3,4-dihydroxybenzoyl)-hydrazin-hydrochlorid werden in 3 ml absolutem Dimethylacetamid gelöst. Nach 24-stündigem Rühren bei Raumtemperatur wird die rötliche Lösung im Hochvakuum eingeengt. Der Rückstand wird in 6 ml Wasser suspendiert und das Produkt abgenutscht. Nach Waschen mit Wasser und Diäthyläther und anschliessendem Trocknen im Hochvakuum erhält man 50 mg (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-(hydroxycarbamoyl)-5-(hydroxymethyl)-s-triazolo[1,5-a]pyrimidin-7-yl]thio]methyl]-8-oxo-5-thia-1-azabicyclo[4.2.0]oct-2-en-2-carbonsäure als beiges Pulver.

$^1H$ NMR (DMSO-$d_6$): Signale u.a. bei $\delta$ 1,46 (s, 3H), 1,50 (s, 3H), 3,65 (d, J = 18 Hz, 1H), 3,87 (d, J = 18 Hz, 1H), 4,42 (d, J = 12,5 Hz, 1H), 4,52 (d, J = 12,5 Hz, 1H), 4,66 (s, 2H), 5,24 (d, J = 5 Hz, 1H), 5,91 (dd, J = 5 Hz und J = 8 Hz, 1H), 6,74 (d, J = 7,5 Hz, 1H), 6,87 (s, 1H), 7,26 (dd, J = 7,5 Hz und J = 2 Hz, 1H), 7,32 (d, J = 2 Hz, 1H), 7,48 (s, 1H), 9,20 (s, 1H), 9,22 (s, 1H), 9,39 (s, 1H), 9,53 (s, 1H), 9,67 (d, J = 8 Hz, 1H), 10,01 (s, 1H), 11,69 (s, 1H).

Die als Ausgangsmaterial eingesetzte (6R,7R)-7-(2-Amino-4-thiazolglyoxylamido)-3-[[[2-(hydroxycarbamoyl)-5-(hydroxymethyl)-s-triazolo[1,5-a]pyrimidin-7-yl]thio]methyl]-8-oxo-5-thia-1-azabicyclo-[4.2.0]oct-2-en-2-carbonsäure kann wie folgt hergestellt werden:

Ein Gemisch aus 71 mg (0,5 mMol) 5-Amino-4H-s-triazol-3-carbonsäuremethylester und 188 mg (1,0 mMol) 4-Acetoxy-3-oxo-buttersäureäthylester wird 40 Minuten bei ca. 160 °C gehalten. Nach dem Abkühlen wird das Produkt aus Methanol kristallisiert und umkristallisiert. Man erhält 110 mg Methyl 5-(acetoxymethyl)-4,7-dihydro-7-oxo-s-triazolo[1,5-a]pyrimidin-2-carboxylat als beige Kristalle vom Schmelzpunkt 223 °C (Zers.).

Eine Suspension von 100 mg (0,38 mMol) Methyl 5-(acetoxymethyl)-4,7-dihydro-7-oxo-s-triazolo[1,5-a]-pyrimidin-2-carboxylat in 7 ml Phosphoroxychlorid wird 6 Stunden unter Rückfluss gehalten und anschliessend im Vakuum eingeengt. Der Rückstand wird in Dichlormethan gelöst. Die Lösung wird mit Eis/Wasser gewaschen, über Natriumsulfat getrocknet und eingeengt. Nach Kristallisation aus Methanol/Diäthyläther

erhält man 70 mg Methyl 5-(acetoxymethyl)-7-chlor-s-triazolo[1,5-a]pyrimidin-2-carboxylat als braune Kristalle vom Schmelzpunkt 120 ° C.

380 mg (1,33 mMol) Methyl 5-(acetoxymethyl)-7-chlor-s-triazolo[1,5-a]pyrimidin-2-carboxylat werden in 20 ml Methanol gelöst. Nach Zugabe von 296 mg (4 mMol) Natriumhydrogensulfid-monohydrat wird die Mischung 5 Stunden bei 60 ° C gerührt. Das Reaktionsgemisch wird eingeengt und mit verdünnter wässriger Salzsäure auf pH 3 gebracht. Das Produkt wird abgenutscht, mit Wasser/Methanol gewaschen und aus Methanol kristallisiert. Man erhält 290 mg Methyl 5-(hydroxymethyl)-7-mercapto-s-triazolo[1,5-a]pyrimidin-2-carboxylat als braunes Pulver.

$^1$H NMR ($DMSO-d_6$): Signale u.a. bei $\delta$ 3,89 (s, 3H), 4,36 (s, 2H), 5,35 (breit, 1H), 7,00 (s, 1H).

100 mg (0,416 mMol) Methyl 5-(hydroxymethyl)-7-mercapto-s-triazolo[1,5-a]pyrimidin-2-carboxylat werden in etwa 5 ml Methanol gelöst. Unter Rühren bei Raumtemperatur gibt man innerhalb 48 Stunden 275 mg (8,3 mMol) Hydroxylamin dazu. Das Reaktionsgemisch wird mit 3N wässriger Salzsäure sauer gestellt (pH 5,5). Das Produkt wird abgenutscht und aus Methanol kristallisiert. Man erhält 60 mg 7-Mercapto-N-hydroxy-5-(hydroxymethyl)-s-triazolo[1,5-a]pyrimidin-2-carboxamid als beige Kristalle vom Schmelzpunkt 238-239 ° C (Zers.).

$^1$H NMR ($DMSO-d_6$): Signale u.a. bei 4,36 (s, 2H), 5,34 (breit, 1H), 6,98 (s, 1H).

300 mg (1,24 mMol) 7-Mercapto-N-hydroxy-5-(hydroxymethyl)-s-triazolo[1,5-a]pyrimidin-2-carboxamid und 305 mg (1,12 mMol) 7-Aminocephalosporansäure werden in 50 ml Sulfolan/Dichlormethan (1:1 v/v) suspendiert. Bei 0 ° C gibt man 5 ml Bortrifluorid-diäthylätherat dazu. Das Reaktionsgemisch wird 2 1/2 Stunden bei 0 ° C und 24 Stunden bei Raumtemperatur gerührt. Nach Zugabe von 30 ml Dichlormethan wird das Ganze 15 Minuten gerührt und anschliessend filtriert. Das Nutschgut wird in Wasser gelöst und mit gesättigter wässriger Natriumhydrogencarbonatlösung auf pH 5 eingestellt. Das ausgefallene Produkt wird abgenutscht und nacheinander mit Wasser, Dichlormethan und Diäthyläther gewaschen. Man erhält 340 mg (6R,7R)-7-Amino-3-[[[2-(hydroxycarbamoyl)-5-(hydroxymethyl)-s-triazolo[1,5-a]pyrimidin-7-yl]thio]methyl]-8-oxo-5-thia-1-azabicyclo[4.2.0]oct-2-en-2-carbonsäure als beiges Pulver.

$^1$H NMR ($DMSO-d_6$): Signale u.a. bei $\delta$ 3,58 (d, J = 18 Hz, 1H), 3,79 (d, J = 18 Hz, 1H), 4,40 (d, J = 12,5 Hz, 1H), 4,46 (d, J = 12,5 Hz, 1H), 4,65 (s, 2H), 4,84 (d, J = 5 Hz, 1H), 5,03 (d, J = 5 Hz, 1H), 7,49 (s, 1H), 9,36 (s, 1H), 11,69 (s, 1H).

45 mg (0,1 mMol) (6R,7R)-7-Amino-3-[[[2-(hydroxycarbamoyl)-5-(hydroxymethyl)-s-triazolo[1,5-a]-pyrimidin-7-yl]thio]methyl]-8-oxo-5-thia-1-azabicyclo[4.2.0]oct-2-en-2-carbonsäure werden in 4 ml absolutem Dimethylacetamid suspendiert. Bei 0 ° C gibt man 61 mg (0,3 mMol) O,N-Bis-trimethylsilyl-acetamid dazu, erwärmt auf Raumtemperatur und rührt noch 50 Minuten nach. Bei 0 ° C fügt man zur Lösung 39 mg (0,12 mMol) 2-Amino-4-thiazol-thioglyoxylsäure-S-(2-benzothiazolyl)ester hinzu und rührt anschliessend 24 Stunden bei Raumtemperatur. Nach Zugabe von 2 ml Aethanol und 15-minütigem Rühren wird das Lösungsmittel bei Raumtemperatur im Hochvakuum abgezogen. Das Produkt wird durch Zugabe von Aethanol kristallisiert. Nach Trocknen bei Raumtemperatur im Hochvakuum erhält man 50 mg (6R,7R)-7-(2-Amino-4-thiazolglyoxylamido)-3-[[[2-(hydroxycarbamoyl)-5-(hydroxymethyl)-s-triazolo[1,5-a]pyrimidin-7-yl]thio]-methyl]-8-oxo-5-thia-1-azabicyclo[4.2.0]oct-2-en-2-carbonsäure als beiges Pulver.

$^1$H NMR ($DMSO-d_6$): Signale u.a. bei $\delta$ 3,63 (d, J = 18 Hz, 1H), 3,84 (d, J = 18 Hz, 1H), 4,44 (d, J = 12,5 Hz, 1H), 4,52 (d, J = 12,5 Hz, 1H), 4,66 (s, 2H), 5,21 (d, J = 5 Hz, 1H), 5,78 dd (J = 5 Hz und J = 8 Hz), 7,41 (s, 2H), 7,50 (s, 1H), 7,83 (s, 1H), 9,37 (s, 1H), 9,81 (d, J = 8 Hz, 1H), 11,69 (s, 1H).

## Beispiel 53

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[5-(hydroxycarbamoyl)-2-methyl-s-triazolo[1,5-a]pyrimidin-7-yl]thio]-methyl]-8-oxo-5-thia-1-azabicyclo[4.2.0]oct-2-en-2-carbonsäure

55 mg (0,093 mMol) (6R,7R)-7-(2-Amino-4-thiazolglyoxylamido)-3-[[[5-(hydroxycarbamoyl)-2methyl-s-triazolo[1,5-a]pyrimidin-7-yl]thio]methyl]-8-oxo-5-thia-1-azabicyclo[4.2.0]oct-2-en-2-carbonsäure und 36,9 mg (0,121 mMol) 1-[2-(Aminooxy)-2-methylpropionyl]-2-(3,4-dihydroxybenzoyl)hydrazin-hydrochlorid werden in 7 ml absolutem Dimethylacetamid gelöst. Nach 20-stündigem Rühren bei Raumtemperataur werden nochmals 18,4 mg (0,060 mMol) 1-[2-(Aminooxy)-2-methylpropionyl]-2-(3,4-dihydroxybenzoyl)hydrazin-hydrochlorid zugegeben. Nach weiteren 24 Stunden wird das Lösungsmittel im Hochvakuum bei Raumtemperatur abgezogen und der Rückstand mit Wasser versetzt. Das ausgefallene Produkt wird abfiltriert, mit Wasser gut ausgewaschen und im Hochvakuum bei Raumtemperatur getrocknet. Man erhält 37 mg (6R,7R)-7-[(Z)-2-(2-Amino-4-thiazolyl)-2-[[1-[3-(3,4-dihydroxybenzoyl)carbazoyl]-1-methyläthoxy]imino]-acetamido]-3-[[[5-(hydroxycarbamoyl)-2-methyl-s-triazolo[1,5-a]pyrimidin-7-yl]thio]methyl]-8-oxo-5-thia-1-

azabicyclo[4.2.0]oct-2-en-2-carbonsäure als beiges Pulver.

$^1$H NMR (DMSO-$d_6$): Signale u.a. bei δ 1,47 (s, 3H), 1,50 (s, 3H), 3,64 (d, J = 18 Hz, 1H), 3,85 (d, J = 18 Hz, 1H), 4,47 (d, J = 12,5 Hz, 1H), 4,57 (d, J = 12,5 Hz, 1H), 5,24 (d, J = 5 Hz, 1H), 5,92 (dd, J = 5 Hz und J = 8 Hz, 1H), 6,76 (d, J = 7,5 Hz, 1H), 6,86 (s, 1H), 7,25 (dd, J = 7,5 Hz und J = 2Hz, 1H), 7,30 (d, J = 2Hz, 1H), 7,71 (s, 1H), 9,18 (s, 1H), 9,23 (s, 1H), 9,34 (s, 1H), 9,54 (s, 1H), 9,66 (d, J = 8 Hz, 1H), 10,01 (s, 1H), 11,88 (s, 1H).

Die als Ausgangsverbindung eingesetzte (6R,7R)-7-(2-Amino-4-thiazolglyoxylamido)-3-[[[5-(hydroxycarbamoyl)-2-methyl-s-triazolo[1,5-a]pyrimidin-7-yl]thio]methyl]-8-oxo-5-thia-1-azabicyclo[4.2.0]oct-2-en-2-carbonsäure kann wie folgt hergestellt werden:

112 mg 3-Amino-5-methyl-1H-s-triazol und 320 mg 2-Oxo-bernsteinsäuredimethylester werden in 5 ml Eisessig gelöst. Nach 22-stündigem Kochen unter Rückfluss wird das Lösungsmittel im Vakuum abdestilliert. Der Rückstand wird in warmem Methanol aufgeschlämmt. Nach dem Abkühlen wird das Produkt abfiltriert und mit Methanol mehrmals gewaschen. Nach Trocknen im Hochvakuum erhält man 73 mg Methyl-7-hydroxy-2-methyl-s-triazolo[1,5-a]pyrimidin-5-carboxylat als beiges Pulver, Smp. >220 °C).

$^1$H NMR (DMSO-$d_6$): Signale u.a. bei δ 2,44 (s, 3H), 3,88 (s, 3H), 6,54 (s, 1H).

1,0 g (4,8 mMol Methyl-7-hydroxy-2-methyl-s-triazolo[1,5-a]pyrimidin-5-carboxylat wird in 30 ml Phosphoroxychlorid suspendiert. Man gibt 0,38 ml (4,8 mMol) Pyridin dazu und rührt 5 Stunden bei 90 °C. Anschliessend engt man ein und gibt zum Rückstand Eis/Wasser und gesättigte wässrige Kochsalzlösung. Das Produkt wird mit Dichlormethan extrahiert. Nach Trocknen und Eindampfen des Extraktes verbleiben 0,81 g Methyl 7-chlor-2-methyl-s-triazolo[1,5-a]pyrimidin-5-carboxylat als beige Kristalle mit Schmelzpunkt 170-174 °C.

$^1$H NMR (DMSO-$d_6$): Signale u.a. bei δ 2,61 (s, 3H), 3,97 (s, 3H), 8,08 (s, 1H).

0,80 g (3,53 mMol) Methyl-7-chlor-2-methyl-s-triazolo[1,5-a]pyrimidin-5-carboxylat werden in 35 ml Methanol suspendiert und mit 0,78 g (10,6 mMol) Natriumhydrogensulfidhydrat versetzt. Nach 5-ständigem Rühren bei 60 °C wird eingeengt und der Rückstand in 20 ml Wasser gelöst. Man säuert bis zu pH 3 mit 3N wässriger Salzsäure an. Das ausgefallene Produkt wird abgenutscht und mit Wasser und Methanol gewaschen. Nach Umkristallisation aus Methanol/Dichlormethan erhält man 0,66 g Methyl-7-mercapto-2-methyl-s-triazolo[1,5-a]pyrimidin-5-carboxylat als gelbe Kristalle vom Schmelzpunkt 237 °C (Zers.).

$^1$H NMR (DMSO-$d_6$): Signale u.a. bei δ 2,54 (s, 3H), 3,88 (s, 3H), 7,51 (s, 1H).

500 mg (2,23 mMol) Methyl-7-mercapto-2-methyl-s-triazolo[1,5-a]pyrimidin-5-carboxylat werden in 10 ml absolutem Methanol suspendiert. Innerhalb 4 Tage werden unter Rühren 2,95 mg (8,9 mMol) Hydroxylamin zugegeben. Das Reaktionsgemisch wird mit 3N wässriger Salzsäure angesäuert (pH 6). Das Produkt wird abgenutscht und nacheinander mit Wasser, Methanol und Diäthyläther gewaschen. Man erhält 540 mg N-Hydroxy-7-mercapto-2-methyl-s-triazolo[1,5-a]pyrimidin-5-carboxamid als gelbe Kristalle vom Schmelzpunkt 178-179 °C.

$^1$H NMR (DMSO-$d_6$): Signale u.a. bei δ 2,38 (s, 3H), 7,21 (s, 1H).

250 mg (1,1 mMol) N-Hydroxy-7-mercapto-2-methyl-s-triazolo[1,5-a]pyrimidin-5-carboxamid und 275 mg (1,0 mMol) 7-Amino-cephalosporinsäure werden in 15 ml Sulfolan/Dichlormethan (1:1 v/v) suspendiert und bei 0 °C mit 3,5 ml Bortrifluorid-diäthylätherat versetzt. Man rührt 2 Stunden bei 0-5 °C und 3 Stunden bei Raumtemperatur. Anschliessend fügt man 60 ml Dichlormethan zu. Unlösliches Material wird abgenutscht, in Wasser gelöst und mit gesättigter Natriumbicarbonatlösung versetzt, bis der pH-Wert 3 erreicht ist. Das ausgefallene Produkt wird abgenutscht und nacheinander mit Wasser und Diäthyläther gewaschen. Man erhält 295 mg (6R,7R)-7-Amino-3-[[[5-(hydroxycarbamoyl)-2-methyl-s-triazolo[1,5-a]pyrimidin-7-yl]thio]-methyl]-8-oxo-5-thia-1-azabicyclo[4.2.0]oct-2-en-2-carbonsäure als beiges Pulver.

$^1$H NMR (DMSO-$d_6$): Signale u.a. bei δ 2,54 (s, 3H), 3,56 (d, J = 18 Hz, 1H), 3,78 (d, J = 18 Hz, 1H), 4,45 (d, J = 12,5 Hz, 1H), 4,51 (d, J = 12,5 Hz, 1H), 4,82 (d, J = 5 Hz, 1H), 4,98 (d, J = 5 Hz, 1H), 7,72 (s, 1H), 9,34 (s, 1H), 11,88 (s, 1H).

160 mg (0,36 mMol) (6R,7R)-7-Amino-3-[[[5-(hydroxycarbamoyl)-2-methyl-s-triazolo[1,5-a]pyrimidin-7-yl]thio]methyl]-8-oxo-5-thia-1-azabicyclo[4.2.0]oct-2-en-2-carbonsäure werden in 2 ml absolutem Dimethylacetamid suspendiert. Bei 0 °C gibt man 146 mg (0,72 mMol) O,N-Bistrimethyisilyl-acetamid dazu und rührt 2 Stunden. Die Lösung wird anschliessend mit 116 mg (0,36 mMol) 2-Amino-4-thiazol-thioglyoxylsäure-S-(2-benzothiazolyl)ester. versetzt und 2 Stunden bei 0 °C und 16 Stunden bei Raumtemperatur gerührt. Man gibt etwas Aethanol dazu und engt nach 20 Minuten im Hochvakuum bei Raumtemperatur ein. Der Rückstand wird mit Wasser versetzt. Das ausgefallene Produkt wird abfiltriert und nacheinander mit Wasser, Aethanol, Essigester und Diäthyläther gewaschen. Man erhält 170 mg (6R,7R)-7-(2-Amino-4-thiazolglyoxylamido)-3-[[[5-(hydroxycarbamoyl)-2-methyl-s-triazolo[1,5-a]pyrimidin-7-yl]thio]methyl]-8-oxo-5-thia-1-azabicyclo[4.2.0]oct-2-en-2-carbonsäure als beiges Pulver.

$^1$H NMR (DMSO-$d_6$): Signale u.a. bei δ 2,54 (s, 3H), 3,62 (d, J = 18 Hz, 1H), 3,81 (d, J = 18 Hz, 1H), 4,47 (d,

J = 12,5 Hz, 1H), 4,60 (d, J = 12,5 Hz, 1H), 5,19 (d, J = 5 Hz, 1H), 5,79 (dd, J = 5 Hz und J = 8 Hz, 1H), 7,41 (s, 2H), 7,74 (s, 1H), 7,82 (s, 1H), 9,34 (s, 1H), 9,80 (d, J = 8 Hz, 1H), 11,89 (s, 1H).

Beispiel 54

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[5-(hydroxycarbamoyl)-2-(hydroxymethyl)-s-triazolo[1,5-a]pyrimidin-7-yl]-thio]methyl]-8-oxo-5-thia-1-azabicyclo[4.2.0]oct-2-en-2-carbonsäure

50 mg (0,082 mMol) (6R,7R)-7-(2-Amino-4-thiazolglyoxylamido)-3-[[[5-(hydroxycarbamoyl)-2-(hydroxymethyl)-s-triazolo[1,5-a]pyrimidin-7-yl]thio]methyl]-8-oxo-5-thia-1-azabicyclo[4.2.0]oct-2-en-2-carbonsäure und 34 mg (0,11 mMol) 1-[2-(Aminooxy)-2-methylpropionyl]-2-(3,4-dihydroxybenzoyl)-hydrazin-hydrochlorid werden in 4 ml absolutem Dimethylacetamid gelöst. Nach 24-ständigem Rühren bei Raumtemperatur wird das Lösungsmittel bei Raumtemperatur abgezogen. Der Rückstand wird mit Wasser behandelt. Das ausgefallene Produkt wird abgenutscht und nacheinander mit Wasser und Diäthyläther gewaschen. Man erhält 50 mg (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[5-(hydroxycarbamoyl)-2-(hydroxymethyl)-s-triazolo[1,5-a]pyrimidin-7-yl]-thio]methyl]-8-oxo-5-thia-1-azabicyclo[4.2.0]oct-2-en-2-carbonsäure als beiges Pulver.

$^1$H NMR (DMSO-$d_6$): Signale u.a. bei δ 1,47 (s, 3H), 1,48 (s, 3H), 3,64 (d, J = 18 Hz, 1H), 3,88 (d, J = 18 Hz, 1H), 4,48 (d, J = 12,5 Hz, 1H), 4,58 (d, J = 12,5 Hz, 1H), 4,68 (s, 2H), 5,23 (d, J = 5 Hz, 1H), 5,92 (dd, J = 5 Hz und J = 8 Hz), 6,75 (d, J = 7,5 Hz), 6,84 (s, 1H), 7,25 (dd, J = 7,5 Hz und J = 2 Hz, 1H), 7,29 (d, J = 2 Hz, 1H), 7,3 (s, 3H), 9,18 (s, 1H), 9,22 (s, 1H) 9,36 (breit, 1H), 9,54 (s, 1H), 9,67 (d, J = 8 Hz, 1H), 9,98 (s, 1H), 11,90 (s, 1H).

Die als Ausgangsmaterial eingesetzte (6R,7R)-7-(2-Amino-4-thiazolglyoxylamido)-3-[[[5-(hydroxycarbamoyl)-2-(hydroxymethyl)-s-triazolo[1,5-a]pyrimidin-7-yl]thio]methyl]-8-oxo-5-thia-1-azabicyclo-[4.2.0]oct-2-en-2-carbonsäure kann wie folgt hergestellt werden;

2,5 g (11,1 mMol) Methyl-4,7-dihydro-2-(hydroxymethyl)-7-oxo-s-triazolo[1,5-a]pyrimidin-5-carboxylat werden in 20 ml Dichlormethan suspendiert. Nach Zugabe von 1,76 g (22,3 mMol) Pyridin und 2,27 g (22,2 mMol) Essigsäureanhydrid rührt man 8 Stunden bei 55 °C. Man engt im Vakuum ein und fällt das Produkt mit Diäthyläther aus. Nach dem Umkristallisieren aus Methanol erhält man 1,2 g Methyl-2-(acetoxymethyl)-4,7-dihydro-7-oxo-s-triazolo[1,5-a]pyrimidin-5-carboxylat als weisse Kristalle vom Schmelzpunkt 206 °C.

500 mg (1,87 mMol) Methy-2-(acetoxymethyl)-4,7-dihydro-7-oxo-s-triazolo[1,5-a]pyrimidin-5-carboxylat werden in 20 ml Phosphoroxychlorid suspendiert. Die Suspension wird 5 Stunden unter Rückfluss gesetzt. Man engt im Wasserstrahlvakuum ein, versetzt den Rückstand mit Dichlormethan und Eis/Wasser und extrahiert mehrmals mit Dichlormethan. Nach Trocknen und Eindampfen des Extraktes wird das Produkt aus Methanol kristallisiert. Man erhält 400 mg Methyl-2-(acetoxymethyl)-7-chlor-s-triazolo[1,5-a]pyrimidin-5-carboxylat.

$^1$H NMR ($CDCl_3$): Signale bei δ 2,21 (s, 3H), 4,09 (s, 3H), 5,47 (s, 2H), 7,99 (s, 1H).

370 mg (1,3 mMol) Methyl-2-(acetoxymethyl)-7-chlor-s-triazolo[1,5-a]pyrimidin-5-carboxylat werden in 10 ml Methanol gelöst. Nach Zugabe von 289 mg (3,9 mMol) Natriumhydrogensulfid-hydrat rührt man 4 Stunden bei 60 °C. Die gelbe Lösung wird im Vakuum eingeengt. Der Rückstand wird in Wasser gelöst und das Produkt mit 3N wässriger Salzsäure bei pH 3 ausgefällt. Der Niederschlag wird abgenutscht und aus Methanol kristallisiert. Man erhält 360 mg Methyi-2-(acetoxymethyl)-7-mercapto-s-triazolo[1,5-a]pyrimidin-5-carboxylat.

$^1$H NMR (DMSO-$d_6$): Signale u.a. bei δ 2,13 (s, 3H), 3,89 (s, 3H), 5,28 (s, 2H), 7,42 (s, 1H).

80 mg (0,28 mMol) Methyl-2-(acetoxymethyl)-7-mercapto-s-triazolo[1,5-a]pyrimidin-5-carboxylat werden in 3 ml absolutem Methanol gelöst. Innerhalb 4 Tage werden unter Rühren 188 mg (5,7 mMol) Hydroxylamin zugegeben. Das Produkt wird mit 3N wässriger Salzsäure ausgefällt (pH 6), abgenutscht und mit Methanol gewaschen. Nach Kristallisation aus Methanol erhält man 50 mg N-Hydroxy-2-(hydroxymethyl)-7-mercapto-s-triazolo[1,5-a]pyrimidin-5-carboxamid als gelbes Pulver.

$^1$H NMR (DMSO-$d_6$): Signale u.a. bei δ 4,54 (s, 2H), 7,24 (s, 1H).

190 mg (0,79 mMol) N-Hydroxy-2-(hydroxymethyl)-7-mercapto-s-triazolo[1,5-a]pyrimidin-5-carboxamid und 193 mg (0,71 mMol) 7-Aminocephalosporansäure werden in 20 ml Sulfolan/Dichlormethan (1:1 v/v) suspendiert. Bei 0 °C werden 4,2 ml Bortrifluorid-diäthylätherat dazugegeben. Man rührt 2 Stunden bei 0 °C und 20 Stunden bei Raumtemperatur. 80 ml Dichlormethan werden zugefügt. Das ausgefällte Material wird abgetrennt und in Wasser gelöst. Durch Zugabe von gesättigter wässriger Natriumhydrogencarbonatlösung wird auf pH 4 eingestellt. Das ausgefällte Produkt wird abgenutscht und nacheinander mit Wasser, Aethanol und Diäthyläther gewaschen. Man erhält 130 mg (6R,7R)-7-Amino-3-[[[5-(hydroxycarbamoyl)-2-(hydroxymethyl)-s-triazolo[1,5-a]pyrimidin-7-yl]thio]methyl]-8-oxo-5-thia-1-azabicyclo[4.2.0]oct-2-en-2-carbonsäure als beiges Pulver.
$^1$H NMR (DMSO-$d_6$): Signale u.a. bei $\delta$ 3,56 (d, J = 18 Hz, 1H), 3,80 (d, J = 18 Hz, 1H), 4,44 (d, J = 12,5 Hz, 1H), 4,54 (d, J = 12,5 Hz, 1H), 4,69 (s, 2H), 4,82 (d, J = 5 Hz, 1H), 5,01 (d, J = 5 Hz, 1H), 5,63 (breit, 1H), 7,76 (s, 1H), 9,37 (s, 1H), 11,90 (s, 1H).

45 mg (0,1 mMol) (6R,7R)-7-Amino-3-[[[-5-(hydroxycarbamoyl)-2-(hydroxymethyl)-s-triazolo[1,5-a]-pyrimidin-7-yl]thio]methyl]-8-oxo-5-thia-1-azabicyclo[4.2.0]oct-2-en-carbonsäure werden in 4 ml absolutem Dimethylacetamid suspendiert. Man gibt bei 0 °C 61 mg (0,3 mMol) O,N-Bis-trimethylsilylacetamid dazu und rührt 70 Minuten bei Raumtemperatur. Anschliessend wird die Lösung auf 0 °C abgekühlt und mit 39 mg (0,12 mMol) 2-Amino-4-thiazol-thioglyoxylsäure-S-(2-benzothiazolyl)ester versetzt. Nach 24-stündigem Rühren bei Raumtemperatur gibt man 0,2 ml Aethanol dazu und engt bei Raumtemperatur im Hochvakuum ein. Der Rückstand wird mit Aethanol kristallisiert. Man erhält nach Abnutschen und Waschen mit Aethanol 53 mg (6R,7R)-7-(2-Amino-4-thiazolglyoxylamido)-3-[[[5-(hydroxycarbamoyl)-2-(hydroxymethyl)-s-triazolo-[1,5-a]pyrimidin-7-yl]thio]methyl]-8-oxo-5-thia-1-azabicyclo[4.2.0]oct-2-en-2-carbonsäure als beiges Pulver.
$^1$H NMR (DMSO-$d_6$): Signale u.a. bei $\delta$ 3,64 (d, J = 18 Hz, 1H), 3,82 (d, J = 18 Hz, 1H), 4,70 (s, 2H), 5,20 (d, J = 5 Hz, 1H), 5,78 (dd, J = 5 Hz und J = 8 Hz), 7,4 (3H), 7,82 (s, 1H), 9,36 (breit, 1H), 9,80 (d, J = 8 Hz, 1H), 11,89 (s, 1H).

## Beispiel 55

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[5-[2-(hydroxycarbamoyl)äthyl]-2-methyl-s-triazolo[1,5-a]pyrimidin-7-yl]-thio]methyl]-8-oxo-5-thia-1-azabicyclo[4.2.0]oct-2-en-2-carbonsäure

41 mg (0,066 mMol) (6R,7R)-7-(2-Amino-4-thiazolglyoxylamido)-3-[[[5-[2-(hydroxycarbamoyl)äthyl]-2-methyl-s-triazolo[1,5-a]pyrimidin-7-yl]thio]methyl]-8-oxo-5-thia-1-azabicyclo[4.2.0]oct-2-en-2-carbonsäure und 29 mg 1-[2-(Aminooxy)-2-methylpropionyl]-2-(3,4-dihydroxybenzoyl)hydrazin-hydrochlorid werden in 1 ml absolutem Dimethylacetamid gelöst. Nach 20-ständigem Rühren bei Raumtemperatur wird das Lösungsmittel im Hochvakuum bei Raumtemperatur abgezogen. Der Rückstand wird aus Aethanol/Diäthyläther kristallisiert. Das Produkt wird abfiltriert, mit Diäthyläther, Wasser und wieder mit Diäthyläther gewaschen. Man erhält nach Trocknen im Hochvakuum bei Raumtemperatur 45 mg (6R,7R)-7-[(Z)-2-(2-Amino-4-thiazolyl)-2-[[1-[3-(3,4-dihydroxybenzoyl)carbazoyl]-1-methyläthoxy]imino]acetamido][-3-[[[5-[2-(hydroxycarbamoyl)äthyl]-2-methyl-s-triazolo[1,5-a]pyrimidin-7-yl]thio]methyl]-8-oxo-5-thia-1-azabicyclo-[4.2.0]oct-2-en-2-carbonsäure als beiges Pulver.
$^1$H NMR (DMSO-$d_6$): Signale u.a. bei $\delta$ 1,47 (6H), 3,06 (t, J = 8 Hz, 2H), 3,62 (d, J = 18 Hz, 1H), 3,84 (d, J = 18 Hz, 1H), 4,33 (d, J = 12,5 Hz, 1H), 4,47 (d, J = 12,5 Hz, 1H), 5,26 (d, J = 5 Hz, 1H), 5,90 (dd, J = 5 Hz und J = 8 Hz, 1H), 6,76 (dd, 1H), 6,86 (s, 1H), 7,16-7,38 (5H), 8,74 (s (breit), 1H), 9,18 (s, 1H), 9,21 (s, 1H), 9,54 (s, 1H), 9,67 (d, J = 8 Hz, 1H), 10,02 (s, 1H), 10,5 (s, 1H).

Die als Ausgangsmaterial eingesetzte (6R,7R)-7-(2-Amino-4-thiazolglyoxylamido)-3-[[[5-[2-(hydroxycarbamoyl)äthyl]-2-methyl-s-triazolo[1,5-a]pyrimidin-7-yl]thio]methyl]-8-oxo-5-thia-1-azabicyclo-[4.2.0]oct-2-en-2-carbonsäure kann wie folgt hergestellt werden:

19,8 g (ca. 0,12 Mol) 3-Amino-5-methyl-1H-s-triazol (ca. 70%-ig) und 30 g (0,16 Mol) 3-Oxo-adipinsäuredimethylester werden unter Argonbegasung in einem 160 °C warmen Bad 1 Stunde zusammen gescbmolzen. Dabei werden die flüchtigen Produkte im Wasserstrahlvakuum abgezogen. Der feste Rückstand wird nach dem Abkühlen in Dichlormethan/Methanol (1:1 v/v) gelöst. Die trübe gelbliche Lösung wird filtriert und konzentriert. Die dabei entstandenen Kristalle werden abgenutscht und mit Aethanol, anschliessend noch mit Diäthyläther, gewaschen. Man erhält 20 g Methyl-4,7-dihydro-2-methyl-7-oxo-s-triazolo[1,5-a]pyrimidin-5-propionat als weisse Kristalle vom Schmelzpunkt 222-225 °C.

Ein Gemisch aus 5,0g (21 mMol) Methyl-4,7-dihydro-2-methyl-7-oxo-s-triazolo[1,5-a]pyrimidin-5-propionat, 250 ml Phosphoroxychlorid und 2,5 ml Pyridin wird ca. 2 Stunden bei 100 °C gerührt und anschliessend im Wasserstraalvakuum bei 50 °C eingeengt. Der Rückstand wird in 600 ml Dichlormethan und 200 ml gesättigte, wässrige Kochsalzlösung aufgenommen. Nach kräftiger Durchmischung wird die wässrige Phase abgetrennt und mit Dichlormethan gewaschen. Die organischen Lösungen werden vereinigt, über Natriumsulfat getrocknet und eingeengt. Es verbleiben 5,4 g Methyl-7-chlor-2-methyl-s-triazolo[1,5-a]pyrimidin-5-propionat, welches beim Stehen fest wird.
$^1$H NMR (DMSO-$d_6$): Signale u.a. bei $\delta$ 2,86 (t, J = 8 Hz, 2H), 3,17 (t, J = 8 Hz, 2H), 3,59 (s, 3H), 7,64 (s, 1H).

Zu einem Gemisch von 5,4 g (ca. 21 mMol) Methyl-7-chlor-2-methyl-s-triazolo[1,5-a]pyrimidin-5-propionat in 200 ml Methanol gibt man 7,0 g (94 mMol) Natriumhydrogensulfid-hydrat. Man rührt das Gemisch 2

Stunden bei 60°C. Anschliessend zieht man das Methanol im Vakuum ab und versetzt den Rückstand mit 150 ml kaltem Wasser. Man stellt das Gemisch auf pH 3, nutscht das Produkt ab und wäscht es nacheinander mit Wasser, Aethanol und Diäthyläther. So erhält man 5 g Methyl-2-methyl-7-mercapto-s-triazolo[1,5-a]pyrimidin-5-propionat als gelbe Kristalle vom Schmelzpunkt 222°C (Zers.).
$^1$H NMR (DMSO-$d_6$): Signale u.a. bei 2,46 (s, 3H), 2,7-2,9 (4H), 3,60 (s, 3H), 6,93 (s, 1H).

100 mg Methyl-2-methyl-7-mercapto-s-triazolo[1,5-a]pyrimidin-5-propionat werden in 10 ml Methanol gelöst und portionenweise mit Hydroxylamin versetzt. Nach 5-tägigem Stehen bei Raumtemperatur wird das Lösungsmittel abgezogen und das Produkt aus Aethanol kristallisiert. Man erhält 60 mg N-Hydroxy-5-mercapto-2-methyl-s-triazolo[1,5-a]pyrimidin-5-propionamid.
$^1$H NMR (DMSO-$d_6$): Signale u.a. bei δ 2,31 (s, 3H), 2,38 (t, J=8 Hz, 2H), 2,69 (t, J=8 Hz, 2H), 6,55 (s, 1H), 8,72 (s, 1H), 10,43 (s, 1H).

Zu einer Suspension von 305 mg (1,12 mMol) 7-Aminocephalosporansäure und 318 mg (1,25 mMol N-Hydroxy-5-mercapto-2-methyl-s-triazolo[1,5-a]pyrimidin-5-propionamid in 4 ml Dichlormethan/Sulfolan (1:1 v/v) tropft man bei 0°C 2 ml Bortrifluorid-diäthylätherat. Anschliessend rührt man 16 Stunden bei Raumtemperatur. Die Lösung wird mit 50 ml Dichlormethan versetzt. Eine beige Substanz kristallisiert aus. Sie wird abfiltriert, mit Diäthyläther gewaschen und in 5 ml Wasser gelöst. Man stellt die Lösung mit wässriger Natriumhydrogencarbonatlösung auf etwa pH 4. Durch Zugabe von ca. 100 ml Aethanol wird das Produkt kristallisiert. Die Kristalle werden abfiltriert, mit Aethanol gewaschen und im Hochvakuum bei Raumtemperatur getrocknet. Man erhält 311 mg (6R,7R)-7-Amino-3-[[[5-[2-(hydroxycarbamoyl)äthyl]-2-methyl-s-triazolo[1,5-a]pyrimidin-7-yl]thio]methyl]-8-oxo-5-thia-1-azabicyclo[4.2.0]oct-2-en-2-carbonsäure als beiges Pulver.
$^1$H NMR (DMSO-$d_6$): Signale u.a. bei δ 3,06 (t, J=8 Hz, 2H), 3,53 (d, J=18 Hz, 1H), 3,77 (d, J=18 Hz, 1H), 4,32 (d, J=12,5 Hz, 1H), 4,45 (d, J=12,5 Hz, 1H), 4,81 (d, J=5 Hz, 1H), 5,02 (d, J=5 Hz, 1H), 7,26 (s, 1H), 8,74 (breites Signal, 1H), 10,50 (s, 1H).

93 mg (0,2 mMol) (6R,7R)-7-Amino-3-[[[5-[2-(hydroxycarbamoyl)äthyl]-2-methyl-s-triazolo[1,5-a]-pyrimidin-7-yl]thio]methyl]-8-oxo-5-thia-1-azabicyclo[4.2.0]oct-2-en-2-carbonsäure werden in 1 ml absolutem Dimethylacetamid suspendiert. Nach Zugabe von 0,11 ml (0,4 mMol) N,O-Bis(trimethylsilyl)-acetamid bei 0°C und 1-stündigem Rühren bei 0°C sowie 2-stündigem Rühren bei Raumtemperatur gibt man bei 0°C 64 mg (0,2 mMol) 2-Amino-4-thiazol-thioglyoxylsäure-S-(2-benzothiazolyl)ester zur erhaltenen gelben Lösung. Das Reaktionsgemisch wird 30 Minuten bei 0°C und 18 Stunden bei Raumtemperatur gerührt. Das Lösungsmittel wird im Hochvakuum abgezogen. Der Rückstand wird in etwa 5 ml Aethanol aufgenommen und kristallisiert. Die Kristalle werden im Hochvakuum bei Raumtemperatur getrocknet.. Man erhält 100 mg (6R,7R)-7-(2-Amino-4-thiazolglyoxylamido)-3-[[[5-[2-(hydroxycarbamoyl)äthyl]-2-methyl-s-triazolo[1,5-a]-pyrimidin-7-yl]thio]methyl]-8-oxo-5-thia-1-azabicyclo[4.2.0]oct-2-en-2-carbonsäure als beiges Pulver.
$^1$H NMR (DMSO-$d_6$): Signale u.a. bei δ 3,07 (t, J=8 Hz, 2H), 3,60 (d, J=18 Hz, 1H), 3,79 (d, J=18 Hz, 1H), 4,36 (d, J=12,5 Hz, 1H), 4,52 (d, J=12,5 Hz, 1H), 5,19 (d, J=5 Hz, 1H), 5,75 (dd, J=5 Hz und J=8 Hz, 1H), 7,28 (s, 1H), 7,40 (s, 2H), 7,82 (s, 1H), 8,75 (s (breit), 1H), 9,80 (d, J=8 Hz, 1H), 10,52 (s, 1H).

## Beispiel 56

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[5-(3,4-dihydroxyphenyl)-1H-tetrazol-1-yl]methyl]-8-oxo-5-thia-1-azabicyclo[4.2.0]oct-2-en-2-carbonsäure

150 mg (6R,7R)-7-(2-Amino-4-thiazolglyoxylamido)-3-[[5-(3,4-dihydroxyphenyl)-1H-tetrazol-1-yl]methyl]-8-oxo-5-thia-1-azabicyclo[4.2.0]oct-2-en-2-carbonsäure und 150 mg 1-[2-(Aminooxy)-2-methylpropionyl]-2-(3,4-dihydroxybenzoyl)hydrazin-hydrochlorid werden in 3 ml Dimethylacetamid 24 Stunden bei Raumtemperatur gerührt. Das Dimethylacetamid wird am Hochvakuum abgedampft. Der Rückstand wird mit 10 ml Wasser versetzt. Der entstandene Niederschlag wird abfiltriert, mit wenig kaltem Wasser gewaschen und in etwa 5 ml Wasser resuspendiert. Der pH-Wert wird mit 1N wässriger Natronlauge auf 7 gestellt, worauf alles in Lösung geht. Die Lösung wird an Opti-up $C_{12}$® mit Wasser und 5% Acetonitril chromatographiert. Die einheitlichen Produktfraktionen werden eingeengt und lyophilisiert. Man erhält 110 mg (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[5-(3,4-dihydroxyphenyl)-1H-tetrazol-1-yl]methyl]-8-oxo-5-thia-1-azabicyclo[4.2.0]oct-2-en-2-carbonsäure als weisses Lyophilisat.
$^1$H NMR (DMSO-$d_6$) 250 MHz: δ(ppm) 1,47 (s, 3H), 1,50 (s, 3H), 3,48 (m, 2H), 5,18 (d, J=5 Hz, 1H), 5,36 (d, J=14 Hz, 1H), 5,54 (d, J=14 Hz, 1H), 5,92 (d,d, J=5 Hz, J=8 Hz, 1H), 6,7-7,4 (m, 9 H) 9,20 (s, 1H), 9,25 (s, 1H), 9,52 (s, 1H), 9,57 (s, 1H), 9,70 (d, J=8 Hz, 1H), 9,73 (s, 1H), 10,04 (s, 1H), 14 (s, breit, 1H).

Beispiel 57

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido)-3-[[5-(3,4-dihydroxyphenyl)-2H-tetrazol-2-yl]methyl]-8-oxo-5-thia-1-azabicyclo[4.2.0]oct-2-en-2-carbonsäure

Wird in Beispiel 56 (6R,7R)-7-(2-Amino-4-thiazolglyoxyiamido)-3-[[5-(3,4-dihydroxyphenyl)-2H-tetrazol-2-yl]methyl]-8-oxo-5-thia-1-azabicyclo[4.2.0]oct-2-en-2-carbonsäure anstelle von (6R,7R)-7-(2-Amino-4-thiazolglyoxylamido)-3-[[5-(3,4-dihydroxyphenyl)-1H-tetrazol-1-yl]methyl]-8-oxo-5-thia-1-azabicyclo[4.2.0]oct-2-en-2-carbonsäure eingesetzt, so erhält man unter sonst gleichen Bedingungen:

(6R,7R)-7-[(Z)-2-(2-Amino-4-thiazolyl)-2-[[1-[3-(3,4-dihydroxybenzoyl)carbazoyl]-1-methyläthoxy]imino]-acetamido]-3-[[5-(3,4-dihydroxyphenyl)-2H-tetrazol-2-yl]methyl]-8-oxo-5-thia-1-azabicyclo[4.2.0]oct-2-en-2-carbonsäure:

$^1$H-NMR (DMSO-$d_6$, 250 MHz): Signale bei δ(ppm) 1,47 (s, 3H), 1,51 (s, 3H), 3,58 (m, 2H), 5,25 (d, J = 5 Hz, 1H), 5,65 (d, J = 14 Hz, H), 5,86 (d, J = 14 Hz, 1H), 5,94 (d,d, J = 5 Hz, J = 8 Hz, 1H), 6,75-7,5 (m, 9H), 9,21 (s, 1H), 9,24 (s, 1H), 9,39 (s, 1H), 9,47 (s, 1H), 9,59 (s, 1H), 9,70 (d, J = 8 Hz, 1H), 10,04 (s, 1H), 14 (s, breit, 1H).

Beispiel 58

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-(3,4-dihydroxyphenyl)-5-methyl-s-triazolo[1,5-a]pyrimidin-7-yl]thio]-methyl]-8-oxo-5-thia-1-azabicyclo[4.2.0]oct-2-en-2-carbonsäure

1,3 g (6R,7R)-7-(2-Amino-4-thiazolglyoxylamido)-3-[[[2-(3,4-dihydroxyphenyl)-5-methyl-s-triazolo[1,5-a]-pyrimidin-7-yl]thio]methyl]-8-oxo-5-thia-1-azabicyclo[4.2.0]oct-2-en-2-carbonsäure und 0,79 g 1-[2-(Aminooxy)-2-methylpropionyl]-2-(3,4-dihydroxybenzoyl)hydrazin-hydrochlorid werden in 10 ml Dimethylacetamid gelöst. Nach Stehen über Nacht wird im Vakuum eingedampft. Der Rückstand wird mit 25 ml Wasser digeriert und der gebildete Niederschlag abgesaugt und getrocknet. Man erhält 1,7 g (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-(3,4-dihydroxyphenyl)-5-methyl-s-triazolo[1,5-a]pyrimidin-7-yl]thio]methyl]-8-oxo-5-thia-1-azabicyclo[4.2.0]oct-2-en-2-carbonsäure als farbloses Pulver.

$^1$H-NMR (DMSO-$d_6$, 250 MHz): Signale bei δ (ppm) 1,49 (s, 3H), 1,52 (s, 3H), 2,57 (s, 3H), 3,64 (d, J = 18 Hz, 1H), 3,89 (d, J = 18 Hz, 1H), 4,35 (d, J = 13 Hz, 1H), 4,54 (d, J = 13 Hz,), 5,26 (d, J = 5 Hz, 1H), 5,91 (dd, J = 5 und 8 Hz, 1H), 6,90 (s, 1H), 6,7-7,65 (m, ca. 9H), 9,23 (s, 1H), 9,70 (d, J = 8 Hz, 1H), 10,05 (s, 1H).

Beispiel 59

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-(3-chlor-4,5-dihydroxyphenyl)-5,6-dimethyl-s-triazolo[1,5-a]pyrimidin-7-yl]thio]methyl]-8-oxo-5-thia-1-azabicyclo[4.2.0]oct-2-en-2-carbonsäure-Natriumsalz (1:1)

In analoger Weise wie in Beispiel 58 wird ausgehend von (6R,7R)-7-(2-Amino-4-thiazolglyoxylamido)-3-[[[2-(3-chlor-4,5-dihydroxyphenyl)-5,6-dimethyl-s-triazolo[1,5-a]pyrimidin-7-yl]thio]methyl]-8-oxo-5-thia-1-azabicyclo[4.2.0]oct-2-en-2-carbonsäure die Verbindung (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-(3-chlor-4,5-dihydroxyphenyl)-5,6-dimethyl-s-triazolo[1,5-a]pyrimidin-7-yl]thio]methyl]-8-oxo-5-thia-1-azabicyclo[4.2.0]oct-2-en-2-carbonsäure-Natriumsalz (1:1) hergestellt.

$^1$H-NMR (DMSO-$d_6$, 250 MHz): Signale bei δ (ppm) 2,42 (s, 3H), 2,59 (s, 3H), 3,47 (d, J = 18 Hz, 1H), 3,64 (d, J = 18 Hz, 1H), 4,37 (d, J = 13 Hz, 1H), 4,50 (d, J = 13 Hz, 1H), 5,07 (d, J = 6 Hz, 1H), 5,71 (dd, J = 6 und 9 Hz, 1H), 6,75 (d, J = 9 Hz, 1H), 6,85 (s, 1H), 7,22 (m, 1H), 7,33 (breit, 2H), 7,53 (d, J = 1 Hz, 1H), 7,65 (d, J = 1 Hz, 1H), 9,27 (s, breit, 1H), 9,55 (d, J = 9 Hz, 1H), 9,9 (breit, 1H).

Beispiel 60

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[5-(3,4-dihydroxyphenyl)pyrazolo[1,5-a]pyrimidin-7-yl]thio]methyl]-8-oxo-5-thia-1-azabicyclo[4.2.0]oct-2-en-2-carbonsäure

625 mg (6R,7R)-7-(2-Amino-4-thiazolglyoxylamido)-3-[[[5-(3,4-dihydroxyphenyl)pyrazolo[1,5-a]pyrimidin-7-yl]thio]methyl]-8-oxo-5-thia-1-azabicyclo[4.2.0]oct-2-en-2-carbonsäure und 500 mg 1-[2-(Aminooxy)-2-methylpropionyl]-2-(3,4-dihydroxybenzoyl)hydrazin-hydrochlorid werden in 10 ml Dimethylacetamid gelöst und 24 Stunden bei Raumtemperatur belassen. Danach dampft man im Vakuum bei 30 °C ein. Der Rückstand wird mit 30 ml Wasser verrührt, wobei er fest wird. Man nutscht ab, wäscht mit Wasser und trocknet bei 0,1 mmHg/40 °C und erhält 800 mg beiges (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[5-(3,4-dihydroxyphenyl)pyrazolo-[1,5-a]-pyrimidin-7-yl]thio]methyl]-8-oxo-5-thia-1-azabicyclo[4.2.0]oct-2-en-2-carbonsäure.

| Elementaranalyse: | | | | |
|---|---|---|---|---|
| Gefunden: | 48,99% C, | 3,75% H, | 15,92% N, | 10,80% S; |
| Berechnet: | 49,31% C, | 3,68% H, | 15,97% N, | 10,97% S. |

$^1$H-NMR (DMSO-$d_6$, 250 MHz): Signale bei $\delta$ (ppm) 1,47 (s, 3H), 1,50 (s, 3H), 3,69 (s, J = 18 Hz, 1H), 3,89 (d, J = 18 Hz, 1H), 4,52 (m, 2H), 5,37 (d, J = 5 Hz, 1H), 5,90 (dd, J = 5 und 8,5 Hz, 1H), 6,67 (d, J = 2 Hz, 1H), 6,76 (d, J = 8,5 Hz, 1H), 6,77 (m, 2H), 7,2-7,4 (m, 6H), 7,55 (m, 1H), 8,18 (d, J = 2 Hz, 1H), 9,2 (m, 3H), 9,55 (m, 2H), 9,67 (d, J = 8,5 Hz, 1H), 10,02 (s, 1H).

## Beispiel 61

Herstellung von (6R,7R)-7-[(Z)-2-(2-Amino-4-thiazolyl)-2-[[1-[3-(3,4-dihydroxybenzoyl)carbazoyl]-1-methyläthoxy]imino]acetamido]-3-[[[2-[[(3,4-dihydroxybenzoyl)amino]methyl]-5-methyl-s-triazolo[1,5-a]-pyrimidin-7-yl]thio]methyl]-8-oxo-5-thia-azabicyclo[4.2.0]oct-2-en-2-carbonsäure-Natriumsalz (1:1)

Diese Verbindung wird in Analogie zu Beispiel 42 aus (6R,7R)-7-(2-Amino-4-thiazolglyoxylamido)-3-[[[2-[(3,4-dihydroxybenzoyl)amino]methyl]-5-methyl-s-triazolo[1,5-a]pyrimidin-7-yl]thio]methyl]-8-oxo-5-thia-1-azabicyclo[4.2.0]oct-2-en-2-carbonsäure und 1-[2-(Aminooxy)-2-methylpropionyl]-2-(3,4-dihydroxybenzoyl)-hydrazin-hydrochlorid als leicht gelbliches Lyophilisat hergestellt.
$^1$H-NMR, DMSO$d_6$, $\delta$ (ppm) unter anderem 1,46 (s, 3H), 1,51 (s, 3H), 2,50 (s, 3H), 3,38 (d, J = 17,5Hz,1H), 3,62 (d, J = 17,5Hz,1H), 4,29 (d, J = 12,5Hz, 1H), 4,60 (d, J = 5Hz, 1H), 4,71 (d, J = 12,5Hz, 1H), 5,15 (d, J = 5Hz, 1H), 5,70 (d, d, $J_1$ = 5Hz, $J_2$ = 8Hz, 1H), 6,75 (m, 3H), 7,25 (m, 6H), 7,54 (s, 1H), 8,81 (t, J = 5Hz, 1H), 9,25 (s, 1H), 9,55 (d, J = 8Hz, 1H), 9,90 (s, 1H)

Die als Ausgangsmaterial eingesetzte (6R,7R)-7-(2-Amino-4-thiazolglyoxylamido)-3-[[[2-[(3,4-dihydroxy-benzoyl)-amino]methyl]-5-methyl-s-triazolo[1,5-a]pyrimidin-7-yl]thio]methyl]-8-oxo-5-thia-1-azabicyclo[4.2.0]-oct-2-en-2-carbonsäure kann wie folgt hergestellt werden:

1,8 g 2-(Hydroxymethyl)-5-methyl-s-triazolo[1,5-a]pyrimidin-7(4H)-on (USP 2.835.581) wird in 5 ml Dimethylformamid suspendiert, bei 0 °C mit 0,45 ml Phosphortribromid versetzt und 1/2 Stunde bei dieser Temperatur gerührt. Das Dimethylformamid wird im Hochvakuum abgedampft und der Rückstand mit eiskaltem Wasser digeriert. Der entstandene Niederschlag wird abgenutscht und im Hochvakuum getrocknet (40 °C). Man erhält 1,97 g 2-Brom-methyl-5-methyl[1.2.4]triazolo[1,5-a]pyrimidin-7-ol als weisse Kristalle vom Schmelzpunkt 217-222 °C.

3,6 g 2-Brom-methyl-5-methyl[1.2.4]triazolo[1,5-a]pyrimidin-7-ol werden in 50 ml Dimethylformamid gelöst, mit 0,99 g Natriumazid versetzt und 2,5 Stunden bei Raumtemperatur gerührt. Das Dimethylformamid wird im Hochvakuum bei 40 °C abgedampft und der Rückstand mit 35 ml Wasser versetzt. Die dabei entstandene Suspension wird 45 Minuten im Eisbad gerührt. Ein weisser Niederschlag wird abgenutscht, mit wenig Wasser nachgewaschen und im Hochvakuum getrocknet. Man erhält 2,0 g 2-Azidomethyl-5-methyl[1.2.4]triazolo[1,5-a]pyrimidin-7-ol als weisse Kristalle vom Schmelzpunkt 193-194 °C.

3,1 g 2-Azidomethyl-5-methyl[1.2.4]triazolo[1,5-a]pyrimidin-7-ol werden in 20 ml frisch destilliertem Phosphoroxychlorid und 1,1 ml Pyridin suspendiert und 4,5 Stunden bei 50 °C gerührt. Das Phosphoroxychlorid wird im Vakuum abgedampft und der Rückstand in 40 ml Aethylacetat und 40 ml Wasser aufgenommen. Die entstandene Suspension wird 45 Minuten im Eisbad gerührt. Das Produkt wird abgenutscht und im Vakuum bei 40 °C getrocknet. Man erhält 2,1 g 2-Azidomethyl-7-chlor-5-methyl[1.2.4]-triazolo[1,5-a]pyrimidin als weisse Kristalle vom Schmelzpunkt 130-132 °C.

2,98 g 2-Azidomethyl-7-chlor-5-methyl[1.2.4]triazolo[1,5-a]pyrimidin werden in 70 ml Aethanol suspendiert, mit 1,1 g Thioharnstoff versetzt und 45 Minuten am Rückfluss erhitzt. Das Reaktionsgemisch wird 30 Minuten im Eisbad gerührt. Der gebildete Niederschlag wird abgenutscht und im Vakuum bei 40 °C getrocknet. Man erhält 2,76 g 2-Azidomethyl-5-methyi[1.2.4]triazolo[1,5-a]pyrimidin-7-thiol als gelbe Kristalle

vom Schmelzpunkt 214-215 ° C.

Eine Lösung von 6,25 ml n-Butyllitnium (1,6M in n-Hexan) in 50 ml Tetrahydrofuran wird bei -78 ° C mit 3,5 g 5-Brom-2,2-diphenyl-1,3-benzodioxol versetzt und 10 Minuten gerührt. Die erhaltene Lösung wird mit etwa 10 g festem Kohlendioxid versetzt und anschliessend auf Raumtemperatur erwärmt. Der gebildete Niederschlag wird abfiltriert und getrocknet. Man erhält 2,66 g 2,2-Diphenyl-1,3-benzodioxol-5-carbonsäure-Lithiumsalz (1:1) als ein weisses Pulver. Dieses wird in 20 ml Dimethylformamid gelöst und mit 3,03 g O-Benzotriazol-1-yl-N,N,N',N'-tetramethyl-uronium-hexafluorophosphat (HBTU) 1 Stunde bei Raumtemperatur gerührt. Das Lösungsmittel wird im Hochvakuum abgedampft, der Rückstand in 15 ml Wasser und 15 ml Aethylacetat aufgenommen und 45 Minuten im Eisbad gerührt. Der gebildete Feststoff wird abgenutscht und in 40 ml Aethylacetat bei Siedehitze gelöst. Man filtriert von wenig Ungelöstem und versetzt die Mutterlauge mit n-Hexan. Das Gemisch wird nun eingeengt, bis eine noch gut ffltrierbare Suspension entsteht. Das Produkt wird abgenutscht und im Vakuum bei 40 ° C getrocknet. Man erhält 2,55 g 1H-Benzotriazol-1-yl-2,2-diphenyl-1,3-benzodioxol-5-carboxylat als weisse Kristalle vom Schmelzpunkt 158-160 ° C.

Eine trockene Lösung von 2,0 g Natriumhydrogensulfid-monohydrat (Trocknungsmittel Molekularsieb 4Å) in 100 ml Dimethylformamid wird mit 2,2 g 2-Azidomethyl-5-methyl[1.2.4]triazolo[1,5-a]pyrimidin-7-thiol versetzt und bis zum Ende der Gasentwicklung (etwa 15 Minuten) gerührt. Hierauf werden 4,35g 1H-Benzotriazol-1-yl-2,2-diphenyl-1,3-benzodioxol-5-carboxylat zugegeben und das Reaktionsgemisch 20 Stunden bei Raumtemperatur und 24 Stunden bei 45 ° C gerührt. Das Lösungsmittel wird im Hochvakuum abgedampft, der Rückstand in 200 ml Aethylacetat und 200 ml Wasser aufgenommen und 30 Minuten im Eisbad gerührt. Der entstandene Niederschlag wird abgenutscht, mit Wasser und Aethylacetat gewaschen und im Vakuum bei 40 ° C getrocknet. Das gelbe Produkt wird in 100 ml Methanol gelöst, von wenig Ungelöstem durch Filtration getrennt und zur Trockene eingeengt. Der Rückstand wird in 100 ml Acetonitril 1 Stunde im Dampfbad am Rückfluss erhitzt. Nach Abkühlen im Eisbad wird das Produkt abgenutscht und getrocknet. Man erhält 4,25 g N-[(7-Mercapto-5-methyl-s-triazolo[1,5-a]pyrimidin-2-yl)methyl]-2,2-diphenyl-1,3-benzodioxol-5-carboxamid als gelben Feststoff vom Schmelzpunkt 267-271 ° C (Zers.).

22,7 g N-[(7-Mercapto-5-methyl-s-triazolo[1,5-a]pyrimidin-2-yl)methyl]-2,2-diphenyl-1,3-benzodioxol-5-carboxamid werden in 210 ml Trifluoressigsäure und 5 ml Wasser 1 Stunde bei Raumtemperatur gerührt. Es wird von wenig Ungelöstem filtriert und die Mutterlauge zur Trockene eingedampft. Der gelbe Rückstand wird mit 900 ml Aethylacetat versetzt, 1 Stunde im Ultraschallbad gerührt und anschliessend im Eisbad abgekühlt. Der gelbe Feststoff wird abfiltriert und im Vakuum bei 40 ° C getrocknet. Man erhält 14,4 g 3,4-Dihydroxy-N-(7-mercapto-5-methyl[1.2.4]triazolo[1,5-a]pyrimidin-2-ylmethyl)-benzamid als gelbes Pulver.
$^1$-NMR, DMSO$d_6$, δ (ppm) unter anderem 2,32 (s, 1H), 4,57 (d, J = 6H,2H), 6,75 (m, 2H), 7,25 (m, 2H), 8,80 (t, J = 6H,1H), 9,15 (s, 1H), 9,51 (s, 1H), 14,00 (s, breit, 1H)

3,4-Dihydroxy-N-(7-mercapto-5-methyl[1.2.4]triazolo[1,5-a]pyrimidin-2-ylmethyl)-benzamid wird in Analogie zu Beispiel 1 durch Umsetzung mit 7-Aminocephalosporansäure in (6R,7R)-7-Amino-3-[[[2-[[(3,4-dihydroxybenzoyl)amino]methyl]-5-methyl-s-triazolo[1,5-a]pyrimidin-7-yl]thio]methyl]-8-oxo-5-thia-1-azabicyclo-[4.2.0]oct-2-en-2-carbonsäure übergeführt.
$^1$H-NMR, DMSO$d_6$, δ (ppm) unter anderem 2,56 (s, 3H), 3,52 (d, J = 17,5Hz, 1H), 3,74 (d, J = 17,5Hz, 1H), 4,30 (d, J = 12,5Hz, 1H), 4,43 (d, J = 12,5Hz, 1H), 4,61 (d, J = 6Hz, 2H), 4,80 (d, J = 5Hz, 1H), 5,01 (d, J = 5Hz, 1H), 6,75 (d, J = 8,5Hz, 1H), 7,27 (m, 3H), 8,79 (t, J = 6Hz, 1H), 9,15 (s, breit, 1H), 9,48 (s, breit, 1H)

(6R,7R)-7-Amino-3[[[2-[[(3,4-dihydroxybenzoyl)amino]methyl]-5-methyl-s-triazolo[1,5-a]pyrimidin-7-yl]-thio]methyl]-8-oxo-5-thia-1-azabicyclo-[4.2.0]oct-2-en-2-carbonsäure wird in Analogie zu Beispiel 1 durch Umsetzung mit 2-Amino-4-thiazol-thioglyoxylsäure-S-(2-benzothiazolyl)ester in (6R,7R)-7-(2-Amino-4-thiazol-glyoxylamido)-3-[[[2-[[(3,4-dihydroxybenzoyl)amino]methyl]-5-methyl-s-triazolo[1,5-a]pyrimidin-7-yl]thio]-methyl]-8-oxo-5-thia-1-azabicyclo[4.2.0]oct-2-en-2-carbonsäure übergeführt.
$^1$H-NMR, DMSO$d_6$, δ (ppm) unter anderem 2,56 (s, 3H), 3,59 (d, J = 17,5Hz, 1H), 3,78 (d, J = 17,5Hz, 1H), 4,34 (d, J = 12,5Hz, 1H), 4,48 (d, J = 12,5Hz, 1H), 4,60 (d, J = 6Hz, 2H), 5,20 (d, J = 5Hz, 1H), 5,78 (d, d, $J_1$ = 5Hz, $J_2$ = 8Hz, 1H), 6,77 (d, J = 8Hz, 1H), 7,35 (m, 5H), 7,82 (s, 1H), 8,81 (t, J = 6Hz, 1H), 9,17 (s, 1H), 9,50 (s, 1H), 9,81 (d, J = 8Hz, 1H)

Beispiel A

Herstellung von Trockenampullen für die intramuskuläre Verabreichung:

Es wird in üblicher Weise ein Lyophilisat von 1 g des Natriumsalzes der 7-[(Z)-2-(2-Amino-4-thiazolyl)-2-[[(2-fluor-3,4-dihydroxybenzyl)oxy]imino]acetamido]-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]oct-2-en-2-carbonsäure

hergestellt und in eine Ampulle abgefüllt. Vor der Verabreichung wird das Lyophilisat mit 2,5 ml einer 2%igen wässrigen Lidokainhydrochloridlösung versetzt.

Das gleiche Verfahren ist z.B. auf die Endprodukte der Beispiele 5, 6, 10, 11, 14, 15, 43, 44, 47 und 61 anwendbar.

## Patentansprüche

1. Cephalosporinderivate der allgemeinen Formel

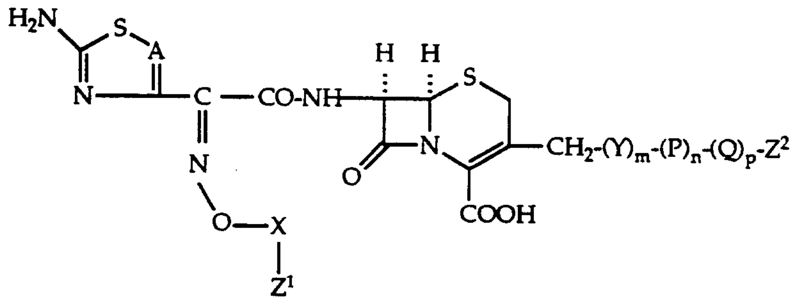

I

worin $Z^1$ und $Z^2$ je einzeln einen durch zwei vicinale Gruppen $-OR^1$ substituierten, 6-gliedrigen aromatischen Ring bedeutet (worin $R^1$ Wasserstoff oder niederes Alkanoyl darstellt), welcher zusätzlich noch 1 oder 2 O- oder N-Atome enthalten und/oder durch Halogen, Carboxy oder Carboxy-niederes Alkyl substituiert sein kann, oder $-X-Z^1$ und $-(Q)p-Z^2$ je einzeln eine Gruppe $-X^1-CONR^2OH$ bzw. $-(Q^1)p-CONR^2OH$, worin $R^2$ Wasserstoff, niederes Alkyl oder Phenyl darstellt, bedeutet; und worin ferner A einen Stickstoffatom oder eine Methingruppe (-CH=), X ggfs. durch Carboxy substituiertes und ggfs. mit einer der Gruppen -S-, -SO-, $-SO_2-$, -CO-, -OCO-, -NHCO-, $-NHSO_2-$ und -CONHNHCO-verknüpftes niederes Alkylen, Phenylen oder niederes Alkylen-Phenylen, $X^1$ niederes Alkylen, Phenylen oder niederes Alkylen-Phenylen, Y eine der Gruppen -O-, -OCO-, $-OCH_2-$, -S-, -SCO-, -SO- und $-SO_2-$, P einen ggfs. durch niederes Alkyl, Phenyl, Hydroxy oder Oxo substituierten 5- oder 6-gliedrigen N-Monoheterocyclus oder einen ggfs. durch niederes Alkyl, Carboxy, niederes Alkoxycarbonyl, Carbamoyl, Hydroxymethyl oder niederes Alkanoyloxymethyl substituierten 8- bis 10-gliedrigen N-Biheterocyclus, Q ggfs. mit einer der Gruppen -CO-, -OCO-, $-SO_2-$, -NHCO- und $-NHSO_2$-verknüpftes niederes Alkylen, Phenylen oder niederes Alkylen-Phenylen oder eine der Gruppen -S-, -SO-, $-SO_2-$, -CO-, -OCO-, -NHCO-, $-NHSO_2-$ und -CONHNHCO-, $Q^1$ niederes Alkylen, Phenylen oder niederes Alkylen-Phenylen und m, n und p jeweils die Zahl 0 oder 1 darstellt;
sowie leicht hydrolysierbare Ester und pharmazeutisch verträgliche Salze dieser Verbindungen und Hydrate von Verbindungen der Formel I bzw. von deren Estern und Salzen.

2. Verbindungen nach Anspruch 1, dadurch gekennzeichnet, dass X ggfs. durch Carboxy substituiertes und ggfs. mit einer der Gruppen -S-, -SO-, $-SO_2-$, -CO-, -OCO-, -NHCO- und $-NHSO_2-$ verknüpftes niederes Alkylen, Phenylen oder niederes Alkylen-Phenylen darstellt.

3. Verbindungen nach Anspruch 1, dadurch gekennzeichnet, dass X die Gruppe -(niederes Alkylen)-CONHNHCO- darstellt.

4. Verbindungen nach einem der Ansprüche 1-3, dadurch gekennzeichnet, dass $Z^1$ und $Z^2$ beide durch jeweils zwei vicinale Gruppen $-OR^1$ substituierte 6-gliedrige aromatische Ringe bedeuten.

5. Verbindungen nach Anspruch 1 oder 2, dadurch gekennzeichnet, dass $-X-Z^1$ eine Gruppe $-X^1-CONR^2OH$ und $Z^2$ einen durch zwei vicinale Gruppen $-OR^1$ substituierten 6-gliedrigen aromatischen Ring bedeutet.

6. Verbindungen nach einem der Ansprüche 1-3, dadurch gekennzeichnet, dass $Z^1$ einen durch zwei vicinale Gruppen $-OR^1$ substituierten 6-gliedrigen aromatischen Ring und $-(Q)_p-Z^2$ eine Gruppe $-(Q^1)_p-CONR^2OH$ bedeutet.

7. Verbindungen nach einem der Ansprüche 1-6, dadurch gekennzeichnet, dass P einen ggfs. durch niederes Alkyl, Carboxy, niederes Alkoxycarbonyl, Carbamoyl, Hydroxymethyl oder niederes Alkanoyloxymethyl substituierten 8- bis 10-gliedrigen N-Biheterocyclus darstellt.

8. Verbindungen nach Anspruch 7, dadurch gekennzeichnet, dass P die 2-(5-Methyl-s-triazolo[1,5-a]-pyrimidin-7-yl)-en-Gruppen der Formel

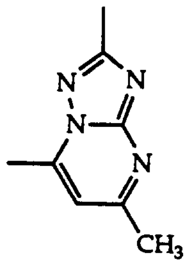

darstellt.

9. Verbindungen nach einem der Ansprüche 1-8, dadurch gekennzeichnet, dass $(Q)_p$-$Z^2$ die (3,4-Dihydroxybenzolsulfonamido)-methylgruppe darstellt.

10. Verbindungen nach einem der Ansprüche 1-8, dadurch gekennzeichnet, dass $(Q)_p$-$Z^2$ die [(3,4-Dihydroxybenzoyl)oxy]methylgruppe darstellt.

11. Verbindungen nach einem der Ansprüche 1-8, dadurch gekennzeichnet, dass $(Q)_p$-$Z^2$ die [(3,4-Dihydroxyphenyl)sulfonyl]methylgruppe darstellt.

12. Verbindungen nach einem der Ansprüche 1-8, dadurch gekennzeichnet, dass $(Q)_p$-$Z^2$ die [(3,4-Dihydroxybenzoyl)amino]methylgruppe darstellt.

13. Verbindungen nach einem der Ansprüche 1-8, dadurch gekennzeichnet, dass $(Y)_m(P)_n$-$(Q)_p$-$Z^2$ die (3,4-Dihydroxyphenyl)sulfonylgruppe darstellt.

14. Verbindungen nach einem der Ansprüche 1, 2 und 4-13, dadurch gekennzeichnet, dass X-$Z^1$ die 3,4-Dihydroxybenzylgruppe darstellt.

15. Verbindungen nach einem der Ansprüche 1, 2 und 4-13, dadurch gekennzeichnet, dass X-$Z^1$ die 2-Fluor-3,4-dihydroxybenzylgruppe darstellt.

16. Verbindungen nach einem der Ansprüche 1 und 3-12, dadurch gekennzeichnet, dass X-$Z^1$ die 1-[3-(3,4-Dihydroxybenzoyl)carbazoyl]-1-methyläthylgruppe darstellt.

17. (6R,7R)-7-[(Z)-2-(2-Amino-4-thiazolyl)-2-[[(3,4-dihydroxybenzyl)oxy]imino]acetamido]-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]-oct-2-en-2-carbonsäure und pharmazeutisch verträgliche Salze dieser Verbindung sowie Hydrate dieser Verbindung bzw. Salze.

18. 7-[(Z)-2-(2-Amino-4-thiazolyl)-2-[[(2-fluor-3,4-dihydroxybenzyl)oxy]imino]acetamido]-3-[[[2-[(3,4-dihydroxybenzolsulfonamido)methyl]-5-methyl-s-triazolo[1,5-a]pyrimidin-7-yl]thio]methyl]-8-oxo-5-thia-1-azabicyclo[4.2.0]-oct-2-en-2-carbonsäure und pharmazeutisch verträgliche Salze dieser Verbindung sowie Hydrate dieser Verbindung bzw. Salze.

19. (6R,7R)-7-[(Z)-2-(2-Amino-4-thiazolyl)-2-[[(3,4-dihydroxybenzyl)oxy]imino]acetamido]-3-[[[2-[[(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure und pharmazeutisch verträgliche Salze dieser Verbindung sowie Hydrate dieser Verbindung bzw. Salze.

**20.** (6R,7R)-7-[(Z)-2-(2-Amino-4-thiazolyl)-2-[[[(3,4-dihydroxy-2-fluorbenzyl)oxy]imino]acetamido]-3-[[[2-[[-(3,4-dihydroxybenzoyl)oxy]methyl]-5-methyl-s-triazolo[1,5-a]pyrimidin-7-yl]thio]methyl]-8-oxo-5-thia-1-azabicyclo[4.2.0]oct-2-en-2-carbonsäure und pharmazeutisch verträgliche Salze dieser Verbindung sowie Hydrate dieser Verbindung bzw. Salze.

**21.** (6R,7R)-7-[(Z)-2-(2-Amino-4-thiazolyl)-2-[[(3,4-dihydroxybenzyl)oxy]imino]acetamido]-3-[[[2-[[(3,4-dihydroxyphenyl)sulfonylmethyl]-5-methyl-s-triazolo[1,5-a]pyrimidin-7-yl]thio]methyl]-8-oxo-5-thia-1-azabicyclo[4.2.0]oct-2-en-2-carbonsäure und pharmazeutisch verträgliche Salze dieser Verbindung sowie Hydrate dieser Verbindung bzw. Salze.

**22.** (6R,7R)-7-[(Z)-2-(2-Amino-4-thiazolyl)-2-[[(2-fluor-3,4-dihydroxybenzyl)oxy]imino]acetamido]-3-[[[2-[[(3,4-dihydroxyphenyl)sulfonyl]methyl]-5-methyl-s-triazolo[1,5-a]pyrimidin-7-yl]thio]methyl]-8-oxo-5-thia-1-azabicyclo[4.2.0]oct-2-en-2-carbonsäure und pharmazeutisch verträgliche Salze dieser Verbindung sowie Hydrate dieser Verbindung bzw. Salze.

**23.** (6R,7R)-7-[(Z)-2-(2-Amino-4-thiazolyl)-2-[[1-[3-(3,4-dihydroxybenzoyl)carbazoyl]-1-methyläthoxy]imino]-acetamido]-3-[[[2-[(3,4-dihydroxybenzolsulfonamido)methyl]-5-methyl-s-triazolo[1,5-a]pyrimidin-7-yl]-thio]methyl]-8-oxo-5-thia-1-azabicyclo[4.2.0]oct-2-en-2-carbonsäure und pharmazeutisch verträgliche Salze dieser Verbindung sowie Hydrate dieser Verbindung bzw. Salze.

**24.** (6R,7R)-7-[(Z)-2-(2-Amino-4-thiazolyl)-2-[[1-[3-(3,4-dihydroxybenzoyl)carbazoyl]-1-methyläthoxy]imino]-acetmamido]-3-[[[2-[(3,4-dihydroxybenzoyl)oxy]methyl]-5-methyl-s-triazolo[1,5-a]pyrimidin-7-yl]thio]-methyl]-8-oxo-5-thia-1-azabicyclo[4.2.0]oct-2-en-2-carbonsäure und pharmazeutisch verträgliche Salze dieser Verbindung sowie Hydrate dieser Verbindung bzw. Salze.

**25.** (6R,7R)-7-[(Z)-2-(2-Amino-4-thiazolyl)-2-[[1-[3-(3,4-dihydroxybenzoyl)carbazoyl]-1-methyläthoxy]imino]-acetamido]-3-[[[2-[(3,4-dihydroxyphenyl)sulfonyl]methyl]-5-methyl-s-triazolo[1,5-a]pyrimidin-7-yl]thio] methyl]-8-oxo-5-thia 1-azabicyclo[4.2.0]oct-2-en-2-carbonsäure und pharmazeutisch verträgliche Salze dieser Verbindung sowie Hydrate dieser Verbindung bzw. Salze.

**26.** (6R,7R)-7-[(Z)-2-(2-Amino-4-thiazolyl)-2-[[1-[3-(3,4-dihydroxybenzoyl)carbazoyl]-1-methyläthoxy]imino]-acetamido]-3-[[[2-[[(3,4-dihydroxybenzoyl)amino]methyl]-5-methyl-s-triazolo[1,5-a]pyrimidin-7-yl]thio]-methyl]-8-oxo-5-thia-1-azabicyclo[4.2.0]oct-2-en-2-carbonsäure und pharmazeutisch verträgliche Salze dieser Verbindung sowie Hydrate dieser Verbindung bzw. Salze.

**27.** Verbindungen der allgemeinen Formel

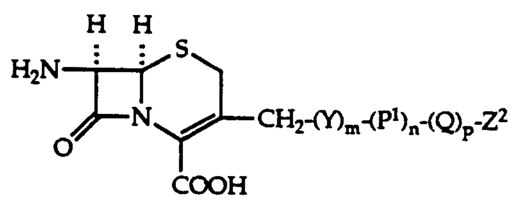

IIa

worin Y, Q, $Z^2$, m, n und p die oben gegebenen Bedeutungen haben und $P^1$ einen ggfs. durch niederes Alkyl, Carboxy, niederes Alkoxycarbonyl, Carbamoyl, Hydroxymethyl oder niederes Alkanoyloxymethyl substituierten 8- bis 10-gliedrigen N-Biheterocyclus darstellt,
sowie leicht hydrolysierbare Ester davon und Säureadditionssalze dieser Verbindungen.

**28.** Verbindungen der allgemeinen Formel

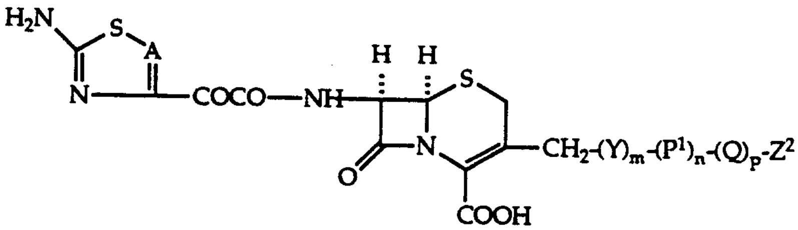

IVa

worin A, X, Y, Q, $Z^1$, $Z^2$, m, n und p die oben gegebenen Bedeutungen haben und $P^1$ einen ggfs. durch niederes Alkyl, Carboxy, niederes Alkoxycarbonyl, Carbamoyl, Hydroxymethyl oder niederes Alkanoyloxymethyl substituierten 8- bis 10-gliedrigen N-Biheterocyclus darstellt,
sowie leicht hydrolysierbare Ester davon.

**29.** Verbindungen der allgemeinen Formel

$HS\text{-}(P^1)_n(Q)_p\text{-}Z^2$ VIIa

worin Q, $Z^2$, n und p die oben gegebenen Bedeutungen haben und $P^1$ einen ggfs. durch niederes Alkyl, Carboxy, niederes Alkoxycarbonyl, Carbamoyl, Hydroxymethyl oder niederes Alkanoyloxymethyl substituierten 8- bis 10-gliedrigen N-Biheterocyclus darstellt.

**30.** Verbindungen der allgemeinen Formel

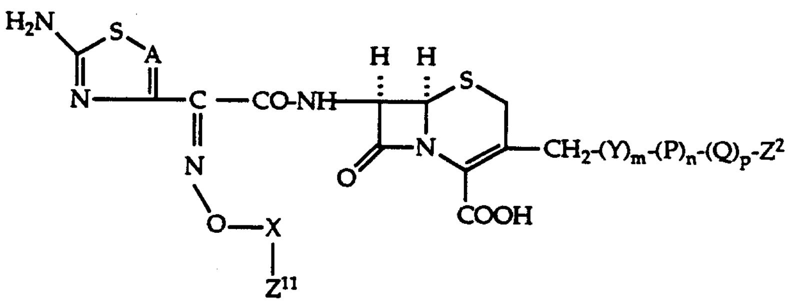

Ia

worin A, X, Y, P, Q, $Z^2$, m, n und p die oben gegebenen Bedeutungen haben, und $Z^{11}$ eine ggfs. im Benzodioxolrest halogenierte 2,2-Diphenyl-1,3-benzodioxol-5-yl-Gruppe darstellt,
sowie Salze dieser Verbindungen.

**31.** Verbindungen nach einem der Ansprüche 1-26 zur Anwendung als therapeutische Wirkstoffe.

**32.** Verbindungen nach einem der Ansprüche 1-26 zur Anwendung als antibiotisch wirksame Stoffe.

**33.** Verfahren zur Herstellung der Verbindungen gemäss einem der Ansprüche 1-26, dadurch gekennzeichnet, dass man
a) eine Verbindung der allgemeinen Formel

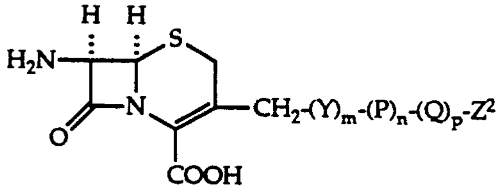

II

worin Y, P, Q, $Z^2$, m, n und p die oben angegebenen Bedeutungen haben,

oder einen leicht hydrolysierbaren Ester davon oder ein Säureadditionssalz einer dieser Verbindungen mit einer Carbonsäure der allgemeinen Formel

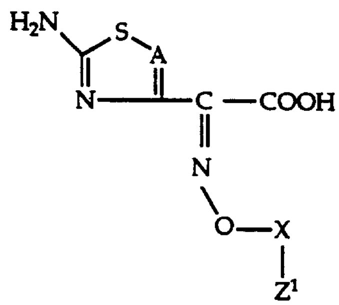

III

worin A, X und $Z^1$ die oben gegebenen Bedeutungen haben,
oder mit einem reaktionsfahigen Derivat dieser Verbindung acyliert, oder dass man

b) eine Verbindung der allgemeinen Formel

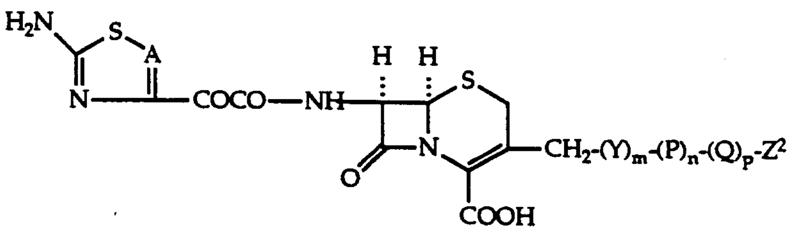

IV

worin A, Y, P, Q, $Z^2$, m, n und p die oben gegebenen Bedeutungen haben,
oder einen leicht hydrolysierbaren Ester davon mit einer Verbindung der allgemeinen Formel

$H_2N-O-X-Z^1$ V

worin X und $Z^1$ die oben gegebenen Bedeutungen haben, oder mit einem Säureadditionssalz davon umsetzt, oder dass man

c) zur Herstellung einer Verbindung der Formel I, worin m 1 und Y -S-darstellt, bzw. eines leicht hydrolysierbaren Esters davon eine Verbindung der allgemeinen Formel

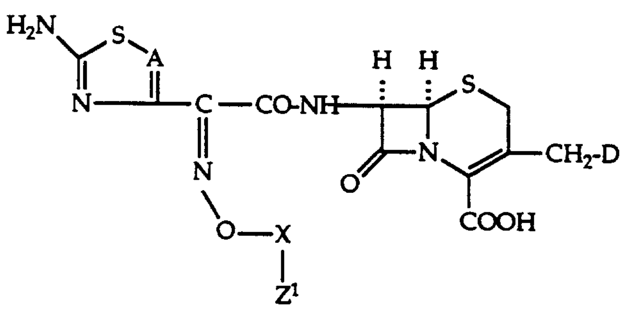

VI

worin A, X, $Z^1$ die oben gegebenen Bedeutungen haben und D eine Abgangsgruppe darstellt,
oder einen leicht hydrolysierbaren Ester davon mit einer Verbindung der allgemeinen Formel

$HS-(P)_n-(Q)_p-Z^2$ VII

worin P, Q, $Z^2$, n und p die oben gegebenen Bedeutungen haben,
umsetzt, oder dass man

d) zur Herstellung eines leicht hydrolysierbaren Esters einer Verbindung der Formel I eine Carbonsäure der Formel I einer entsprechenden Veresterung unterwirft, oder dass man
e) zur Herstellung von Salzen und Hydraten einer Verbindung der Formel I bzw., von Hydraten dieser Salze eine Verbindung der Formel I in ein Salz oder Hydrat bzw. ein Hydrat dieses Salzes überführt.

**34.** Arzneimittel enthaltend eine Verbindung nach einem der Ansprüche 1-26 und einen therapeutisch inerten Träger.

**35.** Antibiotisch wirksame Mittel enthaltend eine Verbindung nach einem der Ansprüche 1-26 und einen therapeutisch inerten Träger.

**36.** Verwendung von Verbindungen nach einem der Ansprüche 1-26 bei der Bekämpfung oder Verhütung von Krankheiten.

**37.** Verwendung von Verbindungen nach einem der Ansprüche 1-26 bei der Bekämpfung oder Verhütung von Infektionskrankheiten.

**38.** Verwendung von Verbindungen nach einem der Ansprüche 1-26 bei der Herstellung von antibiotisch wirksamen Arzneimitteln.